(19) Europäisches Patentamt
European Patent Office
Office européen des brevets

(11) **EP 1 925 672 A1**

(12) **EUROPEAN PATENT APPLICATION**

(43) Date of publication:
**28.05.2008 Bulletin 2008/22**

(21) Application number: **08102091.9**

(22) Date of filing: **21.06.2002**

(51) Int Cl.:
*C12N 15/82* (2006.01)　　*C12N 15/29* (2006.01)
*C07K 14/415* (2006.01)　　*C12N 5/10* (2006.01)
*C07K 16/16* (2006.01)　　*A01H 5/10* (2006.01)
*G01N 33/50* (2006.01)　　*C12Q 1/68* (2006.01)
*G11B 27/00* (2006.01)

(84) Designated Contracting States:
**AT BE CH CY DE DK ES FI FR GB GR IE IT LI LU MC NL PT SE TR**

(30) Priority: **22.06.2001 US 300112 P**
　　　　　**24.08.2001 US 314662 P**
　　　　　**26.09.2001 US 325277 P**
　　　　　**21.11.2001 US 332132 P**

(62) Document number(s) of the earlier application(s) in accordance with Art. 76 EPC:
**02775690.7 / 1 402 042**

(71) Applicant: **Syngenta Participations AG**
**4058 Basel (CH)**

(72) Inventors:
• **Kreps, Joel**
　**San Diego, NC 92121 (US)**
• **Briggs, Steven**
　**Del Mar, CA 92014 (US)**
• **Cooper, Bret**
　**USDA**
　**Beltsville, MD 20705 (US)**

• **Glazebrook, Jane**
　**University of Minnesota**
　**St. Paul, MN 55108 (US)**
• **Goff, Stephen**
　**Research Triangle Park, NC 27709 (US)**
• **Katagiri, Fumiaki**
　**University of Minnesota**
　**St. Paul, MN 55108 (US)**
• **Moughamer, Todd**
　**Research Triangle Park, NC 27709 (US)**
• **Provart, Nicolas**
　**University of Toronto**
　**Toronto M6H 1K5 (CA)**
• **Ricke, Darrell**
　**Winchester, MA 01890 (US)**
• **Zhu, Tong**
　**Research Triangle Park, NC 27709 (US)**

Remarks:
•The complete document including Reference Tables and the Sequence Listing is available on CD-ROM from the European Patent Office, Vienna sub-office
•This application was filed on 27-02-2008 as a divisional application to the application mentioned under INID code 62.

(54) **Abiotic stress responsive polynucleotides and polypeptides**

(57)　Abiotic stress responsive polynucleotides and polypeptides are disclosed. Also disclosed are vectors, expression cassettes, host cells, and plants containing such polynucleotides. Also provided are methods for using such polynucleotides and polypeptides, for example, to after the responsiveness of a plant to abiotic stress.

EP 1 925 672 A1

**Description**

**Cross Reference to Related Applications**

This application claims priority of U.S. Provisional Patent Application 60/300,112 filed June 22, 2001; U.S. Provisional Patent Application 60/314,662 filed August 24, 2001; U.S. Provsional Patent Application 60/325,277 filed September 26, 2001; and U.S. Provisional Patent Application 60/332,132 filed November 21, 2001, each of which is incorporated by reference in its entirety for all purposes including, but not limited to, all text figures, tables, sequence listings, supplemental tables, supplemental figures, appendices and material submitted on electronic media.

**Reference to Material Submitted on Compact Disc**

The sequence listing accompanying this application is contained on compact disc pursuant to PCT Rules 89*bis* and 89*ter,* and PCT Administrative Rules Part 8. The compact disc containing the sequence listing has been submitted in triplicate on compact discs labeled "Copy 1", "Copy 2" and "Copy 3." An additional copy labeled "CFR" has also been provided. Each compact disc contains a text file named 60073_Seq_Lst which is 38.8 MB in size and which was created on June 20, 2002.

**Field of the Invention**

The present invention relates to polynucleotides and polypeptides the expression of which is altered in response to abiotic stress and to regulatory elements that are responsive to abiotic stress.

**Background**

Recent advances in the speed and ease of large-scale DNA sequencing and the computational power of bioinformatic data analysis have permitted scientists to develop methods for studying biological processes on a genome-wide scale, giving rise to the field known as genomics. Genomics is viewed as a powerful tool for answering fundamental and complex questions regarding the structure, function and evolution of biological systems.

Structural genomics includes the sequencing and mapping of a genome, where the material sequenced may be genomic DNA, or the sequencing of other materials such as expressed sequence tags (ESTs) and development of genetic maps based on information from visible and/or molecular markers, or physical maps constructed, for example, using yeast artificial chromosomes (YACs) and/or bacterial artificial chromosomes (BACs). Sequencing and mapping of the genomes of various organisms provide the tools for comparative genomics, which can be used to study how genes and genomes are structured and how they evolved, and may provide insight into the functions of genes and other DNA regions by studying their parallels in other organisms.

Functional genomics utilizes the tools of genomics research to elucidate function, largely by large-scale measurements of gene expression to create expression profiles of the genome, and by gene mutagenesis and/or knock-out to observe the *in vivo* effects of altered genes. Functional genomics encompasses gene discovery, gene expression, protein and nucleic acid structure and function, gene and gene product interactions, and genomic approaches to breeding and comparative studies relevant to ecology and evolutionary biology. Important tools of functional genomics include: arrays, typically microarrays, of nucleotide sequences to simultaneously measure the expression of hundreds or thousands of genes; differential display, which makes it possible to analyze and compare transcribed genes to detect differentially expressed mRNAs; transcript profiling or cDNA-amplified fragment length polymorphism (cDNA-AFLP) to analyze gene expression; gene knock-out using classical mutagenesis or insertional mutagenesis; and bioinformatic tools to analyze experimental data and make predictions.

Comparative functional genomics provides the opportunity to translate the knowledge gained from one organism, often a model system such as *Arabidopsis,* to another system such as a crop plant, by comparing genome organization, function, and evolution. Comparative functional genomics facilitates a greater understanding of: phylogenomics, the study of the evolution of genes and gene families using DNA sequence information from organisms selected at major branch points along the phylogenetic continuum; biochemical pathways, the orderly flow of materials and information in living organisms genomic analysis of complex traits, with the objective to elucidate the mechanisms of multigenic control; and host-environment interactions, aimed at understanding the molecular genetic basis for host responses to changes in environment on a genomic scale.

Proteomics is a "post-genomics" set of methodologies that encompasses studies of protein structure, function, and interaction. Regulomics is the study of gene expression at the level of genetic network regulatory mechanisms to identify and characterize regulatory elements (both cis-acting and trans-acting) that control gene expression. Furthermore, the associated genes, including key mediators in diseases and metabolic processes, can be identified and the disease-related genetic regulatory circuits can be constructed, facilitating the discovery of new and better points of therapeutic intervention. Pharmacogenomics refers to using genomic information for the design and discovery of new drugs and new therapeutic approaches.

Computational genomics refers both to computational processes for analyzing sequence and expression data from *in vivo* experiments and also to computational processes for carrying out "*in silico*" experiments, including electronic hybridization (sequence comparison), predictions of gene structure, protein structure, protein function, direct protein-protein interactions, and higher-order interactions on a systems-wide basis. As such, the term "computational" can be applied widely, to denote computational comparative genomics, computational functional genomics, computational comparative functional genomics, computational comparative regulatory genomics, and the like. Computational genomics goes beyond the analysis of empirical information such as structure, function, and sequence, to develop theoretical frameworks using genomic information to create and test new proteins, to carry out protein design, to evolve genes and genomes *in silico,* to develop multidimensional phylogenies, to predict function based on context-dependent information including gene neighborhood and clusters of orthologous groups of proteins (COGs), and to use sequence data with genetic algorithms for modeling surfaces or solving complex problems.

Integrative approaches to using biological and computational results obtained using genomics and post-genomics methodologies permit the development of systems-level tools to understand, model, and predict the biological functions. Combinatorial biology can be practiced by integrating biological information and computational information. Cellomics is an example of a systems-level approach to cellular or organismal function in space and over time.

Germplasm improvement has been practiced for millennia to direct the evolution of plants and animals. Germplasm improvement can be directed to increasing both quantity and quality of an agricultural commodity, and may involve enhancing pre-existing traits or introducing traits that do not naturally occur in a given organism. Traditional or conventional breeding based on selection and crossing to introduce or enhance desired traits has been the avenue of germplasm improvement prior to the development of methods for genetic engineering. Genomics makes important contributions to both traditional and molecular methods of germplasm improvement. Genomics accelerates the discovery of genes that confer key traits and provides maps, markers, and other tools for enhancing traditional breeding. Molecular methods of germplasm improvement utilize the products of genomics research in the design of genetic constructs to achieve a desired purpose.

Plants and plant products provide the primary sustenance, either directly or indirectly, for all animal life, including humans. For the majority of the world's human population and for many animals, plants and plant products provide the sole source of nutrition. As the world population increases, the best hope to prevent widespread famine is to increase the quantity and improve the quality of food crops, and to make the crops available to the regions of the world most in need of food.

Throughout history, a continual effort has been made to increase the yield and nutritious value of food crops. For centuries, plants having desirable characteristics such as greater resistance to drought conditions or increased size of fruit were crossbred and progeny plants exhibiting the desired characteristics were selected and used to produce seed or cuttings for propagation. Using such classical genetic methods, plants having, for example, greater disease resistance, increased yield, and better flavor have been obtained.

A new paradigm for germplasm improvement in the cereals is based on the extensive similarities among the world's food cereals and other grasses in terms of chromosomal gene content and gene order. (Ahn et al., Mol Gen Genet 241:483, 1993) Due to the conservation of gene order, or synteny, within cereal genomes, a gene on the chromosome of one grass species can be expected to be present in a predicted location on a specific chromosome of a number of other grass family species. (Bennetzen et al. Proc Natl Acad Sci 95:1975, 1998). Chromosomes of the various species, most of which differ in chromosome numbers, can be arrayed in concentric circles such that a radial line from the central species with the smallest genome will pass through regions of similar genic content in each of the other species. This concept has led the plant genetics community to view the grass family as a single genetic system. Recognition of these relationships has led to the prospect of gaining sufficient genomic information from one species to understand much of the genetics of a broad array of species. The identification of genes controlling important pathways such as for insect resistance, isolation of genes of various types, determination of directional pathways of evolution and location of useful genes from exotic sources, decision making on biodiversity conservation, and many other applications in plant breeding will be easier because of the heightened understanding of genetic relationships. Although synteny is a well-studied phenomenon in the cereal (grass) genomes, similar results are emerging for all groups of species, both plant and animal.

Rice is an important cereal crop for human consumption, with approximately half a billion tons produced annually. Rice has a genome size that is considerably smaller than the other major cereals, which results in a higher gene density relative to the other cereals. This smaller genome size and higher gene density makes rice an attractive model system for cereal gene discovery efforts and germplasm improvement through traditional breeding and molecular methods. Although large-scale sequencing and mapping of the rice genome is currently underway, there is currently no complete, assembled, and annotated genome available for rice.

Microarray technology is a powerful tool that can be used to identify the presence and level of expression of a large number of nucleotide sequences in a single assay. A microarray is formed by linking a large number of discrete polynucleotide sequences, for example, a population of polynucleotides representative of a genome of an organism, to a solid support such as a microchip, glass slide, or the like, in a defined pattern. By contacting the microarray with a nucleic acid sample obtained from a cell of interest, and detecting those polynucleotides expressed in the cell can hybridize specifically to complementary sequences on the chip, the pattern formed by the hybridizing polynucleotides allows the identification of clusters of nucleotide sequences that are expressed in the cell. Furthermore, where each polynucleotide linked to the solid support is known, the identity of the hybridizing sequences from the nucleic acid sample can be identified.

A strength of microarray technology is that it allows the identification of differential gene expression simply by comparing patterns of hybridization. For example, by comparing the hybridization pattern of nucleic acid molecules obtained from cells of an individual suffering from a disease with the nucleic acids obtained from the corresponding cells of a healthy individual genes that are differentially expressed can be identified. The identification of such differentially expressed genes provides a means to identify new genes, and can provide insight as to the etiology of a disease.

Microarray technology has been widely used to identify patterns of gene expression associated with particular stages of development or of disease conditions in animal model systems, and is being applied to the identification of specific patterns of gene expression in humans. The recent availability of information for the genomes of plants provides a means to adapt microarray technology to the study of plant gene expression.

The identification of plant genes involved in conferring a selective advantage on the plant to an environmental challenge would facilitate the generation and yield of plants, thereby increasing the available food supply to an increasing world population. In addition, such knowledge provides a basis for diagnostic tests to identify stresses to which plants are subjected allowing implementation of practices to counter the stress. Thus, a need exists to identify plant genes and nucleotide sequences that are involved in modulating the response of a plant to changing environmental conditions. The present invention satisfies this need and provides additional advantages.

## Summary

The present invention relates to clusters or groups of polynucleotides the expression of which is altered in response to one or more stress conditions and in particular abiotic stresses. Such clusters include, for example, plant polynucleotides whose expression is altered in response to two or more different stress conditions; and plant polynucleotides the expression of which is altered in response to one stress condition, but not to others. The identification of such clusters, using microarray technology, has allowed the identification of plant stress-regulated polynucleotides in rice; and homologs and orthologs thereof in other plant species and in particular cereals. Thus, the invention provides isolated polynucleotide sequences of stress-regulated nucleotide sequences from cereals, and homologs and orthologs thereof; variants of such sequences, and nucleotide sequences encoding substantially similar cereal stress-regulated polypeptides expressed there from. Such sequences include, for example, sequences encoding transcription factors; enzymes, including kinases; and structural proteins, including channel proteins. Accordingly, the present invention also relates to an isolated polynucleotide disclosed herein comprising a coding region that encodes a stress-regulated polypeptide from a cereal, and portions thereof. Also included is a regulatory element selected from the regulatory elements disclosed herein or functional portions thereof, which is involved in regulating the response of a cereal to a stress condition, for example, exposure to an abnormal level of salt, osmotic pressure, temperature or any combination thereof.

The present invention also relates to a recombinant polynucleotide, which comprises a cereal stress-regulated nucleotide sequence disclosed herein or functional portion thereof operatively linked to a heterologous nucleotide sequence. In one embodiment, the recombinant polypeptide comprises a cereal stress-regulated regulatory element disclosed herein operatively linked to a heterologous nucleotide sequence, which is not regulated by the regulatory element in a naturally occurring plant. The heterologous nucleotide sequence, when expressed from the regulatory element, can confer a desirable phenotype to a plant cell containing the recombinant polynucleotide. In another embodiment, the recombinant polynucleotide comprises a coding region disclosed herein, or a functional portion thereof, of a plant stress-regulated polynucleotide operatively linked to a heterologous promoter. The heterologous promoter provides a means to express the encoded stress-regulated polypeptide constitutively, or in a tissue-specific or phase-specific manner.

One aspect of the present invention provides a method for determining whether a test plant, for example a cereal, has been exposed to at least one stress condition, for example an abiotic stress, comprising determining polynucleotide expression in the test plant to produce an expression profile and comparing the expression profile of the test plant to the expression profile of at least one reference plant that has been exposed to at least one stress, for example, an abiotic stress. In one embodiment the expressed polynucleotides are selected from the group consisting of any of the polynucleotide sequences contained in the sequence listing. In another embodiment, the test and reference plants are rice plants and the expressed polynucleotides are selected from the group consisting of SEQ ID NOs. 1-4131, 8263-8353, 8445-8829, 17505-17506 or a functional portion thereof; the group consisting of SEQ ID NOs. 2159-3176; the group consisting of SEQ ID NOs. 1964-2158; the group consisting of SEQ ID NOs. 855-1963; or the group consisting of SEQ ID NOs. 1-854.

Another aspect provides an isolated nucleic acid sequence comprising a plant nucleotide sequence, of at least 10 nucleotides long, that hybridizes under stringent conditions, or high stringency conditions, to the complement of any one of SEQ ID NOs. 1-4131, 8263-8353, 8445-8829, 17505-17506, or a functional portion thereof, which is operably linked to a regulatory element or functional portion thereof. In one embodiment, the regulatory element or functional portion thereof alters transcription of an operatively linked nucleic acid sequence in response to an abiotic stress. In one embodiment, the regulatory element, or functional portion thereof, is selected from the group consisting of SEQ ID NOs. 13374-17504 or a functional portion thereof.

Also provided are expression cassettes, plants and seeds comprising any of the above isolated sequences.

Another aspect provides an isolated polynucleotide comprising a plant, for example, a cereal, nucleotide sequence containing a coding region for an abiotic stress responsive polypeptide, selected from the group consisting of SEQ ID NOs. 1-4131, 8263-8353, 8445-8829, 17505-17506, or a functional portion thereof; or a sequence that hybridizes under stringent conditions or highly stringent conditions, to the complement of any one of SEQ ID NOs. 1-4131, 8263-8353, 8445-8829, 17505-17506, or a functional portion thereof In one embodiment, the sequence is selected from the group consisting of SEQ ID NOs. 1-854. In another embodiment, the sequence is selected from the group consisting of SEQ ID NOs. 855-4131, 8445-8829 and 17505-17506.

A further aspect provides an isolated polynucleotide comprising a rice nucleotide sequence containing a coding region for an abiotic stress responsive polypeptide, selected from the group consisting of SEQ ID NOs. 1-4131, 8263-8353, 8445-8829, and 17505-17506, or a functional protion thereof; or a sequence that hybridizes under stringent conditions or highly stringent conditions, to the complement of any one of SEQ ID NOs. 1-4131, 8263-8353, 8445-8829, and 17505-17506, or a functional protion thereof. Additional aspects include, any of the afore disclosed nucleotide sequences, or functional portions thereof, wherein the polynucleotide is located in the drought tolerance QTL located on rice chromosome 3. Another embodiment provides any of the previously disclosed polynucleotides wherein said polynucleotide is from a genomic region syntenic with a maize cold tolerance QTL. A futher embodiment provides any of the previously disclosed polynucleotides wherein the polynucleotide is present in the drought tolerence QTL on rice chromosome 8. Still a futher embodiment provides any of the previously disclosed polynucleotides wherein the polynucleotide encodes a protein comprising a Universal Stress Protein A domain.

One specific embodiment comprises an isolated nucleic acid molecule comprising a polynucleotide selected from the group consisting of: a) any one of the nucleotide sequences selected from the group consisting of SEQ ID NOs. 1-4131, 8263-8353, 8445-8829 and 17505-17506; b) a function portion of any of the sequences of a); c) a polynucleotide that is substantially similar to a sequence of a) or b); d) a sequence of at least 15 nucletides that hybridizes under stringent conditions to a polynucleotide of a), b) or c); e) the complement of any sequence of a), b), c) or d); f) the reverse complement of any sequence of a), b), c) or d); g) a polynucleotide encoding any polypeptide selected from the group consisting of SEQ ID NOs. 4132-8262, 8354-8444, and 8830-9214; and h) an allelic variant of any of the above.

Another aspect provides an isolated polypeptide selected from the group consisting of SEQ ID NOs. 4132-8262, 8354-8444, and 8830-9214 or a functional portion thereof. Specific embodiments include, but are not limted to, an isolated polypeptide comprising: a) any one of the amino acid sequences selected from the group consisting of SEQ ID NOs. 4132-8262, 8354-8444, and 8830-9214; b) a functional portion of a); c) an amino acid sequence substantially similar to any sequence of a) or b); d) an amino acid sequence of a)-c) wherein there has been at least one conservative amino acid substitution; and e) an allelic variant of any of a)-d).

The invention further relates to a method of producing a transgenic plant, which comprises at least one plant cell that exhibits altered responsiveness to a stress condition. In one embodiment, the method can be performed by introducing a functional portion of plant stress-regulated nucleotide sequence into a plant cell genome, whereby the functional portion of the plant stress-regulated nucleotide sequence modulates a response of the plant cell to a stress condition. The functional portion of the plant stress-regulated nucleotide sequence can encode a stress-regulated polypeptide or functional peptide portion thereof, wherein expression of the stress-regulated polypeptide or functional peptide portion thereof either increases the stress tolerance of the transgenic plant, or decreases the stress tolerance of the transgenic plant. The functional portion of the plant stress-regulated nucleotide sequence encoding the stress-regulated polypeptide or functional peptide portion thereof can be operatively linked to a heterologous promoter. The functional portion of the plant stress-regulated nucleotide sequence also can comprise a stress-regulated regulatory element or a functional portion thereof, such as a minimal promoter. The stress-regulated regulatory element can integrate into the plant cell genome in a site-specific manner, whereupon it can be operatively linked to a heterologous nucleotide sequence, which can be expressed in response to a stress condition specific for the regulatory element; or can be a mutant regulatory element, which is not responsive to the stress condition, whereby upon integrating into the plant cell genome, the mutant regulatory element disrupts an endogenous stress-regulated regulatory element of a plant stress-regulated nucleotide sequence, thereby altering the responsiveness of the plant stress-regulated nucleotide sequence to the stress condition.

One particular aspect provides a method for producing a transgenic plant comprising introducing into at least one plant cell a recombinant nucleic acid construct comprising i) any one of SEQ ID NOs. 1-4131, 8263-8353, 8445-8829 and 17505-17506 or the complement thereof; ii) a polynucleotide substantially similar to any one of SEQ ID NOs. 1-4131, 8263-8353, 8445-8829 and 17505-17506 or the complement thereof; iii) any one of SEQ ID NOs. 13374-17504 or the complement thereof; or iv) a polynucleotide substantially similar any one of SEQ ID NO 13374-17504 or the complement thereof.

Further aspects include plants and uniform populations of plants made by the above methods as well as seeds and progeny from such plants.

In another embodiment, a transgene introduced into a plant cell according to a method of the invention can encode a polypeptide that regulates expression from an endogenous plant stress-regulated nucleotide sequence. Such a polypeptide can be, for example, a recombinantly produced polypeptide comprising a zinc finger domain, which is specific for the regulatory element, and an effector domain, which can be a repressor domain or an activator domain. The polynucleotide encoding the recombinant polypeptide can be operatively linked to and expressed from a constitutively active, inducible or tissue specific or phase specific regulatory element. Expression of the recombinant polypeptide from a plant stress-regulated promoter such as any of SEQ ID NOs. 13374-17504 or a functional portion thereof can be particularly advantageous in that the polypeptide can be coordinately expressed with the endogenous plant stress-regulated nucleotide sequences upon exposure to a stress condition. The invention also provides transgenic plants produced by a method as disclosed, as well as to a plant cell obtained from such transgenic plant, wherein said plant cell exhibits altered responsiveness to the stress condition; a seed produced by the transgenic plant; and a cDNA or genomic DNA library prepared from the transgenic plant, or from a plant cell from said transgenic plant, wherein said plant cell exhibits altered responsiveness to the stress condition.

Thus, one aspect provides an isolated nucleic acid comprising a regulatory element comprising a sequence selected from the group consisting of SEQ ID NOs. 13374-17504 or a functional portion thereof, wherein said regulatory element alters transcription of an operatively linked polynucleotide in a plant cell, for example a cereal, in response to an abiotic stress. An additional aspect provides an isolated nucleic acid comprising a regulatory element having a sequence substantially similar to a sequence selected from the group consisting of SEQ ID NOs. 13374-17504 or a functional portion thereof, wherein said regulatory element alters transcription of an operatively linked polynucleotide in a plant cell in response to an abiotic stress.

Further aspects provide an expression cassette comprising as operatively linked components, a regulatory element comprising a sequence from the group consisting of SEQ ID NOs. 13374-17504 or a functional portion thereof, wherein said regulatory element alters transcription of an operatively linked polynucleotide in a plant cell, for example a cereal, in response to an abiotic stress; a coding region; and a termination sequence. Another aspect provides an expression cassette comprising as operatively linked components, a regulatory element comprising a sequence substantially similar to a sequence selected from the group consisting of SEQ ID NOs. 13374-17504 or a functional portion thereof, wherein said regulatory element alters transcription of an operatively linked polynucleotide in a plant cell, for example a cereal, in response to an abiotic stress; a coding region; and a termination sequence. Also provided are host cells and seeds comprising such expression cassettes, plants containing such host cells and seeds and progeny of plants containing said host cells. In related aspects, the coding region of the expression cassettes comprise sequences encoding marker proteins and sequences involved in gene silencing such as antisense sequences, double stranded RNAi sequences, a triplexing agent, and sequences comprising dominant negative mutations. In additional related aspects, the coding regions comprise sequences encoding polypeptides that alter the response of a plant to an abiotic stress.

The present invention also relates to a method of modulating the responsiveness of a plant, for example a cereal, cell to a stress condition. Such a method can be performed, for example, by introducing a functional portion of a stress-regulated polynucleotide described herein into the plant cell, thereby modulating the responsiveness of the plant cell to a stress condition. Such a method can result in the responsiveness of the plant cell being increased upon exposure to the stress condition, which, in turn, can result in increased or decreased tolerance of the plant cell to a stress condition; or can result in the responsiveness of the plant cell to the stress condition being decreased, which, in turn, can result in increased or decreased tolerance of the plant cell to a stress condition. In one embodiment, the functional portion of the plant stress-regulated polynucleotide can integrate into the genome of the plant cell, thereby modulating the responsiveness of the plant cell to the stress condition. In another embodiment, the functional portion of the plant stress-regulated nucleotide sequence encodes a stress-regulated polypeptide or functional peptide portion thereof, and can be operatively linked to a heterologous promoter. The functional portion of the plant stress-regulated polynucleotide also can contain a mutation, whereby upon integrating into the plant cell genome, the polynucleotide disrupts (knocks-out) an endogenous plant stress-regulated sequence, thereby modulating the responsiveness of the plant cell to the stress condition. Depending on whether the knocked-out gene encodes an adaptive or a maladaptive stress-regulated polypeptide, the responsiveness of the plant will be modulated accordingly. In still another embodiment, the functional portion of the plant stress-regulated polynucleotide can comprise a stress-regulated regulatory element, which can be operatively linked to a heterologous nucleotide sequence, the expression of which can modulate the responsiveness of the plant cell to a stress condition. Such a heterologous nucleotide sequence can encode, for example, a stress-inducible transcription factor such as DREB1A. The heterologous nucleotide sequence also can encode a polynucleotide that is specific for a plant stress-regulated nucleotide sequence, for example, an antisense molecule, an RNAi molecule, a ribozyme, and a triplexing agent, any of which, upon expression in the plant cell, reduces or inhibits expression of a stress-regulated polypeptide encoded by the nucleotide sequence, thereby modulating the responsiveness of the plant cell to a stress condition, for example, an abnormal level of cold, osmotic pressure, salinity or any combination thereof. Accordingly, the invention also relates to a plant cell, for example a cereal obtained by such a method, and to a plant, for example a cereal, comprising such a plant cell.

The present invention also relates to a method of expressing a heterologous nucleotide sequence in a plant cell, for example a cereal. Such a method can be performed, for example, by introducing into the plant cell a plant stress-regulated regulatory element operatively linked to the heterologous nucleotide sequence, whereby, upon exposure of the plant cell to a stress condition, the heterologous nucleotide sequence is expressed in the plant cell. In one embodiment, the stress regulated element is any of the sequences described herein that are capable of altering transcription of an operatively linked sequence in response to an abiotic stress, for example, SEQ ID NOs. 13374-17504 or a functional portion thereof. The heterologous nucleotide sequence can encode a selectable marker, a diagnostic marker, or a polypeptide that confers a desirable trait upon the plant cell, for example, a polypeptide that improves the nutritional value, digestability or ornamental value of the plant cell, or a plant comprising the plant cell.

The present invention further relates to a method of modulating the activity of a biological pathway in a plant cell, wherein the pathway involves a stress-regulated polypeptide or a non-protein regulatory molecule, for example a ribozyme, such as those encoded by sequences contained in any of SEQ ID NOs. 1-4131, 8263-8353, 8445-8829 and 17505-17506. Such a method can be performed by introducing a functional portion of a plant stress-regulated polynucleotide into the plant cell, thereby modulating the activity of the biological pathway. The method can be performed with respect to a pathway involving any of the stress-regulated polypeptides as disclosed herein or encoded by the polynucleotides disclosed herein, as well as using homologs or orthologs thereof. Also included are stress-regulated polypeptides or non-protein regulatory molecules that are encoded by sequences that are substantially similar to SEQ ID NOs. 1-4131, 8263-8353, 8445-8829 and 17505-17506.

The present invention also relates to a method of identifying a polynucleotide that modulates a stress response in a plant cell, for example a cereal. In one embodiment the method comprises determining polynucleotide expression in a plant exposed to at least one abiotic stress to produce an expression profile and identifying sequences whose expression is altered at least two fold compared to plants not exposed to the stress. Such an expression profile can be obtained, for example, by contacting an array of probes representative of a plant cell genome with nucleic acid molecules expressed in a plant cell exposed to the stress; detecting a nucleic acid molecule that is expressed at a level different from a level of expression in the absence of the stress. The method can further comprise introducing the differentially expressed nucleic acid molecule into a plant cell; and detecting a modulated response of the genetically modified plant cell to a stress, thereby identifying a polynucleotide that modulates a stress response in a plant cell. In one embodiment, the differentially expressed nucleic acid is selected from the group consisting of SEQ ID NOs. 1-4131, 8263-8353, 8445-8829 and 17505-17506. The abiotic stress can be any abiotic stress such as exposure to an abnormal level of cold, osmotic pressure, salinity or any combination thereof. The contacting is under conditions that allow for specific hybridization of a nucleic acid molecule with a probe having sufficient complementarity, for example, under stringent or highly stringent hybridization conditions. Expression of the polynucleotide can increase or decrease the tolerance of the plant cell to the stress, and the nucleic acid molecule can be expressed at a level that is less than or greater than the level of expression in the absence of the stress.

The present invention additionally relates to a method of identifying a stress condition to which a plant cell, for example a cereal, was exposed by comparing an expression profile from a test plant suspected of having been exposed to at least one stress to an expression profile obtained from a reference plant, preferably of the same species, which has been exposed to the suspected stress. Such a method can be performed, for example, by contacting nucleic acid molecules expressed in the test plant cell with an array of probes representative of the plant cell genome; detecting a profile of expressed nucleic acid molecules characteristic of a stress response, and comparing the expression pattern in the test plant to the expression pattern obtained from a reference plant thereby identifying the stress condition to which the plant cell was exposed. The contacting is under conditions that allow for specific hybridization of a nucleic acid molecule with probes having sufficient complementarity, for example, under stringent hybridization conditions. In one embodiment, the stress is an abiotic stress. The profile can be characteristic of exposure to a single stress condition, for example, an abnormal level of cold, osmotic pressure, or salinity, or can be characteristic of exposure to more than one stress condition, for example, cold, increased osmotic pressure and increased salinity. In one embodiment, the polynucleotides whose expression is detected are selected from the group consisting of SEQ ID NOs. 1-4131, 8263-8353, 8445-8829 and 17505-17506. In one embodiment, the plant is a rice plant. It is contemplated that in any of the above embodiments that the number of polynucleotides whose expression will be determined will be greater than one. Thus within the scope of the invention are embodiment in which the number of different polynucleotides whose expression is determined for any one plant is at least 10, at least 25, at least 50, at least 100, at least 250, at least 500, and at least 750.

The present invention further relates to a transgenic plant, for example a cereal, which contains a nucleic acid construct comprising a function portion of any of the plant stress-regulated polynucleotides of SEQ ID NOs. 1-4131, 8263-8353, 8445-8829 and 17505-17506. In one embodiment, the transgenic plant exhibits altered responsiveness to a stress condition as compared to a corresponding reference plant not containing the construct. Such a transgenic plant can contain, for example, a construct that disrupts an endogenous stress-regulated nucleotide sequence in the plant, thereby reducing or inhibiting expression of the gene in response to a stress condition. Such a knock-out can increase or decrease tolerance of the plant to a stress condition. The transgene also can comprise a coding sequence of a plant stress-regulated nucleotide sequence, such as any of SEQ ID NOs. 1-4131, 8263-8353, 8445-8829 and 17505-17506, which can be operatively linked to a heterologous regulatory element such as a constitutively active regulatory element, an regulated regulatory element, a tissues specific or phase specific regulatory element, or the like. In another embodiment, the transgenic plant contains a nucleic acid construct comprising a plant stress-regulated regulatory element, such as any of SEQ ID NOs. 13374-17504, which can be operatively linked to a heterologous nucleotide sequence that can encode a polypeptide or functional RNA. Expression of the heterologous polypeptide can confer a desirable characteristic on the plant, for example, can improve the nutritional or ornamental value of the transgenic plant. In still another embodiment, the transgenic plant contains multiple nucleic acid constructs, which can be multiple copies of the same construct, or can be two or more different constructs.

The present invention also relates to a plant stress-regulated regulatory element, which is contained in a plant stress-responsive polynucleotide disclosed herein for example any of SEQ ID NOs. 13374-17504; a homolog or ortholog thereof. The invention also provides a method of identifying an agent, for example a transcription factor, that specifically binds to, or activates a plant stress-responsive regulatory element and in particular a stress responsive-regulatory element from any of SEQ ID NOs. 13374-17504. Such a method can be performed, for example, by contacting the regulatory element with a plant cell extract, and identifying polypeptides that specifically bind to the regulatory element. Confirmation that the specifically binding polypeptide is a transcription factor can be demonstrated using, for example, the stress-regulated regulatory element operably linked to a reporter gene, and detecting expression of the reporter gene. Control constructs comprising a regulatory element, other than a plant stress-regulated regulatory element, operatively linked to a reporter molecule can be used to confirm that the transcription factor is specific for the plant stress-regulated regulatory element. A polynucleotide encoding such a transcription factor also can be obtained.

The present invention also relates to a method of using a functional portion of a plant stress-regulated nucleotide sequence disclosed herein to confer a selective advantage on a plant cell, for example a cereal plant cell. In one embodiment, such a method is performed by introducing a plant stress-regulated regulatory element disclosed herein into a plant cell such as those described herein, wherein, upon exposure of the plant cell to a stress condition to which the regulatory element is responsive, a nucleotide sequence operatively linked to the regulatory element is expressed, thereby conferring a selective advantage to plant cell. The operatively linked nucleotide sequence can be, for example, a transcription factor, the expression of which induces the further expression of nucleotide sequences involved in a stress response, thereby enhancing the response of a plant to the stress condition. In another embodiment, a coding sequence of a plant stress-regulated sequence described herein is introduced into the cell, thereby providing the plant with a selective advantage in response to a stress condition. In still another embodiment, the method results in the knock-out of any of the plant stress-regulated sequences described herein in a first population of plants, thereby providing a selective advantage to a stress condition in a second population of plants.

The invention further relates to a method of identifying an agent that modulates the activity of a stress-regulated regulatory element of a plant, for example a cereal. In a particular embodiment, is provided a method for identifying an agent that alters the activity of an abiotic stress responsive regulatory element comprising contacting the agent or a composition containing an agent to be tested with at least one abiotic stress responsive regulatory element, for example selected from the group consisting of SEQ ID NOs. 13374-17504, or a functional portion thereof, and determining the effect of the agent on the ability of the regulatory sequence to regulate transcription. In one embodiment, the regulatory element can be operatively linked to a heterologous polynucleotide encoding a reporter molecule, and an agent that modulates the activity of the stress-regulated regulatory element can be identified by detecting a change in expression of the reporter molecule due to contacting the regulatory element with the agent. Such a method can be performed *in vitro* in a plant cell-free system, or in a plant cell in culture or in a plant *in situ.* In another embodiment, the agent is contacted with a transgenic plant containing an introduced plant stress-regulated regulatory element, and an agent that modulates the activity of the regulatory element is identified by detecting a phenotypic change in the transgenic plant. The methods of the invention can be performed in the presence or absence of the stress condition to which the particularly regulatory element is responsive.

Another aspect provides a method for identifying an agent that alters abiotic stress responsive polynucleotide expression in a plant or plant cell, for example a cereal, comprising contacting a plant or plant cell with a test agent; subjecting the plant cell or plant cell to an abiotic stress or combination of stresses before, during or after contact with the agent to be tested; obtaining an expression profile of the plant or plant cell and comparing the expression profile of the plant or plant cell to an expression profile from a plant or plant cell not exposed to the abiotic stress or combination of stresses. In one embodiment, the expression profile comprises expression data for at least one sequence selected from the group consisting of SEQ ID NOs. 1-4131, 8263-8353, 8445-8829, and 17505-17506. In additional embodiments, the plant or plant cell is a rice plant. By at least one, it should be realized that the above embodiments contemplate that the expression profile discussed above can contain expression data for greater than one of the described sequences, for example at least 10 sequences, at least 25 sequences, at least 50 sequences, at least 100 sequences, at least 250 sequences, at least 500 sequences or at least 750 sequences.

Still another aspect provides nucleotide probes for the diagnosis of abiotic stress in plants, for example cereals, comprising nucleotide sequences of at least 15, 25, 50 or 100 nucleotides, that hybridize under stringent or highly stringent conditions to at least one sequence selected from the group consisting of SEQ ID NOs. 1-4131, 8263-8353, 8445-8829, and 17505-17506.

An additional aspect provides a method for selecting plants, for example cereals, having an altered resistance to abiotic stress comprising obtaining nucleic acid molecules from the plants to be selected; contacting the nucleic acid molecules with one or more probes that selectively hybridize under stringent or highly stringent conditions to a nucleic acid sequence selected from the group consisting of SEQ ID NOs. 1-4131, 8263-8353, 8445-8829, and 17505-17506; detecting the hybridization of the one or more probes to the nucleic acid sequences wherein the presence of the hybridization indicates the presence of a gene associated with altered resistance to abiotic stress; and selecting plants on the basis of the presence or absence of such hybridization. In one embodiment, marker-assisted selection is accomplished in rice. In another embodiment, marker assisted selection is accomplished in wheat using one or more probes which selectively hybridize under stringent or highly stringent conditions to sequences selected from the group consisting of SEQ ID NOs. 9881-11967. In yet another embodiment, marker assisted selection is accomplished in maize or corn using one or more probes which selectively hybridize under stringent or highly stringent conditions to sequences selected from the group consisting of SEQ ID NOs. 11968-13373. In still another embodiment, marker assisted selection is accomplished in banana using one or more probes which selectively hybridize under stringent or highly stringent conditions to sequences selected from the group consisting of SEQ ID NOs. 9215-9880. In each case marker-assisted selection can be accomplished using a probe or probes to a single sequence or multiple sequences. If multiple sequences are used they can be used simultaneously or sequentially.

A further aspect provides a method for monitoring a population of plants comprising providing at least one sentinel plant, for example a cereal, containing a recombinant polynucleotide comprising a stress responsive regulatory sequence selected from the group consisting of SEQ ID NOs. 13374-17504 which is operatively linked to a nucleotide sequence encoding a detectable marker, for example a fluorescent protein such as a green fluorescent protein, a yellow fluorescent protein, a cyan fluorescent protein, a red fluorescent protein or an enhanced or modified form thereof.

A further aspect provides a computer readable medium having stored thereon computer executable instructions for performing a method comprising receiving data on nucleotide sequence expression in a test plant of at least one nucleic acid molecule having at least 70%, at least 80%, at least 90% or at least 95%, sequence identity to a nucleotide sequence selected from the group consisting of SEQ ID NOs. 1-4131, 8263-8353, 8445-8829 and 17505-17506; and comparing expression data from said test plant to expression data for the same nucleotide sequence or sequences in a plant which has been exposed to at least one abiotic stress.

Yet a further aspect provides a computer readable medium having stored thereon a data structure comprising, sequence data for at least one nucleic acid molecule having at least 70%, at least 80%, at least 90%, or at least 95%, nucleic acid sequence identity to a polynucleotide selected from the group consisting of SEQ ID NOs. 1-4131, 8263-8353, 8445-8829 and 17505-17506, or the complement thereof; and a module receiving the nucleic acid molecule sequence data which compares the nucleic acid molecule sequence data to at least one other nucleic acid sequence.

An additional aspect provides a monoclonal or polyclonal antibody to any of the abiotic stresss related polypeptides disclosed herein, for example, SEQ ID NOs. 4132-8261, 8263-8353 and 8830-9214.

A futher aspect provides a method for identifying a homolog or ortholog of an abiotic stress responsive polynucleotide comprising determining the nucleotide sequence of a plurality of isolated polynucleotides to create a set of nucleotide sequences and translating the nucleotide sequences in the set to derive one or more putative amino acid sequences, based on one or more of the possible reading frames of the nucleotide sequences and their complementary sequences. Selecting an amino acid sequence of an abiotic stress-responsive protein and comparing the amino acid sequence of the abiotic stress-responsive protein with at least one of the putative amino acid sequences. Identifying putative amino acid sequences having homology with at least a region of the amino acid sequence of the abiotic stress-responsive protein; and correlating putative amino acid sequences having homology to translated nucleotide sequences. In one embodiment the amino acid sequence of an abiotic stress responsive protein is selected from the group consisting of SEQ ID NOs. 4132-8262, 8354-8444, and 8830-9214. In another embodiment, the method further comprises, assembling a plurality of the translated nucleotide sequences that encode the putative amino acid sequences based on regions of overlap comprising at least 10 base pairs of identical sequences between two translated nucleotide sequences to form one or more nucleotide contigs. Translating the one or more nucleotide contigs into one or more amino acid contigs.

Comparing the one or more amino acid contigs with the amino aicd sequence of the abiotic stress responsive protein to determine homology between the amino acid contig and at least a region of the abiotic stress responsive protein; and identifying at least one homolog of at least a region of the abiotic stress responsive protein based on the homology determined.

## Detailed Description

The following detailed description is provided to aid those skilled in the art in practicing the present invention. Even so, this detailed description should not be construed to unduly limit the present invention as modifications and variations in the embodiments discussed herein can be made by those of ordinary skill in the art without departing from the scope of the present invention.

All publications, patents, patent applications, public databases, public database enteries, and other references cited in this application are herein incorporated by reference in their entirety as if each individual publication, patent, patent application, public database, public database entry, or other reference were specifically and individually indicated to be incorporated by reference.

The present invention relates to clusters or groups of polynucleotides and the polypeptides encoded thereby, the expression of which is altered in response to abiotic stress conditions and to regulatory elements that are responsive to abiotic stress. In one embodiment, the invention provides polynucleotides and their associated polypeptides whose expression is altered at least 2X by abiotic stress. By a two fold alteration is meant that the change in expression level can be described by using a multiplier or divisor of at least two. For example, if the expression level were set at 100 prior to stress exposure, at 2X alteration would result in an expression level of > 200 or < 50. Abiotic stress conditions, such as a shortage or excess of solar energy, water and nutrients, and salinity, high and low temperature, or pollution (e.g., heavy metals), can have a major impact on plant growth and can significantly reduce the yield, for example, of cultivars. Under conditions of abiotic stress, the growth of plant cells is inhibited by arresting the cell cycle in late G1, before DNA synthesis, or at the G2/M boundary (see Dudits, Plant Cell Division, Portland Press Research, Monograph; Francis, Dudits, and Inze, eds., 1997; chap. 2, page 21; Bergounioux, Protoplasma 142:127-136, 1988). The identification of stress-regulated polynucleotide clusters, using microarray technology, provides a means to identify plant stress-regulated nucleotide sequences.

The polynucleotides and polypeptides disclosed herein are expected to be functional in a wide variety of plants and especially cereals. The wide-spread applicability of the sequences disclosed is supported by the finding of expressed sequence tags from banana, wheat and maize or corn having homology to the disclosed polynucleotide sequences. Table 2 shows list of such sequences and the SEQ ID NOs of the corresponding banana, wheat and/or maize ESTs.

As used herein, the term "cluster," when used in reference to stress-regulated polynucleotides, refers to polynucleotides that have been selected by drawing Venn diagrams, and selecting those nucleotide sequences that are regulated only by a selected stress condition. The selected stress condition can be a single stress condition, for example, cold, osmotic stress or salinity stress, or can be a selected combination of stress conditions, for example, cold, osmotic stress and salinity stress. In addition, a cluster can be selected based on specifying that all of the nucleotide sequences are coordinately regulated, for example, they all start at a low level and are induced to a higher level. However, a cluster of saline stress-regulated nucleotide sequences, for example, that was selected for coordinate regulation from low to high, also can be decreased in response to cold stress or osmotic stress. By varying the parameters used for selecting a cluster of nucleotide sequences, those nucleotide sequences that are expressed in a specific manner following a stress can be identified.

As used herein in reference to a polynucleotide or nucleic acid sequence, "isolated" means a polynucleotide or nucleic acid sequence that is free of one or both of the nucleotide sequences which flank the polynucleotide in the naturally-occurring genome of the organism from which the polynucleotide is derived. The term includes, for example, a polynucleotide or fragment thereof that is incorporated into a vector or expression cassette; into an autonomously replicating plasmid or virus; into the genomic DNA of a prokaryote or eukaryote; or that exists as a separate molecule independent of other polynucleotides. It also includes a recombinant polynucleotide that is part of a hybrid polynucleotide, for example, one encoding a polypeptide sequence.

As used herein "polynucleotide" and "oligonucleotide" are used interchangeably and refer to a polymeric (2 or more monomers) form of nucleotides of any length, either ribonucleotides or deoxyribonucleotides. Although nucleotides are usually joined by phosphodiester linkages, the term also includes polymeric nucleotides containing neutral amide backbone linkage composed of aminoethyl glycine units. This term refers only to the primary structure of the molecule. Thus, these terms include double- and single-stranded DNA and RNA as well DNA/RNA hybrids that may be single-stranded, but are more typically double-stranded. In addition, the term also refers to triple-stranded regions comprising RNA or DNA or both RNA and DNA. The strands in such regions may be from the same molecule or from different molecules. The regions may include all or one or more of the molecules, but more typically involve only a region of some of the molecules. The terms also include known types of modifications, for example, labels, methylation, "caps", substitution of one or more of the naturally occurring nucleotides with an analog, internucleotide modifications such as, for example, those with uncharged linkages (e.g. methyl phosphonates, phophotriesters, phosphoamidates, carbamates etc.), those containing pendant moieties, such as, for example, proteins (including for e.g., nucleases, toxins, antibodies, signal peptides, poly-L-lysine, etc.), those with intercalators (e.g., acridine, psoralen, tec,), those containing alkylators, those with modified linkages (e.g. alpha anomeric nucleic acids, etc.), as well as unmodified forms of the polynucleotide. Polynucleotides include both sense and antisense, or coding and template strands. The terms include naturally occurring and chemically synthesized molecules.

As used herein, "sequence" means the linear order in which monomers occur in a polymer, for example, the order of amino acids in a polypeptide or the order of nucleotides in a polynucleotide.

A "recombinant" nucleic acid is one produced by human intervention in the nucleotide sequence, typically selection or production. Alternatively, it can be a nucleic acid made by generating a sequence comprising fusion of two or more fragments which are not naturally contiguous to each other. Thus, for example, products made by transforming cells with any unnaturally occurring vector is encompassed, as are nucleic acids comprising sequences derived using any synthetic oligonucleotide process. Such is often done to replace a codon with a redundant codon encoding the same or a conservative amino acid, while typically introducing or removing a sequence recognition site. Alternatively, it is performed to join together nucleic acid segments of desired functions to generate a single genetic entity comprising a desired combination of functions not found in the commonly available natural forms. Restriction enzyme recognition sites are often the target of such artificial manipulations, but other site specific targets, e.g., promoters, DNA replication sites, regulation sequences, control sequences, or other useful features may be incorporated by design.

As used herein, the term "abiotic stress" or "stress" or "stress condition" refers to the exposure of a plant, plant cell, or the like, to a non-living ("abiotic") physical or chemical agent or condition that has an adverse effect on metabolism, growth, development, propagation and/or survival of the plant (collectively "growth"). A stress can be imposed on a plant due, for example, to an environmental factor such as water (e.g., flooding, drought, dehydration), anaerobic conditions (e.g., a low level of oxygen), abnormal osmotic conditions, salinity or temperature (e.g., hot/heat, cold, freezing, frost), a deficiency of nutrients or exposure to pollutants, or by a hormone, second messenger or other molecule. Anaerobic stress, for example, is due to a reduction in oxygen levels (hypoxia or anoxia) sufficient to produce a stress response. A flooding stress can be due to prolonged or transient immersion of a plant, plant part, tissue or isolated cell in a liquid medium such as occurs during monsoon, wet season, flash flooding or excessive irrigation of plants, or the like. A cold stress or heat stress can occur due to a decrease or increase, respectively, in the temperature from the optimum range of growth temperatures for a particular plant species. Such optimum growth temperature ranges are readily determined or known to those skilled in the art. Dehydration stress can be induced by the loss of water, reduced turgor, or reduced water content of a cell, tissue, organ or whole plant. Drought stress can be induced by or associated with the deprivation of water or reduced supply of water to a cell, tissue, organ or organism. Salinity-induced stress (salt-stress) can be associated with or induced by a perturbation in the osmotic potential of the intracellular or extracellular environment of a cell.

As disclosed herein, plant stress-regulated polynucleotides and clusters of stress-regulated polynucleotides have been identified. Surprisingly, several of the stress-regulated polynucleotides previously were known to encode polypeptides having defined cellular functions, including roles as transcription factors, enzymes such as kinases, and structural proteins such as channel proteins, but were not identified as being stress-regulated. The identification of rice stress-regulated nucleotide sequences has provided a means to identify homologous and orthologous nucleotide sequences in other plant species using procedures described herein.

As used herein, the term "substantially similar", when used with respect to a nucleotide sequence, means a nucleotide sequence corresponding to a reference nucleotide sequence, wherein the corresponding sequence encodes a polypeptide having substantially the same structure and function as the polypeptide encoded by the reference nucleotide sequence, e.g., where only changes in amino acids not affecting the polypeptide function occur. Desirably, the substantially similar nucleotide sequence encodes the polypeptide encoded by the reference nucleotide sequence. The percentage of identity between the substantially similar nucleotide sequence and the reference nucleotide sequence is at least 60%, at least 75%, at least 90%, at least 95%, or at least 99%, including 100%. A nucleotide sequence is "substantially similar" to reference nucleotide sequence that hybridizes to the reference nucleotide sequence in 7% sodium dodecyl sulfate (SDS), 0.5 M $NaPO_4$, 1 mM EDTA at 50°C with washing in 2X SSC, 0.1% SDS at 50°C; in 7% sodium dodecyl sulfate (SDS), 0.5 M $NaPO_4$, 1 mM EDTA at 50°C with washing in 1X SSC, 0.1% SDS at 50°C (stringent conditions); in 7% sodium dodecyl sulfate (SDS), 0.5 M $NaPO_4$, 1 mM EDTA at 50°C with washing in 0.5X SSC, 0.1% SDS at 50°C (high stringency); in 7% sodium dodecyl sulfate (SDS), 0.5 M $NaPO_4$, 1 mM EDTA at 50°C with washing in 0.1X SSC, 0.1 % SDS at 50°C (very high stringency); or in 7% sodium dodecyl sulfate (SDS), 0.5 M $NaPO_4$, 1 mM EDTA at 50°C with washing in 0.1X SSC, 0.1% SDS at 65°C (extremely high stringency).

As is well known in the art, stringency is related to the $T_m$ of the hybrid formed. The $T_m$ (melting temperature) of a nucleic acid hybrid is the temperature at which 50% of the bases are base-paired. For example, if one the partners in a hybrid is a short oligonucleotide of approximately 20 bases, 50% of the duplexes are typically strand separated at

the $T_m$. In this case, the $T_m$ reflects a time-independent equilibrium that depends on the concentration of oligonucleotide. In contrast, if both strands are longer, the $T_m$ corresponds to a situation in which the strands are held together in structure possibly containing alternating duplex and denatured regions. In this case, the $T_m$ reflects an intramolecular equilibrium that is independent of time and polynucleotide concentration.

As is also well known in the art, $T_m$ is dependent on the composition of the polynucleotide (e.g. length, type of duplex, base composition, and extent of precise base pairing) and the composition of the solvent (e.g. salt concentration and the presence of denaturants such formamide). On equation for the calculation of $T_m$ can be found in Sambrook et al. (Molecular Cloning, 2nd ed., Cold Spring Harbor Press, 1989) and is:

$$T_m = 81.5EC - 16.6(\log_{10}[Na^+]) = 0.41(\% \, G + C) - 0.63(\% \, formamide) - 600/L)$$

Where L is the length of the hybrid in base pairs, the concentration of $Na^+$ is in the range of 0.01M to 0.4M and the G + C content is in the range of 30% to 75%. Equations for hybrids involving RNA can be found in the same reference. Alternative equations can be found in Davis et al., Basic Methods in Molecular Biology, 2nd ed., Appleton and Lange, 1994, Sec 6-8.

Likewise, the term "substantially similar," when used in reference to a polypeptide sequence, means that an amino acid sequence relative to a reference (query) sequence shares at least about 65% amino acid sequence identity, at least about 75% amino acid sequence identity, at least about 85%, at least about 90% , or at least about 95% or greater amino acid sequence identity. Generally, sequences having an $E \leq 10^{-8}$ are considered to be substantially similar to a query sequence. Such sequence identity can take into account conservative amino acid changes that do not substantially affect the function of a polypeptide.

Homology or identity is often measured using sequence analysis software such as the Sequence Analysis Software Package of the Genetics Computer Group (University of Wisconsin Biotechnology Center, 1710 University Avenue, Madison, WI 53705). Such software matches similar sequences by assigning degrees of homology to various deletions, substitutions and other modifications. The terms "homology" and "identity," when used herein in the context of two or more nucleic acids or polypeptide sequences, refer to two or more sequences or subsequences that are the same or have a specified percentage of amino acid residues or of nucleotides that are the same when compared and aligned for maximum correspondence over a comparison window or designated region as measured using any number of sequence comparison algorithms or by manual alignment and visual inspection.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

The term "comparison window" is used broadly herein to include reference to a segment of any one of the number of contiguous positions, for example, about 20 to 600 positions, for example, amino acid or nucleotide position, usually about 50 to about 200 positions, more usually about 100 to about 150 positions, in which a sequence may be compared to a reference sequence of the same number of contiguous positions after the two sequences are optimally aligned. Methods of alignment of sequence for comparison are well known in the art. Optimal alignment of sequences for comparison can be conducted, for example, by the local homology algorithm of Smith and Waterman (Adv. Appl. Math. 2:482,1981), by the homology alignment algorithm of Needleman and Wunsch (J. Mol. Biol. 48:443, 1970), by the search for similarity method of Person and Lipman (Proc. Natl. Acad. Sci., USA 85:2444, 1988), each of which is incorporated herein by reference; by computerized implementations of these algorithms (GAP, BESTFIT, FASTA, and TFASTA in the Wisconsin Genetics Software Package, Genetics Computer Group, 575 Science Dr., Madison, WI); or by manual alignment and visual inspection. Other algorithms for determining homology or identity include, for example, in addition to a BLAST program (Basic Local Alignment Search Tool at the National Center for Biological Information), ALIGN, AMAS (Analysis of Multiply Aligned Sequences), AMPS (Protein Multiple Sequence Alignment), ASSET (Aligned Segment Statistical Evaluation Tool), BANDS, BESTSCOR, BIOSCAN (Biological Sequence Comparative Analysis Node), BLIMPS (BLocks IMProved Searcher), FASTA, Intervals & Points, BMB, CLUSTAL V, CLUSTAL W, CONSENSUS, LCONSENSUS, WCONSENSUS, Smith-Waterman algorithm, DARWIN, Las Vegas algorithm, FNAT (Forced Nucleotide Alignment Tool), Framealign, Framesearch, DYNAMIC, FILTER, FSAP (Fristensky Sequence Analysis Package), GAP (Global Alignment Program), GENAL, GIBBS, GenQuest, ISSC (Sensitive Sequence Comparison), LALIGN (Local Sequence Alignment), LCP (Local Content Program), MACAW (Multiple Alignment Construction & Analysis Workbench), MAP (Multiple Alignment Program), MBLKP, MBLKN, PIMA (Pattern-Induced Multi-sequence Alignment), SAGA (Sequence Alignment by Genetic Algorithm) and WHAT-IF. Such alignment programs can also be used to screen genome databases to identify polynucleotide sequences having substantially identical sequences.

A number of genome databases are available for comparison. Several databases containing genomic information annotated with some functional information are maintained by different organizations, and are accessible via the internet, for example, http://wwwtigr.org/tdb; http://www.genetics.wisc.edu; http://genome-www.stanford-edu/~ball; http://hiv-web.lanl.gov; http://www.ncbi.nlm.nih.gov; http://www.ebi.ac.uk; http://Pasteur.fr/other/biology; and http:// www.genome.wi.mit.edu.

In particular, the BLAST and BLAST 2.0 algorithms using default parameters are particularly useful for identifying polynucleotides and polypeptides encompassed within the present invention (Altschul et al. (Nucleic Acids Res. 25: 3389-3402, 1977; J. Mol. Biol. 215:403-410, 1990). Software for performing BLAST analyses is publicly available through the National Center for Biotechnology Information (http://www.ncbi.nlm.nih.gov). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al., *supra,* 1977,1990). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always >0). For amino acid sequences, a scoring matrix is used to calculate the cumulative score. Extension of the word hits in each direction is halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, M=5, N=4 and a comparison of both strands. For amino acid sequences, the BLASTP program uses as defaults a wordlength of 3, and expectations (E) of 10, and the BLOSUM62 scoring matrix (see Henikoff and Henikoff, Proc. Natl. Acad. Sci., USA 89:10915,1989) alignments (B) of 50, expectation (E) of 10, M=5, N=-4, and a comparison of both strands.

The BLAST algorithm also performs a statistical analysis of the similarity between two sequences (see, for example, Karlin and Altschul, Proc. Natl. Acad. Sci., USA 90:5873, 1993). One measure of similarity provided by BLAST algorithm is the smallest sum probability (P(N)), which provides an indication of the probability by which a match between two nucleotide or amino acid sequences would occur by chance. For example, a nucleic acid is considered similar to a references sequence if the smallest sum probability in a comparison of the test nucleic acid to the reference nucleic acid is less than about 0.2, less than about 0.01, or less than about 0.001.

In one embodiment, protein and nucleic acid sequence homologies are evaluated using the Basic Local Alignment Search Tool ("BLAST"). In particular, five specific BLAST programs are used to perform the following tasks:

(1) BLASTP and BLAST3 compare an amino acid query sequence against a protein sequence database;
(2) BLASTN compares a nucleotide query sequence against a nucleotide sequence database;
(3) BLASTX compares the six-frame conceptual translation products of a query nucleotide sequence (both strands) against a protein sequence database;
(4) TBLASTN compares a query protein sequence against a nucleotide sequence database translated in all six reading frames (both strands); and
(5) TBLASTX compares the six-frame translations of a nucleotide query sequence against the six-frame translations of a nucleotide sequence database.

The BLAST programs identify homologous sequences by identifying similar segments, which are referred to herein as "high-scoring segment pairs," between a query amino or nucleic acid sequence and a test sequence which may be obtained from a protein or nucleic acid sequence database. High-scoring segment pairs can identified (*i.e.*, aligned) by means of a scoring matrix, many of which are known in the art. In one embodiment, the scoring matrix used is the BLOSUM62 matrix (Gonnet et al., Science 256:1443-1445, 1992; Henikoff and Henikoff, Proteins 17:49-61, 1993). In another embodiment, the PAM or PAM250 matrices may also be used (Schwartz and Dayhoff, eds., "Matrices for Detecting Distance Relationships: Atlas of Protein Sequence and Structure" (Washington, National Biomedical Research Foundation 1978)). BLAST programs are accessible through the U.S. National Library of Medicine, for example, at www.ncbi.nlm.nih.gov.

The parameters used with the above algorithms may be adapted depending on the sequence length and degree of homology studied. In some embodiments, the parameters may be the default parameters used by the algorithms in the absence of instructions from the user.

As disclosed herein, clusters of stress-regulated polynucleotides (and their products), some of which also have been described as having cellular functions such as enzymatic activity or roles as transcription factors, are involved in the response of plant cells and in particular plant cells of cereal crops to various abiotic stresses. As such, the polynucleotides in a cluster may share common stress-regulated regulatory elements, including, for example, cold-regulated regulatory elements, salinity-regulated regulatory elements, and osmotic pressure-regulated regulatory elements, as well as regulatory elements that are responsive to a combination of stress conditions, but not to any of the individual stress conditions, alone. The identification of such clusters of polynucleotides thus provides a means to identify the stress-regulated regulatory elements that control the level of expression of these nucleotide sequences.

As used herein, the term "stress-regulated polynucleotide" or "stress-responsive polynucleotide" means a polynucleotide sequence of a plant the transcription of which is altered, in response to exposure to a stress condition and/or the regulatory elements involved in the response. In general, such regulatory elements will be contained within a sequence including approximately two kilobases upstream of the transcription or translation start site and two kilobases downstream of the transcription or translation termination site. In the absence of a stress condition, the stress-regulated nucleotide sequence can normally be unexpressed in the cells, can be expressed at a basal level, which is induced to a higher level in response to the stress condition, or can be expressed at a level that is reduced in response to the stress condition. The coding region of a plant stress-regulated nucleotide sequence encodes a stress-regulated or stress-responsive polypeptide or a functional non-protein molecule such as a ribozyme or other functional RNA. A stress-regulated polypeptide can have an adaptive effect on a plant, thereby allowing the plant to better tolerate stress conditions; or can have a maladaptive effect, thereby decreasing the ability of the plant to tolerate the stress conditions.

"Genome" refers to the complete genetic material of an organism, specifically a plant, in particular, nuclear genetic material but inclusive of plastid genetic material.

A "functional RNA" refers to an antisense RNA, ribozyme, or other RNA that is not translated.

The present invention provides an isolated plant stress-regulated regulatory element, which regulates expression of an operatively linked nucleotide sequence in a plant in response a stress condition. A stress-responsive or stress-regulated regulatory element is one that alters transcription of an operatively linked polynucleotide in response to a stress, for example, an abiotic stress. Such alteration includes an increase in the transcription or a decrease in transcription of the operatively linked polynucleotide. As disclosed herein, a plant stress-regulated regulatory element can be isolated from a polynucleotide sequence of a plant stress-regulated nucleotide sequence as set forth in the accompanying sequence listing or a functional portion of said sequence. Methods for identifying and isolating the stress-regulated regulatory element from the disclosed polynucleotides, or genomic DNA clones corresponding thereto, are well known in the art. For example, methods of making deletion constructs or linker-scanner constructs can be used to identify nucleotide sequences that are responsive to a stress condition. Generally, such constructs include a reporter gene operatively linked to the sequence to be examined for regulatory activity. By performing such assays, a plant stress-regulated regulatory element can be defined within a sequence of about 500 nucleotides or fewer, generally at least about 200 nucleotides or fewer, particularly about 50 to 100 nucleotides, and more particularly at least about 20 nucleotides or fewer. In one embodiment, the minimal (core) sequence required for regulating a stress response of a plant is identified.

As used herein, the term "regulatory element" or "regulatory region" means a nucleotide sequence that, when operatively linked to a coding region, effects transcription of the coding region such that a ribonucleic acid (RNA) molecule is transcribed from the coding region. A regulatory element generally can increase or decrease the amount of transcription of a nucleotide sequence, for example, a coding sequence, operatively linked to the element with respect to the level at which the nucleotide sequence would be transcribed absent the regulatory element. Regulatory elements are well known in the art and include promoters, enhancers, silencers, inactivated silencer intron sequences, 3'-untranslated or 5'-untranslated sequences of transcribed sequence, for example, a poly-A signal sequence, or other protein or RNA stabilizing elements, or other gene expression control elements known to regulate gene expression or the amount of expression of a gene product. A regulatory element can be isolated from a naturally occurring genomic DNA sequence or can be synthetic, for example, a synthetic promoter. In one embodiment, the plant stress-regulated regulatory element is a plant stress-regulated promoter from a cereal.

Regulatory elements can be constitutively expressed regulatory elements, which maintain gene expression at a relative level of activity (basal level), or can be regulated regulatory elements. Constitutively expressed regulatory elements can be expressed in any cell type, or can be tissue specific, which are expressed only in particular cell types, phase specific, which are expressed only during particular developmental or growth stages of a plant cell, or the like. A regulatory element such as a tissue specific or phase specific regulatory element or an inducible regulatory element useful in constructing a recombinant polynucleotide or in a practicing a method of the invention can be a regulatory element that generally, in nature, is found in a plant genome. However, the regulatory element also can be from an organism other than a plant, including, for example, from a plant virus, an animal virus, or a cell from an animal or other multicellular organism.

One well-known type of regulatory element useful in the practice of the present invention is the promoter. Useful promoters include, but are not limited to, constitutive, inducible, temporally regulated, developmentally regulated, spatially-regulated, chemically regulated, stress-responsive, tissue-specific, viral and synthetic promoters. Promoter sequences are known to be strong or weak. A strong promoter provides for a high level of gene expression, whereas a weak promoter provides for a very low level of gene expression. An inducible promoter is a promoter that provides for the turning on and off of gene expression in response to an exogenously added agent, or to an environmental or developmental stimulus. A bacterial promoter such as the $P_{tac}$ promoter can be induced to varying levels of gene expression depending on the level of isothiopropylgalactoside added to the transformed bacterial cells. An isolated promoter sequence that is a strong promoter for heterologous nucleic acid is advantageous because it provides for a sufficient level of gene expression to allow for easy detection and selection of transformed cells and provides for a high level of gene expression when desired.

Within a plant promoter region there are several domains that are necessary for full function of the promoter. The first of these domains lies immediately upstream of the structural gene and forms the "core promoter region" containing consensus sequences, normally 70 base pairs immediately upstream of the gene. The core promoter region contains the characteristic CAAT and TATA boxes plus surrounding sequences, and represents a transcription initiation sequence that defines the transcription start point for the structural gene.

The presence of the core promoter region defines a sequence as being a promoter. The core promoter region, however, is insufficient to provide full promoter activity. A series of regulatory sequences upstream of the core constitute the remainder of the promoter. These regulatory sequences determine expression level, the spatial and temporal pattern of expression and, for an important subset of promoters, expression under inductive conditions (regulation by external factors such as light, temperature, chemicals, hormones).

To define a minimal promoter region, a DNA segment representing the promoter region is removed from the 5' region of the gene of interest and operably linked to the coding sequence of a marker (reporter) gene by recombinant DNA techniques well known to the art. The reporter gene is operably linked downstream of the promoter, so that transcripts initiating at the promoter proceed through the reporter gene. Reporter genes generally encode proteins that are easily measured, including, but not limited to, chloramphenicol acetyl transferase (CAT), beta-glucuronidase (GUS), green fluorescent protein (GFP), beta-galactosidase (beta-GAL), and luciferase.

The construct containing the reporter gene under the control of the promoter is then introduced into an appropriate cell type by transfection techniques well known to the art. In one embodiment, cell lysates are prepared and appropriate assays, which are well known in the art, for the reporter protein are performed. For example, if CAT were the reporter gene of choice, the lysates from cells transfected with constructs containing CAT under the control of a promoter under study are mixed with isotopically labeled chloramphenicol and acetyl-coenzyme A (acetyl-CoA). The CAT enzyme transfers the acetyl group from acetyl-CoA to the 2- or 3-position of chloramphenicol. The reaction is monitored by thin-layer chromatography, which separates acetylated chloramphenicol from unreacted material. The reaction products are then visualized by autoradiography.

The level of enzyme activity corresponds to the amount of enzyme that was made, which in turn reveals the level of expression from the promoter of interest. This level of expression can be compared to other promoters to determine the relative strength of the promoter under study. In order to be sure that the level of expression is determined by the promoter, rather than by the stability of the mRNA, the level of the reporter mRNA can be measured directly, such as by Northern blot analysis.

Once activity is detected, mutational and/or deletional analyses may be employed to determine the minimal region and/or sequences required to initiate transcription. Thus, sequences can be deleted at the 5' end of the promoter region and/or at the 3' end of the promoter region, and nucleotide substitutions introduced. These constructs are then introduced to cells and their activity determined.

The choice of promoter will vary depending on the temporal and spatial requirements for expression, and also depending on the target species. In some cases, expression in multiple tissues is desirable. While in others, tissue-specific, e.g., leaf-specific, seed-specific, petal-specific, anther-specific, or pith-specific, expression is desirable. Although many promoters from dicotyledons have been shown to be operational in monocotyledons and *vice versa,* typically dicotyledonous promoters are selected for expression in dicotyledons, and monocotyledonous promoters for expression in monocotyledons. There is, however, no restriction to the provenance of selected promoters. It is sufficient that the promoters are operational in driving the expression of the nucleotide sequences in the desired cell.

A range of naturally-occurring promoters are known to be operative in plants and have been used to drive the expression of heterologous (both foreign and endogenous) genes and nucleotide sequences in plants: for example, the constitutive 35S cauliflower mosaic virus (CaMV) promoter, the ripening-enhanced tomato polygalacturonase promoter (Schuch et al., Plant Mol. Biol., 13:303, 1989), the E8 promoter (Diekman & Fischer, EMBO J., 7:3315 1988) and the fruit specific 2A1 promoter (Pear et al., Plant Mol. Biol., 13:639, 1989). Many other promoters, e.g., U2 and U5 snRNA promoters from maize, the promoter from alcohol dehydrogenase, the Z4 promoter from a gene encoding the Z4 22 kD zein protein, the Z10 promoter from a gene encoding a 10 kD zein protein, a Z27 promoter from a gene encoding a 27 kD zein protein, the A20 promoter from the gene encoding a 19 kD -zein protein, inducible promoters, such as the light inducible promoter derived from the pea rbcS gene and the actin promoter from rice, e.g., the actin 2 promoter (WO 00/70067); seed specific promoters, such as the phaseolin promoter from beans, may also be used. The stress-regulated nucleotide sequences of this invention can also be expressed under the regulation of promoters that are chemically regulated. This enables the nucleic acid sequence or encoded polypeptide to be synthesized only when the crop plants are treated with the inducing chemicals. Chemical induction of gene expression is detailed in EP 0 332 104 and U.S. Patent 5,614,395.

In some instances it may be desirable to link a constitutive promoter to the stress regulated nucleotide sequences of the present invention. Examples of some constitutive promoters which have been described include the rice actin 1 (Wang et al., Molec. Cell Biol., 12:3399, 1992; U.S. Patent No. 5,641,876), CaMV 35S (Odell et al., Nature, 313:810, 1985), CaMV 19S (Lawton et al., Mol. Cell Biol., 7:335, 1987), *nos,* Adh, sucrose synthase; and the ubiquitin promoters.

In other situations it may be desirable to limit expression of stress-related sequences to specific tissues or stages of development. As used herein, the term "tissue specific or phase specific regulatory element" means a nucleotide sequence that effects transcription in only one or a few cell types, or only during one or a few stages of the life cycle of a plant, for example, only for a period of time during a particular stage of growth, development or differentiation. The terms "tissue specific" and "phase specific" are used together herein in referring to a regulatory element because a single regulatory element can have characteristics of both types of regulatory elements. For example, a regulatory element active only during a particular stage of plant development also can be expressed only in one or a few types of cells in the plant during the particular stage of development. As such, any attempt to classify such regulatory elements as tissue specific or as phase specific can be difficult. Accordingly, unless indicated otherwise, all regulatory elements having the characteristic of a tissue specific regulatory element, or a phase specific regulatory element, or both are considered together for purposes of the present invention.

Examples of tissue specific promoters which have been described include the lectin (Vodkin, Prog. Clin Biol. Res., 138:87, 1983; Lindstrom et al., Der. Genet., 11:160, 1990) corn alcohol dehydrogenase 1 (Vogel et al., EMBO J., 11: 157, 1992; Dennis et al., Nuc. Acid Res., 12:3983, 1984), corn light harvesting complex (Bansal et al., Proc. Natl. Acad. Sci. USA, 89:3654, 1992), corn heat shock protein (Odell et al., Nature, 313:810, 1985), pea small subunit RuBP carboxylase (Poulsen et al., Mol. Gen. Genet., 205:193, 1986), Ti plasmid mannopine synthase (Langridge et al., Proc. Natl. Acad. Sci., USA, 86:3219, 1989), Ti plasmid nopaline synthase (Langridge et al., Proc. Natl. Acad. Sci., USA, 86: 3219, 1989), petunia chalcone isomerase (vanTunen et al., EMBO J., 7:1257, 1988), bean glycine rich protein 1 (Keller et al., Genes Dev., 3:1639, 1989), truncated CaMV 35s (Odell et al., Nature, 313:810, 1985), potato patatin (Wenzler et al., Plant Molec. Biol., 13:347, 1989), root cell (Yamamoto et al., Nuc. Acid Res., 18:7449, 1990), maize zein (Reina et al., Nuc. Acids Res., 18:6425-26, 1990; Kriz et al., Mol. Gen. Genet., 207:90, 1987; Wandelt et al., Nuc. Acids Res., 17:2354, 1989; Langridge et al., Cell, 34:1015, 1983; Reina et al., Nuc. Acids Res., 18:6425-26 1990), globulin-1 (Belanger et a]., Genetics, 129:863, 1991), a-tubulin, cab (Sullivan et al., Mol. Gen. Genet., 215:431, 1989), PEPCase (Hudspeth & Grula, Plant Molec. Biol., 12:579, 1989), R gene complex-associated promoters (Chandler et al., Plant Cell, 1:1175, 1989), histone, and chalcone synthase promoters (Franken et al., EMBO J., 10:2605, 1991). Tissue specific enhancers are described in Fromm et al. Bio/Technology, 8:833, 1989.

Several other tissue-specific regulated genes and/or promoters have been reported in plants. These include genes encoding the seed storage proteins (such as napin, cruciferin, beta-conglycinin, and phaseolin) zein or oil body proteins (such as oleosin), or genes involved in fatty acid biosynthesis (including acyl carrier protein, stearoyl-ACP desaturase, fatty acid desaturases (fad 2-1)), and other genes expressed during embryo development (such as Bce4, see, for example, EP 255378 and Kridl et al., Seed Sci. Res., 1:209, 1991). Particularly useful for seed-specific expression is the pea vicilin promoter (Czako et al., Mol. Gen. Genet., 235:33, 1992). (See also U.S. Pat. No. 5,625,136, herein incorporated by reference.) Other useful promoters for expression in mature leaves are those that are switched on at the onset of senescence, such as the SAG promoter from Arabidopsis (Gan et al., Science, 270:1986, 1995).

A class of fruit-specific promoters expressed at or during antithesis through fruit development, at least until the beginning of ripening, is discussed in U.S. 4,943,674. cDNA clones that are preferentially expressed in cotton fiber have been isolated (John et al., Proc. Natl. Acad. Sci. USA, 89:5769, 1992). cDNA clones from tomato displaying differential expression during fruit development have been isolated and characterized (Mansson et al., Gen. Genet., 200:356, 1985, Slater et al., Plant Molec. Biol., 5:137, 1985). The promoter for polygalacturonase gene is active in fruit ripening. The polygalacturonase gene is described in U.S. Patent No. 4,535,060, U.S. Patent No. 4,769,061, U.S. Patent No. 4,801,590, and U.S. Patent No. 5,107,065, which disclosures are incorporated herein by reference.

Other examples of tissue-specific promoters include those that direct expression in leaf cells following damage to the leaf (for example, from chewing insects), in tubers (for example, patatin gene promoter), and in fiber cells (an example of a developmentally-regulated fiber cell protein is E6 (John et al., Proc. Natl. Acad. Sci. USA, 89:5769, 1992). The E6 gene is most active in fiber, although low levels of transcripts are found in leaf, ovule and flower.

Additional tissue specific or phase specific regulatory elements include, for example, the *AGL8*/*FRUITFULL* regulatory element, which is activated upon floral induction (Hempel et al., Development 124:3845-3853, 1997) root specific regulatory elements such as the regulatory elements from the RCP1 gene and the LRP1 gene (Tsugeki and Fedoroff, Proc. Natl. Acad., USA 96:12941-12946, 1999; Smith and Fedoroff, Plant Cell 7:735, 1995); flower specific regulatory elements such as the regulatory elements from the *LEAFY* gene and the *APETELA1* gene (Blazquez et al., Development 124:3835-3844, 1997; Hempel et al., supra, 1997); seed specific regulatory elements such as the regulatory element from the oleosin gene (Plant et al., Plant Mol. Biol. 25:193-205, 1994), and dehiscence zone specific regulatory element. Additional tissue specific or phase specific regulatory elements include the Zn13 promoter, which is a pollen specific promoter (Hamilton et al., Plant Mol. Biol. 18:211-218, 1992); the *UNUSUAL FLORAL ORGANS (UFO)* promoter, which is active in apical shoot meristem; the promoter active in shoot meristems (Atanassova et al., Plant J. 2:291, 1992), the cdc2a promoter and cyc07 promoter (see, for example, Ito et al., Plant Mol. Biol. 24:863, 1994; Martinez et al., Proc. Natl. Acad. Sci., USA 89:7360, 1992; Medford et al., Plant Cell 3:359, 1991; Terada et al., Plant J. 3:241, 1993; Wissenbach et al., Plant J. 4:411, 1993); the promoter of the *APETELA3* gene, which is active in floral meristems (Jack et al., Cell 76:703, 1994; Hempel et a]., *supra,* 1997); a promoter of an agamous-like (AGL) family member, for example, AGL8, which is active in shoot meristem upon the transition to flowering (Hempel et al., *supra,* 1997); floral abscission zone promoters; L1-specific promoters; and the like.

The tissue-specificity of some "tissue-specific" promoters may not be absolute and may be tested by one skilled in the art using the diphtheria toxin sequence. One can also achieve tissue-specific expression with "leaky" expression by a combination of different tissue-specific promoters (Beals et al., Plant Cell, 9:1527, 1997). Other tissue-specific promoters can be isolated by one skilled in the art (see U.S. 5,589,379). Several inducible promoters ("gene switches") have been reported, many of which are described in the review by Gatz (Cur. Opin. Biotech, 7:168, 1996) and Gatz (Ann. Rev. Plant. Physiol. Plant Mol. Biol., 48:89, 1997). These include tetracycline repressor system, Lac repressor system, copper-inducible systems, salicylate-inducible systems (such as the PR1a system), glucocorticoid- (Aoyama et al., N-H Plant J., 11:605, 1997) and ecdysome-inducible systems. Also included are the benzene sulphonamide- (U.S. Patent No. 5,364,780) and alcohol-(WO 97/06269 and WO 97/06268) inducible systems and glutathione S-transferase promoters.

In some instances it might be desirable to inhibit expression of a native DNA sequence within a plant's tissues to achieve a desired phenotype. In this case, such inhibition might be accomplished with transformation of the plant to comprise a constitutive, tissue-independent promoter operably linked to an antisense nucleotide sequence, such that constitutive expression of the antisense sequence produces an RNA transcript that interferes with translation of the mRNA of the native DNA sequence.

Inducible regulatory elements also are useful for purposes of the present invention. As used herein, the term "inducible regulatory element" means a regulatory element that, when exposed to an inducing agent, effects an increased level of transcription of a nucleotide sequence to which it is operatively linked as compared to the level of transcription, if any, in the absence of an inducing agent. Inducible regulatory elements can be those that have no basal or constitutive activity and only effect transcription upon exposure to an inducing agent, or those that effect a basal or constitutive level of transcription, which is increased upon exposure to an inducing agent. Inducible regulatory elements that effect a basal or constitutive level of expression generally are useful in a method or composition of the invention where the induced level of transcription is substantially greater than the basal or constitutive level of expression, for example, at least about two-fold greater, or at least about five-fold greater. Particularly useful inducible regulatory elements do not have a basal or constitutive activity, or increase the level of transcription at least about ten-fold greater than a basal or constitutive level of transcription associated with the regulatory element.

Inducible promoters that have been described include the ABA- and turgor-inducible promoters, the promoter of the auxin-binding protein gene (Schwob et al., Plant J., 4:423, 1993), the UDP glucose flavonoid glycosyl-transferase gene promoter (Ralston et al., Genetics, 119:185, 1988), the MPI proteinase inhibitor promoter (Cordero et al., Plant J., 6:141, 1994), and the glyceraldehyde-3-phosphate dehydrogenase gene promoter (Kohler et al., Plant Molec. Biol.' 29: 1293, 1995; Quigley et al., J. Mol. Evol., 29:412, 1989; Martinez et al., J. Mol. Biol., 208:551, 1989).

The term "inducing agent" is used to refer to a chemical, biological or physical agent or environmental condition that effects transcription from an inducible regulatory element. In response to exposure to an inducing agent, transcription from the inducible regulatory element generally is initiated *de novo* or is increased above a basal or constitutive level of expression. Such induction can be identified using the methods disclosed herein, including detecting an increased level of RNA transcribed from a nucleotide sequence operatively linked to the regulatory element, increased expression of a polypeptide encoded by the nucleotide sequence, or a phenotype conferred by expression of the encoded polypeptide.

An inducing agent useful in a method of the invention is selected based on the particular inducible regulatory element. For example, the inducible regulatory element can be a metallothionein regulatory element, a copper inducible regulatory element or a tetracycline inducible regulatory element, the transcription from which can be effected in response to metal ions, copper or tetracycline, respectively (Furst et al., Cell 55:705-717, 1988; Mett et al., Proc. Natl. Acad. Sci., USA 90:4567-4571, 1993; Gatz et al., Plant J. 2:397-404, 1992; Roder et al., Mol. Gen. Genet. 243:32-38, 1994). The inducible regulatory element also can be an ecdysone regulatory element or a glucocorticoid regulatory element, the transcription from which can be effected in response to ecdysone or other steroid (Christopherson et al., Proc. Natl. Acad. Sci., USA 89:6314-6318, 1992; Schena et al., Proc. Natl. Acad. Sci., USA 88:10421-10425, 1991). In addition, the regulatory element can be a cold responsive regulatory element or a heat shock regulatory element, the transcription of which can be effected in response to exposure to cold or heat, respectively (Takahashi et al., Plant Physiol. 99: 383-390, 1992). Additional regulatory elements useful in the methods or compositions of the invention include, for example, the spinach nitrite reductase gene regulatory element (Back et al., Plant Mol. Biol. 17:9, 1991); a light inducible regulatory element (Feinbaum et al., Mol. Gen. Genet. 226:449,1991; Lam and Chua, Science 248:471, 1990), a plant hormone inducible regulatory element (Yamaguchi-Shinozaki et al., Plant Mol. Biol. 15:905, 1990; Kares et al., Plant Mol. Biol. 15:225, 1990), and the like.

An inducible regulatory element also can be a plant stress-regulated regulatory element of the invention. In addition to the known stress conditions that specifically induce or repress expression from such elements, the present invention provides methods of identifying agents that mimic a stress condition. Accordingly, such stress mimics are considered inducing or repressing agents with respect to a plant stress-regulated regulatory element. In addition, a recombinant polypeptide comprising a zinc finger domain, which is specific for the regulatory element, and an effector domain, particularly an activator, can be useful as an inducing agent for a plant stress-regulated regulatory element. Furthermore, such a recombinant polypeptide provides the advantage that the effector domain can be a repressor domain, thereby providing a repressing agent, which decreases expression from the regulatory element. In addition, use of such a method of modulating expression of an endogenous plant stress-regulated nucleotide sequence provides the advantage that the polynucleotide encoding the recombinant polypeptide can be introduced into cells of the plant, thus providing a transgenic plant that can be regulated coordinately with the endogenous plant stress-regulated nucleotide sequence upon exposure to a stress condition. A polynucleotide encoding such a recombinant polypeptide can be operatively linked to and expressed from a constitutively active, inducible or tissue specific or phase specific regulatory element.

In one embodiment, the promoter may be a gamma zein promoter, an oleosin ole16 promoter, a globulinI promoter, an actin I promoter, an actin c1 promoter, a sucrose synthetase promoter, an INOPS promoter, an EXM5 promoter, a globulin2 promoter, a b-32, ADPG- pyrophosphorylase promoter, an LtpI promoter, an Ltp2 promoter, an oleosin olel7 promoter, an oleosin ole18 promoter, an actin 2 promoter, a pollen-specific protein promoter, a pollen-specific pectate lyase promoter, an anther-specific protein promoter, an anther-specific gene RTS2 promoter, a pollen-specific gene promoter, a tapeturn-specific gene promoter, tapeturn- specific gene RAB24 promoter, a anthranilate synthase alpha subunit promoter, an alpha zein promoter, an anthranilate synthase beta subunit promoter, a dihydrod-ipicolinate synthase promoter, a ThiI promoter, an alcohol dehydrogenase promoter, a cab binding protein promoter, an H3C4 promoter, a RUBISCO SS starch branching enzyme promoter, an ACCase promoter, an actin3 promoter, an actin7 promoter, a regulatory protein GF14-12 promoter, a ribosomal protein L9 promoter, a cellulose biosynthetic enzyme promoter, an S-adenosyl-L-homocysteine hydrolase promoter, a superoxide dismutase promoter, a C-kinase receptor promoter, a phosphoglycerate mutase promoter, a root-specific RCc3 mRNA promoter, a glucose-6 phosphate isomerase promoter, a pyrophosphate-fructose 6-phosphatelphosphotransferase promoter, an ubiquitin promoter, a beta-ketoacyl-ACP synthase promoter, a 33 kDa photosystem 11 promoter, an oxygen evolving protein promoter, a 69 kDa vacuolar ATPase subunit promoter, a metallothionein-like protein promoter, a glyceraldehyde-3 -phosphate dehydrogenase promoter, an ABA- and ripening-inducible-like protein promoter, a phenylalanine ammonia lyase promoter, an adenosine triphosphatase S-adenosyl-L-homocysteine hydrolase promoter, an a- tubulin promoter, a cab promoter, a PEPCase promoter, an R gene promoter, a lectin promoter, a light harvesting complex promoter, a heat shock protein promoter, a chalcone synthase promoter, a zein promoter, a globulin-1 promoter, an ABA promoter, an auxin-binding protein promoter, a UDP glucose flavonoid glycosyl-transferase gene promoter, an NTI promoter, an actin promoter, an opaque 2 promoter, a b70 promoter, an oleosin promoter, a CaMV 35S promoter, a CaMV 19S promoter, a histone promoter, a turgor-inducible promoter, a pea small subunit RuBP carboxylase promoter, a Ti plasmid mannopine synthase promoter, Ti plasmid nopaline synthase promoter, a petunia chalcone isomerase promoter, a bean glycine rich protein I promoter, a CaMV 35S transcript promoter, a potato patatin promoter, or a S-E9 small subunit RuBP carboxylase promoter.

In addition to promoters, a variety of 5' and 3' transcriptional regulatory sequences are also available for use in the present invention. Transcriptional terminators are responsible for the termination of transcription and correct mRNA polyadenylation. The 3' nontranslated regulatory DNA sequence usually includes from about 50 to about 1,000, typically about 100 to about 1,000, nucleotide base pairs and contains plant transcriptional and translational termination sequences. Appropriate transcriptional terminators and those which are known to function in plants include the CaMV 35S terminator, the *tml* terminator, the nopaline synthase terminator, the pea rbcS E9 terminator, the terminator for the T7 transcript from the octopine synthase gene of *Agrobacterium tumefaciens,* and the 3' end of the protease inhibitor I or II genes from potato or tomato, although other 3' elements known to those of skill in the art can also be employed. Alternatively, one also could use a gamma coixin, oleosin 3 or other terminator from the genus Coix.

Suitable 3' elements include those from the nopaline synthase gene of *Agrobacterium tumefaciens* (Bevan et al., Nature, 304:184, 1983), the terminator for the T7 transcript from the octopine synthase gene of *Agrobacterium tumefaciens,* and the 3' end of the protease inhibitor I or II genes from potato or tomato.

As the DNA sequence between the transcription initiation site and the start of the coding sequence, i.e., the untranslated leader sequence, can influence gene expression, one may also wish to employ a particular leader sequence. Suitable leader sequences are contemplated to include those that comprise sequences predicted to direct optimum expression of the attached sequence, i.e., to include a consensus leader sequence that may increase or maintain mRNA stability and prevent inappropriate initiation of translation. The choice of such sequences will be known to those of skill in the art in light of the present disclosure. Sequences that are derived from genes that are highly expressed in plants are desirable.

Other sequences that have been found to enhance gene expression in transgenic plants include intron sequences (e.g., from *Adh1, bronze1, actin1, actin2* (WO 00/760067), or the sucrose synthase intron) and viral leader sequences (e.g., from TMV, MCMV and AMV). For example, a number of non-translated leader sequences derived from viruses are known to enhance expression. Specifically, leader sequences from Tobacco Mosaic Virus (TMV), Maize Chlorotic Mottle Virus (MCMV), and Alfalfa Mosaic Virus (AMV) have been shown to be effective in enhancing expression (e.g., Gallie et al., Nuc. Acids Res., 15:8693, 1987; Skuzeski et al., Plant Mol. Biol., 15:65, 1990). Other leaders known in the art, include but are not limited to: Picornavirus leaders, for example, EMCV leader (Encephalomyocarditis 5 noncoding region) (Elroy-Stein et al., Proc. Natl. Acad. Sci. USA, 86:6126, 1989); Potyvirus leaders, for example, TEV leader (Tobacco Etch Virus); MDMV leader (Maize Dwarf Mosaic Virus); Human immunoglobulin heavy-chain binding protein (BiP) leader, (Macejak et al., Nature, 353:90, 1991); Untranslated leader from the coat protein mRNA of alfalfa mosaic virus (AMV RNA 4), (Jobling et al., Nature, 325:622, 1987; Tobacco mosaic virus leader (TMV), (Gallie et al., Molecular Biology of RNA, 237 1989; and Maize Chlorotic Mottle Virus leader (MCMV) (Lommel et al., Virology. 81: 382,1991. See also, Della-Cioppa et al., Plant Physiol., 84:965, 1987.

Regulatory elements such as Adh intron 1 (Callis et al., Genes Devel., 1:1183, 1987), sucrose synthase intron (Vasil et a]., Plant Physiol., 91:1575, 1989) or TMV omega element (Gallie, et al., Molecular Biology of RNA, 237 1989 1989), may further be included where desired.

Examples of enhancers include elements from the CaMV 35S promoter, octopine synthase genes (Ellis el al., EMBO J., 6:3203, 1987), the rice actin I gene, the maize alcohol dehydrogenase gene (Callis et al., Genes Devel., 1: 1183, 1987), the maize shrunken I gene (Vasil et al., Plant Physiol., 91:1575, 1989), TMV Omega element (Gallie et al., Molecular Biology of RNA, 1989) and promoters from non-plant eukaryotes (e.g. yeast; Ma et al., Nature, 334:631, 1988).

Vectors for use in accordance with the present invention may be constructed to include the ocs enhancer element. This element was first identified as a 16 bp palindromic enhancer from the octopine synthase (ocs) gene of ultilane (Ellis et al., EMBO J., 6:3203, 1987), and is present in at least 10 other promoters (Bouchez et al., EMBO J., 8: 4197, 1989). The use of an enhancer element, such as the ocs element and particularly multiple copies of the element, will act to increase the level of transcription from adjacent promoters when applied in the context of monocot transformation.

The methods of the invention provide genetically modified plant cells, which can contain, for example, a coding region, or functional portion thereof, of a plant stress-regulated polynucleotide operatively linked to a heterologous inducible regulatory element; or a plant stress-regulated regulatory element operatively linked to a heterologous nucleotide sequence encoding a polypeptide of interest. In such a plant, the expression from the inducible regulatory element can be effected by exposing the plant cells to an inducing agent in any of numerous ways depending, for example, on the inducible regulatory element and the inducing agent. For example, where the inducible regulatory element is a cold responsive regulatory element present in the cells of a transgenic plant, the plant can be exposed to cold conditions, which can be produced artificially, for example, by placing the plant in a thermostatically controlled room, or naturally, for example, by planting the plant in an environment characterized, at least in part, by attaining temperatures sufficient to induce transcription from the promoter but not so cold as to kill the plants. By examining the phenotype of such transgenic plants, those plants that ectopically express a gene product that confers increased resistance of the plant to cold can be identified. Similarly, a transgenic plant containing a metallothionein promoter can be exposed to metal ions such as cadmium or copper by watering the plants with a solution containing the inducing metal ions, or can be planted in soil that is contaminated with a level of such metal ions that is toxic to most plants. The phenotype of surviving plants can be observed, those expressing desirable traits can be selected.

As used herein, the term "phenotype" refers to a physically detectable characteristic. A phenotype can be identified visually by inspecting the physical appearance of a plant following exposure, for example, to increased osmotic conditions; can be identified using an assay to detecting a product produced due to expression of reporter gene, for example, an RNA molecule, a polypeptide such as an enzyme, or other detectable signal such as disclosed herein; or by using any appropriate tool useful for identifying a phenotype of a plant, for example, a microscope, a fluorescence activated cell sorter, or the like.

A transgenic plant containing an inducible regulatory element such as a steroid inducible regulatory element can be exposed to a steroid by watering the plants with a solution containing the steroid. The use of an inducible regulatory element that is induced upon exposure to a chemical or biological inducing agent that can be placed in solution or suspension in an aqueous medium can be particularly useful because the inducing agent can be applied conveniently to a relatively large crop of transgenic plants containing the inducible regulatory element, for example, through a watering system or by spraying the inducing agent over the field. As such, inducible regulatory elements that are responsive to an environmental inducing agent, for example, cold; heat; metal ions or other potentially toxic agents such as a pesticides, which can contaminate a soil; or the like; or inducible regulatory elements that are regulated by inducing agents that conveniently can be applied to plants, can be particularly useful in a method or composition of the invention, and allow the identification and selection of plants that express desirable traits and survive and grow in environments that otherwise would not support growth of the plants.

For purposes of modulating the responsiveness of a plant to a stress condition, it can be useful to introduce a modified plant stress-regulated regulatory element into a plant. Such a modified regulatory element can have any desirable characteristic, for example, it can be inducible to a greater level than the corresponding wild-type promoter, or it can be inactivated such that, upon exposure to a stress, there is little or no induction of expression of a nucleotide sequence operatively linked to the mutant element. A plant stress-regulated regulatory element can be modified by incorporating random mutations using, for example, *in vitro* recombination or DNA shuffling (Stemmer et al., Nature 370: 389-391, 1994; U.S. Patent No. 5,605,793). Using such a method, millions of mutant copies of the polynucleotide, for example, stress-regulated regulatory element, can be produced based on the original nucleotide sequence, and variants with improved properties, such as increased inducibility can be recovered.

A mutation method such as DNA shuffling encompasses forming a mutagenized double-stranded polynucleotide from a template double-stranded polynucleotide, wherein the template double-stranded polynucleotide has been cleaved into double stranded random fragments of a desired size, and comprises the steps of adding to the resultant population of double-stranded random fragments one or more single or double stranded oligonucleotides, wherein the oligonucleotides comprise an area of identity and an area of heterology to the double stranded template polynucleotide; denaturing the resultant mixture of double stranded random fragments and oligonucleotides into single stranded fragments; incubating the resultant population of single stranded fragments with a polymerase under conditions that result in the annealing of the single stranded fragments at the areas of identity to form pairs of annealed fragments, the areas of identity being sufficient for one member of a pair to prime replication of the other, thereby forming a mutagenized double-stranded polynucleotide; and repeating the second and third steps for at least two further cycles, wherein the resultant mixture in the second step of a further cycle includes the mutagenized double-stranded polynucleotide from the third step of the previous cycle, and the further cycle forms a further mutagenized double-stranded polynucleotide. Typically, the concentration of a single species of double stranded random fragment in the population of double stranded random fragments is less than 1% by weight of the total DNA. In addition, the template double stranded polynucleotide can comprise at least about 100 species of polynucleotides. The size of the double stranded random fragments can be from about 5 base pairs to 5 kilobase pairs. In a further embodiment, the fourth step of the method comprises repeating the second and the third steps for at least 10 cycles.

A plant stress-regulated regulatory element of the invention is useful for expressing a nucleotide sequence operatively linked to the element in a cell, particularly a plant cell. As used herein, the term "expression" refers to the transcription and/or translation of an endogenous gene or a transgene in plants. In the case of an antisense molecule, for example, the term "expression" refers to the transcription of the polynucleotide encoding the antisense molecule.

As used herein, the term "operatively linked," when used in reference to a plant stress-regulated regulatory element, means that the regulatory element is positioned with respect to a second nucleotide sequence such that the regulatory element effects transcription or transcription and translation of the nucleotide sequence in substantially the same manner, but not necessarily to the same extent, as it does when the regulatory element is present in its natural position in a genome. Transcriptional promoters, for example, generally act in a position and orientation dependent manner and usually are positioned at or within about five nucleotides to about fifty nucleotides 5' (upstream) of the start site of transcription of a gene in nature. In comparison, enhancers and silencers can act in a relatively position or orientation independent manner and, therefore, can be positioned several hundred or thousand nucleotides upstream or downstream from a transcription start site, or in an intron within the coding region of a gene, yet still be operatively linked to a coding region so as to effect transcription.

The second nucleotide sequence, i.e., the sequence operatively linked to the plant stress-regulated regulatory element, can be any nucleotide sequence, including, for example, a coding region of a gene or cDNA; a sequence encoding an antisense molecule, an RNAi molecule, ribozyme, triplexing agent (see, for example, Frank-Kamenetskii and Mirkin, Ann. Rev. Biochem. 64:65-95, 1995), or the like; or a sequence that, when transcribed, can be detected in the cell using, for example, by hybridization or amplification, or when translated produces a detectable signal. The term "coding region" is used broadly herein to include a nucleotide sequence of a genomic DNA or a cDNA molecule comprising all or part of a coding region of the coding strand. A coding region can be transcribed from an operatively linked regulatory element, and can be translated into a full-length polypeptide or a peptide portion of a polypeptide, preferably a peptide portion having the same functional characteristics as the full-length polypeptide. It should be recognized that, in a nucleotide sequence comprising a coding region, not all of the nucleotides in the sequence need necessarily encode the polypeptide and, particularly, that a gene transcript can contain one or more introns, which do not encode an amino acid sequence of a polypeptide but, nevertheless, are part of the coding region, particularly the coding strand, of the gene.

The present invention also relates to a recombinant polynucleotide, which contains a functional portion of a plant stress-regulated nucleotide sequence operatively linked to a heterologous nucleotide sequence. As used herein, the term "functional portion" of plant stress-regulated sequence means a contiguous nucleotide sequence of the plant stress-regulated sequence that provides a function within a plant or plant cell. The portion can be any portion of the sequence, particularly a coding sequence, or a sequence encoding a peptide portion of the stress-regulated polypeptide; the stress-regulated regulatory element such as a promoter or minimal promoter; a sequence useful as an antisense molecule or triplexing agent; or a sequence useful for disrupting (knocking-out) an endogenous plant stress-regulated nucleotide sequence.

A heterologous nucleotide sequence is a nucleotide sequence that is not normally part of the plant stress-regulated polynucleotide from which the functional portion of the plant stress-regulated polynucleotide-component of the recombinant polynucleotide is obtained; or, if it is a part of the plant stress-regulated polynucleotide sequence from which the functional portion is obtained, it is an orientation other than it would normally be in, for example, is an antisense sequence, or comprises at least partially discontinuous as compared to the genomic structure, for example, a single exon operatively linked to the regulatory element. In general, where the functional portion of the plant stress-regulated nucleotide sequence comprises the coding sequence in a recombinant polynucleotide of the invention, the heterologous nucleotide sequence will function as a regulatory element. The regulatory element can be any heterologous regulatory element, including, for example, a constitutively active regulatory element, an inducible regulatory element, or a tissue specific or phase specific regulatory element, as disclosed above. Conversely, where the functional portion of the plant stress-regulated polynucleotide comprises the stress-regulated regulatory element of a recombinant polynucleotide of the invention, the heterologous nucleotide sequence generally will be a nucleotide sequence that can be transcribed and, if desired, translated. Where the heterologous nucleotide sequence is expressed from a plant stress-regulated regulatory element, it generally confers a desirable phenotype to a plant cell containing the recombinant polynucleotide, or provides a means to identify a plant cell containing the recombinant polynucleotide. It should be recognized that a "desirable" phenotype can be one that decreases the ability of a plant cell to compete where the plant cell, or a plant containing the cell, is an undesired plant cell. Thus, a heterologous nucleotide sequence can allow a plant to grow, for example, under conditions in which it would not normally be able to grow.

A heterologous nucleotide sequence can be, or encode, a selectable marker. As used herein, the term "selectable marker" is used herein to refer to a molecule that, when present or expressed in a plant cell, provides a means to identify a plant cell containing the marker. As such, a selectable marker can provide a means for screening a population of plants, or plant cells, to identify those having the marker. A selectable marker also can confer a selective advantage to the plant cell, or a plant containing the cell. The selective advantage can be, for example, the ability to grow in the presence of a negative selective agent such as an antibiotic or herbicide, compared to the growth of plant cells that do not contain the selectable marker. The selective advantage also can be due, for example, to an enhanced or novel capacity to utilize an added compound as a nutrient, growth factor or energy source. A selectable advantage can be conferred, for example, by a single polynucleotide, or its expression product, or to a combination of polynucleotides whose expression in a plant cell gives the cell with a positive selective advantage, a negative selective advantage, or both.

Examples of selectable markers include those that confer antimetabolite resistance, for example, dihydrofolate reductase, which confers resistance to methotrexate (Reiss, Plant Physiol. (Life Sci. Adv.) 13:143-149, 1994); neomycin phosphotransferase, which confers resistance to the aminoglycosides neomycin, kanamycin and paromycin (Herrera-Estrella, EMBO J. 2:987-995, 1983) and hygro, which confers resistance to hygromycin (Marsh, Gene 32:481-485, 1984), trpB, which allows cells to utilize indole in place of tryptophan; hisD, which allows cells to utilize histinol in place of histidine (Hartman, Proc. Natl. Acad. Sci., USA 85:8047, 1988); mannose-6-phosphate isomerase which allows cells to utilize mannose (WO 94/20627); ornithine decarboxylase, which confers resistance to the ornithine decarboxylase inhibitor, 2-(difluoromethyl)-DL-ornithine (DFMO; McConlogue, 1987, In: Current Communications in Molecular Biology, Cold Spring Harbor Laboratory ed.); and deaminase from *Aspergillus terreus,* which confers resistance to Blasticidin S (Tamura, Biosci. Biotechnol. Biochem. 59:2336-2338, 1995). Additional selectable markers include those that confer herbicide resistance, for example, phosphinothricin acetyltransferase gene, which confers resistance to phosphinothricin (White et al., Nucl. Acids Res. 18:1062, 1990; Spencer et al., Theor. Appl. Genet. 79:625-631, 1990), a mutant EPSPV-synthase, which confers glyphosate resistance (Hinchee et al., Bio/Technology 91:915-922, 1998), a mutant acetolactate synthase, which confers imidazolione or sulfonylurea resistance (Lee et al., EMBO J. 7:1241-1248, 1988), a mutant psbA, which confers resistance to atrazine (Smeda et al., Plant Physiol. 103:911-917, 1993), or a mutant protoporphyrinogen oxidase (see U.S. Patent No. 5, 767, 373), or other markers conferring resistance to an herbicide such as glufosinate. In addition, markers that facilitate identification of a plant cell containing the polynucleotide encoding the marker include, for example, luciferase (Giacomin, Plant Sci. 116:59-72, 1996; Scikantha, J. Bacteriol. 178:121, 1996), green fluorescent protein (Gerdes, FEBS Lett. 389:44-47, 1996) or fl-glucuronidase (Jefferson, EMBO J. 6:3901-3907, 1997), and numerous others as disclosed herein or otherwise known in the art. Such markers also can be used as reporter molecules.

A heterologous nucleotide sequence can encode an antisense molecule, particularly an antisense molecule specific for a plant stress-regulated nucleotide sequence, for example, the gene from which the regulatory component of the recombinant polynucleotide is derived. Such a recombinant polynucleotide can be useful for reducing the expression of a plant stress-regulated polypeptide in response to a stress condition because the antisense molecule, like the polypeptide, only will be induced upon exposure to the stress. A heterologous nucleotide sequence also can be, or can encode, a ribozyme or a triplexing agent. In addition to being useful as heterologous nucleotide sequences, such molecules also can be used directly in a method of the invention, for example, to modulate the responsiveness of a plant cell to a stress condition. Thus, an antisense molecule, ribozyme, or triplexing agent can be contacted directly with a target cell and, upon uptake by the cell, can effect their antisense, ribozyme or triplexing activity; or can be encoded by a heterologous nucleotide sequence that is expressed in a plant cell from a plant stress-regulated regulatory element, whereupon it can effect its activity.

An antisense polynucleotide, ribozyme or triplexing agent is complementary to a target sequence, which can be a DNA or RNA sequence, for example, messenger RNA, and can be a coding sequence, a nucleotide sequence comprising an intron-exon junction, a regulatory sequence, or the like. The degree of complementarity is such that the polynucleotide, for example, an antisense polynucleotide, can interact specifically with the target sequence in a cell. Depending on the total length of the antisense or other polynucleotide, one or a few mismatches with respect to the target sequence can be tolerated without losing the specificity of the polynucleotide for its target sequence. Thus, few if any mismatches would be tolerated in an antisense molecule consisting, for example, of twenty nucleotides, whereas several mismatches will not affect the hybridization efficiency of an antisense molecule that is complementary, for example, to the full length of a target mRNA encoding a cellular polypeptide. The number of mismatches that can be tolerated can be estimated, for example, using well known formulas for determining hybridization kinetics (see Sambrook et al., "Molecular Cloning; A Laboratory Manual" 2nd Edition (Cold Spring Harbor Laboratory Press, Cold Spring Harbor, NY; 1989) or can be determined empirically using methods as disclosed herein or otherwise known in the art, particularly by determining that the presence of the antisense polynucleotide, ribozyme, or triplexing agent in a cell decreases the level of the target sequence or the expression of a polypeptide encoded by the target sequence in the cell.

A nucleotide sequence useful as an antisense molecule, a ribozyme or a triplexing agent can inhibit translation or cleave a polynucleotide encoded by plant stress-regulated nucleotide sequence, thereby modulating the responsiveness of a plant cell to a stress condition. An antisense molecule, for example, can bind to an mRNA to form a double stranded molecule that cannot be translated in a cell. Antisense oligonucleotides of at least about 15 to 25 nucleotides are typically used since they are easily synthesized and can hybridize specifically with a target sequence, although longer antisense molecules can be expressed from a recombinant polynucleotide introduced into the target cell. Specific nucleotide sequences useful as antisense molecules can be identified using well-known methods, for example, gene walking methods (see, for example, Seimiya et al., J. Biol. Chem. 272:4631-4636, 1997). Where the antisense molecule is contacted directly with a target cell, it can be operatively associated with a chemically reactive group such as iron-linked EDTA, which cleaves a target RNA at the site of hybridization. A triplexing agent, in comparison, can stall transcription (Maher et al., Antisense Res. Devel. 1:227, 1991; Helene, Anticancer Drug Design 6:569, 1991).

A plant stress-regulated regulatory element can be included in an expression cassette. As used herein, the term "expression cassette" refers to a nucleotide sequence that can direct expression of a particular polynucleotide in an appropriate host cells. Expression cassettes typically comprise as operably linked components, a promoter, a nucleotide sequence whose expression is desired and a termination signal. Expression cassettes also often contain sequences necessary for proper translation of the sequence to be expressed along with selection and marker sequences. Thus, a plant stress-regulated regulatory element can constitute an expression cassette, or component thereof. An expression cassette is particularly useful for directing expression of a nucleotide sequence, which can be an endogenous nucleotide sequence or a heterologous nucleotide sequence, in a cell, particularly a plant cell. In general, an expression cassette can be introduced into a plant cell such that the plant cell, a plant resulting from the plant cell, seeds obtained from such a plant, or plants produced from such seeds are resistant to a stress condition.

Additional regulatory sequences as disclosed herein or other desirable sequences such as selectable markers or the like can be incorporated into an expression cassette containing a plant stress-regulated regulatory element (see, for example, WO 99/47552). Examples of suitable markers include dihydrofolate reductase (DHFR) or neomycin resistance for eukaryotic cells and tetracycline or ampicillin resistance for E. coli. Selection markers in plants include bleomycin, gentamycin, glyphosate, hygromycin, kanamycin, methotrexate, phleomycin, phosphinotricin, spectinomycin, dtreptomycin, sulfonamide and sulfonylureas resistance. Maliga et al., Methods in Plant Molecular Biology, Cold Spring Harbor Laboratory Press, 1995, p. 39. The selection marker can have its own promoter or its expression can be driven by the promoter operably linked to the sequence of interest. Additional sequences such as intron sequences (e.g. from Adh1 or bronze1) or viral leader sequences (e.g. from TMV, MCMV and AIVIV), all of which can enhance expression, can be included in the cassette. In addition, where it is desirable to target expression of a nucleotide sequence operatively linked to the stress-regulated regulatory element, a sequence encoding a cellular localization motif can be included in the cassette, for example, such that an encoded transcript or translation product is translocated to and localizes in the cytosol, nucleus, a chloroplast, or another subcellular organelle. Examples of useful transit peptides and transit peptide sequences can be found in Von Heijne et al. Plant Mol. Biol. Rep. 9: 104, 1991; Clark et al. J. Biol. Chem. 264: 17544, 1989; della-Cioppa et al. Plant Physiol. 84: 965, 1987; Romer et al. Biochem. Biophys. Res. Commun. 196: 1414, 1993; and Shah et al., Science 233: 478, 1986; Archer et al., J. Bioenerg Biomembr., 22:789, 1990; Scandalios, Prog. Clin.

Biol. Res, 344:515, 1990; Weisbeek et al., J. Cell Sci. Suppl., 11:199, 1989; Bruce, Trends Cell Biol., 10:440, 2000. The present invention can utilize native or heterologous transit peptides. The encoding sequence for a transit peptide can include all or a portion of the encoding sequence for a particular transit peptide, and may also contain portions of the mature protein encoding sequence associated with a particular transit peptide.

A functional portion of a plant stress-regulated plant polynucleotide, or an expression cassette, can be introduced into a cell as a naked DNA molecule, can be incorporated in a matrix such as a liposome or a particle such as a viral particle, or can be incorporated into a vector. Such vectors can be cloning or expression vectors, but other uses are within the scope of the present invention. A cloning vector is a self-replicating DNA molecule that serves to transfer a DNA segment into a host cell. The three most common types of cloning vectors are bacterial plasmids, phages, and other viruses. An expression vector is a cloning vector designed so that a coding sequence inserted at a particular site will be transcribed and translated into a protein.

Incorporation of the polynucleotide into a vector can facilitate manipulation of the polynucleotide, or introduction of the polynucleotide into a plant cell. A vector can be derived from a plasmid or a viral vector such as a T-DNA vector (Horsch et al., Science 227:1229-1231, 1985). If desired, the vector can comprise components of a plant transposable element, for example, a Ds transposon (Bancroft and Dean, Genetics 134:1221-1229, 1993) or an Spm transposon (Aarts et al., Mol. Gen. Genet. 247:555-564, 1995).

In addition to containing the polynucleotide portion of a plant stress-regulated polynucleotide, a vector can contain various nucleotide sequences that facilitate, for example, rescue of the vector from a transformed plant cell; passage of the vector in a host cell, which can be a plant, animal, bacterial, or insect host cell; or expression of an encoding nucleotide sequence in the vector, including all or a portion of a rescued coding region. As such, the vector can contain any of a number of additional transcription and translation elements, including constitutive and inducible promoters, enhancers, and the like (see, for example, Bitter et al., Meth. Enzymol. 153:516-544, 1987). For example, a vector can contain elements useful for passage, growth or expression in a bacterial system, including a bacterial origin of replication; a promoter, which can be an inducible promoter; and the like. In comparison, a vector that can be passaged in a mammalian host cell system can have a promoter such as a metallothionein promoter, which has characteristics of both a constitutive promoter and an inducible promoter, or a viral promoter such as a retrovirus long terminal repeat, an adenovirus late promoter, or the like. A vector also can contain one or more restriction endonuclease recognition and cleavage sites, including, for example, a polylinker sequence, to facilitate rescue of a nucleotide sequence operably linked to the polynucleotide portion.

The present invention also relates to a method of using a polynucleotide portion of a plant stress-regulated nucleotide sequence to confer a selective advantage on a plant cell. Such a method can be performed by introducing, for example, a plant stress-regulated regulatory element into a plant cell, wherein, upon exposure of the plant cell to a stress condition to which the regulatory element is responsive, a nucleotide sequence operatively linked to the regulatory element is expressed, thereby conferring a selective advantage to plant cell. The operatively linked nucleotide sequence can be a heterologous nucleotide sequence, which can be operatively linked to the regulatory element prior to introduction of the regulatory sequence into the plant cell; or can be an endogenous nucleotide sequence into which the regulatory element was targeted by a method such as homologous recombination. The selective advantage conferred by the operatively linked nucleotide sequence can be such that the plant is better able to tolerate the stress condition; or can be any other selective advantage.

As used herein, the term "selective advantage" refers to the ability of a particular organism to better propagate, develop, grow, survive, or otherwise tolerate a condition as compared to a corresponding reference organism that does not contain a plant-stress regulated polynucleotide of the present invention. In one embodiment, a selective advantage is exemplified by the ability of a desired plant, plant cell, or the like, that contains an introduced plant stress-regulated regulatory element, to grow better than an undesired plant, plant cell, or the like, that does not contain the introduced regulatory element. For example, a recombinant polynucleotide comprising a plant stress-regulated regulatory element operatively linked to a heterologous nucleotide sequence encoding an enzyme that inactivates a herbicide can be introduced in a desired plant. Upon exposure of a mixed population of plants comprising the desired plants, which contain the recombinant polynucleotide, and one or more other populations of undesired plants, which lack the recombinant polynucleotide, to a stress condition that induces expression of the regulatory element and to the herbicide, the desired plants will have a greater likelihood of surviving exposure to the toxin and, therefore, a selective advantage over the undesired plants.

In another embodiment, a selective advantage is exemplified by the ability of a desired plant, plant cell, or the like, to better propagate, develop, grow, survive, or otherwise tolerate a condition as compared to an undesired plant, plant cell, or the like, that contains an introduced plant stress-regulated regulatory element. For example, a recombinant polynucleotide comprising a plant stress-regulated regulatory element operatively linked to a plant cell toxin can be introduced into cells of an undesirable plant present in a mixed population of desired and undesired plants, for example, food crops and weeds, respectively, then the plants can be exposed to stress conditions that induce expression from the plant stress-regulated regulatory element, whereby expression of the plant cell toxin results in inhibition of growth or death of the undesired plants, thereby providing a selective advantage to the desired plants, which no longer have to compete with the undesired plants for nutrients, light, or the like. In another example, a plant stress-regulated regulatory element operatively linked to a plant cell toxin can be introduced into cells of plants used as a nurse crop. Nurse crops, also called cover or companion crops, are planted in combination with plants of interest to provide, among other things, shade and soil stability during establishment of the desired plants. Once the desired plants have become established, the presence of the nurse crop may no longer be desirable. Exposure to conditions inducing expression of the gene linked to the plant stress-regulated regulatory element allows elimination of the nurse crop. Alternatively nurse crops can be made less tolerant to abiotic stress by the inhibition of any of the stress-regulated sequences disclosed herein. Inhibition can be accomplished by any of the method described herein. Upon exposure of the nurse crop to the stress, the decreased ability of the nurse crop to respond to the stress will result in elimination of the nurse crop, leaving only the desired plants.

The invention also provides a means of producing a transgenic plant, which comprises plant cells that exhibit altered responsiveness to a stress condition. As such, the present invention further provides a transgenic plant, or plant cells or tissues derived therefrom, which are genetically modified to respond to stress differently than a corresponding wild-type plant or plant not containing constructs of the present invention would respond. As used herein, the term "responsiveness to a stress condition" refers to the ability of a plant to express a plant stress-regulated polynucleotide upon exposure to the stress condition. A transgenic plant cell contains a functional portion of a plant stress-regulated polynucleotide, or a mutant form thereof, for example, a knock-out mutant. A knock-out mutant form of a plant stress-regulated nucleotide sequence can contain, for example, a mutation such that a STOP codon is introduced into the reading frame of the translated portion of the gene such that expression of a functional stress-regulated polypeptide is prevented; or a mutation in the stress-regulated regulatory element such that inducibility of the element in response to a stress condition is inhibited. Such transgenic plants of the invention can display any of various idiotypic modifications is response to an abiotic stress, including altered tolerance to the stress condition, as well as increased or decreased plant growth, root growth, yield, or the like, as compared to the corresponding wild-type plant.

The term "plant" is used broadly herein to include any plant at any stage of development, or to part of a plant, including a plant cutting, a plant cell, a plant cell culture, a plant organ, a plant seed, and a plantlet. A plant cell is the structural and physiological unit of the plant, comprising a protoplast and a cell wall. A plant cell can be in the form of an isolated single cell or a cultured cell, or can be part of higher organized unit, for example, a plant tissue, plant organ, or plant. Thus, a plant cell can be a protoplast, a gamete producing cell, or a cell or collection of cells that can regenerate into a whole plant. As such, a seed, which comprises multiple plant cells and is capable of regenerating into a whole plant, is considered plant cell for purposes of this disclosure. A plant tissue or plant organ can be a seed, protoplast, callus, or any other groups of plant cells that is organized into a structural or functional unit. Particularly useful parts of a plant include harvestable parts and parts useful for propagation of progeny plants. A harvestable part of a plant can be any useful part of a plant, for example, flowers, pollen, seedlings, tubers, leaves, stems, fruit, seeds, roots, and the like. A part of a plant useful for propagation includes, for example, seeds, fruits, cuttings, seedlings, tubers, rootstocks, and the like.

A transgenic plant can be regenerated from a transformed plant cell. As used herein, the term "regenerate" means growing a whole plant from a plant cell; a group of plant cells; a protoplast; a seed; or a piece of a plant such as a callus or tissue. Regeneration from protoplasts varies from species to species of plants. For example, a suspension of protoplasts can be made and, in certain species, embryo formation can be induced from the protoplast suspension, to the stage of ripening and germination. The culture media generally contains various components necessary for growth and regeneration, including, for example, hormones such as auxins and cytokinins; and amino acids such as glutamic acid and proline, depending on the particular plant species. Efficient regeneration will depend, in part, on the medium, the genotype, and the history of the culture. If these variables are controlled, however, regeneration is reproducible.

Regeneration can occur from plant callus, explants, organs or plant parts. Transformation can be performed in the context of organ or plant part regeneration. (see Meth. Enzymol. Vol. 118; Klee et al. Ann. Rev. Plant Physiol. 38: 467 (1987)). Utilizing the leaf disk-transformation-regeneration method, for example, disks are cultured on selective media, followed by shoot formation in about two to four weeks (see Horsch et al., Science 227:1229, 1985). Shoots that develop are excised from calli and transplanted to appropriate root-inducing selective medium. Rooted plantlets are transplanted to soil as soon as possible after roots appear. The plantlets can be repotted as required, until reaching maturity.

In vegetatively propagated crops, the mature transgenic plants are propagated utilizing cuttings or tissue culture techniques to produce multiple identical plants. Selection of desirable transgenotes is made and new varieties are obtained and propagated vegetatively for commercial use. In seed propagated crops, the mature transgenic plants can be self crossed to produce a homozygous inbred plant. The resulting inbred plant produces seeds that contain the introduced plant stress-induced regulatory element, and can be grown to produce plants that express a polynucleotide or polypeptide in response to a stress condition that induces expression from the regulatory element. As such, the invention further provides seeds produced by a transgenic plant obtained by a method of the invention.

In addition, transgenic plants comprising different recombinant sequences can be crossbred, thereby providing a means to obtain transgenic plants containing two or more different transgenes, each of which contributes a desirable characteristic to the plant. Methods for breeding plants and selecting for crossbred plants having desirable characteristics or other characteristics of interest are well known in the art.

A method of the invention can be performed by introducing a functional portion of a plant stress-regulated nucleotide sequence into the plant. As used herein, the term "introducing" means transferring a polynucleotide into a plant cell. A polynucleotide can be introduced into a cell by a variety of methods well known to those of ordinary skill in the art. For example, the polynucleotide can be introduced into a plant cell using a direct gene transfer method such as electroporation or microprojectile mediated transformation, or using *Agrobacterium* mediated transformation. Non-limiting examples of methods for the introduction of polynucleotides into plants are provided in greater detail herein. As used herein, the term "transformed" refers to a plant cell containing an exogenously introduced polynucleotide portion of a plant stress-regulated nucleotide sequence that is or can be rendered active in a plant cell, or to a plant comprising a plant cell containing such a polynucleotide.

It should be recognized that one or more polynucleotides, which are the same or different can be introduced into a plant, thereby providing a means to obtain a genetically modified plant containing multiple copies of a single transgenic sequence, or containing two or more different transgenic sequences, either or both of which can be present in multiple copies. Such transgenic plants can be produced, for example, by simply selecting plants having multiple copies of a single type of transgenic sequence; by co-transfecting plant cells with two or more populations of different transgenic sequences and identifying those containing the two or more different transgenic sequences; or by crossbreeding transgenic plants, each of which contains one or more desired transgenic sequences, and identifying those progeny having the desired sequences.

Methods for introducing a polynucleotide into a plant cell to obtain a transformed plant also include direct gene transfer (see European Patent A 164 575), injection, electroporation, biolistic methods such as particle bombardment, pollen-mediated transformation, plant RNA virus-mediated transformation, liposome-mediated transformation, transformation using wounded or enzyme-degraded immature embryos, or wounded or enzyme-degraded embryogenic callus, and the like. Transformation methods using *Agrobacterium tumefaciens* tumor inducing (Ti) plasmids or root-inducing (Ri) plasmids, or plant virus vectors are well known in the art (see, for example, WO 99/47552; Weissbach & Weissbach, "Methods for Plant Molecular Biology", Academic Press, 1988), section VIII, pages 421-463; Grierson and Corey, "Plant Molecular Biology" 2d Ed. Blackie, London, 1988), Chapters 7-9; Horsch et al., Science 227:1229, 1985). The wild-type form of *Agrobacterium,* for example, contains a Ti plasmid, which directs production of tumorigenic crown gall growth on host plants. Transfer of the tumor inducing T-DNA region of the Ti plasmid to a plant genome requires the Ti plasmid-encoded virulence genes as well as T-DNA borders, which are a set of direct DNA repeats that delineate the region to be transferred. An *Agrobacterium* based vector is a modified form of a Ti plasmid, in which the tumor inducing functions are replaced by a nucleotide sequence of interest that is to be introduced into the plant host.

Methods of using *Agrobacterium* mediated transformation include cocultivation of *Agrobacterium* with cultured isolated protoplasts; transformation of plant cells or tissues with *Agrobacterium;* and transformation of seeds, apices or meristems with *Agrobacterium.* In addition, *in planta* transformation by *Agrobacterium* can be performed using vacuum infiltration of a suspension of *Agrobacterium* cells (Bechtold et al]., C.R. Acad. Sci. Paris 316:1194, 1993).

*Agrobacterium* mediated transformation can employ cointegrate vectors or binary vector systems, in which the components of the Ti plasmid are divided between a helper vector, which resides permanently in the *Agrobacterium* host and carries the virulence genes, and a shuttle vector, which contains the gene of interest bounded by T-DNA sequences. Binary vectors are well known in the art (see, for example, DeFramond, BioTechnology 1:262, 1983; Hoekema et al., Nature 303:179, 1983) and are commercially available (Clontech; Palo Alto CA). For transformation, *Agrobacterium* can be cocultured, for example, with plant cells or wounded tissue such as leaf tissue, root explants, hypocotyledons, stem pieces or tubers (see, for example, Glick and Thompson, "Methods in Plant Molecular Biology and Biotechnology" , Boca Raton FL, CRC Press, 1993). Wounded cells within the plant tissue that have been infected by *Agrobacterium* can develop organs *de novo* when cultured under the appropriate conditions; the resulting transgenic shoots eventually give rise to transgenic plants, which contain an exogenous polynucleotide portion of a plant stress-regulated nucleotide sequence.

*Agrobacterium* mediated transformation has been used to produce a variety of transgenic plants, including, for example, transgenic cruciferous plants such as *Arabidopsis,* mustard, rapeseed and flax; transgenic leguminous plants such as alfalfa, pea, soybean, trefoil and white clover; and transgenic solanaceous plants such as eggplant, petunia, potato, tobacco and tomato (see, for example, Wang et al., "Transformation of Plants and Soil Microorganisms", Cambridge University Press, 1995). In addition, *Agrobacterium* mediated transformation can be used to introduce an exogenous polynucleotide sequence, for example, a plant stress-regulated regulatory element into apple, aspen, belladonna, black currant, carrot, celery, cotton, cucumber, grape, horseradish, lettuce, morning glory, muskmelon, neem, poplar, strawberry, sugar beet, sunflower, walnut, asparagus, rice and other plants (see, for example, Glick and Thompson, *supra,* 1993; Hiei et al., Plant J. 6:271-282, 1994; Shimamoto, Science 270:1772-1773, 1995).

Suitable strains of *Agrobacterium tumefaciens* and vectors as well as transformation of Agrobacteria and appropriate growth and selection media are well known in the art (GV3101, pMK90RK), Koncz, Mol. Gen. Genet. 204: 383-396, 1986; (C58C1, pGV3850kan), Deblaere, Nucl. Acid Res. 13:4777, 1985; Bevan, Nucleic Acid Res. 12:8711, 1984; Koncz, Proc. Natl. Acad. Sci. USA 86:8467-8471, 1986; Koncz, Plant Mol. Biol. 20:963-976, 1992; Koncz, "Specialized vectors for gene tagging and expression studies", in: Plant Molecular Biology Manual Vol. 2, Gelvin and Schilperoort (Eds.), Dordrecht, The Netherlands: Kluwer Academic Publ. (1994), 1-22; European Patent A-1 20 516; Hoekema, "The Binary Plant Vector System", Offsetdrukkerij Kanters B. V., Alblasserdam (1985), Chapter V; Fraley, Crit. Rev. Plant. Sci., 4:1-46; An, EMBO J. 4:277-287, 1985).

Where a polynucleotide portion of a plant stress-regulated nucleotide sequence is contained in vector, the vector can contain functional elements, for example "left border" and "right border" sequences of the T-DNA of *Agrobacterium,* which allow for stable integration into a plant genome. Furthermore, methods and vectors that permit the generation of marker-free transgenic plants, for example, where a selectable marker gene is lost at a certain stage of plant development or plant breeding, are known, and include, for example, methods of co-transformation (Lyznik, Plant Mol. Biol. 13:151-161, 1989; Peng, Plant Mol. Biol. 27:91- 104, 1995), or methods that utilize enzymes capable of promoting homologous recombination in plants (see, e.g., W097108331; Bayley, Plant Mol. Biol. 18:353-361, 1992; Lloyd, Mol. Gen. Genet. 242:653-657, 1994; Maeser, Mol. Gen. Genet. 230:170-176, 1991; Onouchi, Nucl. Acids Res. 19:6373-6378, 1991; see, also, Sambrook et al., *supra,* 1989).

A direct gene transfer method such as electroporation also can be used to introduce a polynucleotide portion of a plant stress-regulated nucleotide sequence into a cell such as a plant cell. For example, plant protoplasts can be electroporated in the presence of the regulatory element, which can be in a vector (Fromm et al., Proc. Natl. Acad. Sci., USA 82:5824, 1985). Electrical impulses of high field strength reversibly permeabilize membranes allowing the introduction of the nucleic acid. Electroporated plant protoplasts reform the cell wall, divide and form a plant callus. Microinjection can be performed as described in Potrykus and Spangenberg (eds.), Gene Transfer To Plants, Springer Verlag, Berlin, NY, 1995. A transformed plant cell containing the introduced polynucleotide can be identified by detecting a phenotype due to the introduced polynucleotide, for example, increased or decreased tolerance to a stress condition.

Microprojectile mediated transformation also can be used to introduce a polynucleotide into a plant cell (Klein et al., Nature 327:70-73 (1987)). This method utilizes microprojectiles such as gold or tungsten, which are coated with the desired nucleic acid molecule by precipitation with calcium chloride, spermidine or polyethylene glycol. The microprojectile particles are accelerated at high speed into a plant tissue using a device such as the BIOLISTIC PD- 1 000 (Biorad; Hercules CA).

Microprojectile mediated delivery ("particle bombardment") is especially useful to transform plant cells that are difficult to transform or regenerate using other methods. Methods for the transformation using biolistic methods are well known (Wan, Plant Physiol. 104:37-48, 1984; Vasil, Bio/Technology 11:1553-1558, 1993; Christou, Trends in Plant Science 1:423-431, 1996). Microprojectile mediated transformation has been used, for example, to generate a variety of transgenic plant species, including cotton, tobacco, corn, hybrid poplar and papaya (see Glick and Thompson, *supra,* 1993). Important cereal crops such as wheat, oat, barley, sorghum and rice also have been transformed using microprojectile mediated delivery (Duan et al., Nature Biotech. 14:494-498, 1996; Shimamoto, Curr. Opin. Biotech. 5:158-162, 1994). A rapid transformation regeneration system for the production of transgenic plants such as a system that produces transgenic wheat in two to three months (see European Patent No. EP 0709462A2) also can be useful for producing a transgenic plant using a method of the invention, thus allowing more rapid identification of gene functions. The transformation of most dicotyledonous plants is possible with the methods described above. Transformation of monocotyledonous plants also can be transformed using, for example, biolistic methods as described above, protoplast transformation, electroporation of partially permeabilized cells, introduction of DNA using glass fibers, *Agrobacterium* mediated transformation, and the like.

Plastid transformation also can be used to introduce a polynucleotide portion of a plant stress-regulated nucleotide sequence into a plant cell (U.S. Patent Nos. 5,451,513, 5,545,817, and 5,545,818; WO 95/16783; McBride et al., Proc. Natl. Acad. Sci., USA 91:7301-7305, 1994). Chloroplast transformation involves introducing regions of cloned plastid DNA flanking a desired nucleotide sequence, for example, a selectable marker together with polynucleotide of interest into a suitable target tissue, using, for example, a biolistic or protoplast transformation method (e.g., calcium chloride or PEG mediated transformation). One to 1.5 kb flanking regions ("targeting sequences") facilitate homologous recombination with the plastid genome, and allow the replacement or modification of specific regions of the plastome. Using this method, point mutations in the chloroplast 16S rRNA and rps12 genes, which confer resistance to spectinomycin and streptomycin, can be utilized as selectable markers for transformation (Svab et al., Proc. Natl. Acad. Sci., USA 87:8526-8530, 1990; Staub and Maliga, Plant Cell 4:39-45, 1992), resulted in stable homopiasmic transformants; at a frequency of approximately one per 100 bombardments of target leaves. The presence of cloning sites between these markers allowed creation of a plastid targeting vector for introduction of foreign genes (Staub and Maliga, EMBO J. 12:601-606, 1993). Substantial increases in transformation frequency are obtained by replacement of the recessive rRNA or r-protein antibiotic resistance genes with a dominant selectable marker, the bacterial aadA gene encoding the spectinomycin-detoxifying enzyme aminoglycoside-3'-adenyltransf erase (Svab and Maliga, Proc. Natl. Acad. Sci., USA 90:913-917, 1993). Approximately 15 to 20 cell division cycles following transformation are generally required to reach a homoplastidic state. Plastid expression, in which genes are inserted by homologous recombination into all of the several thousand copies of the circular plastid genome present in each plant cell, takes advantage of the enormous copy number advantage over nuclear-expressed genes to permit expression levels that can readily exceed 10% of the total soluble plant protein.

Plants suitable to treatment according to a method of the invention can be monocots or dicots and include, but are not limited to, corn *(Zea mays), Brassica* sp. (e.g., *B. napus, B. rapa, B. juncea),* particularly those *Brassica* species useful as sources of seed oil, alfalfa *(Medicago sativa),* rice *(Oryza sativa),* rye *(Secale cereale),* sorghum *(Sorghum bicolor, Sorghum vulgare),* millet (e.g., pearl millet *(Pennisetum glaucum),* proso millet *(Panicum miliaceum),* foxtail millet *(Setaria italica),* finger millet *(Eleusine coracana)),* sunflower *(Helianthus annuus),* safflower (*Carthamus tinctorius),* wheat *(Triticum aestivum),* soybean *(Glycine max),* tobacco *(Nicotiana tabacum),* potato *(Solanum tuberosum),* peanuts *(Arachis hypogaea),* cotton *(Gossypium barbadense, Gossypium hirsutum),* sweet potato *(Ipomoea batatus),* cassava *(Manihot esculenta),* coffee *(Cofea* spp.), coconut *(Cocos nucifera),* pineapple *(Ananas comosus),* citrus trees *(Citrus* spp.), cocoa *(Theobroma cacao),* tea *(Camellia sinensis),* banana *(Musa* spp.), avocado *(Persea ultilane),* fig *(Ficus casica),* guava *(Psidium guajava),* mango *(Mangifera indica),* olive *(Olea europaea),* papaya *(Carica papaya),* cashew *(Anacardium occidentale),* macadamia *(Macadamia integrifolia),* almond *(Prunus amygdalus),* sugar beets *(Beta vulgaris),* sugarcane *(Saccharum* spp.), oats, duckweed *(Lemna),* barley, tomatoes *(Lycopersicon esculentum),* lettuce (e.g., *Lactuca sativa),* green beans *(Phaseolus vulgaris),* lima beans *(Phaseolus limensis),* peas *(Lathyrus* spp.), and members of the genus *Cucumis* such as cucumber (C. *sativus),* cantaloupe (C. *cantalupensis),* and musk melon (*C. melo).*

Ornamentals such as azalea *(Rhododendron* spp.), hydrangea *(Macrophylla hydrangea),* hibiscus *(Hibiscus rosasanensis),* roses *(Rosa* spp.), tulips *(Tulipa* spp.), daffodils *(Narcissus* spp.), petunias *(Petunia hybrida),* carnation *(Dianthus caryophyllus),* poinsettia *(Euphorbia pulcherrima),* and chrysanthemum are also included. Additional ornamentals within the scope of the invention include impatiens, Begonia, Pelargonium, Viola, Cyclamen, Verbena, Vinca, Tagetes, Primula, Saint Paulia, Agertum, Amaranthus, Antihirrhinum, Aquilegia, Cineraria, Clover, Cosmo, Cowpea, Dahlia, Datura, Delphinium, Gerbera, Gladiolus, Gloxinia, Hippeastrum, Mesembryanthemum, Salpiglossos, and Zinnia.

Conifers that may be employed in practicing the present invention include, for example, pines such as loblolly pine *(Pinus taeda),* slash pine *(Pinus elliotii),* ponderosa pine *(Pinus ponderosa)*, lodgepole pine *(Pinus contorta),* and Monterey pine *(Pinus radiata),* Douglas-fir *(Pseudotsuga menziesii);* Western hemlock *(Tsuga ultilane);* Sitka spruce *(Picea glauca);* redwood *(Sequoia sempervirens);* true firs such as silver fir *(Abies amabilis)* and balsam fir *(Abies balsamea);* and cedars such as Western red cedar *(Thuja plicata)* and Alaska yellow-cedar *(Chamaecyparis nootkatensis).*

Leguminous plants which may be used in the practice of the present invention include beans and peas. Beans include guar, locust bean, fenugreek, soybean, garden beans, cowpea, mungbean, lima bean, fava bean, lentils, chickpea, etc. Legumes include, but are not limited to, *Arachis,* e.g., peanuts, *Vicia,* e.g., crown vetch, hairy vetch, adzuki bean, mung bean, and chickpea, *Lupinus,* e.g., lupine, trifolium, *Phaseolus,* e.g., common bean and lima bean, *Pisum,* e.g., field bean, *Melilotus,* e.g., clover, *Medicago,* e.g., alfalfa, Lotus, e.g., trefoil, lens, e.g., lentil, and false indigo. Forage and turf grass for use in the methods of the invention include alfalfa, orchard grass, tall fescue, perennial ryegrass, creeping bent grass, and redtop. Other plants within the scope of the invention include *Acacia,* aneth, artichoke, arugula, blackberry, canola, cilantro, clementines, escarole, eucalyptus, fennel, grapefruit, honey dew, jicama, kiwifruit, lemon, lime, mushroom, nut, okra, orange, parsley, persimmon, plantain, pomegranate, poplar, radiata pine, radicchio, Southern pine, sweetgum, tangerine, triticale, vine, yams, apple, pear, quince, cherry, apricot, melon, hemp, buckwheat, grape, raspberry, chenopodium, blueberry, nectarine, peach, plum, strawberry, watermelon, eggplant, pepper, cauliflower, Brassica, e.g., broccoli, cabbage, ultilan sprouts, onion, carrot, leek, beet, broad bean, celery, radish, pumpkin, endive, gourd, garlic, snapbean, spinach, squash, turnip, ultilane, chicory, groundnut and zucchini.

Angiosperms are divided into two broad classes based on the number of cotyledons, which are seed leaves that generally store or absorb food; a monocotyledonous angiosperm has a single cotyledon, and a dicotyledonous angiosperm has two cotyledons. Angiosperms produce a variety of useful products including materials such as lumber, rubber, and paper; fibers such as cotton and linen; herbs and medicines such as quinine and vinblastine; ornamental flowers such as roses and orchids; and foodstuffs such as grains, oils, fruits and vegetables.

Angiosperms encompass a variety of flowering plants, including, for example, cereal plants, leguminous plants, oilseed plants, hardwood trees, fruit-bearing plants and ornamental flowers, which general classes are not necessarily exclusive. Cereal plants, which produce an edible grain cereal and are suitable for use in the present invention, include, for example corn, rice, wheat, barley, oat, rye, millet, orchardgrass, guinea grass, sorghum and turfgrass. Leguminous plants include members of the pea family *(Fabaceae)* and produce a characteristic fruit known as a legume. Examples of leguminous plants include, for example, soybean, pea, chickpea, moth bean, broad bean, kidney bean, lima bean, lentil, cowpea, dry bean, and peanut, as well as alfalfa, birdsfoot trefoil, clover and sainfoin. Oilseed plants, which have seeds that are useful as a source of oil, include soybean, sunflower, rapeseed (canola) and cottonseed.

Angiosperms also include hardwood trees, which are perennial woody plants that generally have a single stem (trunk). Examples of such trees include alder, ash, aspen, basswood (linden), beech, birch, cherry, cottonwood, elm, eucalyptus, hickory, locust, maple, oak, persimmon, poplar, sycamore, walnut, sequoia, and willow. Trees are useful, for example, as a source of pulp, paper, structural material and fuel.

Angiosperms are fruit-bearing plants that produce a mature, ripened ovary, which generally contains seeds. A fruit can be suitable for human or animal consumption or for collection of seeds to propagate the species. For example, hops are a member of the mulberry family that are prized for their flavoring in malt liquor. Fruit-bearing angiosperms also include grape, orange, lemon, grapefruit, avocado, date, peach, cherry, olive, plum, coconut, apple and pear trees and blackberry, blueberry, raspberry, strawberry, pineapple, tomato, cucumber and eggplant plants. An ornamental flower is an angiosperm cultivated for its decorative flower. Examples of commercially important ornamental flowers include rose, orchid, lily, tulip and chrysanthemum, snapdragon, camellia, carnation and petunia plants. The skilled artisan will recognize that the methods of the invention can be practiced using these or other angiosperms, as desired, as well as gymnosperms, which do not produce seeds in a fruit.

A method of producing a transgenic plant can be performed by introducing a functional portion of plant stress-regulated polynucleotide into a plant cell genome, whereby the functional portion of the plant stress-regulated polynucleotide modulates a response of the plant cell to a stress condition, thereby producing a transgenic plant, which comprises plant cells that exhibit altered responsiveness to the stress condition. In one embodiment, the functional portion of the plant stress-regulated polynucleotide encodes a stress-regulated polypeptide or functional peptide portion thereof, wherein expression of the stress-regulated polypeptide or functional peptide portion thereof either increases the stress tolerance of the transgenic plant, or decreases the stress tolerance of the transgenic plant. The functional portion of the plant stress-regulated nucleotide sequence encoding the stress-regulated polypeptide or functional peptide portion thereof can be operatively linked to a heterologous promoter.

In another embodiment, the polynucleotide portion of the plant stress-regulated nucleotide sequence comprises a stress-regulated regulatory element. The stress-regulated regulatory element can integrate into the plant cell genome in a site-specific manner, whereupon it can be operatively linked to an endogenous nucleotide sequence, which can be expressed in response to a stress condition specific for the regulatory element; or can be a mutant regulatory element, which is not responsive to the stress condition, whereby upon integrating into the plant cell genome, the mutant regulatory element disrupts an endogenous stress-regulated regulatory element of a plant stress-regulated nucleotide sequence, thereby altering the responsiveness of the plant stress-regulated nucleotide sequence to the stress condition. Accordingly, the invention also provides genetically modified plants, including transgenic plants, produced by such a method, and a plant cell obtained from such genetically modified plant, wherein said plant cell exhibits altered responsiveness to the stress condition; a seed produced by a transgenic plant; and a cDNA library prepared from a transgenic plant.

Also provided is a method of modulating the responsiveness of a plant cell to a stress condition. Such a method can be performed, for example, by introducing a functional portion of a plant stress-regulated nucleotide sequence into the plant cell, thereby modulating the responsiveness of the plant cell to a stress condition. As disclosed herein, the responsiveness of the plant cell can be increased or decreased upon exposure to the stress condition, and the altered responsiveness can result in increased or decreased tolerance of the plant cell to a stress condition. The functional portion of the plant stress-regulated polynucleotide can, but need not, be integrated into the genome of the plant cell, thereby modulating the responsiveness of the plant cell to the stress condition. Accordingly, the invention also provide a genetically modified plant, including a transgenic plant, which contains an introduced polynucleotide portion of a plant stress-regulated nucleotide sequence, as well as plant cells, tissues, and the like, which exhibit modulated responsiveness to a stress condition.

The functional portion of the plant stress-regulated polynucleotide can encode a stress-regulated polypeptide or functional peptide portion thereof, which can be operatively linked to a heterologous promoter. As used herein, reference to a "functional peptide portion of a plant stress-regulated polypeptide" means a contiguous amino acid sequence of the polypeptide that has at least 50%, at least 75%, at least 90%, or at least 95% the activity of the full length polypeptide, or that has an antagonist activity with respect to the full length polypeptide, or that presents an epitope unique to the polypeptide. Thus, by expressing a functional peptide portion of a plant stress-regulated polypeptide in a plant cell, the peptide can act as an agonist or an antagonist of the polypeptide, thereby modulating the responsiveness of the plant cell to a stress condition. It should be noted that while the functional peptide portion has an activity of the full length polypeptide, the activity need not be of the same magnitude. Thus, the functional peptide portion can have an activity with is greater or lesser in magnitude than the full length polypeptide.

A functional portion of the plant stress-regulated polynucleotide also can contain a mutation, whereby upon integrating into the plant cell genome, the polynucleotide disrupts (knocks-out) an endogenous plant stress-regulated nucleotide sequence, thereby modulating the responsiveness of said plant cell to the stress condition. Depending on whether the knocked-out gene encodes an adaptive or a maladaptive stress-regulated polypeptide, the responsiveness of the plant will be modulated accordingly. Thus, a method of the invention provides a means of producing a transgenic plant having a knock-out phenotype of a plant stress-regulated nucleotide sequence.

Alternatively, the responsiveness of a plant or plant cell to a stress condition can be modulated by use of a suppressor construct containing dominant negative mutation for any of the stress-regulated polynucleotides described herein. Expression of a suppressor construct containing a dominant mutant mutation generates a mutant transcript that, when coexpressed with the wild-type transcript inhibits the action of the wild-type transcript. Methods for the design and use of dominant negative constructs are well known in the art and can be found, for example, in Herskowitz, Nature, 329:219-222. 1987 and Lagna and Hemmati-Brivanlou, Curr. Topics Devel. Biol., 36:75-98, 1998.

The functional portion of the plant stress-regulated polynucleotide sequence also can comprise a stress-regulated regulatory element, which can be operatively linked to a heterologous nucleotide sequence, which, upon expression from the regulatory element in response to a stress condition, modulates the responsiveness of the plant cell to the stress condition. Such a heterologous nucleotide sequence can encode, for example, a stress-inducible transcription factor such as DREB1A, which, upon exposure to the stress condition, is expressed such that it can amplify the stress response (see Kasuga et al., Nat. Biotechnol., 17:287-291, 1999). The heterologous nucleotide sequence also can encode a polynucleotide that is specific for a plant stress-regulated nucleotide sequence, for example, an antisense molecule, a ribozyme, and a triplexing agent, either of which, upon expression in the plant cell, reduces or inhibits expression of a stress-regulated polypeptide encoded by the gene, thereby modulating the responsiveness of the plant cell to a stress condition, for example, an abnormal level of cold, osmotic pressure, and salinity. As used herein, the term "abnormal," when used in reference to a condition such as temperature, osmotic pressure, salinity, or any other condition that can be a stress condition, means that the condition varies sufficiently from a range generally considered optimum for growth of a plant that the condition results in an induction of a stress response in a plant. Methods of determining whether a stress response has been induced in a plant are disclosed herein or otherwise known in the art.

A plant stress-regulated regulatory element can be operatively linked to a heterologous polynucleotide sequence, such that the regulatory element can be introduced into a plant genome in a site-specific matter by homologous recombination. For example, a mutant plant stress-regulated regulatory element for a maladaptive stress-induced polypeptide can be transformed into a plant genome in a site specific manner by *in* vivo mutagenesis, using a hybrid RNA-DNA oligonucleotide ("chimeroplast" (TIBTECH 15:441- 447, 1997; W0 95/15972; Kren, Hepatology 25:1462-1468, 1997; Cole-Strauss, Science 273:1386-1389, 1996). Part of the DNA component of the RNA-DNA oligonucleotide is homologous to a nucleotide sequence comprising the regulatory element of the maladaptive gene, but includes a mutation or contains a heterologous region which is surrounded by the homologous regions. By means of base pairing of the homologous regions of the RNA-DNA oligonucleotide and of the endogenous nucleic acid molecule, followed by a homologous recombination the mutation contained in the DNA component of the RNA-DNA oligonucleotide or the heterologous region can be transferred to the plant genome, resulting in a "mutant" gene that, for example, is not induced in response to a stress and, therefore, does not confer the maladaptive phenotype. Such a method similarly can be used to knock-out the activity of a stress-regulated nucleotide sequence, for example, in an undesirable plant. Such a method can provide the advantage that a desirable wild-type plant need not compete with the undesirable plant, for example, for light, nutrients, or the like.

A method of modulating the responsiveness of a plant cell to a stress condition also can be performed by introducing a mutation in the chromosomal copy of a plant stress-regulated nucleotide sequence, for example, in the stress-regulated regulatory element, by transforming a cell with a chimeric oligonucleotide composed of a contiguous stretch of RNA and DNA residues in a duplex conformation with double hairpin caps on the ends. An additional feature of the oligonucleotide is the presence of 2'-0- methylation at the RNA residues. The RNA/DNA sequence is designed to align with the sequence of a chromosomal copy of the target regulatory element and to contain the desired nucleotide change (see U.S. Patent No. 5,501,967).

A plant stress-regulated regulatory element also can be operatively linked to a heterologous polynucleotide such that, upon expression from the regulatory element in the plant cell, confers a desirable phenotype on the plant cell. For example, the heterologous polynucleotide can encode an aptamer, which can bind to a stress-induced polypeptide. Aptamers are nucleic acid molecules that are selected based on their ability to bind to and inhibit the activity of a protein or metabolite. Aptamers can be obtained by the SELEX (Systematic Evolution of Ligands by Exponential Enrichment) method (see U.S. Patent No. 5,270,163), wherein a candidate mixture of single stranded nucleic acids having regions of randomized sequence is contacted with a target, and those nucleic acids having a specific affinity to the target are partitioned from the remainder of the candidate mixture, and amplified to yield a ligand enriched mixture. After several iterations a nucleic acid molecule (aptamer) having optimal affinity for the target is obtained. For example, such a nucleic acid molecule can be operatively linked to a plant stress-regulated regulatory element and introduced into a plant. Where the aptamer is selected for binding to a polypeptide that normally is expressed from the regulatory element and is involved in an adaptive response of the plant to a stress, the recombinant molecule comprising the aptamer can be useful for inhibiting the activity of the stress-regulated polypeptide, thereby decreasing the tolerance of the plant to the stress condition.

The invention provides a genetically modified plant, which can be a transgenic plant, that is tolerant or resistant to a stress condition. As used herein, the term "tolerant" or "resistant," when used in reference to a stress condition of a plant, means that the particular plant, when exposed to a stress condition, shows less of an effect, or no effect, in response to the condition as compared to a corresponding reference plant (naturally occurring wild-type plant or a plant not containing a construct of the present invention). As a consequence, a plant encompassed within the present invention grows better under more widely varying conditions, has higher yields and/or produces more seeds. Thus, a transgenic plant produced according to a method of the invention can demonstrate protection (as compared to a corresponding reference plant) from a delay to complete inhibition of alteration in cellular metabolism, or reduced cell growth or cell death caused by the stress. Preferably, the transgenic plant is capable of substantially normal growth under environmental conditions where the corresponding reference plant shows reduced growth, metabolism or viability, or increased male or female sterility.

The determination that a plant modified according to a method of the invention has increased resistance to a stress-inducing condition can be made by comparing the treated plant with a control (reference) plant using well known methods. For example, a plant having increased tolerance to saline stress can be identified by growing the plant on a medium such as soil, which contains a higher content of salt in the order of at least about 10% compared to a medium the corresponding reference plant is capable of growing on. Advantageously, a plant treated according to a method of the invention can grow on a medium or soil containing at least about 50%, or more than about 75%, or more than about 100%, or more than about 200% salt than the medium or soil on which a corresponding reference plant can grow. In particular, such a treated plant can grow on medium or soil containing at least 40 mM, at least 100 mM, at least 200 mM, or at least 300 mM salt, including, for example, a water soluble inorganic salt such as sodium sulfate, magnesium sulfate, calcium sulfate, sodium chloride, magnesium chloride, calcium chloride, potassium chloride, or the like; salts of agricultural fertilizers, and salts associated with alkaline or acid soil conditions; particularly NaCl.

In another embodiment, the invention provides a plant that is less tolerant or less resistant to a stress condition as compared to a corresponding reference plant. As used herein, the term "less tolerant" or "less resistant," when used in reference to a stress condition of a plant, means that the particular plant, when exposed to a stress condition, shows an alteration in response to the condition as compared to a corresponding reference plant. In one embodiment, the alteration is response is at least 5%, in another at least 10% and in still another at least 25% when compared to the reference plant. As a consequence, such a plant, which generally is an undesirable plant species, is less likely to grow when exposed to a stress condition than an untreated plant.

The present invention also relates to a method of expressing a heterologous nucleotide sequence in a plant cell. Such a method can be performed, for example, by introducing into the plant cell a plant stress-regulated regulatory element operatively linked to the heterologous nucleotide sequence, whereby, upon exposure of the plant cell to stress condition, the heterologous nucleotide sequence is expressed in the plant cell. The heterologous nucleotide sequence can encode a selectable marker, or a polypeptide that confers a desirable trait upon the plant cell, for example, a polypeptide that improves the nutritional value, digestibility or ornamental value of the plant cell, or a plant comprising the plant cell. Accordingly, the invention provides a transgenic plant that, in response to a stress condition, can produce a heterologous polypeptide from a plant stress-regulated regulatory element. Such transgenic plants can provide the advantage that, when grown in a cold environment for example, expression of the heterologous polypeptide from a plant cold-regulated regulatory element can result in increased nutritional value of the plant.

The present invention further relates to a method of modulating the activity of a biological pathway in a plant cell, wherein the pathway involves a stress-regulated polypeptide. As used herein, reference to a pathway that "involves" a stress-regulated polypeptide means that the polypeptide is required for normal function of the pathway. For example, plant stress-regulated polypeptides as disclosed herein include those acting as kinases or as transcription factors, which are well known to be involved in signal transduction pathways. As such, a method of the invention provides a means to modulate biological pathways involving plant stress-regulated polypeptides, for example, by altering the expression of the polypeptides in response to a stress condition. Thus, a method of the invention can be performed, for example, by introducing a polynucleotide portion of a plant stress-regulated nucleotide sequence into the plant cell, thereby modulating the activity of the biological pathway. A method of the invention can be performed with respect to a pathway involving any of the stress-regulated polypeptides as encoded by a polynucleotide of disclosed herein, including for example, a stress-regulated transcription factor, an enzyme, including a kinase, a channel protein, or the like.

The present invention also relates to a method of identifying a polynucleotide that modulates a stress response in a plant cell. Such a method can be performed, for example, by contacting an array of probes representative of a plant cell genome and nucleic acid molecules expressed in plant cell exposed to the stress; detecting a nucleic acid molecule that is expressed at a level different from a level of expression in the absence of the stress; introducing the nucleic acid molecule that is expressed differently into a plant cell; and detecting a modulated response of the plant cell containing the introduced nucleic acid molecule to a stress, thereby identifying a polynucleotide that modulates a stress response in a plant cell. The contacting is under conditions that allow for specific hybridization of a nucleic acid molecule with probe having sufficient complementarity, for example, under stringent or highly stringent, hybridization conditions.

As used herein, the term "array of probes representative of a plant cell genome" means an organized group of oligonucleotide probes that are linked to a solid support, for example, a microchip or a glass slide, wherein the probes can hybridize specifically and selectively to nucleic acid molecules expressed in a plant cell. Such an array is exemplified herein by a GeneChip® Arabidopsis Genome Array (Affymetrix; see Examples). In general, an array of probes that is "representative" of a plant genome will identify at least about 30% of the expressed nucleic acid molecules in a plant cell, at least about 50% or 70%, at least about 80% or 90%, or will identify all of the expressed nucleic acid molecules. It should be recognized that the greater the representation, the more likely all nucleotide sequences of cluster of stress-regulated nucleotide sequences will be identified.

In addition, any polynucleotide of the present disclosure can be used for diagnostic purposes or to find related stress-responsive sequences in other species. Any polynucleotide provided herein may be attached in overlapping areas or at random locations on the solid support. Alternatively the polynucleotides of the invention may be attached in an ordered array wherein each polynucleotide is attached to a distinct region of the solid support that does not overlap with the attachment site of any other polynucleotide. In one instance, such an ordered array of polynucleotides is designed to be "addressable" where the distinct locations are recorded and can be accessed as part of an assay procedure. Addressable polynucleotide arrays typically include a plurality of different oligonucleotide probes that are coupled to a surface of a substrate in different known locations. The knowledge of the precise location of each polynucleotides location makes these "addressable" arrays particularly useful in hybridization assays. Any addressable array technology known in the art can be employed with the polynucleotides of the invention. One particular embodiment of these polynucleotide arrays is known as the Genechips™, and has been generally described in US Patent 5,143,854 and PCT publications WO 90/15070 and 92/10092. These arrays may generally be produced using mechanical synthesis methods or light directed synthesis methods that incorporate a combination of photolithographic methods and solid phase oligonucleotide synthesis. The immobilization of arrays of oligonucleotides on solid supports has been rendered possible by the development of a technology generally identified as "Very Large Scale Immobilized Polymer Synthesis" (VLSIPS™) in which, typically, probes are immobilized in a high density array on a solid surface of a chip. Examples of VLSIPS™ technologies are provided in US Patents 5,143,854 and 5,412,087 and in PCT Publications WO 90/15070, WO 92/10092 and WO 95/11995, which describe methods for forming oligonucleotide arrays through techniques such as light-directed synthesis techniques. Further presentation strategies aimed at providing arrays of nucleotides immobilized on solid supports were developed to order and display the oligonucleotide arrays on the chips in an attempt to maximize hybridization patterns and sequence information as disclosed in PCT Publications WO 94/12305, WO 94/11530, WO 97/29212 and WO 97/31256.

In another embodiment, an oligonucleotide probe matrix may advantageously be used to detect mutations occurring in a polynucleotide disclosed hereiin. For this particular purpose, probes are specifically designed to have a nucleotide sequence allowing their hybridization to the genes that carry known mutations (either by deletion, insertion or substitution of one or several nucleotides). By "known mutations" it is meant, mutations of a polynucleotide including any of those disclosed herein, that have been identified using techniques known in the art.

Another technique that is used to detect mutations in a polynucleotide including any stress-responsive sequence disclosed herein is the use of a high-density DNA array, where single base mutations are encompassed by this technique. Each oligonucleotide probe constituting a unit element of the high density DNA array is designed to match a specific subsequence of the genomic DNA or cDNA of interest. Thus, an array containing oligonucleotides complementary to subsequences of the target gene sequence is used to determine the identity of the target sequence with the "wild-type" nucleotide sequence, measure its amount, and detect differences between the target sequence and the reference wild-type nucleotide sequence. One such design termed a "4L tiled array", is implemented using a set of four probes (A, C, G, T), for example 15-nucleotide oligomers. In each set of four probes, the perfect complement will hybridize more strongly than mismatched probes. Consequently, a nucleotide target of length L is scanned for mutations with a tiled array containing 4L probes; the whole probe set containing all the possible mutations in the known wild reference sequence. The hybridization signals of the 15-mer probe set tiled array are perturbed by a single base change in the target sequence. As a consequence, there is a characteristic loss of signal or a "footprint" for the probes flanking a mutation position. This technique was described by Chee et al. (Science 274:610, 1996).

Polynucleotides identified herein include those nucleotide sequences that are induced or repressed in response to a combination of stress conditions, but not to any of the stress conditions alone; and polynucleotides that are induced or repressed in response to a selected stress condition, but not to other stress conditions. Furthermore, polynucleotides whose response to a stress condition is temporally regulated are also included, such as polynucleotides that are induced early, late or continuously in a stress response. In addition, the polynucleotides are represented by a variety of cellular proteins, including transcription factors, enzymes such as kinases, channel proteins, and the like.

The present invention additionally relates to a method of identifying a stress condition to which a plant cell was exposed. Such a method can be performed, for example, by contacting nucleic acid molecules expressed in the plant cell with an array of probes representative of the plant cell genome; and detecting a profile of expressed nucleic acid molecules characteristic of a stress response, thereby identifying the stress condition to which the plant cell was exposed. The contacting generally is under conditions that allow for specific hybridization of a nucleic acid molecule with probes having sufficient complementarity, for example, under stringent or highly stringent hybridization conditions. The profile can be characteristic of exposure to a single stress condition, for example, an abnormal level of cold, osmotic pressure, or salinity, or can be characteristic of exposure to more than one stress condition, for example, cold, increased osmotic pressure and increased salinity.

The polynucleotides for which expression is determined and so the probes used may be varied depending on the particular plant and/or stress involved. In one embodiment, the plant is a cereal and expression is determined for at least one polynucleotide selected from the group consisting of those sequences disclosed herein. In another embodiment, the plant is a rice plant. It will be apparent to those of skill in the art, that though the use of various technologies, for example microarrays, it is possible and in many cases desirable to determine the expression of multiple stress-regulated polynucleotides at once. Thus, the preceding various embodiments include the identification of a stress condition to which a plant was exposed in which expression data is obtained on at least 10, at least 25, at least 50, at least 100, at least 250, at least 500, or at least 750 of the various groups of polynucleotide sequences described above.

In one embodiment, the expression profile is produced by isolating RNA, for example mRNA from the test plant. Methods for the isolation of RNA from plants are well known in the art and can be found in standard reference texts such as those cited herein. In one embodiment, the RNA is transformed into cDNA by the use of reverse transcriptase using protocols that are well known to those skilled in the art of molecular biology. The RNA or cDNA is then hybridized to probes to the stress-regulated polynucleotides described herein under stringent, high stringency, or very high stringency conditions and hybridization detected. It is envisioned that multiple probes will be used for each polynucleotide expressed and that expression of multiple polynucleotides will be determined as detailed above.

The method can be used to determine exposure to any stress to which results in altered expression of the described polynucleotide sequences. In one embodiment the stress is a single or combination abiotic stress such as cold stress, saline stress, osmotic stress or any combination thereof. In one embodiment, the expression profile from the test plant is also compared to a control plant of the same species, for example an isogenic plant, that has not been exposed to a stress.

In one embodiment of the invention, nucleic acid samples from the plant cells to be collected can be contacted with an array, then the profile can be compared with known profiles prepared from nucleic acid samples of plants exposed to known stresses. By creating a panel of such profiles, representative of various stress conditions, an unknown stress condition to which a plant was exposed can be identified simply by comparing the unknown profile with the known profiles and determining which known profile that matches the unknown profile. In one embodiment, the comparison is automated. Such a method can be useful, for example, to identify a cause of damage to a crop, where the condition causing the stress is not known or gradually increases over time. For example, accumulation in soils over time of salts from irrigation water can result in gradually decreasing crop yields. Because the accumulation is gradual, the cause of the decreased yield may not be readily apparent. Using the present methods, it is possible to evaluate the stress to which the plants are exposed, thus revealing the cause of the decreased yields.

The present invention, therefore includes a computer readable medium containing executable instructions form receiving expression data for sequences substantially similar to any of those disclosed herein and comparing expression data from a test plant to a reference plant that has been exposed to an abiotic stress. Also provided is a computer-readable medium containing sequence data for sequences substantially similar to any of the sequences described herein, or the complements thereof, and a module for comparing such sequences to other nucleic acid sequences.

Also provided are plants and plant cells comprising plant stress-regulatory elements of the present invention operably linked to a nucleotide sequence encoding a detectable signal. Such plants can be used as diagnostic or "sentinel plants" to provide early warning that nearby plants are being stressed so that appropriate actions can be taken. In one embodiment, the signal is one that alters the appearance of the plant. For example, an osmotic stress regulatory element of the present invention can be operably linked to a nucleotide sequence encoding a fluorescent protein such as green fluorescent protein, a yellow fluorescent protein, a cyan fluorescent protein, or a red fluorescent protein. When subjected to osmotic stress, the expression of the green fluorescent protein in the sentinel plant provides a visible signal so that appropriate actions can be taken to remove or alleviate the stress. The use of fluorescent proteins in plants is known in the art and can be found, for example, in Leffel et al., Biotechniques 23:912, 1997.

The invention further relates to a method of identifying an agent that modulates the activity of a stress-regulated regulatory element of a plant. As used herein, the term "modulate the activity," when used in reference to a plant stress-regulated regulatory element, means that expression of nucleotide sequence from the regulatory element is increased or decreased. In particular, expression can be increased or decreased with respect to the basal activity of the promoter, i.e., the level of expression, if any, in the absence of a stress condition that normally induces expression from the regulatory element; or can be increased or decreased with respect to the level of expression in the presence of the inducing stress condition. As such, an agent can act as a mimic of a stress condition, or can act to modulate the response to a stress condition.

Such a method can be performed, for example, by contacting the regulatory element with an agent suspected of having the ability to modulate the activity of the regulatory element, and detecting a change in the activity of the regulatory element. In one embodiment, the regulatory element can be operatively linked to a heterologous polynucleotide encoding a reporter molecule, and an agent that modulates the activity of the stress-regulated regulatory element can be identified by detecting a change in expression of the reporter molecule due to contacting the regulatory element with the agent. Such a method can be performed *in vitro* in a plant cell-free system, or in a plant cell in culture or in a plant *in situ.*

A method of the invention also can be performed by contacting the agent with a genetically modified cell or a transgenic plant containing an introduced plant stress-regulated regulatory element, and an agent that modulates the activity of the regulatory element is identified by detecting a phenotypic change in the modified cell or transgenic plant.

A method of the invention can be performed in the presence or absence of the stress condition to which the particularly regulatory element is responsive. As such, the method can identify an agent that modulates the activity of plant stress-regulated promoter in response to the stress, for example, an agent that can enhance the stress response or can reduce the stress response. In particular, a method of the invention can identify an agent that selectively activates the stress-regulated regulatory elements of a cluster of plant stress-regulated nucleotide sequences, but does not affect the activity of other stress-regulated regulatory olynucleotides. As such, the method provides a means to identify an agent that acts as a stress mimic. Such agents can be particularly useful to prepare a plant to an expected stress condition. For example, a agent that acts as a cold mimic can be applied to a field of plants prior to the arrival of an expected cold front. Thus, the cold stress response can be induced prior to the actual cold weather, thereby providing the plants with the protection of the stress response, without the plants suffering from any initial damage due to the cold. Similarly, an osmotic pressure mimic can be applied to a crop of plants prior a field being flooded by a rising river.

In one embodiment, the present invention provides a method for marker-assisted selection. Marker-assisted selection is a well-known method in the art and involves the selection of plant having desirable phenotypes based on the presence of particular nucleotide sequences known as markers. The use of makers allows plants to be selected early in development, often before the phenotype would normally manifest itself. Because it allows for early selection, marker-assisted selection decreases the amount of time need for selection and thus allows more rapid genetic progress. Briefly, marker-assisted selection involves obtaining nucleic acid from a plant to be selected. The nucleic acid obtained is then probed with probes that selectively hybridize under stringent or highly stringent, conditions to a nucleotide sequence or sequences associated with the desired phenotype. In one embodiment, the probes hybridize to any of the stress-responsive nucleotide sequences or regulatory elements disclosed herein. The presence of any hybridization products formed is detected and plants are then selected on the presence or absence of the hybridization products.

The isolated polynucleotides of the invention can be used to create various types of genetic and physical maps of the genome of rice or other plants. Such maps are used to devise positional cloning strategies for isolating novel genes from the mapped crop species. The sequences of the present invention are also useful for chromosome mapping, chromosome identification, tagging genes of known and useful function, tagging genes to which a function has not yet been assigned, and including the uses set forth in U.S. Patent No. 5,817,479.

In addition, because the genomes of closely related species are largely syntenic (that is, they display the same ordering of genes within the genome), these maps can be used to isolate novel alleles from wild relatives of crop species by positional cloning strategies. This shared synteny is very powerful for using genetic maps from one species to map genes in another. For example, a gene mapped in rice provides information for the gene location in maize and wheat.

In one embodiment, the stress responsive sequences of the present invention are located in and can be used to identify Quantitative Trait Loci (QTLs) for a variety of uses, including marker-assisted breeding. Many important crop traits are quantitative traits and result from the combined interactions of several genes. These genes reside at different loci in the genome, often on different chromosomes, and generally exhibit multiple alleles at each locus. Developing markers, tools, and methods to identify and isolate the QTLs involved in a trait, enables marker-assisted breeding to enhance desirable traits or suppress undesirable traits. The sequences of the invention can be used to identify QTLs and isolate alleles as described by Li et al. in a study of QTLs involved in resistance to a pathogen of rice. (Li et al., Mol Gen Genet, 261:58, 1999).

In particular SEQ ID Nos. listed in Table 1 have been mapped to a drought resistance OTL located on chromosome 8 of rice (Zhang et al., Thero. Appl. Genet., 103:19-29, 2000). This OTL is syntenic with drought resistance OTLs located on wheat chromosome 7S and barley chromosome 1. In addition to supporting the role of these sequences in drought resistance, these data show that support a role for these sequences in drought resistance for a variety of cereals. Likewise, additional SEQ ID Nos. listed in Table 1 are located in the drought resistance QTL on chromosome 3 of rice (Zhang et al., Thero. Appl. Genet., 103:19-29, 2000). This QTL is syntenic with a drought resistance QTL located on maize choromosome 1 again, supporting the role of the polynucletotides in drought resistance in a variety of species.

In addition to isolating QTL alleles in rice, other cereals, and other monocot and dicot crop species, the sequences of the invention can also be used to isolate alleles from the corresponding QTL(s) of wild relatives. Transgenic plants having various combinations of QTL alleles can then be created and the effects of the combinations measured. Once an ideal allele combination has been identified, crop improvement can be accomplished either through biotechnological means or by directed conventional breeding programs. (Flowers et al., J Exp Bot, 51:99, 2000; Tanksley and McCouch, Science, 277:1063, 1997).

Polynucleotides derived from sequences of the present invention are useful to detect the presence in a test sample of at least one copy of a nucleotide sequence containing the same or substantially the same sequence, or a fragment, complement, or variant thereof. The sequence of the probes and/or primers of the instant invention need not be identical to those provided in the Sequence Listing or the complements thereof. Some variation in probe or primer sequence and/or length can allow additional family members to be detected, as well as orthologous genes and more taxonomically distant related sequences. Similarly probes and/or primers of the invention can include additional nucleotides that serve as a label for detecting duplexes, for isolation of duplexed polynucleotides, or for cloning purposes.

Probes and primers of the invention include isolated, purified, or recombinant polynucleotides containing a contiguous span of between at least 12 to at least 1000 nucleotides of any of sequences disclosed herein or the complements thereof, with each individual number of nucleotides within this range also being part of the invention. Examples are isolated, purified, or recombinant polynucleotides containing a contiguous span of at least 12, 15, 18, 20, 25, 30, 35, 40, 50, 60, 70, 80, 90, 100, 150, 200, 300, 400, 500, 750, or 1000 nucleotides of any of the sequences disclosed herein or the complements thereof. The appropriate length for primers and probes will vary depending on the application. For use as PCR primers, probes are 12-40 nucleotides, typically 18-30 nucleotides long. For use in mapping, probes are 50 to 500 nucleotides, typically 100-250 nucleotides long. For use in Southern hybridizations, probes as long as several kilobases can be used. The appropriate length for primers and probes under a particular set of assay conditions may be empirically determined by one of skill in the art.

The primers and probes can be prepared by any suitable method, including, for example, cloning and restriction of appropriate sequences and direct chemical synthesis by a method such as the phosphodiester method of Narang et al. (Meth Enzymol, 68: 90, 1979), the diethylphosphoramidite method, the triester method of Matteucci et al. (J Am Chem Soc, 103: 3185, 1981), or according to Urdea et al. (Proc Natl Acad Sci. USA, 80: 7461, 1981), the solid support method described in EP 0 707 592, or using commercially available automated oligonucleotide synthesizers.

Detection probes are generally nucleotide sequences or uncharged nucleotide analogs such as, for example peptide nucleotides which are disclosed in International Patent Application WO 92/20702, morpholino analogs which are described in U.S. Patent Nos. 5,185,444, 5,034,506 and 5,142,047. The probe may have to be rendered "non-extendable" such that additional dNTPs cannot be added to the probe. Analogs are usually non-extendable, and nucleotide probes can be rendered non-extendable by modifying the 3' end of the probe such that the hydroxyl group is no longer capable of participating in elongation. For example, the 3' end of the probe can be functionalized with the capture or detection label to thereby consume or otherwise block the hydroxyl group. Alternatively, the 3' hydroxyl group simply can be cleaved, replaced or modified so as to render the probe non-extendable.

Any of the polynucleotides of the present invention can be labeled, if desired, by incorporating a label detectable by spectroscopic, photochemical, biochemical, immunochemical, or chemical means. For example, useful labels include radioactive substances ($^{32}$P, $^{35}$S, $^{3}$H, $^{125}$I), fluorescent dyes (5-bromodesoxyuridine, fluorescein, acetylaminofluorene, digoxigenin) or biotin. In one embodiment, polynucleotides are labeled at their 3' and 5' ends. Examples of non-radioactive labeling of nucleotide fragments are described in the French patent No. FR-7810975 and by Urdea et al. (Nuc Acids Res, 16:4937, 1988). In addition, the probes according to the present invention may have structural characteristics such that they allow the signal amplification, such structural characteristics being, for example, branched DNA probes as described in EP 0 225 807.

A label can also be used to capture the primer so as to facilitate the immobilization of either the primer or a primer extension product, such as amplified DNA, on a solid support. A capture label is attached to the primers or probes and can be a specific binding member that forms a binding pair with the solid's phase reagent's specific binding member, for example biotin and streptavidin. Therefore depending upon the type of label carried by a polynucleotide or a probe, it may be employed to capture or to detect the target DNA. Further, it will be understood that the polynucleotides, primers or probes provided herein, may, themselves, serve as the capture label. For example, in the case where a solid phase reagent's binding member is a nucleotide sequence, it may be selected such that it binds a complementary portion of a primer or probe to thereby immobilize the primer or probe to the solid phase. In cases where a polynucleotide probe itself serves as the binding member, those skilled in the art will recognize that the probe will contain a sequence or "tail" that is not complementary to the target. In the case where a polynucleotide primer itself serves as the capture label, at least a portion of the primer will be free to hybridize with a nucleotide on a solid phase. DNA labeling techniques are well known in the art.

Any of the polynucleotides, primers and probes of the present invention can be conveniently immobilized on a solid support. Solid supports are known to those skilled in the art and include the walls of wells of a reaction tray, test tubes, polystyrene beads, magnetic beads, nitrocellulose strips, membranes, microparticles such as latex particles, sheep (or other animal) red blood cells, duracytes and others. The solid support is not critical and can be selected by one skilled in the art. Thus, latex particles, microparticles, magnetic or non-magnetic beads, membranes, plastic tubes, walls of microtiter wells, glass or silicon chips, sheep (or other suitable animal's) red blood cells and duracytes are all suitable examples. Suitable methods for immobilizing nucleotides on solid phases include ionic, hydrophobic, covalent interactions and the like. A solid support, as used herein, refers to any material that is insoluble, or can be made insoluble by a subsequent reaction. The solid support can be chosen for its intrinsic ability to attract and immobilize the capture reagent. Alternatively, the solid phase can retain an additional receptor that has the ability to attract and immobilize the capture reagent. The additional receptor can include a charged substance that is oppositely charged with respect to the capture reagent itself or to a charged substance conjugated to the capture reagent. As yet another alternative, the receptor molecule can be any specific binding member which is immobilized upon (attached to) the solid support and which has the ability to immobilize the capture reagent through a specific binding reaction. The receptor molecule enables the indirect binding of the capture reagent to a solid support material before the performance of the assay or during the performance of the assay. The solid phase thus can be a plastic, derivatized plastic, magnetic or non-magnetic metal, glass or silicon surface of a test tube, microtiter well, sheet, bead, microparticle, chip, sheep (or other suitable animal's) red blood cells, duracytes and other configurations known to those of ordinary skill in the art. The polynucleotides of the invention can be attached to or immobilized on a solid support individually or in groups of at least 2, 5, 8, 10, 12, 15, 20, or 25 distinct polynucleotides of the invention to a single solid support. In addition, polynucleotides other than those of the invention may be attached to the same solid support as one or more polynucleotides of the invention.

Probes and primers of the invention can be used to identify and/or isolate polynucleotides related to the stress responsive sequences provided in the Sequence Listing, or allelic variants of the stress responsive sequences. Generally, related polynucleotides have similar sequences or encode polypeptides with similar biological activity, but are found at other loci within an organism, or are found in other organisms. Identification and isolation of related sequences can provide important tools for functional genomics, to study the evolution of genomes, and to predict gene and protein function, interaction, and regulation. Related sequences including paralogs and orthologs are particularly important in identifying Clusters of Orthologous Groups (COGs) of proteins, which aids protein function prediction and the functional and phylogenetic annotation of newly sequenced genomes.

Hybridization of the stress-responsive sequences of the invention to nucleotides obtained from other organisms can be used to identify and isolate paralogous sequences, or paralogs, which are additional members of gene families. The terms "paralogous sequence" and "paralog" as used herein encompass both full-length genes and regions and fragments thereof. Paralogs may be located in the same or a different region of the genome in which the sequence used as a probe is located. Paralogs generally have a high sequence identity with the probe sequence or the gene from which the probe was prepared; however, paralogs may have overall sequence identity with a probe sequence as low as 20 to 30% and still be recognizable as members of the same gene family with similar functions, as reported by Takata et al. for RAD51B paralogs (Mol Cell Biol, 20:6476, 2000). When overall sequence identity is not high, the sequence similarity among paralogs of a gene family is often concentrated into one or a few portions of the sequence, notably in a portion encoding a protein or RNA that has an enzymatic or structural function. The degree of identity in the amino acid sequence of the domain that defines the gene family can be as low as 20%, but is often at least 50%, at least 75%, at least 80 to 95%, or at least 85 to 99%. Paralogs may differ in their expression profiles, indicating that they may act at different time, a different place, or at a different developmental stage, even when their function appears to be similar. Differences in function among paralogs may suggest that paralogs encode polypeptides that are "remodeled" during plant evolution, for example to create new forms of oxidized carotenoids in tomato as described by Bouvier et al. (Eur J Biochem, 267: 6346, 2000).

In one embodiment, paralogs may be isolated by hybridizing a stress-resonsive sequence probe to a Southern blot containing the appropriate genomic DNA or cDNA of the organism. To search for paralogs within a species, low stringency hybridization is usually performed, but will depend on size, distribution and degree of sequence divergence of domains that define the gene family. Given the resulting hybridization data, one or ordinary skill in the art could distinguish and isolate the correct DNA fragments by size, restriction sites and stated hybridization conditions from a gel or from a library. Alternately, paralogs may be isolated by large-scale sequencing followed by BLAST analysis of sequences to identify putative paralogs, as described by Ospina-Giraldo et al,. (Fungal Genet Biol, 29:81, 2000). In another embodiment, paralogs may be isolated using reverse-transcriptase polymerase chain reaction (RT-PCR) using primers to conserved regions of sequence. Paralogs may be cloned using standard techniques to screen libraries using at least one stress-responsive sequence as a probe.

The stress-responsive sequences disclosed herein can also be used to determine orthologous sequences. An orthologous sequence, or orthologous gene, or ortholog, has a high degree of sequence similarity to a known sequence or gene of interest, with the similarity often occurring along the entire length of the coding portion of the gene. The terms "orthologous sequence" and "ortholog" as used herein encompass both full-length genes and functional regions and fragments thereof. An ortholog often encodes a gene product that performs a similar function in the organism. Functions for orthologous genes are expected to be the same as or very similar to that of the gene from which the probe was prepared. The degree of identity is a function of evolutionary separation and, in closely related species, the degree of sequence identity can be 98 to 100%. Orthologous sequences sometimes have significantly lower levels of sequence identity, for example as described by Weise et al. (Plant Cell, 12:1345, 2000) where orthologs of sucrose transporters from *Arabidopsis,* tomato, and potato had 47% similarity to the previously characterized sucrose transporter. The amino acid sequence of a protein encoded by an orthologous gene can be less than 50% identical, but tends to be at least 50%, or at least 70% or at least 80% identical, or at least 90%, or at least 95% identical to the amino acid sequence of the reference protein.

To find orthologs, probes are hybridized to nucleotides from a species of interest under low stringency conditions and blots are then washed under conditions of increasing stringency. It is preferable that the wash stringency be such that sequences that are 85 to 100% identical will hybridize. More preferably, sequences 90 to 100% identical will hybridize and most preferably only sequences greater than 95% identical will hybridize. The low stringency condition is preferably one where sequences containing as much as 40-45% mismatches will be able to hybridize. This condition is established by $T_m$- 40°C to $T_m$-48°C. One of ordinary skill in the art will recognize that, due to degeneracy in the genetic code, amino acid sequences that are identical can be encoded by DNA sequences as little as 67% identical. Thus, it is preferable to make an overlapping series of shorter probes, on the order of 24 to 45 nucleotides, and individually hybridize them to the same arrayed library to avoid the problem of degeneracy introducing large numbers of mismatches.

In one embodiment, orthologous sequences, or orthologs, may be isolated by hybridizing an stress-responsive sequence probe to a Southern blot containing the appropriate genomic DNA or cDNA of a different organism, for example, another cereal. Alternately, orthologs may be isolated by large-scale sequencing followed by BLAST analysis of sequences to identify putative orthologous sequences and full-length orthologs. In another embodiment, orthologs may be isolated using reverse-transcriptase polymerase chain reaction (RT-PCR) using primers to conserved regions of sequence in a stress responsive sequence. Orthologs and/or orthologous sequences may be cloned using standard techniques to screen libraries using at least one stress-responsive sequence as a probe.

As evolutionary divergence increases, genome sequences also tend to diverge. Thus, one of skill will recognize that searches for orthologous genes between more divergent species will require the use of lower stringency conditions compared to searches between closely related species. Also, degeneracy is more of a problem for searches in the genome of a species more distant evolutionarily from the species that is the source of the stress-responsive probe sequences.

Identification of the relationship of nucleotide or amino acid sequences among plant species can be done by comparison of the subject nucleotide or amino acid sequence with the sequences of the present application presented in the Sequence Listing.

Sequences disclosed herein can also be used to isolate corresponding DNA by Southern blotting. Probes for Southern blotting to distinguish individual restriction fragments can range in size from 15 to 20 nucleotides to several thousand nucleotides. Typically, the probe is 100 to 1000 nucleotides long for identifying members of a gene family when it is found that repetitive sequences would complicate the hybridization. For identifying an entire corresponding gene in another species, the probe is more often the length of the gene, typically 2000 to 10,000 nucleotides, but probes 50-1,000 nucleotides long might be used. Some genes, however, might require probes up to 15,000 nucleotides long or overlapping probes constituting the full-length sequence to span their lengths.

In one embodiment, the probe derived from sequences of the present invention is homogeneous, having a single sequence. In another embodiment, a probe designed to represent or identify members of a gene family having diverse sequences can be generated using PCR to amplify genomic DNA or RNA templates using primers derived from stress-responsive sequences that include sequences that define the gene family.

For identifying corresponding genes in another species, the probe for Southern blotting most preferably would be the genomic copy of the probe gene. This allows all elements of the gene to be identified in the other species. The next most preferable probe is a cDNA spanning the entire coding sequence which allows the entire mRNA-coding portion of the gene to be identified; in this case it is possible that some introns in the gene might be missed. Probes for Southern blotting can easily be generated from stress-responsive sequences by making primers having the sequence at the ends of the sequence and using rice *(Oryza sativa)* genomic DNA as a template. In instances where the sequence includes sequence conserved among species, primers including the conserved sequence can be used for PCR with genomic DNA from a species of interest to obtain a probe. Similarly, if the sequence includes a domain of interest, that portion of the sequence can be used to make primers and, with appropriate template DNA, used to make a probe to identify genes containing the domain. Alternatively, the PCR products can be resolved, for example by gel electrophoresis, and cloned and/or sequenced. In this manner, the variants of the domain among members of a gene family, both within and across species, can be examined.

The sequences of the invention can be used for library screening to isolate the corresponding DNA from the same organism or other organisms. Either cDNA or genomic DNA can be isolated. Libraries of genomic DNA, or lambda, cosmid, BAC or YAC, or other large insert genomic library from the plant of interest can be constructed using standard molecular biology techniques as described in detail by Sambrook et al., (Molecular Cloning: A Laboratory Manual, Cold Spring Harbor Laboratory Press, 1989) and by Ausubel et al. (Current Protocols in Molecular Biology, Greene Publishing, 1992) with updates).

To screen a phage library, recombinant lambda clones are plated out on appropriate bacterial medium using an appropriate E. *coli* host strain. The resulting plaques are lifted from the plates using nylon or nitrocellulose filters. The plaque lifts are processed through denaturation, neutralization, and washing treatments following the standard protocols outlined by Ausubel et al. (1992 *supra).* The plaque lifts are hybridized to either radioactively labeled or non-radioactively labeled stress responsive DNA at room temperature for about 16 hours, usually in the presence of 50% formamide and 5X SSC (sodium chloride and sodium citrate) buffer and blocking reagents. The plaque lifts are then washed at 42°C with 1% sodium dodecyl sulfate (SDS) and at a particular concentration of SSC. The SSC concentration used is dependent upon the stringency at which hybridization occurred in the initial Southern blot analysis performed. For example, if a fragment hybridized under medium stringency such as $T_m$ - 20°C, then this condition is maintained or adjusted to a less stringent condition such as $T_m$ - 30°C, to wash the plaque lifts. Positive clones showing hybridization to the probe are detected by exposure to X-ray films or chromogen formation or any other suitable detection method, and subsequently isolated for purification using the same general protocol outlined above. Once the clone is purified, restriction analysis can be conducted to narrow the region corresponding to the gene of interest. Restriction analysis and succeeding subcloning steps can be done using procedures described by, for example, Sambrook et al. (1989, *supra).*

To screen a YAC library, the procedures outlined for the lambda library are essentially similar except the YAC clones are harbored in bacterial colonies. The YAC clones are plated out at reasonable density on nitrocellulose or nylon filters supported by appropriate bacterial medium in petri plates. Following the growth of the bacterial clones, the filters are processed through the denaturation, neutralization, and washing steps following the procedures of Ausubel et al. (1992, *supra).* The same hybridization procedures for lambda library screening are followed.

To isolate cDNA, similar procedures using appropriately modified vectors are employed. For instance, the library can be constructed in a lambda vector appropriate for cloning cDNA such as λgt11. Alternatively, the cDNA library can be made in a plasmid vector. cDNA for cloning can be prepared by any of the methods known in the art, but is preferably prepared as described above. Preferably, a cDNA library will include a high proportion of full-length clones.

Identification and isolation of alleles and paralogs within a species, and orthologs from other species, is particularly desirable because of their potential use as a tool for crop improvement especially for quantitative traits such as resistance to abiotic stress. By identifying and isolating numerous alleles for each locus from a single species or from different species, transgenic plants having various combinations of alleles can be created and the effects of the combinations measured. Once a more favorable ideal allele combination has been identified, crop improvement can be accomplished either through biotechnological means or by traditional (conventional) breeding programs. (Tanksley et al., Science 277:1063, 1997). In a similar manner, substitution of at least one paralogous or orthologous sequence in at least one locus will introduce diversity at each substituted locus, and the substituted sequences will contribute to a trait that is influenced by combined interactions of the products of several genes residing at different loci in the genome. When favorable combinations of substituted sequences and endogenous sequences at loci whose products interact are identified, crop improvement can be accomplished through further biotechnological manipulation or by traditional breeding programs, including sexual crossing and also apomixis.

The results from hybridization of the sequences of the invention to Southern blots containing DNA from another species can be used to generate restriction fragment maps for the corresponding genomic regions. These maps provide additional information about the relative positions of restriction sites within fragments, further distinguishing mapped DNA from the remainder of the genome. Physical maps can be made by digesting genomic DNA with different combinations of restriction enzymes.

Sequence analysis and mapping of related sequences (paralogs and orthologs) can be used in phylogenetic analyses of the evolution of the sequences in question, including the determination of gene duplication and rearrangements. In addition, expression studies of related sequences can be used to further understand the evolutionary history and function of the paralogs and orthologs, and to suggest future uses for the sequences.

Isolated polynucleotides within the scope of the invention also include allelic variants of the specific sequences presented in the sequence listing. An "allelic variant" is a sequence that is a variant from that of the stress-responsive sequence, but represents the same chromosomal locus in the organism. Allelic variants can arise by normal genetic variation in a population. Allelic variants can also be produced by genetic engineering methods, for example by the method of chimeraplasty using chimeric oligonucleotides to introduce a single nucleotide base substitution in a target sequence, as described by Beetham et al. (Proc. Natl. Acad. Sci. USA, 96:8774, 1999) and Zhu et al., (Proc. Natl. Acad. Sci., USA, 96:8768, 1999). An allelic variant can be one that is found naturally occurring in a plant, including a cultivar or ecotype. An allele can give rise to detectably distinct phenotypic and expression profiles. An allelic variant may or may not give rise to a phenotypic change, and may or may not be expressed. An expressed allele can result in a detectable change in the phenotype of the trait represented by the locus. Allelic variations can occur in any portion of the gene sequence, including regulatory regions as well as structural regions. The stress responsive sequences of the present invention are useful to detect and/or isolate allelic variants, and may be used to introduce an allelic variant at a locus. Thus, present sequences can be used to manipulate the allelic diversity of a plant or a population.

With respect to nucleotide sequences, degeneracy of the genetic code provides the possibility to substitute at least one base of the base sequence of a gene with a different base without causing the amino acid sequence of the polypeptide produced from the gene to be changed. Hence, the sequences of the present invention may also have any base sequence that has been changed from a sequence as provided in the Sequence Listing by substitution in accordance with degeneracy of genetic code. References describing codon usage include: Carels et al., J Mol Evol 46:45, 1998 and Fennoy et al., Nucl Acids Res, 21:5294, 1993.

One embodiment of the invention comprises stress-responsive polypeptides containing a universal stress protein A (USPA) domain and polynucleotides encoding said polypeptides. The gene encoding proteins containing the USPA domain was originally found in E. *coli* (Nystrom and Neidhardt, Mol. Microbiol., 6:3187-3198, 1992). The *uspA* gene is unique in its almost universal responsiveness to diverse stresses. Rice stress responsive polypeptides containing a USPA domain are listed in Table 1.

**Examples**

The following examples are intended to provide illustrations of the application of the present invention. The following examples are not intended to completely define or otherwise limit the scope of the invention.

***Example 1****: Isolation and sequencing of DNA fragments*

*1.1 Isolation and sequencing of genomic DNA fragments*

Genomic DNA was isolated from nuclei of *Oryza sativa* L. ssp *japonica* cv Nipponbare and then sheared to produce fragments of approximately 500 bp. Using a method derived from the method of Mao et al. (Genome Res 10: 982 (2000)), seeds were germinated on cheese cloth immersed in water and grown for 4-6 weeks under greenhouse conditions. After plants reached a height of approximately 5-8 inches, the upper parts of the green leaves were harvested and wrapped in aluminum foil at 4°C overnight. Leaf material was then stored at -80°C or directly used for extraction of nuclei. Intact nuclei were isolated by homogenization (in a blender for fresh material or by grinding with mortar and pestle for frozen material) in a buffer containing 10 mM Trizma base, 80 mM KCl, 10 mM EDTA, I mM spermidine, 1 mM spermine, 0.5 M sucrose, 0.5% Triton-X-100, 0.15% β-mercaptoethanol pH 9.5. The homogenate was filtered and nuclei recovered by gentle centrifugation using a fixed-angle rotor at 1,800 g at 4°C for 20 minutes. The pellet recovered after centrifugation was gently resuspended with the assistance of a small paint brush soaked in ice cold wash buffer and wash buffer added. Particulate matter remaining in the suspension was removed by filtering the resuspended nuclei into a 50 ml centrifuge tube through two layers of miracloth by gravity and centrifuging the filtrate at 57 g (500 rpm), 4C for 2 minutes to remove intact cells and tissue residues. The supernatant fluid was transferred into a fresh centrifuge tube and nuclei were pelleted by centrifugation at 1,800 g, 4C for 15 minutes in a swinging bucket centrifuge.

DNA was isolated from the nuclear preparation by phenol/chloroform extraction, as in Sambrook *et al* (supra). Isolated total genomic DNA was physically sheared (Hydroshear) to generate for generating random DNA fragments, and fragments of approximately 500 bp were recovered. DNA was eluted and the ends filled in using $T_4$ DNA polymerase, Klenow fragments, and dNTPs. Double-stranded DNA was linkered and cloned into a proprietary medium-copy vector derived from pSC101.

Vector inserts were amplified by PCR and sequenced using the MegaBACE sequencing system (Molecular Dynamics, Amersham). The amplification reaction was diluted before use and was not purified using an exonuclease/ alkaline phosphatase procedure. Sequencing reactions were performed using DYEnamic ET Terminator Kit. The reactions contained approximately 50 ng of amplicon, DYEnamic ET Terminator premix, and 5 pmol of -40 M13 forward primer. The sequencing reaction is amplified for 30 cycles, and reaction products are concentrated and purified using ethanol precipitation. The sample was electrokinetically injected into the capillary at 3 kV for 45 sec and separated via electrophoresis at 9 kV for 120 min.

*1.2 Isolation and sequencing of cDNA fragments*

*Construction of rice cDNA library.* Total RNA was purified from rice plant tissue using standard total RNA purification methods. PolyA+ RNA was isolated from the total RNA using the Qiagen Oligotex mRNA purification system (Qiagen, Valencia, CA), and cDNA was generated using cDNA synthesis reagents from Life Technologies (Rockville, MD). First strand cDNA synthesis was catalyzed by reverse transcriptase using oligo dT primers with a NotI restriction site. Second strand synthesis was catalyzed by DNA polymerase. An oligonucleotide linker with a SalI restriction endonuclease site was attached to the 5' end of the cDNAs using DNA ligase. The cDNAs were digested with NotI and SalI restriction endonucleases and inserted into an E. coli-replicating plasmid harboring a selectable marker. *E. coli* was transfected with the recombinant plasmids and grown on selectable media. E. *coli* colonies were individually picked off the selectable media and placed into storage plates.

*Sequencing the rice cDNA library,* The DNA sequence of the cDNA cloned into the plasmid purified from an *E. coli* colony was determined using standard dideoxy sequencing methods. Oligonucleotide primers respectively corresponding to plasmid DNA regions upstream of the 5' end of the cDNA insert (Forward reaction) and downstream of the 3' end of the cDNA insert (Reverse reaction) were used in the dideoxy sequencing reactions. If the DNA sequence determined as a result of the Forward and Reverse reactions from the cDNA overlapped, the two sequences could be merged into a contig using computerized analysis software (Consed, University of Washington,Seattle), to assemble a full-length sequence of the cDNA. In cases where DNA sequence from the Forward and Reverse reactions from a single clone did not overlap sufficiently to be assembled into a contig, such that there was a region of unsequenced DNA to bridge the DNA from the Forward and Reverse reaction in order to form a contig, the DNA sequence of the separating region was determined using one of two dideoxy sequencing methods. In a "primer walking" approach, a primer specifically corresponding to the 3' end of the DNA sequence determined from the Forward reaction was used in a second dedeoxy sequencing reaction. The primer walking procedure was repeated until the DNA sequence that separated the original Forward and Reverse was resolved and a contig could be assembled. Alternatively, the clone harboring the cDNA was subjected to transposon in vitro insertion dideoxysequencing (Epicentre, Madison, WI). In this procedure, the insertion process was random and the result was multiple DNA sequence coverage over the targeted cDNA, where the sequences thus obtained were assembled into a contig.

**Example 2:** *GeneChip®Standard Protocol*

The standard protocol for using the GeneChip® to quantitatively measure plant gene expression was carried out as outlined below:

Quantitation of total RNA

1. Total RNA from plant tissue was extracted and quantified.Quantified total RNA using GeneQuant I $OD_{260}$=40 mg RNA/ml; A26dA280=1 -9 to about 2.1
2. Ran gel to check the integrity and purity of the extracted RNA

Synthesis of double-stranded cDNA

Gibco/BRL Superscript Choice System for cDNA Synthesis (Cat#1B090-019) was employed to prepare cDNAs. T7-$(dT)_{24}$ oligonucleotides were prepared and purified by HPLC. (5'- GGCCAGTGAATTGTAATACGACTCACTATAG-GGAGGCGG-$(dT)_{24}$-3'; SEQ ID NO: 17507).

Step 1. Primer hybridization:

Incubated at 70°C for 10 minutes
Spun quickly and put on ice briefly

Step 2. Temperature adjustment:

Incubated at 42°C for 2 minutes

Step 3. First strand synthesis carried out using,

DEPC-water- 1 :1
RNA (10 :g final)-10 :1
T7-$(dT)_{24}$ Primer (100 pmol final)-1 :1 pmol
5X 1$^{st}$ strand cDNA buffer-4 :1
0.1 M DTT (10 mM final)- 2 :1
10 mM dNTP mix (500 :M final)-1 :1
Superscript II RT 200 U/:1- 1 :1
Total of 20 :1

Mixed well
Incubated at 42°C for 1 hour
Step 4. Second strand synthesis:

Placed reactions on ice, quick spin

DEPC-water- 91 :1
5X 2nd strand cDNA buffer- 30 :1
10 mM dNTP mix (250 mM final) - 3 :1
*E. coli* DNA ligase (10 U/:l)-1 :1 :1
*E. coli DNA* polymerase 1-10 U/:1- 4 :1
RnaseH 2U/:1-1 :1
T4 DNA polymerase 5 U/:1-2 :1
0.5 M EDTA (0.5 M final)-10 :1
Total 162 :1

Mixed/spun down/incubated 16°C for 2 hours
Step 5. Completing the reaction:
Incubated at 16°C for 5 minutes

Purification of double stranded cDNA

1. Centrifuged PLG (Phase Lock Gel, Eppendorf 5 Prime Inc., pI-188233) at 14,000X, transfered 162 :1 of cDNA to PLG
2. Added 162 :1 of Phenol:Chloroform:Isoamyl alcohol (pH 8.0), centrifuge 2 minutes
3. Transfered the supernatant to a fresh 1.5 ml tube, add

| | |
|---|---|
| Glycogen (5 mglml) | 2 |
| 0.5 M NH$_4$OAC (0.75xVol) | 120 |
| ETOH (2.5xVol, -20°C) | 400 |

4. Mixed well and centrifuge at 14,000X for 20 minutes
5. Removed supernatant, added 0.5 ml 80% EtOH (-20°C)
6. Centrifuged for 5 minutes, air dry or by speed vac for 5-10 minutes
7. Added 44 :1 DEPC H$_2$O
Analyzed quantity and size distribution of cDNA
Ran a gel using 1:1 ratio of the double-stranded synthesis product to loading buffer

Synthesis of biotinylated cRNA

(used Enzo BioArray High Yield RNA Transcript Labeling Kit Cat#900182)

| | |
|---|---|
| Purified cDNA | 22 :1 |
| 10X Hy buffer | 4 :1 |
| 10X biotin ribonucleotides | 4 :1 |
| 10XDTT | 4 :1 |
| 10X Rnase inhibitor mix | 4 :1 |
| 20X T7 RNA polymerase | 2 :1 |
| Total | 40 :1 |

Centrifuged 5 seconds, and incubated for 4 hours at 37°C
Gently mixed every 30-45 minutes

Purification and quantification of cRNA

(used Qiagen Rneasy Mini kit Cat# 74103)

| | | |
|---|---|---|
| cRNA | 40 :1 | |
| DEPC H$_2$O | 60 :1 | |
| RLT buffer | 350 :1 | mix by vortexing |
| EtOH | 250 :1 | mix by pipetting |

(continued)

Total     700 :1

Waited 1 minute or more for the RNA to stick
Centrifuged at 2000 rpm for 5 minutes

RPE buffer    500:1

Centrifuged at 10,000 rpm for 1 minute

RPE buffer    500 :1

Centrifuged at 10,000 rpm for 1 minute
Centrifuged at 10,000 rpm for 1 minute to dry the column

DEPC $H_2O$    30 :1

Waited for 1 minute, then elute cRNA from by centrifugation, 10K 1 minute

DEPC $H_2O$    30 :1

Repeated previous step
Determined concentration and dilute to 1 :g/:l concentration
Fragmentation of cRNA

| cRNA (1 :g/:l) | 15 :1 |
| 5X Fragmentation Buffer* | 6 :1 |
| DEPC $H_2O$ | 9 :1 |
| | 30 :1 |

| *5x Fragmentation Buffer | |
| --- | --- |
| 1M Tris (pH8.1) | 4.0 ml |
| MgOAc | 0.64 g |
| KOAC | 0.98 g |
| DEPC $H_2$0 | |
| Total | 20 ml |
| Filter Sterilize | |

Array washed and stained in:

Stringent Wash Buffer**
Non-Stringent Wash Buffer***
SAPE Stain****
Antibody Stain*****

Washed on fluidics station using the appropriate antibody amplification protocol

**Stringent Buffer: 12X MES 83.3 ml, 5 M NaCl 5.2 ml, 10% Tween 1.0 ml, $H_2O$ 910 ml,
Filter Sterilize
***Non-Stringent Buffer: 20X SSPE 300 ml, 10% Tween 1.0 ml, $H_2O$ 698 ml,

Filter Sterilize, Antifoam 1.0.
****SAPE stain: 2X Stain Buffer 600 :1, BSA 48 :1, SAPE 12:1, $H_2O$ 540 :1.
*****Antibody Stain: 2X Stain Buffer 300 :1, $H_2O$ 266.4 :1, BSA 24 :1, Goat IgG 6 :1, Biotinylated Ab 3.6 :1

***Example 3:*** *Profiling of plant stress-regulated genes*

A GeneChip® Rice Genome Array (Affymetrix, Santa Clara, CA) was used to identify clusters of genes that were coordinately induced in response to various stress conditions. The GeneChip® Rice Genome Array contains probes synthesized *in situ* and is designed to measure temporal and spatial gene expression of approximately 18,000 genes which covers approximately 40-50% of the genome..

The Affymetrix GeneChip® array was used to define nucleotide sequences/ pathways affected by various abiotic stresses and to define which are uniquely regulated by one stress and those that respond to multiple stress, and to identify candidate nucleotide sequences for screening for insertional mutants. Of the approximately 18,000 nucleotide sequences represented on the Affymetrix GeneChip® array, certain nucleotide sequences showed at least a 2-fold change in expression in at least one sample, relative to no-treatment controls.

The following describes in more detail how the experiments were done. Transcriptional profiling was performed by hybridizing fluorescence labeled cRNA with the oligonucleotides probes on the chip, washing, and scanning. Each gene is represented on the chip by about sixteen oligonucleotides (25-mers). Expression level is related to fluorescence intensity. Starting material contained 1 to 10 μg total RNA; detection specificity was about 1:10$^6$; approximately a 2-fold change was detectable, with less than 2% false positive; the dynamic range was approximately 500x. Nucleotide sequences having up to 70% to 80% identity could be discriminated using this system.

*3.1 Growth conditions*

Rice plants were grown for 6 weeks in convirons in plastic pots filled with sand. The conditions of the conviron are 12 h/12h light/dark, 25°C, ~50% RH and light intensity at 300 μEi. The plants were fertilized three times per week with one-half-strength Hoagland Solution containing 25 μM $KH_2PO_4$.

*3.2 Abiotic stress treatment*

Six weeks after placing the rice plants in convirons, stresses were applied as follows:

- *Control* - no treatment;
- *Drought* = 25% PolyEthyleneGlycol (PEG) 8000 (PEG is a more controllable method for creating a water-deficit, the osmotic pressure from PEG will mimic the water-deficit experienced during drought)
  *Osmotic Stress* = 260.0 mM Mannitol (equivalent osmolarity of a 150.0 mM NaCl solution)
  *NaCl* = 150.0 mM
  *Cold* = 14°C (the temperature at which pollen mother cell development is affected)

The abiotic stress treatments was applied at time 0 and then at the same time of day on subsequent days (ie. Time 0, 24, 48 and 72 hours).

*3.3 Tissue sampling*

After the onset of treatment, 3 time points were harvested, namely, 3hr, 27 hr and 75 hr. Leaves and roots were harvested separately and the tissue flash-frozen in liquid nitrogen. These time points are set to be the exact same time of day at all 3 time points to eliminate the effects of circadian rhythms in gene expression. RNA was purified, and the samples were analyzed using the GeneChip® Rice Genome Array (Affymetrix, Santa Clara, CA) following the manufacturer's protocol.

*3.4. Data analysis*

Raw fluorescence values as generated by Affymetrix software were processed as follows: the values were brought into Microsoft Excel® and values of 25 or less were set to 25 (an empirically determined baseline as disclosed in Zhu and Wang, Plant Physiol. 124:1472-1476; 2000). The values from the stressed samples were then converted to fold change relative to control by dividing the values from the stressed samples by the values from the no-treatment control samples. Expression patterns that were altered at least 2-fold with respect to the control were selected. This method gave very robust results and resulted in a larger number of nucleotide sequences called as stress-regulated than previous methods had permitted.

Based on the profiles obtained following hybridization of nucleic acid molecules obtained from plant cells exposed to various stress conditions to the probes in the microarray, clusters of nucleotide sequences that were altered at least two-fold in response to the stress conditions were identified.

**Example 4** *Identification of Abiotic Stress Responsive Genes by Yeast Two Hybrid System*

An automated, high-throughput yeast two hybrid assay technology provided by Myriad Genetics Inc., (Salt Lake City, UT) was used to search for protein interactions with a bait protein known to be inducible by chilling in rice.

Multiple prtein fragments that encode recognizable motifs, or domains, were constructed as baits from the ORF encoding the protein to be studied. A screening protocol, which uses Myriad's proprietary strains and vectors, was then used to search the individual baits against two activation domain libraries of greater than five million cDNA clones of assorted peptide motifs. The libraries were derived from RNA isolated from leaves, stems and roots of rice plants grown in normal conditions plus tissues form plants exposed to various stresses (input trait library) and from various seed stages, callus, and early and late panicle (output trait library). Both hybrid proteins were expressed in a yeast reporter strain where an interaction between the test proteins results in transcription of the reporter genes *TRP1* and *LEU2*, allowing growth on selective medium lacking tryptophan and leucine. Positives obtained from these searches were isolated and their identity was determined by sequence analysis against proprietary and public nucleic acid and protein databases.

To further characterize the polynucleotides encoding interacting proteins, the sequences of the baits and preys were compared with the gene fragments represented on a proprietary GeneChip® Rice Genome Array (Affymetrix, Santa Clara, CA) and where a polynucleotide was identitified on the chip, its expression was experimentally determined. Experiments included evaluating differential gene expression from various plant tissues comprising seed, root, leaf and stem, panicle, and pollen.

Using these methods, an ORF (SEQ ID NO: 17505) was identified encoding a protein that interacted with the chilling inducible bait. This ORF is contained in a full-length cDNA (SEQ ID NO: 17506) which also includes 258 bp of 5' UTR and 423 bp of 3'UTR.

**Example 5:** *Rice Orthologs of Arabidopsis Abiotic stress Genes Identified by Reverse Genetics*

Understanding the function of every gene is the major challenge in the age of completely sequenced eukaryotic genomes. Sequence homology can be helpful in identifying possible functions of many genes. However, reverse genetics, the process of identifying the function of a gene by obtaining and studying the phenotype of an individual containing a mutation in that gene, is another approach to identify the function of a gene.

Reverse genetics in *Arabidopsis* has been aided by the establishment of large publicly available collections of insertion mutants (Krysan et a]., Plant Cell, 11:2283-2290, 1999; Tisser et al., Plant Cell, 11:1841-1852, 1999; Speulman et al., Plant Cell 11:1853-1866, 1999; Parinov et al., Plant Cell, 11:2263-2270, 1999; Parinov and Sundaresan, Biotechnology, 11:157-161, 2000). Mutations in genes of interest are identified by screening the population by PCR amplification using primers derived from sequences near the insert border and the gene of interest to screen through large pools of individuals. Pools producing PCR products are confirmed by Southern hybridization and further deconvoluted into subpools until the individual is identified (Sussman et al., Plant Physiology, 124:1465-1467, 2000).

Recently, some groups have begun the process of sequencing insertion site flanking regions from individual plants in large insertion mutant populations, in effect prescreening a subset of lines for genomic insertion sites (Parinov et al., Plant Cell, 11:2263-2270, 1999; Tisser et al., Plant Cell, 11:1841-1852, 1999). The advantage to this approach is that the laborious and time-consuming process of PCR-based screening and deconvolution of pools is avoided.

A large database of insertion site flanking sequences from approximately 100,000 T-DNA mutagenized *Arabidopsis* plants of the Columbia ecotype (GARLIC lines) is prepared. T-DNA left border sequences from individual plants are amplified using a modified thermal asymmetric interlaced-polymerase chain reaction (TAIL-PCR) protocol (Liu et al., Plant J., 8:457-463, 1995). Left border TAIL-PCR products are sequenced and assembled into a database that associates sequence tags with each of the approximately 100,000 plants in the mutant collection. Screening the collection for insertions in genes of interest involves a simple gene name or sequence BLAST query of the insertion site flanking sequence database, and search results point to individual lines. Insertions are confirmed using PCR.

Analysis of the GARLIC insert lines suggests that there are 76,856 insertions that localize to a subset of the genome representing coding regions and promoters of 22,880 genes. Of these, 49,231 insertions lie in the promoters of over 18,572 genes, and an additional 27,625 insertions are located within the coding regions of 13,612 genes. Approximately 25,000 T-DNA left border mTAIL-PCR products (25% of the total 102,765) do not have significant matches to the subset of the genome representing promoters and coding regions, and are therefore presumed to lie in noncoding and/or repetitive regions of the genome.

The *Arabidopsis* T-DNA GARLIC insertion collection is used to investigate the roles of certain genes in abiotic stress. Target genes are chosen using a variety of criteria, including public reports of mutant phenotypes, RNA profiling experiments, and sequence similarity to genes implicated in abiotic stress. Plant lines with insertions in genes of interest are then identified. Each T-DNA insertion line is represented by a seed lot collected from a plant that is hemizygous for a particular T-DNA insertion. Plants homozygous for insertions of interest are identified using a PCR assay. The seed produced by these plants is homozygous for the T-DNA insertion mutation of interest.

Homozygous mutant plants are tested for altered stress response. The genes interrupted in these mutants contribute to the observed phenotype. The genes interrupted in these mutants interfere with the normal response of the plant to abiotic stresses.

Rice orthologs of the *Arabidopsis* genes affecting the plants response to an abiotic stress are identified by similarity searching of a rice database using the Double-Affine Smith-Waterman algorithm (BLASP with e values better than $^{-10}$).

***Example 6*** : *Cloning and Sequencing of Nucleic Acid Molecules from Rice*

*6.1 Genomic DNA:* Plant genomic DNA samples are isolated from a collection of tissues. Individual tissues are collected from a minimum of five plants and pooled. DNA can be isolated according to one of the three procedures, e.g., standard procedures described by Ausubel et al. (1995), a quick leaf prep described by Klimyuk et al. (Plant J., 3:493-494, 1993), or using FTA paper (Life Technologies. Rockville, MD).

For the latter procedure, a piece of plant tissue such as, for example, leaf tissue is excised from the plant, placed on top of the FTA paper and covered with a small piece of parafilm that serves as a barrier material to prevent contamination of the crushing device. In order to drive the sap and cells from the plant tissue into the FTA paper matrix for effective cell lysis and nucleic acid entrapment, a crushing device is used to mash the tissue into the FTA paper. The FTA paper is air dried for an hour. For analysis of DNA, the samples can be archived on the paper until analysis. Two mm punches are removed from the specimen area on the FTA paper using a 2 mm Harris Micro Punch™ and placed into PCR tubes. Two hundred (200) microliters of FTA purification reagent is added to the tube containing the punch and vortexed at low speed for 2 seconds. The tube is then incubated at room temperature for 5 minutes. The solution is removed with a pipette so as to repeat the wash one more time. Two hundred (200) microliters of TE (10 mM Tris, 0.1 mM EDTA, pH 8.0) is added and the wash is repeated two more times. The PCR mix is added directly to the punch for subsequent PCR reactions.

*6.2 Cloning of Candidate cDNA:* A candidate cDNA is amplified from total RNA isolated from rice tissue after reverse transcription using primers designed against the computationally predicted cDNA. Primers designed based on the genomic sequence can be used to PCR amplify the full-length cDNA (start to stop codon) from first strand cDNA prepared from rice cultivar Nipponbare tissue.

The Qiagen RNeasy kit (Qiagen, Hilden, Germany) is used for extraction of total RNA. The Superscript II kit (Invitrogen, Carlsbad, USA) is used for the reverse transcription reaction. PCR amplification of the candidate cDNA is carried out using the reverse primer sequence located at the translation start of the candidate gene in 5' - 3' direction. This is performed with high-fidelity Taq polymerase (Invitrogen, Carlsbad, USA).

The PCR fragment is then cloned into pCR2.1-TOPO (Invitrogen) or the pGEM-T easy vector (Promega Corporation, Madison, Wis.) per the manufacturer's instructions, and several individual clones are subjected to sequencing analysis.

*6.3 DNA sequencing:* DNA preps for 2-4 independent clones are miniprepped following the manufacturer's instructions (Qiagen). DNA is subjected to sequencing analysis using the BigDye™ Terminator Kit according to manufacturer's instructions (Applied Biosystems Inc., Foster City, CA) . Sequencing makes use of primers designed to both strands of the predicted gene of interest. DNA sequencing is performed using standard dye-terminator sequencing procedures and automated sequencers (models 373 and 377; Applied Biosystems). All sequencing data are analyzed and assembled using the Phred/Phrap/Consed software package (University of Washington) to an error ratio equal to or less than $10^{-4}$ at the consensus sequence level.

The consensus sequence from the sequencing analysis is then to be validated as being intact and the correct gene in several ways. The coding region is checked for being full length (predicted start and stop codons present) and uninterrupted (no internal stop codons). Alignment with the gene prediction and BLAST analysis is used to ascertain that this is in fact the right gene.

The clones are sequenced to verify their correct amplification.

***Example 7*****:** *Functional analysis in plants*

A plant complementation assay can be used for the functional characterization of the abiotic stress genes according to the invention.

Rice and Arabidopsis putative orthologue pairs are identified using BLAST comparisons, TFASTXY comparisons, and Double-Affine Smith-Waterman similarity searches. Constructs containing a rice cDNA or genomic clone inserted between the promoter and terminator of the Arabidopsis orthologue are generated using overlap PCR (Horton et al., Gene, 77: 61-68,1989) and GATEWAY cloning (Life Technologies Invitrogen. Carlsbad, CA). For ease of cloning, rice cDNA clones are preferred to rice genomic clones. A three stage PCR strategy is used to make these constructs.

(1) In the first stage, primers are used to PCR amplify: (i) 2Kb upstream of the translation start site of the Arabidopsis orthologue, (ii) the coding region or cDNA of the rice orthologue, and (iii) the 500 bp immediately downstream of the Arabidopsis orthogue's translation stop site. Primers are designed to incorporate onto their 5' ends at least 16 bases of the 3' end of the adjacent fragment, except in the case of the most distal primers which flank the gene construct (the forward primer of the promoter and the reverse primer of the terminator). The forward primer of the promoters contains on their 5' ends partial AttB1 sites, and the reverse primer of the terminators contains on their 5' ends partial AttB2 sites, for Gateway cloning.
(2) In the second stage, overlap PCR is used to join either the promoter and the coding region, or the coding region and the terminator.
(3) In the third stage, either the promoter-coding region product can be joined to the terminator or the coding region-terminator product can be joined to the promoter, using overlap PCR and amplification with full1 Att site-containing primers, to link all three fragments, and put full Att sites at the construct termini.

The fused three-fragment piece flanked by Gateway cloning sites are introduced into the LTI donor vector pDONR201 using the BP clonase reaction, for confirmation by sequencing. Confirmed sequenced constructs are introduced into a binary vector containing Gateway cloning sites, using the LR clonase reaction such as, for example, pAS200.
The pAS200 vector was created by inserting the Gateway cloning cassette RfA into the Acc65I site of pNOV3510.
pNOV3510 was created by ligation of inverted pNOV2114 VSI binary into pCTK7-PTX5'AtPPONOS.
pNOV2114 was created by insertion of virGN54D (Pazour et al., J. Bacteriol. 174:4169-4174, 1992) from pAD1289 (Hansen et al., Proc. Natl. Acad. Sci. USA 91:7603-7607, 1994) into pHiNK085.
pHiNK085 was created by deleting the 35S:PMI cassette and M13 ori in pVictorHiNK.
pPVictorHiNK was created by modifying the T-DNA of pVictor (described in WO 97/04112) to delete M13 derived sequences and to improve its cloning versatility by introducing the BIGLINK polylinker.
The sequence of the pVictor HiNK vector is disclosed in SEQ ID NO: 5 of WO 00/6837, which is incorporated herein by reference. The pVictorHiNK vector contains the following constituents that are of functional importance:

- The origin of replication (ORI) functional in *Agrobacterium* is derived from the *Pseudomonas aeruginosa* plasmid pVS1 (Itoh et al., Plasmid, 11: 206-220 1984; Itoh and Haas, Gene, 36: 27-36, 1985). The pVS1 ORI is only functional in *Agrobacterium* and can be mobilized by the helper plasmid pRK2013 from *E.coli* into A. *tumefaciens* by means of a triparental mating procedure (Ditta et al., Proc. Natl. Acad. Sci USA. 77:7347-7351, 1980).
- The ColE1 origin of replication functional in E. *coli* is derived from pUC19 (Yannisch-Perron et al., Gene, 33:103-119, 1985).
- The bacterial resistance to spectinomycin and streptomycin encoded by a 0.93 kb fragment from transposon Tn7 (Fling et al., Nucl. Acids Res., 13:7095, 1985) functions as selectable marker for maintenance of the vector in *E. coli* and *Agrobacterium.* The gene is fused to the *tac* promoter for efficient bacterial expression (Amman et al., Gene, 25:167-178, 1983).
- The right and left T-DNA border fragments of 1.9 kb and 0.9 kb that comprise the 24 bp border repeats, have been derived from the Ti-plasmid of the nopaline type *Agrobacterium tumefaciens* strains pTiT37 (Yadav et al., Proc. Natl. Acad. Sci. USA., 79:6322-6326, 1982).

The plasmid is introduced *into Agrobacterium tumefaciens* GV3101pMP90 by electroporation. The positive bacterial transformants are selected on LB medium containing 50 $\mu$g/$\mu$l kanamycin and 25 $\mu$g/$\mu$l gentamycin. Plants are transformed by standard methodology (e.g., by dipping flowers into a solution containing the *Agrobacterium)* except that 0.02% Silwet-77 (Lehle Seeds, Round Rock, TX) is added to the bacterial suspension and the vacuum step omitted. Five hundred (500) mg of seeds are planted per 2 ft$^2$ flat of soil and plant transformants are selected by spraying with the herbicide formulated BASTA (2 ml of Finale, AgrEvo Environmental Health, Montvale, NJ, is added to 498 ml water) once every two days, for a week.

***Example 8:*** *Vector construction for overexpression and gene "knockout" experiments*

*8.1 Overexpression*

Vectors used for expression of full-length "abiotic stress candidate genes" of interest in plants (overexpression) are designed to overexpress the protein of interest and are of two general types, biolistic and binary, depending on the plant transformation method to be used.

For biolistic transformation (biolistic vectors), the requirements are as follows:

1. a backbone with a bacterial selectable marker (typically, an antibiotic resistance gene) and origin of replication functional in *Escherichia coli (E. coli;* eg. ColEI), and
2. a plant-specific portion consisting of:

a. a gene expression cassette consisting of a promoter (eg. ZmUBIint MOD), the gene of interest (typically, a full-length cDNA) and a transcriptional terminator (eg. *Agrobacterium tumefaciens nos* terminator);
b. a plant selectable marker cassette, consisting of a promoter (e.g. rice Act1D-BV MOD), selectable marker gene (e.g. phosphomannose isomerase, PMI) and transcriptional terminator (e.g. CaMV terminator).

Vectors designed for transformation by *Agrobacterium tumefaciens (A. tumefaciens;* binary vectors) consist of:

1. a backbone with a bacterial selectable marker functional in both E. *coli* and A. *tumefaciens* (e.g. spectinomycin resistance mediated by the *aadA* gene) and two origins of replication, functional in each of aforementioned bacterial hosts, plus the A. *tumefaciens virG* gene;
2. a plant-specific portion as described for biolistic vectors above, except in this instance this portion is flanked by A. *tumefaciens* right and left border sequences which mediate transfer of the DNA flanked by these two sequences to the plant.

*8.2 Knock out vectors*

Vectors designed for reducing or abolishing expression of a single gene or of a family or related genes (knockout vectors) are also of two general types corresponding to the methodology used to downregulate gene expression: antisense or double-stranded RNA interference (dsRNAi).

(a) Anti-sense
For antisense vectors, a full-length or partial gene fragment (typically, a portion of the cDNA) can be used in the same vectors described for full-length expression, as part of the gene expression cassette. For antisense-mediated down-regulation of gene expression, the coding region of the gene or gene fragment will be in the opposite orientation relative to the promoter; thus, mRNA will be made from the non-coding (antisense) strand *in planta.*
(b) dsRNAi
For dsRNAi vectors, a partial gene fragment (typically, 300 to 500 basepairs long) is used in the gene expression cassette, and is expressed in both the sense and antisense orientations, separated by a spacer region (typically, a plant intron, e.g. the OsSH1 intron 1, or a selectable marker, e.g. conferring kanamycin resistance). Vectors of this type are designed to form a double-stranded mRNA stem, resulting from the basepairing of the two complementary gene fragments *in planta.*

Biolistic or binary vectors designed for overexpression or knockout can vary in a number of different ways, including e.g. the selectable markers used in plant and bacteria, the transcriptional terminators used in the gene expression and plant selectable marker cassettes, and the methodologies used for cloning in gene or gene fragments of interest (typically, conventional restriction enzyme-mediated or Gateway™ recombinase-based cloning). An important variant is the nature of the gene expression cassette promoter driving expression of the gene or gene fragment of interest in most tissues of the plants (constitutive, eg. ZmUBIint MOD), in specific plant tissues (eg. maize ADP-gpp for endosperm-specific expression), or in an inducible fashion (eg. GAL4bsBz1 for estradiol-inducible expression in lines constitutively expressing the cognate transcriptional activator for this promoter).

***Example 9:*** *Insertion ofan "abiotic stress candidate gene" into an expression vector*

A validated rice cDNA clone in pCR2.1-TOPO or the pGEM-T easy vector is subcloned using conventional restriction enzyme-based cloning into a vector, downstream of the maize ubiquitin promoter and intron, and upstream of the Agrobacterium tumefaciens nos 3' end transcriptional terminator. The resultant gene expression cassette (promoter, "abiotic stress candidate gene" and terminator) is further subcloned, using conventional restriction enzyme-based cloning, into the pNOV2117 binary vector (Negrotto et al., Plant Cell Reports 19, 798-803, 2000; plasmid pNOV117 discosed in this article corresponds to pNOV2117 described herein), generating pNOVCAND.

The pNOVCAND binary vector is designed for transformation and over-expression of the "abiotic stress candidate gene" in monocots. It consists of a binary backbone containing the sequences necessary for selection and growth in *Escherichia coli* DH-5α (Invitrogen) and *Agrobacterium tumefaciens* LBA4404 (pAL4404; pSBI), including the bacterial spectinomycin antibiotic resistance *aadA* gene from *E. coli* transposon Tn7, origins of replication for E. *coli* (ColE1) and *A. tumefaciens* (VS1), and the *A. tumefaciens virG* gene. In addition to the binary backbone, which is identical to that of pNOV2114 described herein previously (see Example 7 above), pNOV2117 contains the T-DNA portion flanked by the right and left border sequences, and including the Positech™ (Syngenta) plant selectable marker (WO 94/20627) and the "abiotic stress candidate gene" gene expression cassette. The Positech™ plant selectable marker confers resistance to mannose and in this instance consists of the maize ubiquitin promoter driving expression of the PMI (phosphomannose isomerase) gene, followed by the cauliflower mosaic virus transcriptional terminator.

Plasmid pNOV2117 is introduced into Agrobacterium tumefaciens LBA4404 (pAL4404; pSB1) by electroporation. Plasmid pAL4404 is a disarmed helper plasmid (Ooms et al., Plasmid, 7:15-29, 1982). Plasmid pSB1 is a plasmid with a wide host range that contains a region of homology to pNOV2117 and a 15.2 kb Kpnl fragment from the virulence region of pTiBo542 (Ishida et al., Nat. Biotechnol., 14:745-750, 1996). Introduction of plasmid pNOV2117 into Agrobacterium strain LBA4404 results in a co-integration of pNOV2117 and pSB1.

Alternatively, plasmid pCIB7613, which contains the hygromycin phosphotransferase *(hpt)* gene (Gritz and Davies, Gene, 25:179-188, 1983) as a selectable marker, may be employed for transformation.

Plasmid pCIB7613 (see WO 98/06860, incorporated herein by reference) is selected for rice transformation. In pCIB7613, the transcription of the nucleic acid sequence coding hygromycin-phosphotransferase (HYG gene) is driven by the corn ubiquitin promoter (ZmUbi) and enhanced by corn ubiquitin intron 1. The 3'polyadenylation signal is provided by NOS 3' nontranslated region.

Other useful plasmids include pNADII002 (GAL4-ER-VP16) which contains the yeast GAL4 DNA Binding domain (Keegan et al., Science, 231:699,1986), the mammalian estrogen receptor ligand binding domain (Greene et al., Science, 231:1150, 1986) and the transcriptional activation domain of the HSV VP16 protein (Triezenberg et al., Genes Dev., 2:718-729, 1988). Both *hpt* and GAL4-ER-VP16 are constitutively expressed using the maize Ubiquitin promoter, and pSGCDL1 (GAL4BS Bz1 Luciferase), which carries the firefly luciferase reporter gene under control of a minimal maize Bronzel (Bzl) promoter with 10 upstream synthetic GAL4 binding sites. All constructs use termination signals from the nopaline synthase gene.

### *Example 10*: Rice Transformation

pNOVCAND is transformed into a rice cultivar (Kaybonnet) using Agrobacterium-mediated transformation, and mannose-resistant calli are selected and regenerated.

Agrobacterium is grown on YPC solid plates for 2-3 days prior to experiment initiation. Agrobacterial colonies are suspended in liquid MS media to an OD of 0.2 at λ600nm. Acetosyringone is added to the agrobacterial suspension to a concentration of 200μM and agro is induced for 30min.

Three-week-old calli which are induced from the scutellum of mature seeds in the N6 medium (Chu et al., Sci. Sin., 18:659-668, 1975) are incubated in the agrobacterium solution in a 100 x 25 petri plate for 30 minutes with occasional shaking. The solution is then removed with a pipet and the callus transfered to a MSAs medium which is overlayed with sterile filter paper.

Co-Cultivation is continued for 2 days in the dark at 22°C.

Calli are then placed on MS-Timetin plates for 1 week. After that they are tranfered to PAA + mannose selection media for 3 weeks.

Growing calli (putative events) are picked and transfered to PAA+ mannose media and cultivated for 2 weeks in light.

Colonies are tranfered to MS20SorbKinTim regeneration media in plates for 2 weeks in light. Small plantlets are transferred to MS20SorbKinTim regeneration media in GA7 containers. When they reach the lid, they are transfered to soil in the greenhouse.

Expression of the "abiotic stress candidate gene" in transgenic To plants is analyzed. Additional rice cultivars, such as but not limited to, Nipponbare, Taipei 309 and Fuzisaka 2 are also transformed and assayed for expression of the "abiotic stress candidate gene" product and enhanced protein expression.

**Example 11**: *Analysis of mutant and transgenic plant material*

*11.1 Testing Arabidopsis seedlings using agar plates*

Arabidopsis seedlings can be assayed for abiotic stress phenotypes by measuring root growth rate under control and experimental conditions (Wu et al., Plant Cell, 8:617-627, 1996). Four to five day-old axenic seedlings are produced by germinating surface-sterilized seeds on agar growth medium plates oriented vertically. The seedlings are transferred to agar growth medium containing inhibitory levels of NaCl or Polyethylene glycol or mannitol or kept on the original plate for temperature stress (chilling stress = 4°C, freezing stress $\leq$ 0°C, heat stress $\geq$ 37°C) and control seedlings are transferred to a new plate containing normal growth medium. Upon transfer (or exposure to the temperature extreme), the plates are rotated 180 degrees so that subsequent root growth occurs in the exact opposite direction to previous growth due to the agravitropic root response. Growth subsequent to exposure to the abiotic stress is measured as the growth that occurs after bending of the root. Sensitivity or tolerance to abiotic stress is expressed as percent of experimental growth versus control growth following transfer.

*11.2 Testing Arabidopsis or rice plants growing in soil*

Adult Arabidopsis plants can be assayed for abiotic stress phenotypes by exposing soil-grown plants to abiotic stresses such as NaCl-, osmotic-, drought-, or temperature-stress and scoring for survivability following exposure (Wu et al., Plant Cell, 8:617-627, 1996; Kasuga et al., Nat. Biotech., 17:287-291, 1999). Three-week-old plants are exposed to abiotic stress conditions by sub-irrigating with NaCl, mannitol or polyethylene glycol, or the pots are moved from normal growing temperature to <0°C or > 37°C, or water is withheld. Resistant and sensitive phenotypes can be distinguished within 2 to 5 days of treatment depending on the stress (Kasuga et al., Nat. Biotech., 17:287-291, 1999). Similar methods have been applied to cereal plants such as rice (Babu et al., Crop Sci., 39:150-158, 1999; Saijo et al., Plant J., 23:319-327, 2000). It is also possible to assay abiotic stress phenotypes using plants growing hydroponically (Moons et al., Plant Physiol., 107:177-186, 1995; Kawaski et al., Plant Cell., 13:889-905, 2001). Young plants are grown in liquid nutrient medium and the stress treatments are applied by mixing in the stressful compounds such as NaCl, mannitol or Polyethylene glycol and assessing growth visually.

**Example 12:** *Chromosomal Markers to Identify the Location of a Nucleic Acid Sequence*

The sequences of the present invention can also be used for SSR mapping. SSR mapping in rice has been described by Miyao et al. (DNA Res., 3:233, 1996) and Yang et al. (Mol. Gen. Genet., 245:187, 1994), and in maize by Ahn et al. (Mol. Gen. Genet., 241:483, 1993). SSR mapping can be achieved using various methods. In one instance, polymorphisms are identified when sequence specific probes flanking an SSR contained within a sequence are made and used in polymerase chain reaction (PCR) assays with template DNA from two or more individuals or, in plants, near isogenic lines. A change in the number of tandem repeats between the SSR-flanking sequence produces differently sized fragments (U.S. Patent No. 5,766,847). Alternatively, polymorphisms can be identified by using the PCR fragment produced from the SSR-flanking sequence specific primer reaction as a probe against Southern blots representing different individuals (Refseth et al., ., Electrophoresis, 18:1519, 1997). Rice SSRs can be used to map a molecular marker closely linked to functional gene, as described by Akagi et al. (Genome 39:205, 1996).

The sequences of the present invention can be used to identify and develop a variety of microsatellite markers, including the SSRs described above, as genetic markers for comparative analysis and mapping of genomes.

Some of the polynucleotides disclosed herein contain at least 3 consecutive di-, tri- or tetranucleotide repeat units in their coding region that can potentially be developed into SSR markers. Trinucleotide motifs that can be commonly found in the coding regions of said polynucleotides and easily identified by screening the polynucleotides sequences for said motifs are, for example: CGG; GCC, CGC, GGC, etc. Once such a repeat unit has been found, primers can be designed which are complementary to the region flanking the repeat unit and used in any of the methods described below.

Sequences of the present invention can also be used in a variation of the SSR technique known as inter-SSR (ISSR), which uses microsatellite oligonucleotides as primers to amplify genomic segments different from the repeat region itself (Zietkiewicz et al., Genomics, 20:176, 1994). ISSR employs oligonucleotides based on a simple sequence repeat anchored or not at their 5'- or 3'-end by two to four arbitrarily chosen nucleotides, which triggers site-specific annealing and initiates PCR amplification of genomic segments which are flanked by inversely orientated and closely spaced repeat sequences. In one embodiment of the present invention, microsatellite markers, or substantially similar sequences, or allelic variants thereof, may be used to detect the appearance or disappearance of markers indicating genomic instability as described by Leroy et al. (Electron. J. Biotechnol., 3(2), at http://www.ejb.org (2000)), where alteration of a fingerprinting pattern indicated loss of a marker corresponding to a part of a gene involved in the regulation of cell proliferation. Microsatellite markers are useful for detecting genomic alterations such as the change observed by Leroy et al. (Electron. J Biotechnol, 3(2), supra (2000)) which appeared to be the consequence of microsatellite instability at the primer binding site or modification of the region between the microsatellites, and illustrated somaclonal variation leading to genomic instability. Consequently, sequences of the present invention are useful for detecting genomic alterations involved in somaclonal variation, which is an important source of new phenotypes.

In addition, because the genomes of closely related species are largely syntenic (that is, they display the same ordering of genes within the genome), these maps can be used to isolate novel alleles from wild relatives of crop species by positional cloning strategies. This shared synteny is very powerful for using genetic maps from one species to map genes in another. For example, a gene mapped in rice provides information for the gene location in maize and wheat.

## Example 13: *Quantitative Trait Linked Breeding*

Various types of maps can be used with the sequences of the invention to identify Quantitative Trait Loci (QTLs) for a variety of uses, including marker-assisted breeding. Many important crop traits are quantitative traits and result from the combined interactions of several genes. These genes reside at different loci in the genome, often on different chromosomes, and generally exhibit multiple alleles at each locus. Developing markers, tools, and methods to identify and isolate the QTLs involved in a trait, enables marker-assisted breeding to enhance desirable traits or suppress undesirable traits. The sequences disclosed herein can be used as markers for QTLs to assist marker-assisted breeding. The sequences of the invention can be used to identify QTLs and isolate alleles as described by Li et al. in a study of QTLs involved in resistance to a pathogen of rice. (Li et al., Mol. Gen. Genet., 261:58, 1999). In addition to isolating QTL alleles in rice, other cereals, and other monocot and dicot crop species, the sequences of the invention can also be used to isolate alleles from the corresponding QTL(s) of wild relatives. Transgenic plants having various combinations of QTL alleles can then be created and the effects of the combinations measured. Once an ideal allele combination has been identified, crop improvement can be accomplished either through biotechnological means or by directed conventional breeding programs. (Flowers et al., J. Exp. Bot., 51:99, 2000); Tanksley and McCouch, Science, 277:1063, 1997).

## Example 14: *Marker-Assisted Breeding*

Markers or genes associated with specific desirable or undesirable traits are known and used in marker assisted breeding programs. It is particularly beneficial to be able to screen large numbers of markers and large numbers of candidate parental plants or progeny plants. The methods of the invention allow high volume, multiplex screening for numerous markers from numerous individuals simultaneously.

Markers or genes associated with specific desirable or undesirable traits are known and used in marker assisted breeding programs. It is particularly beneficial to be able to screen large numbers of markers and large numbers of candidate parental plants or progeny plants. The methods of the invention allow high volume, multiplex screening for numerous markers from numerous individuals simultaneously.

A multiplex assay is designed providing SSRs specific to each of the markers of interest. The SSRs are linked to different classes of beads. All of the relevant markers may be expressed genes, so RNA or cDNA techniques are appropriate. RNA is extracted from root tissue of 1000 different individual plants and hybridized in parallel reactions with the different classes of beads. Each class of beads is analyzed for each sample using a microfluidics analyzer. For the classes of beads corresponding to qualitative traits, qualitative measures of presence or absence of the target gene are recorded. For the classes of beads corresponding to quantitative traits, quantitative measures of gene activity are recorded. Individuals showing activity of all of the qualitative genes and highest expression levels of the quantitative traits are selected for further breeding steps. In procedures wherein no individuals have desirable results for all the measured genes, individuals having the most desirable, and fewest undesirable, results are selected for further breeding steps. In either case, progeny are screened to further select for homozygotes with high quantitative levels of expression of the quantitative traits.

## Example 15: *Method of modifying the gene frequency*

The invention further provides a method of modifying the frequency of a gene in a plant population, including the steps of: identifying an SSR within a coding region of a gene; screening a plurality of plants using the SSR as a marker to determine the presence or absence of the gene in an individual plant; selecting at least one individual plant for breeding based on the presence or absence of the gene; and breeding at least one plant thus selected to produce a population of plants having a modified frequency of the gene. The identification of the SSR within the coding region of a gene can be accomplished based on sequence similarity between the nucleic acid molecules of the invention and the region within the gene of interest flanking the SSR.

The results disclosed herein demonstrate that several polynucleotides, some of which were known to function as transcription factors, enzymes, and structural proteins, also are involved in the response of a plant cell to stress. The identification of stress-regulated genes as disclosed herein provides a means to identify stress-regulated regulatory elements present in rice nucleotide sequences, including consensus regulatory elements. Furthermore, the identification of the rice stress-regulated genes provides a means to identify the corresponding homologs and orthologs in other plants, including commercially valuable food crops such as wheat, maize, soy, and barley, and ornamental plants..

Table 1

A = ORFs from drought tolerance QTL in rice chromosome 3
B = Cold induced genes from the rice genomic region syntenic with maize cold tolerance QTL
C = Genes in the drought QTL on chromos 8 showing >=2x change during drought stress
D = Rice proteins that have a universal stress protein domain
E = ORFs from rice genomic region that is syntenic to a maize cold tolerance QTL
F = ORFs from drought tolerance QTL in rice chromosome 8
G = Abiotic stress regulated Universal stress protein domain containing genes
H = Stress-regulated rice genes that are homologs of arabidopsis stress regulated genes
I = Genes from drought QTL on Chromos 3 showing >= 2x change up or down during drought stress

| SEQ ID | A | B | C | D | E | F | G | H | I |
|---|---|---|---|---|---|---|---|---|---|
| 3177 | - | - | - | - | - | X | - | - | - |
| 3178 | - | - | - | - | - | X | - | - | - |
| 3179 | X | - | - | - | - | - | - | - | - |
| 3180 | X | - | - | - | - | - | - | - | - |
| 3181 | X | - | - | - | - | - | - | - | - |
| 3182 | X | - | - | - | - | - | - | - | - |
| 3183 | X | - | - | - | - | - | - | - | - |
| 3184 | X | - | - | - | - | - | - | - | - |
| 3185 | X | - | - | - | - | - | - | - | - |
| 3186 | X | - | - | - | - | - | - | - | - |
| 4379 | - | - | - | - | - | - | - | X | - |
| 1328 | - | - | - | - | - | X | - | - | - |
| 3187 | - | - | - | - | - | X | - | - | - |
| 2554 | - | - | - | - | - | X | - | - | - |
| 3188 | - | - | - | - | - | X | - | - | - |
| 3189 | - | - | - | - | - | X | - | - | - |
| 3190 | - | - | - | - | - | X | - | - | - |
| 5827 | - | - | - | - | - | - | - | X | - |
| 3191 | - | - | - | - | - | X | - | - | - |
| 3192 | - | - | - | - | - | X | - | - | - |
| 3193 | - | - | - | - | - | X | - | - | - |
| 3194 | - | - | - | - | - | X | - | - | - |
| 3195 | - | - | - | - | - | X | - | - | - |
| 4863 | - | - | - | - | - | - | - | X | - |
| 3196 | - | - | - | - | - | X | - | - | - |
| 355 | X | - | - | - | - | - | - | - | - |
| 3197 | - | - | - | - | - | X | - | - | - |
| 3198 | - | - | - | - | - | X | - | - | - |
| 795 | - | - | - | - | X | - | - | - | - |
| 1583 | - | - | - | - | X | - | - | - | - |
| 71 | - | - | - | - | X | - | - | - | - |
| 3199 | - | - | - | - | X | - | - | - | - |
| 3200 | - | - | - | - | X | - | - | - | - |
| 3201 | X | - | - | - | X | - | - | - | - |
| 3202 | X | - | - | - | X | - | - | - | - |

(continued)

| SEQ ID | A | B | C | D | E | F | G | H | I |
|--------|---|---|---|---|---|---|---|---|---|
| 3203 | X | - | - | - | - | - | - | - | - |
| 3204 | X | - | - | - | - | - | - | - | - |
| 1865 | X | - | - | - | - | - | - | - | - |
| 3205 | X | - | - | - | - | - | - | - | - |
| 3206 | X | - | - | - | - | - | - | - | - |
| 3207 | - | - | - | - | - | X | - | - | - |
| 3208 | X | - | - | - | - | - | - | - | - |
| 3209 | X | - | - | - | - | - | - | - | - |
| 3210 | X | - | - | - | - | - | - | - | - |
| 3211 | X | - | - | - | - | - | - | - | - |
| 3212 | X | - | - | - | - | - | - | - | - |
| 3213 | - | - | - | - | X | - | - | - | - |
| 3214 | - | - | - | - | - | X | - | - | - |
| 3215 | - | - | - | - | - | X | - | - | - |
| 3216 | - | - | - | X | - | - | - | - | - |
| 6692 | - | - | - | - | - | - | - | X | - |
| 3217 | - | - | - | - | - | X | - | - | - |
| 3218 | - | - | - | - | - | X | - | - | - |
| 3219 | - | - | - | - | - | X | - | - | - |
| 3220 | - | - | - | - | - | - | - | - | - |
| 3221 | - | - | - | - | X | - | - | - | - |
| 3222 | - | - | - | - | - | X | - | - | - |
| 3223 | - | - | - | - | - | X | - | - | - |
| 3224 | - | - | - | X | - | - | - | - | - |
| 7356 | - | - | - | - | - | - | - | - | X |
| 3226 | - | - | - | - | X | - | - | - | - |
| 3227 | - | - | - | - | X | - | - | - | - |
| 1741 | - | - | - | - | X | - | - | - | - |
| 3228 | X | - | - | - | - | - | - | - | - |
| 3229 | X | - | - | - | - | - | - | - | - |
| 3230 | X | - | - | - | - | - | - | - | - |
| 3231 | X | - | - | - | - | - | - | - | - |
| 3232 | X | - | - | - | - | - | - | - | - |
| 3233 | - | - | - | - | X | - | - | - | - |
| 2401 | X | - | - | - | - | - | - | - | - |
| 3234 | - | - | - | - | - | X | - | - | - |
| 3235 | - | - | - | - | - | X | - | - | - |
| 5394 | - | - | - | - | - | - | - | X | - |
| 3236 | - | - | - | - | X | - | - | - | - |
| 3237 | - | - | - | - | X | - | - | - | - |
| 3238 | X | - | - | - | - | - | - | - | - |
| 3239 | X | - | - | - | - | - | - | - | - |
| 3240 | X | - | - | - | - | - | - | - | - |
| 3241 | - | - | - | - | X | - | - | - | - |
| 3242 | - | - | - | - | X | - | - | - | - |
| 3243 | - | - | - | - | - | X | - | - | - |
| 3244 | - | - | - | - | - | X | - | - | - |
| 3245 | - | - | - | - | - | X | - | - | - |
| 3246 | - | - | - | - | - | X | - | - | - |
| 3247 | - | - | - | - | - | X | - | - | - |

(continued)

| SEQ ID | A | B | C | D | E | F | G | H | I |
|--------|---|---|---|---|---|---|---|---|---|
| 3248 | X | - | - | - | - | - | - | - | - |
| 5939 | - | - | - | - | - | - | - | X | - |
| 3249 | X | - | - | - | - | - | - | - | - |
| 3250 | X | - | - | - | - | - | - | - | - |
| 3251 | X | - | - | - | - | - | - | - | - |
| 1655 | - | - | - | - | - | X | - | - | - |
| 3252 | X | - | - | X | - | - | - | - | - |
| 3253 | - | - | - | - | X | - | - | - | - |
| 3254 | X | - | - | - | - | - | - | - | - |
| 3255 | X | - | - | - | - | - | - | - | - |
| 3256 | - | - | - | - | - | X | - | - | - |
| 7132 | - | - | - | - | - | - | - | X | - |
| 3257 | X | - | - | - | - | - | - | - | - |
| 3258 | X | - | - | - | - | - | - | - | - |
| 3259 | X | - | - | - | - | - | - | - | - |
| 3260 | X | - | - | - | - | - | - | - | - |
| 3261 | X | - | - | - | - | - | - | - | - |
| 3262 | X | - | - | - | - | - | - | - | - |
| 3263 | X | - | - | - | - | - | - | - | - |
| 3264 | - | - | - | - | - | X | - | - | - |
| 2876 | X | - | - | - | - | - | - | - | - |
| 3265 | - | - | - | - | - | X | - | - | - |
| 3266 | - | - | - | - | - | X | - | - | - |
| 5575 | - | - | - | - | - | - | - | X | - |
| 3267 | - | - | - | - | - | X | - | - | - |
| 3268 | X | - | - | - | - | - | - | - | - |
| 3269 | X | - | - | - | - | - | - | - | - |
| 3270 | X | - | - | - | - | - | - | - | - |
| 3271 | X | - | - | - | - | - | - | - | - |
| 3272 | X | - | - | - | - | - | - | - | - |
| 3273 | X | - | - | - | - | - | - | - | - |
| 3274 | - | - | - | - | X | - | - | - | - |
| 3275 | - | - | - | - | X | - | - | - | - |
| 3276 | - | - | - | - | - | X | - | - | - |
| 1828 | - | - | - | - | - | X | - | - | - |
| 792 | - | - | - | - | - | X | - | - | - |
| 3277 | - | - | - | - | - | X | - | - | - |
| 3278 | - | - | - | - | X | - | - | - | - |
| 542 | - | - | - | - | X | - | - | - | - |
| 3279 | - | - | - | - | X | - | - | - | - |
| 3289 | - | - | - | - | X | - | - | - | - |
| 3281 | - | - | - | - | X | - | - | - | - |
| 2367 | - | - | - | - | - | X | - | - | - |
| 3282 | - | - | - | - | - | X | - | - | - |
| 3283 | - | - | - | - | - | X | - | - | - |
| 3284 | - | - | - | - | - | X | - | - | - |
| 3285 | - | - | - | - | - | X | - | - | - |
| 3286 | - | - | - | - | - | X | - | - | - |
| 3287 | - | - | - | - | - | X | - | - | - |
| 7148 | - | - | - | - | - | - | - | X | - |

(continued)

| SEQ ID | A | B | C | D | E | F | G | H | I |
|--------|---|---|---|---|---|---|---|---|---|
| 3288 | - | - | - | - | - | X | - | - | - |
| 3289 | - | - | - | - | - | X | - | - | - |
| 3290 | - | - | - | - | - | X | - | - | - |
| 3291 | - | - | - | - | - | X | - | - | - |
| 2591 | - | - | - | - | X | - | - | - | - |
| 3292 | - | - | - | - | X | - | - | - | - |
| 1090 | - | - | - | - | X | - | - | - | - |
| 3293 | - | - | - | - | - | X | - | - | - |
| 6799 | - | - | - | - | - | - | - | X | - |
| 3294 | X | - | - | - | - | - | - | - | - |
| 3295 | X | - | - | - | - | - | - | - | - |
| 1736 | X | - | - | - | - | - | - | - | - |
| 3296 | X | - | - | - | - | - | - | - | - |
| 3297 | X | - | - | - | - | - | - | - | - |
| 3298 | X | - | - | - | - | - | - | - | - |
| 3299 | - | - | - | X | - | - | - | - | - |
| 307 | X | - | - | - | - | - | - | - | - |
| 3300 | X | - | - | - | - | - | - | - | - |
| 3301 | - | - | - | - | X | - | - | - | - |
| 3302 | X | - | - | - | - | - | - | - | - |
| 3303 | - | - | - | - | X | - | - | - | - |
| 3304 | - | - | - | - | X | - | - | - | - |
| 3305 | - | - | - | - | - | X | - | - | - |
| 3306 | - | - | - | - | - | X | - | - | - |
| 3307 | - | - | - | X | - | - | - | - | - |
| 7439 | - | - | X | - | - | - | - | - | - |
| 765 | - | - | - | - | - | - | - | - | - |
| 3309 | - | - | - | - | - | X | - | - | - |
| 3310 | - | - | - | - | - | X | - | - | - |
| 3311 | - | - | - | - | - | X | - | - | - |
| 545 | - | - | - | - | - | X | - | - | - |
| 3312 | - | - | - | - | - | X | - | - | - |
| 5198 | - | - | - | - | - | - | - | X | - |
| 420 | - | - | - | - | X | - | - | - | - |
| 3313 | - | - | - | - | X | - | - | - | - |
| 2914 | - | - | - | - | X | - | - | - | - |
| 6188 | - | - | - | - | - | - | - | X | - |
| 3314 | - | - | - | - | X | - | - | - | - |
| 3315 | X | - | - | - | - | - | - | - | - |
| 3316 | - | - | - | - | - | X | - | - | - |
| 3317 | - | - | - | - | X | - | - | - | - |
| 3318 | X | - | - | - | - | - | - | - | - |
| 3319 | X | - | - | - | - | - | - | - | - |
| 884 | X | - | - | - | - | - | - | - | - |
| 3320 | X | - | - | - | - | - | - | - | - |
| 3321 | - | - | - | - | - | X | - | - | - |
| 3322 | - | - | - | - | - | X | - | - | - |
| 3323 | - | - | - | - | - | X | - | - | - |
| 3324 | - | - | - | - | - | X | - | - | - |
| 3325 | - | - | - | - | - | X | - | - | - |

(continued)

| SEQ ID | A | B | C | D | E | F | G | H | I |
|--------|---|---|---|---|---|---|---|---|---|
| 3326 | - | - | - | - | X | - | - | - | - |
| 7458 | - | - | X | - | - | - | - | - | - |
| 3328 | - | - | - | - | - | X | - | - | - |
| 3329 | - | - | - | - | - | X | - | - | - |
| 3330 | - | - | - | - | - | X | - | - | - |
| 3331 | - | - | - | - | - | X | - | - | - |
| 5553 | - | - | - | - | - | - | - | X | - |
| 7463 | - | - | X | - | - | - | - | - | - |
| 3333 | X | - | - | - | - | - | - | - | - |
| 3334 | X | - | - | - | - | - | - | - | - |
| 3335 | X | - | - | - | - | - | - | - | - |
| 3336 | X | - | - | - | - | - | - | - | - |
| 3337 | X | - | - | - | - | - | - | - | - |
| 1781 | X | - | - | - | - | - | - | - | - |
| 5912 | - | - | - | - | - | - | - | X | X |
| 3338 | X | - | - | - | - | - | - | - | - |
| 3339 | - | - | - | - | - | X | - | - | - |
| 3340 | - | - | - | - | - | X | - | - | - |
| 3341 | X | - | - | - | - | - | - | - | - |
| 3342 | X | - | - | - | - | - | - | - | - |
| 6706 | - | - | - | - | - | - | - | X | - |
| 3343 | X | - | - | - | - | - | - | - | - |
| 3344 | X | - | - | - | - | - | - | - | - |
| 3345 | X | - | - | - | - | - | - | - | - |
| 3346 | X | - | - | - | - | - | - | - | - |
| 3347 | X | - | - | - | - | - | - | - | - |
| 3348 | X | - | - | - | - | - | - | - | - |
| 3349 | X | - | - | - | - | - | - | - | - |
| 3350 | - | - | - | - | - | X | - | - | - |
| 3351 | X | - | - | - | - | - | - | - | - |
| 3352 | X | - | - | - | - | - | - | - | - |
| 3353 | X | - | - | - | - | - | - | - | - |
| 7037 | - | - | - | - | - | - | - | X | - |
| 3354 | - | - | - | - | X | - | - | - | - |
| 3355 | - | - | - | - | X | - | - | - | - |
| 3356 | - | - | - | - | X | - | - | - | - |
| 3357 | - | - | - | X | - | - | - | - | - |
| 3358 | X | - | - | - | - | - | - | - | - |
| 3359 | - | - | - | - | X | - | - | - | - |
| 6017 | - | - | - | - | - | - | - | X | - |
| 3360 | X | - | - | - | - | - | - | - | - |
| 3361 | X | - | - | - | - | - | - | - | - |
| 3362 | X | - | - | - | - | - | - | - | - |
| 2481 | - | - | - | - | - | X | - | - | - |
| 5384 | - | - | - | - | - | - | - | X | - |
| 3363 | X | - | - | - | - | - | - | - | - |
| 3364 | X | - | - | - | - | - | - | - | - |
| 3365 | - | - | - | - | - | X | - | - | - |
| 3366 | - | - | - | - | - | X | - | - | - |
| 3367 | - | - | - | - | X | - | - | - | - |

(continued)

| SEQ ID | A | B | C | D | E | F | G | H | I |
|--------|---|---|---|---|---|---|---|---|---|
| 3368 | - | - | - | - | - | X | - | - | - |
| 7500 | - | - | - | - | - | - | - | - | X |
| 3370 | - | - | - | - | - | X | - | - | - |
| 2630 | - | - | - | - | X | - | - | - | - |
| 3371 | - | - | - | - | - | X | - | - | - |
| 3372 | - | - | - | - | - | X | - | - | - |
| 3373 | - | - | - | - | - | X | - | - | - |
| 3374 | - | - | - | - | - | X | - | - | - |
| 386 | - | - | - | - | X | - | - | - | - |
| 3375 | - | - | - | - | X | - | - | - | - |
| 3376 | - | - | - | - | - | X | - | - | - |
| 3377 | X | - | - | - | - | - | - | - | - |
| 3378 | X | - | - | - | - | - | - | - | - |
| 182 | - | - | - | - | X | - | - | - | - |
| 6846 | - | - | - | - | - | - | - | X | - |
| 881 | X | - | - | - | - | - | - | - | - |
| 4229 | - | - | - | - | - | - | - | X | - |
| 3379 | - | - | - | - | - | X | - | - | - |
| 3389 | X | - | - | - | - | - | - | - | - |
| 3381 | X | - | - | - | - | - | - | - | - |
| 3382 | X | - | - | - | - | - | - | - | - |
| 1809 | - | - | - | X | - | - | - | - | - |
| 4428 | - | - | - | - | - | - | - | X | - |
| 4721 | - | - | - | - | - | - | - | X | - |
| 3383 | - | - | - | - | X | - | - | - | - |
| 5908 | - | - | - | - | - | - | - | X | - |
| 6874 | - | - | - | - | - | - | - | X | - |
| 235 | X | - | - | - | - | - | - | - | - |
| 3384 | - | - | - | - | - | X | - | - | - |
| 3385 | X | - | - | - | - | - | - | - | - |
| 4230 | - | - | - | - | - | - | - | X | - |
| 3386 | - | - | - | - | - | X | - | - | - |
| 5179 | - | - | - | - | - | - | - | X | - |
| 3387 | - | - | - | X | - | - | - | - | - |
| 2602 | - | - | - | - | X | - | - | - | - |
| 3388 | - | - | - | - | X | - | - | - | - |
| 3389 | - | - | - | - | X | - | - | - | - |
| 4152 | - | - | - | - | - | - | - | X | - |
| 6118 | - | - | - | - | - | - | - | X | - |
| 3390 | - | - | - | - | - | X | - | - | - |
| 3391 | X | - | - | - | - | - | - | - | - |
| 1918 | X | - | - | - | - | - | - | - | - |
| 3392 | X | - | - | - | - | - | - | - | - |
| 3393 | X | - | - | - | - | - | - | - | - |
| 1277 | X | - | - | - | - | - | - | - | - |
| 5408 | - | - | X | - | - | - | - | X | X |
| 3394 | X | - | - | - | - | - | - | - | - |
| 3395 | X | - | - | - | - | - | - | - | - |
| 6982 | - | - | - | - | - | - | - | X | - |
| 3396 | - | - | - | - | X | - | - | - | - |

(continued)

| SEQ ID | A | B | C | D | E | F | G | H | I |
|--------|---|---|---|---|---|---|---|---|---|
| 3397 | - | - | - | - | - | X | - | - | - |
| 3398 | - | - | - | - | - | X | - | - | - |
| 3399 | X | - | - | - | - | - | - | - | - |
| 3400 | X | - | - | - | - | - | - | - | - |
| 2938 | - | - | - | - | - | X | - | - | - |
| 186 | - | - | - | - | X | - | - | - | - |
| 3401 | X | - | - | - | - | - | - | - | - |
| 3402 | X | - | - | - | - | - | - | - | - |
| 3403 | X | - | - | - | - | - | - | - | - |
| 3404 | X | - | - | - | - | - | - | - | - |
| 7535 | - | - | - | - | - | - | - | - | X |
| 1083 | X | - | - | - | - | - | - | - | - |
| 3405 | X | - | - | - | - | - | - | - | - |
| 3406 | X | - | - | - | - | - | - | - | - |
| 340 | - | - | - | - | - | X | - | - | - |
| 4448 | - | - | - | - | - | - | - | X | - |
| 3407 | - | - | - | - | - | X | - | - | - |
| 3408 | X | - | - | - | - | - | - | - | - |
| 3409 | X | - | - | - | - | - | - | - | - |
| 2883 | X | - | - | - | - | - | - | - | - |
| 2143 | X | - | - | - | - | - | - | - | - |
| 2784 | - | - | - | - | - | X | - | - | - |
| 3410 | X | - | - | - | - | - | - | - | - |
| 2486 | X | - | - | - | - | - | - | - | - |
| 3411 | X | - | - | - | - | - | - | - | - |
| 3412 | X | - | - | - | - | - | - | - | - |
| 3413 | - | - | - | - | - | X | - | - | - |
| 3414 | X | - | - | - | - | - | - | - | - |
| 3415 | X | - | - | - | - | - | - | - | - |
| 960 | - | - | - | - | X | - | - | - | - |
| 2360 | - | - | - | - | X | - | - | - | - |
| 3416 | - | - | - | - | X | - | - | - | - |
| 3417 | - | - | - | - | X | - | - | - | - |
| 3418 | - | - | - | - | X | - | - | - | - |
| 3419 | - | - | - | - | X | - | - | - | - |
| 3420 | - | - | - | - | X | - | - | - | - |
| 3421 | - | - | - | - | X | - | - | - | - |
| 3422 | - | - | - | - | X | - | - | - | - |
| 3423 | - | - | - | - | X | - | - | - | - |
| 3424 | - | - | - | - | X | - | - | - | - |
| 3425 | - | - | - | - | X | - | - | - | - |
| 3426 | - | - | - | - | X | - | - | - | - |
| 3427 | - | - | - | - | X | - | - | - | - |
| 719 | - | - | - | - | X | - | - | - | - |
| 7074 | - | - | - | - | - | - | - | X | - |
| 3428 | - | - | - | - | - | X | - | - | - |
| 3429 | - | - | - | - | - | X | - | - | - |
| 3430 | - | - | - | - | - | X | - | - | - |
| 5684 | - | - | - | - | - | - | - | X | - |
| 3431 | X | - | - | - | - | - | - | - | - |

(continued)

| SEQ ID | A | B | C | D | E | F | G | H | I |
|--------|---|---|---|---|---|---|---|---|---|
| 3432 | X | - | - | - | - | - | - | - | - |
| 3433 | X | - | - | - | - | - | - | - | - |
| 3434 | X | - | - | - | - | - | - | - | - |
| 414 | X | - | - | - | - | - | - | - | - |
| 3435 | X | - | - | - | - | - | - | - | - |
| 3436 | - | - | - | - | X | - | - | - | - |
| 3437 | - | - | - | - | X | - | - | - | - |
| 7569 | - | - | - | - | - | - | - | X | - |
| 4498 | - | - | - | - | - | - | - | X | - |
| 3439 | X | - | - | - | - | - | - | - | - |
| 3440 | X | - | - | - | - | - | - | - | - |
| 3441 | - | - | - | - | X | - | - | - | - |
| 3442 | - | - | - | - | - | X | - | - | - |
| 828 | - | - | - | - | - | X | - | - | - |
| 3443 | - | - | - | - | - | X | - | - | - |
| 3444 | - | - | - | - | - | X | - | - | - |
| 7576 | - | X | - | - | - | - | - | - | - |
| 3446 | X | - | - | - | - | - | - | - | - |
| 2506 | - | - | - | - | X | - | - | - | - |
| 3447 | - | - | - | - | X | - | - | - | - |
| 4885 | - | - | - | - | - | - | - | X | - |
| 3448 | - | - | - | - | - | X | - | - | - |
| 3449 | - | - | - | - | - | X | - | - | - |
| 3450 | - | - | - | - | - | X | - | - | - |
| 3451 | - | - | - | - | - | x | - | - | - |
| 3452 | - | - | - | - | - | X | - | - | - |
| 3453 | - | - | - | - | - | X | - | - | - |
| 3454 | - | - | - | - | X | - | - | - | - |
| 3085 | - | - | - | - | X | - | - | - | - |
| 3455 | X | - | - | - | - | - | - | - | - |
| 3456 | X | - | - | - | - | - | - | - | - |
| 3457 | X | - | - | - | - | - | - | - | - |
| 3458 | X | - | - | - | - | - | - | - | - |
| 3459 | - | - | - | - | - | X | - | - | - |
| 3460 | - | - | - | - | - | X | - | - | - |
| 4816 | - | - | - | - | - | - | - | X | - |
| 3461 | - | - | - | - | - | X | - | - | - |
| 3462 | - | - | - | - | - | X | - | - | - |
| 3463 | - | - | - | - | - | X | - | - | - |
| 3464 | - | - | - | - | - | X | - | - | - |
| 3465 | - | - | - | - | - | X | - | - | - |
| 3466 | - | - | - | - | - | X | - | - | - |
| 4487 | - | - | - | - | - | - | - | X | - |
| 3467 | X | - | - | - | - | - | - | - | - |
| 3468 | X | - | - | - | - | - | - | - | - |
| 3469 | X | - | - | - | - | - | - | - | - |
| 3470 | X | - | - | - | - | - | - | - | - |
| 3471 | X | - | - | - | - | - | - | - | - |
| 3472 | - | - | - | - | X | - | - | - | - |
| 3473 | - | - | - | - | X | - | - | - | - |

(continued)

| SEQ ID | A | B | C | D | E | F | G | H | I |
|--------|---|---|---|---|---|---|---|---|---|
| 2041 | - | - | - | - | X | - | - | - | - |
| 3474 | - | - | - | - | X | - | - | - | - |
| 3475 | X | - | - | - | - | - | - | - | - |
| 3476 | X | - | - | - | - | - | - | - | - |
| 7607 | - | - | - | - | - | - | - | - | X |
| 3477 | X | - | - | - | - | - | - | - | - |
| 3478 | X | - | - | - | - | - | - | - | - |
| 2172 | - | - | - | - | - | X | - | - | - |
| 3479 | - | - | - | - | X | - | - | - | - |
| 2402 | - | - | - | - | X | - | - | - | - |
| 3480 | X | - | - | - | - | - | - | - | - |
| 3481 | X | - | - | - | - | - | - | - | - |
| 3482 | X | - | - | - | - | - | - | - | - |
| 3483 | X | - | - | - | - | - | - | - | - |
| 7615 | - | - | - | - | - | - | - | X | - |
| 3485 | - | - | - | - | X | - | - | - | - |
| 3486 | - | - | - | - | X | - | - | - | - |
| 3487 | - | - | - | - | - | X | - | - | - |
| 3488 | - | - | - | - | - | X | - | - | - |
| 3489 | - | - | - | - | - | X | - | - | - |
| 207 | X | - | - | - | - | - | - | - | - |
| 3490 | X | - | - | - | - | - | - | - | - |
| 3491 | X | - | - | - | - | - | - | - | - |
| 3492 | X | - | - | - | - | - | - | - | - |
| 3493 | X | - | - | - | - | - | - | - | - |
| 3494 | - | - | - | - | - | X | - | - | - |
| 3495 | - | - | - | - | - | X | - | - | - |
| 3496 | - | - | - | - | - | X | - | - | - |
| 3497 | - | - | - | - | - | X | - | - | - |
| 3498 | - | - | - | - | - | X | - | - | - |
| 5573 | - | - | - | - | - | - | - | X | - |
| 4148 | - | - | - | - | - | - | - | X | - |
| 4897 | - | - | - | - | - | - | - | X | - |
| 3499 | - | - | - | - | - | X | - | - | - |
| 3500 | - | - | - | - | - | X | - | - | - |
| 3501 | - | - | - | - | X | - | - | - | - |
| 3502 | - | - | - | - | X | - | - | - | - |
| 3101 | - | - | - | - | X | - | - | - | - |
| 282 | - | - | - | - | X | - | - | - | - |
| 3503 | - | - | - | - | X | - | - | - | - |
| 3504 | - | - | - | - | X | - | - | - | - |
| 3505 | - | - | - | - | X | - | - | - | - |
| 3506 | X | - | - | - | - | - | - | - | - |
| 7637 | - | - | - | - | - | - | - | X | X |
| 3507 | X | - | - | - | - | - | - | - | - |
| 3508 | X | - | - | - | - | - | - | - | - |
| 7062 | - | - | - | - | - | - | - | X | - |
| 3509 | - | - | - | - | - | X | - | - | - |
| 3510 | X | - | - | - | - | - | - | - | - |
| 785 | X | - | - | - | - | - | - | - | - |

(continued)

| SEQ ID | A | B | C | D | E | F | G | H | I |
|--------|---|---|---|---|---|---|---|---|---|
| 471 | X | - | - | - | - | - | - | - | - |
| 1783 | X | - | - | - | - | - | - | - | - |
| 3511 | X | - | - | - | - | - | - | - | - |
| 3512 | X | - | - | - | - | - | - | - | - |
| 3513 | X | - | - | - | - | - | - | - | - |
| 3514 | X | - | - | - | - | - | - | - | - |
| 3515 | X | - | - | - | - | - | - | - | - |
| 3516 | - | - | - | - | - | X | - | - | - |
| 3517 | - | - | - | - | - | X | - | - | - |
| 3518 | - | - | - | - | X | - | - | - | - |
| 3519 | X | - | - | - | - | - | - | - | - |
| 3520 | X | - | - | - | - | - | - | - | - |
| 3521 | X | - | - | - | - | - | - | - | - |
| 3522 | X | - | - | - | - | - | - | - | - |
| 4186 | - | - | - | - | - | - | - | X | - |
| 3523 | X | - | - | - | - | - | - | - | - |
| 3524 | X | - | - | - | - | - | - | - | - |
| 3525 | - | - | - | - | - | X | - | - | - |
| 3526 | X | - | - | - | - | - | - | - | - |
| 3527 | X | - | - | - | - | - | - | - | - |
| 3528 | X | - | - | - | - | - | - | - | - |
| 3529 | X | - | - | - | - | - | - | - | - |
| 3530 | X | - | - | - | - | - | - | - | - |
| 3531 | X | - | - | - | - | - | - | - | - |
| 3532 | X | - | - | - | - | - | - | - | - |
| 3533 | X | - | - | - | - | - | - | - | - |
| 3534 | X | - | - | - | - | - | - | - | - |
| 3535 | X | - | - | - | - | - | - | - | - |
| 3536 | - | - | - | - | - | X | - | - | - |
| 3537 | X | - | - | - | - | - | - | - | - |
| 3538 | X | - | - | - | - | - | - | - | - |
| 2235 | - | - | - | - | X | - | - | - | - |
| 3539 | X | - | - | - | - | - | - | - | - |
| 3540 | X | - | - | - | - | - | - | - | - |
| 3541 | X | - | - | - | - | - | - | - | - |
| 3542 | - | - | - | - | - | X | - | - | - |
| 3543 | - | - | - | - | - | X | - | - | - |
| 3544 | - | - | - | - | - | X | - | - | - |
| 2540 | - | - | - | - | - | X - | - | - | - |
| 3545 | - | - | - | - | - | X | - | - | - |
| 1098 | - | - | - | - | - | X | - | - | - |
| 3546 | X | - | - | - | - | - | - | - | - |
| 3095 | X | - | - | - | - | - | - | - | - |
| 3547 | X | - | - | - | - | - | - | - | - |
| 3548 | - | - | - | - | X | - | - | - | - |
| 5148 | - | - | - | - | - | - | - | X | - |
| 3549 | - | - | - | - | - | X | - | - | - |
| 3550 | - | - | - | - | - | X | - | - | - |
| 3551 | X | - | - | - | - | - | - | - | - |
| 3552 | X | - | - | - | - | - | - | - | - |

(continued)

| SEQ ID | A | B | C | D | E | F | G | H | I |
|--------|---|---|---|---|---|---|---|---|---|
| 1042 | - | - | - | - | - | X | - | - | - |
| 3553 | - | - | - | - | - | X | - | - | - |
| 3554 | - | - | - | - | - | X | - | - | - |
| 7685 | - | - | X | - | - | - | - | - | - |
| 1927 | - | - | - | - | X | - | - | - | - |
| 3555 | - | - | - | - | X | - | - | - | - |
| 3556 | X | - | - | - | - | - | - | - | - |
| 1928 | - | - | - | - | - | X | - | - | - |
| 3557 | - | - | - | - | X | - | - | - | - |
| 3558 | - | - | - | - | X | - | - | - | - |
| 3559 | X | - | - | - | - | - | - | - | - |
| 3560 | - | - | - | - | - | X | - | - | - |
| 3561 | - | - | - | X | - | - | - | - | - |
| 3562 | X | - | - | - | - | - | - | - | - |
| 1856 | X | - | - | - | - | - | - | - | - |
| 3563 | X | - | - | - | - | - | - | - | - |
| 3564 | X | - | - | - | - | - | - | - | - |
| 3565 | X | - | - | - | - | - | - | - | - |
| 3566 | X | - | - | - | - | - | - | - | - |
| 3567 | X | - | - | - | - | - | - | - | - |
| 3568 | X | - | - | - | - | - | - | - | - |
| 3569 | X | - | - | - | - | - | - | - | - |
| 1977 | X | - | - | - | - | - | - | - | - |
| 2025 | X | - | - | - | - | - | - | - | - |
| 3570 | - | - | - | - | - | X | - | - | - |
| 3571 | - | - | - | - | - | X | - | - | - |
| 3572 | - | - | - | - | - | X | - | - | - |
| 1929 | - | - | - | - | - | X | - | - | - |
| 3573 | - | - | - | - | - | X | - | - | - |
| 3574 | X | - | - | - | - | - | - | - | - |
| 3575 | - | - | - | - | X | - | - | - | - |
| 3576 | X | - | - | - | - | - | - | - | - |
| 3577 | - | - | - | X | - | - | - | - | - |
| 3578 | X | - | - | - | - | - | - | - | - |
| 3579 | X | - | - | - | - | - | - | - | - |
| 3580 | - | - | - | - | - | X | - | - | - |
| 3581 | - | - | - | - | - | X | - | - | - |
| 3582 | - | - | - | - | - | X | - | - | - |
| 3583 | - | - | - | - | - | X | - | - | - |
| 3584 | - | - | - | - | x | - | - | - | - |
| 3585 | - | - | - | - | X | - | - | - | - |
| 3586 | - | - | - | - | X | - | - | - | - |
| 3587 | - | - | - | - | X | - | - | - | - |
| 3588 | X | - | - | - | - | - | - | - | - |
| 3589 | X | - | - | - | - | - | - | - | - |
| 3590 | - | - | - | - | X | - | - | - | - |
| 1934 | - | - | - | - | - | X | - | - | - |
| 3591 | X | - | - | - | - | - | - | - | - |
| 3592 | X | - | - | - | - | - | - | - | - |
| 759 | X | - | - | - | - | - | - | - | - |

(continued)

| SEQ ID | A | B | C | D | E | F | G | H | I |
|---|---|---|---|---|---|---|---|---|---|
| 3593 | X | - | - | - | - | - | - | - | - |
| 3594 | - | - | - | - | - | X | - | - | - |
| 3595 | X | - | - | - | - | - | - | - | - |
| 3596 | - | - | - | - | - | X | - | - | - |
| 3597 | - | - | - | - | - | X | - | - | - |
| 3598 | - | - | - | - | - | X | - | - | - |
| 4577 | - | - | - | - | - | - | - | X | - |
| 371 | - | - | - | - | X | - | - | - | - |
| 2231 | - | - | - | - | X | - | - | - | - |
| 3599 | - | - | - | - | X | - | - | - | - |
| 3600 | - | - | - | - | X | - | - | - | - |
| 3601 | - | - | - | - | X | - | - | - | - |
| 3602 | - | - | - | - | X | - | - | - | - |
| 4851 | - | - | - | - | - | - | - | X | - |
| 3603 | - | - | - | - | X | - | - | - | - |
| 6301 | - | X | - | - | - | - | - | X | - |
| 3604 | - | - | - | - | - | X | - | - | - |
| 3605 | - | - | - | - | - | X | - | - | - |
| 3606 | - | - | - | - | - | X | - | - | - |
| 3607 | X | - | - | - | - | - | - | - | - |
| 3608 | X | - | - | - | - | - | - | - | - |
| 3609 | X | - | - | - | - | - | - | - | - |
| 3610 | X | - | - | - | - | - | - | - | - |
| 2352 | X | - | - | - | - | - | - | - | - |
| 3611 | X | - | - | - | - | - | - | - | - |
| 3612 | - | - | - | - | X | - | - | - | - |
| 3613 | - | - | - | - | - | X | - | - | - |
| 1854 | - | - | - | - | - | X | - | - | - |
| 3614 | - | - | - | - | - | X | - | - | - |
| 3615 | - | - | - | - | - | X | - | - | - |
| 3616 | - | - | - | - | - | X | - | - | - |
| 3617 | - | - | - | X | - | - | - | - | - |
| 6070 | - | - | - | - | - | - | - | X | - |
| 3618 | X | - | - | - | - | - | - | - | - |
| 3619 | - | - | - | - | - | X | - | - | - |
| 3620 | - | - | - | - | - | X | - | - | - |
| 3621 | - | - | - | - | - | X | - | - | - |
| 3622 | - | - | - | - | - | X | - | - | - |
| 3623 | - | - | - | - | - | X | - | - | - |
| 3624 | - | - | - | - | - | X | - | - | - |
| 3625 | - | - | - | - | - | X | - | - | - |
| 3626 | - | - | - | - | - | X | - | - | - |
| 3627 | - | - | - | - | - | X | - | - | - |
| 3628 | - | - | - | - | - | X | - | - | - |
| 3629 | - | - | - | - | - | X | - | - | - |
| 3630 | - | - | - | - | - | X | - | - | - |
| 3631 | X | - | - | - | - | - | - | - | - |
| 381 | X | - | - | - | - | - | - | - | - |
| 2706 | X | - | - | - | - | - | - | - | - |
| 3632 | X | - | - | - | - | - | - | - | - |

(continued)

| SEQ ID | A | B | C | D | E | F | G | H | I |
|--------|---|---|---|---|---|---|---|---|---|
| 3633 | X | - | - | - | - | - | - | - | - |
| 3634 | X | - | - | - | - | - | - | - | - |
| 7766 | - | - | - | - | - | - | - | X | - |
| 3636 | X | - | - | - | - | - | - | - | - |
| 3637 | X | - | - | - | - | - | - | - | - |
| 2416 | X | - | - | - | - | - | - | - | - |
| 3638 | X | - | - | - | - | - | - | - | - |
| 3639 | X | - | - | - | - | - | - | - | - |
| 3640 | X | - | - | - | - | - | - | - | - |
| 3641 | - | - | - | - | - | X | - | - | - |
| 3642 | - | - | - | - | - | X | - | - | - |
| 3643 | - | - | - | - | - | X | - | - | - |
| 3644 | X | - | - | - | - | - | - | - | - |
| 3645 | X | - | - | - | - | - | - | - | - |
| 3646 | - | - | - | - | - | X | - | - | - |
| 3647 | - | - | - | - | - | X | - | - | - |
| 3648 | - | - | - | - | - | X | - | - | - |
| 3649 | X | - | - | - | - | - | - | - | - |
| 3650 | - | - | - | - | - | X | - | - | - |
| 3651 | X | - | - | - | - | - | - | - | - |
| 4515 | - | - | - | - | - | - | - | X | - |
| 7155 | - | - | - | - | - | - | - | X | - |
| 3652 | - | - | - | - | X | - | - | - | - |
| 3653 | - | - | - | - | X | - | - | - | - |
| 3654 | - | - | - | - | - | X | - | - | - |
| 870 | - | - | - | - | - | X | - | - | - |
| 3655 | - | - | - | - | - | x | - | - | - |
| 3656 | - | - | - | - | - | X | - | - | - |
| 3657 | - | - | - | - | - | X | - | - | - |
| 3658 | - | - | - | - | - | X | - | - | - |
| 1423 | - | - | - | X | - | - | - | - | - |
| 2327 | - | - | - | - | X | - | - | - | - |
| 3659 | X | - | - | - | - | - | - | - | - |
| 3660 | X | - | - | - | - | - | - | - | - |
| 3661 | X | - | - | - | - | - | - | - | - |
| 3662 | - | - | - | - | - | X | - | - | - |
| 3663 | - | - | - | - | - | X | - | - | - |
| 3664 | - | - | - | - | - | X | - | - | - |
| 3665 | - | - | - | - | - | X | - | - | - |
| 3666 | X | - | - | - | - | - | - | - | - |
| 3667 | - | - | - | - | X | - | - | - | - |
| 3668 | X | - | - | - | - | - | - | - | - |
| 3669 | - | - | - | - | - | X | - | - | - |
| 3670 | - | - | - | - | - | X | - | - | - |
| 3671 | - | - | - | - | - | X | - | - | - |
| 3672 | - | - | - | - | - | X | - | - | - |
| 3673 | X | - | - | - | - | - | - | - | - |
| 3674 | X | - | - | - | - | - | - | - | - |
| 3675 | X | - | - | - | - | - | - | - | - |
| 3676 | X | - | - | - | - | - | - | - | - |

(continued)

| SEQ ID | A | B | C | D | E | F | G | H | I |
|--------|---|---|---|---|---|---|---|---|---|
| 3677 | X | - | - | - | - | - | - | - | - |
| 3678 | X | - | - | - | - | - | - | - | - |
| 2104 | - | - | - | X | | - | - | - | - |
| 3679 | - | - | - | - | - | X | - | - | - |
| 3680 | - | - | - | - | X | - | - | - | - |
| 7812 | - | - | X | - | - | - | - | - | - |
| 3682 | X | - | - | - | - | - | - | - | - |
| 3683 | X | - | - | - | - | - | - | - | - |
| 3684 | - | - | - | - | - | X | - | - | - |
| 3685 | - | - | - | - | - | X | - | - | - |
| 3686 | - | - | - | - | - | X | - | | - |
| 3687 | X | - | - | - | - | - | - | - | - |
| 3688 | X | - | - | - | - | - | - | - | - |
| 3689 | - | - | - | - | - | X | - | - | - |
| 3690 | - | - | - | - | - | X | - | - | - |
| 3691 | - | - | - | - | X | - | - | - | - |
| 7223 | - | - | - | - | - | - | - | X | - |
| 3692 | X | - | - | - | - | - | - | - | - |
| 1816 | X | - | - | X | - | - | - | - | - |
| 3693 | X | - | - | - | - | - | - | - | - |
| 4542 | - | - | - | - | - | - | - | X | - |
| 7825 | - | - | - | - | - | - | - | X | - |
| 3695 | - | - | - | - | - | X | - | - | - |
| 3696 | - | - | - | - | - | X | - | - | - |
| 3697 | - | - | - | - | - | X | - | - | - |
| 3698 | - | - | - | - | - | X | - | - | - |
| 3699 | - | - | - | - | - | X | - | - | - |
| 3700 | - | - | - | - | X | - | - | - | - |
| 3701 | - | - | - | - | - | X | - | - | - |
| 3702 | - | - | - | - | - | X | - | - | - |
| 3703 | - | - | - | - | - | X | - | - | - |
| 2012 | - | - | - | - | - | X | - | - | - |
| 3704 | - | - | - | - | - | X | - | - | - |
| 3705 | - | - | - | - | - | X | - | - | - |
| 3706 | - | - | - | - | - | X | - | - | - |
| 3707 | X | - | - | - | - | - | - | - | - |
| 1605 | - | - | - | X | - | - | - | - | - |
| 3708 | X | - | - | - | - | - | - | - | - |
| 3709 | X | - | - | - | - | - | - | - | - |
| 3710 | X | - | - | - | - | - | - | - | - |
| 3711 | X | - | - | - | - | - | - | - | - |
| 6564 | - | - | - | - | - | - | - | X | - |
| 3712 | X | - | - | - | - | - | - | - | - |
| 3713 | - | - | - | - | X | - | - | - | - |
| 3714 | - | - | - | - | X | - | - | - | - |
| 3715 | - | - | - | - | X | - | - | - | - |
| 3716 | - | - | - | - | X | - | - | - | - |
| 3717 | - | - | - | - | X | - | - | - | - |
| 3718 | - | - | - | - | - | X | - | - | - |
| 3719 | - | - | - | - | - | X | - | - | - |

63

(continued)

| SEQ ID | A | B | C | D | E | F | G | H | I |
|--------|---|---|---|---|---|---|---|---|---|
| 3720 | - | - | - | - | - | X | - | - | - |
| 3721 | - | - | - | - | - | X | - | - | - |
| 3158 | - | - | - | - | X | - | - | - | - |
| 3722 | - | - | - | - | - | X | - | - | - |
| 85 | - | - | - | - | - | X | - | - | - |
| 287 | - | - | - | - | - | X | - | - | - |
| 2476 | - | - | - | - | - | X | - | - | - |
| 3723 | - | - | - | - | X | - | - | - | - |
| 3724 | - | - | - | - | X | - | - | - | - |
| 3725 | - | - | - | - | X | - | - | - | - |
| 3726 | - | - | - | - | X | - | - | - | - |
| 3727 | X | - | - | - | - | - | - | - | - |
| 3728 | X | - | - | - | - | - | - | - | - |
| 3729 | X | - | - | - | - | - | - | - | - |
| 5625 | - | - | - | - | - | - | - | X | - |
| 1393 | X | - | - | - | - | - | - | - | - |
| 3730 | X | - | - | - | - | - | - | - | - |
| 3731 | X | - | - | - | - | - | - | - | - |
| 3732 | X | - | - | - | - | - | - | - | - |
| 3733 | X | - | - | - | - | - | - | - | - |
| 1413 | X | - | - | - | - | - | - | - | - |
| 3734 | - | - | - | - | - | X | - | - | - |
| 3735 | - | - | - | - | - | X | - | - | - |
| 3736 | - | - | - | - | - | X | - | - | - |
| 436 | - | - | - | - | - | X | - | - | - |
| 3737 | - | - | - | - | - | X | - | - | - |
| 2453 | - | - | - | - | - | X | - | - | - |
| 2821 | - | - | - | - | - | X | - | - | - |
| 3738 | - | - | - | - | - | X | - | - | - |
| 3739 | - | - | - | - | - | X | - | - | - |
| 3740 | X | - | - | - | - | X | - | - | - |
| 3741 | - | - | - | - | - | X | - | - | - |
| 3144 | - | - | - | - | - | X | - | - | - |
| 3742 | - | - | - | - | - | X | - | - | - |
| 3743 | - | - | - | - | - | X | - | - | - |
| 3744 | - | - | - | - | X | - | - | - | - |
| 3745 | - | - | - | - | - | X | - | - | - |
| 3746 | - | - | - | - | - | X | - | - | - |
| 226 | - | - | - | - | - | X | - | - | - |
| 3747 | X | - | - | - | - | - | - | - | - |
| 3748 | - | - | - | - | - | X | - | - | - |
| 1087 | X | - | - | - | - | - | - | - | - |
| 3749 | X | - | - | - | - | - | - | - | - |
| 3750 | X | - | - | - | - | - | - | - | - |
| 3751 | X | - | - | - | - | - | - | - | - |
| 3752 | - | - | - | - | X | - | - | - | - |
| 3753 | - | - | - | - | X | - | - | - | - |
| 3754 | X | - | - | - | - | - | - | - | - |
| 3755 | X | - | - | - | - | - | - | - | - |
| 3756 | X | - | - | - | - | - | - | - | - |

(continued)

| SEQ ID | A | B | C | D | E | F | G | H | I |
|--------|---|---|---|---|---|---|---|---|---|
| 3757 | X | - | - | - | - | - | - | - | - |
| 3758 | X | - | - | - | - | - | - | - | - |
| 4870 | - | - | - | - | - | - | - | X | - |
| 7890 | - | - | - | - | - | - | - | X | - |
| 3760 | X | - | - | - | - | - | - | - | - |
| 3761 | X | - | - | - | - | - | - | - | - |
| 3762 | - | - | - | - | X | - | - | - | - |
| 3763 | - | - | - | - | X | - | - | - | - |
| 3764 | - | - | - | - | X | - | - | - | - |
| 4279 | - | - | - | - | - | - | - | X | - |
| 3765 | X | - | - | - | - | - | - | - | - |
| 3766 | X | - | - | - | - | - | - | - | - |
| 3767 | X | - | - | - | - | - | - | - | - |
| 3768 | X | - | - | - | - | - | - | - | - |
| 3769 | X | - | - | - | - | - | - | - | - |
| 3770 | - | - | - | - | - | X | - | - | - |
| 3771 | - | - | - | - | X | - | - | - | - |
| 3772 | X | - | - | - | - | - | - | - | - |
| 3773 | X | - | - | - | - | - | - | - | - |
| 3774 | X | - | - | - | - | - | - | - | - |
| 3775 | X | - | - | - | - | - | - | - | - |
| 1310 | X | - | - | - | - | - | - | - | - |
| 1562 | X | - | - | - | - | - | - | - | - |
| 3776 | X | - | - | - | - | - | - | - | - |
| 3777 | X | - | - | - | - | - | - | - | - |
| 3778 | X | - | - | - | - | - | - | - | - |
| 3779 | X | - | - | - | - | - | - | - | - |
| 3780 | - | - | - | - | - | X | - | - | - |
| 3781 | - | - | - | - | - | X | - | - | - |
| 945 | - | - | - | X | - | - | - | - | - |
| 5076 | - | - | - | - | - | - | X | X | - |
| 3782 | X | - | - | - | - | - | - | - | - |
| 3783 | X | - | - | - | - | - | - | - | - |
| 3784 | X | - | - | - | - | - | - | - | - |
| 3785 | X | - | - | - | - | - | - | - | - |
| 3786 | - | - | - | - | X | - | - | - | - |
| 3787 | - | - | - | - | X | - | - | - | - |
| 3788 | - | - | - | - | X | - | - | - | - |
| 3789 | - | - | - | - | - | X | - | - | - |
| 3790 | - | - | - | - | X | - | - | - | - |
| 4963 | - | - | - | - | - | - | - | X | - |
| 3791 | - | - | - | - | - | X | - | - | - |
| 3792 | - | - | - | - | - | X | - | - | - |
| 3793 | - | - | - | - | - | X | - | - | - |
| 3794 | X | - | - | - | - | - | - | - | - |
| 3795 | - | - | - | - | X | - | - | - | - |
| 3796 | - | - | - | - | X | - | - | - | - |
| 3797 | - | - | - | - | X | - | - | - | - |
| 3798 | - | - | - | - | X | - | - | - | - |
| 3799 | X | - | - | - | - | - | - | - | - |

(continued)

| SEQ ID | A | B | C | D | E | F | G | H | I |
|--------|---|---|---|---|---|---|---|---|---|
| 3800 | X | - | - | - | - | - | - | - | - |
| 3801 | - | - | - | - | - | X | - | - | - |
| 2009 | - | - | - | - | - | X | - | - | - |
| 404 | - | - | - | - | - | X | - | - | - |
| 3802 | - | - | - | - | - | X | - | - | - |
| 3803 | - | - | - | - | - | X | - | - | - |
| 4790 | - | - | - | - | - | - | - | X | - |
| 2740 | - | - | - | - | - | X | - | - | - |
| 3804 | - | - | - | - | - | X | - | - | - |
| 4671 | - | - | - | - | - | - | - | X | - |
| 3805 | X | - | - | - | - | - | - | - | - |
| 4600 | - | - | - | - | - | - | - | X | - |
| 3806 | X | - | - | - | - | - | - | - | - |
| 569 | X | - | - | - | - | - | - | - | - |
| 2760 | X | - | - | - | - | - | - | - | - |
| 3807 | X | - | - | - | - | - | - | - | - |
| 3808 | X | - | - | - | - | - | - | - | - |
| 3809 | - | - | - | - | - | X | - | - | - |
| 3810 | - | - | - | - | - | X | - | - | - |
| 7942 | - | - | X | - | - | - | - | - | - |
| 3812 | - | - | - | - | - | X | - | - | - |
| 3813 | - | - | - | - | X | - | - | - | - |
| 3814 | - | - | - | - | X | - | - | - | - |
| 3815 | - | - | - | - | X | - | - | - | - |
| 3816 | - | - | - | - | - | X | - | - | - |
| 56 | - | - | - | - | - | X | - | - | - |
| 3817 | - | - | - | - | - | X | - | - | - |
| 3818 | - | - | - | - | - | X | - | - | - |
| 3819 | - | - | - | - | - | X | - | - | - |
| 156 | - | - | - | - | - | X | - | - | - |
| 3820 | - | - | - | - | - | X | - | - | - |
| 3821 | X | - | - | - | - | - | - | - | - |
| 3822 | X | - | - | - | - | - | - | - | - |
| 3823 | - | - | - | - | X | - | - | - | - |
| 3824 | X | - | - | - | - | - | - | - | - |
| 3825 | X | - | - | - | - | - | - | - | - |
| 3826 | X | - | - | - | - | - | - | - | - |
| 3827 | - | - | - | - | - | X | - | - | - |
| 3828 | - | - | - | - | - | X | - | - | - |
| 3829 | X | - | - | - | - | - | - | - | - |
| 3830 | X | - | - | - | - | - | - | - | - |
| 3831 | X | - | - | - | - | - | - | - | - |
| 3832 | - | - | - | - | - | X | - | - | - |
| 3833 | - | - | - | - | - | X | - | - | - |
| 3834 | - | - | - | - | - | X | - | - | - |
| 3835 | - | - | - | - | - | X | - | - | - |
| 1147 | - | - | - | - | X | - | - | - | - |
| 5278 | - | X | - | - | - | - | - | X | - |
| 3836 | - | - | - | - | X | - | - | - | - |
| 3837 | - | - | - | - | X | - | - | - | - |

(continued)

| SEQ ID | A | B | C | D | E | F | G | H | I |
|--------|---|---|---|---|---|---|---|---|---|
| 3838 | - | - | - | - | X | - | - | - | - |
| 394 | X | - | - | - | - | - | - | - | - |
| 3839 | X | - | - | - | - | - | - | - | - |
| 3840 | - | - | - | - | X | - | - | - | - |
| 3841 | - | - | - | - | X | - | - | - | - |
| 5606 | - | - | - | - | - | - | - | X | - |
| 3842 | X | - | - | - | - | - | - | - | - |
| 3843 | X | - | - | - | - | - | - | - | - |
| 3844 | X | - | - | - | - | - | - | - | - |
| 3845 | - | - | - | - | - | X | - | - | - |
| 3846 | - | - | - | - | - | X | - | - | - |
| 4399 | - | - | - | - | - | - | - | X | - |
| 3847 | X | - | - | - | - | - | - | - | - |
| 1678 | X | - | - | - | - | - | - | - | - |
| 1313 | X | - | - | - | - | - | - | - | - |
| 3848 | X | - | - | - | - | - | - | - | - |
| 3849 | X | - | - | - | - | - | - | - | - |
| 3850 | X | - | - | - | - | - | - | - | - |
| 3851 | X | - | - | - | - | - | - | - | - |
| 3852 | X | - | - | - | - | - | - | - | - |
| 3853 | X | - | - | - | - | - | - | - | - |
| 3854 | X | - | - | - | - | - | - | - | - |
| 3855 | X | - | - | - | - | - | - | - | - |
| 3856 | X | - | - | - | - | - | - | - | - |
| 3857 | X | - | - | - | - | - | - | - | - |
| 7989 | - | - | - | - | - | - | - | - | X |
| 3859 | - | - | - | - | - | X | - | - | - |
| 1053 | - | - | - | - | - | X | - | - | - |
| 3860 | X | - | - | - | - | - | - | - | - |
| 3861 | - | - | - | - | - | X | - | - | - |
| 4665 | - | - | - | - | - | - | - | X | - |
| 3862 | - | - | - | - | - | X | - | - | - |
| 3863 | - | - | - | - | - | X | - | - | - |
| 3864 | - | - | - | - | - | X | - | - | - |
| 3865 | - | - | - | - | - | X | - | - | - |
| 4614 | - | - | - | - | - | - | - | X | - |
| 3866 | X | - | - | - | - | - | - | - | - |
| 3867 | - | - | - | - | X | - | - | - | - |
| 3868 | - | - | - | - | X | - | - | - | - |
| 3869 | X | - | - | - | - | - | - | - | - |
| 2182 | - | - | - | - | - | X | - | - | - |
| 3870 | X | - | - | - | - | - | - | - | - |
| 3871 | X | - | - | - | - | - | - | - | - |
| 3872 | X | - | - | - | - | - | - | - | - |
| 3873 | X | - | - | - | - | - | - | - | - |
| 3874 | X | - | - | - | - | - | - | - | - |
| 5528 | - | - | - | - | - | - | - | X | - |
| 3875 | - | - | - | - | - | X | - | - | - |
| 3876 | - | - | - | - | - | X | - | - | - |
| 2414 | - | - | - | - | - | X | - | - | - |

(continued)

| SEQ ID | A | B | C | D | E | F | G | H | I |
|--------|---|---|---|---|---|---|---|---|---|
| 4715 | - | - | - | - | - | - | - | X | - |
| 3877 | - | - | - | - | - | X | - | - | - |
| 3878 | X | - | - | - | - | - | - | - | - |
| 3879 | X | - | - | - | - | - | - | - | - |
| 3880 | - | - | - | - | - | X | - | - | - |
| 4998 | - | - | - | - | - | - | - | X | - |
| 3881 | - | - | - | - | - | X | - | - | - |
| 3882 | - | - | - | - | - | X | - | - | - |
| 3883 | - | - | - | - | - | X | - | - | - |
| 3884 | - | - | - | - | X | - | - | - | - |
| 3885 | X | - | - | - | - | - | - | - | - |
| 3886 | X | - | - | - | - | - | - | - | - |
| 3887 | X | - | - | - | - | - | - | - | - |
| 3888 | X | - | - | - | - | - | - | - | - |
| 3889 | X | - | - | - | - | - | - | - | - |
| 3890 | - | - | - | - | - | X | - | - | - |
| 6524 | - | - | - | - | - | - | - | X | - |
| 3891 | X | - | - | - | - | - | - | - | - |
| 3892 | X | - | - | - | - | - | - | - | - |
| 3893 | X | - | - | - | - | - | - | - | - |
| 3894 | - | - | - | - | - | X | - | - | - |
| 3895 | - | - | - | - | - | X | - | - | - |
| 2810 | - | - | - | - | - | X | - | - | - |
| 3896 | - | - | - | - | - | X | - | - | - |
| 3897 | - | - | - | - | - | X | - | - | - |
| 3898 | - | - | - | - | X | - | - | - | - |
| 3899 | X | - | - | - | - | - | - | - | - |
| 3900 | X | - | - | - | - | - | - | - | - |
| 3901 | - | - | - | - | X | - | - | - | - |
| 3902 | - | - | - | - | X | - | - | - | - |
| 1946 | - | - | - | - | X | - | - | - | - |
| 3903 | X | - | - | - | - | - | - | - | - |
| 3904 | X | - | - | - | - | - | - | - | - |
| 3905 | - | - | - | - | - | X | - | - | - |
| 3906 | - | - | - | - | - | X | - | - | - |
| 3907 | - | - | - | - | - | X | - | - | - |
| 3903 | - | - | - | - | - | X | - | - | - |
| 2703 | - | - | - | - | - | X | - | - | - |
| 1121 | - | - | - | X | - | - | - | - | - |
| 3909 | - | - | - | - | X | - | - | - | - |
| 3910 | - | - | - | - | X | - | - | - | - |
| 3911 | - | - | - | - | X | - | - | - | - |
| 3912 | - | - | - | - | X | - | - | - | - |
| 4 | - | - | - | - | - | X | - | - | - |
| 3913 | - | - | - | - | - | X | - | - | - |
| 3914 | - | - | - | - | - | X | - | - | - |
| 3113 | - | - | - | - | - | X | - | - | - |
| 3915 | - | - | - | - | - | X | - | - | - |
| 3916 | X | - | - | - | - | - | - | - | - |
| 3917 | - | - | - | - | - | X | - | - | - |

(continued)

| SEQ ID | A | B | C | D | E | F | G | H | I |
|--------|---|---|---|---|---|---|---|---|---|
| 3918 | X | - | - | - | - | - | - | - | - |
| 5518 | - | - | - | - | - | - | - | X | - |
| 3919 | - | - | - | - | - | X | - | - | - |
| 3920 | X | - | - | - | - | - | - | - | - |
| 3921 | X | - | - | - | - | - | - | - | - |
| 1521 | X | - | - | - | - | - | - | - | - |
| 3922 | - | - | - | - | - | X | - | - | - |
| 2893 | - | - | - | X | - | - | - | - | - |
| 7024 | - | - | - | - | - | - | X | X | - |
| 3923 | X | - | - | - | - | - | - | - | - |
| 3924 | X | - | - | - | - | - | - | - | - |
| 3925 | - | - | - | - | X | - | - | - | - |
| 3926 | - | - | - | - | X | - | - | - | - |
| 3927 | - | - | - | - | X | - | - | - | - |
| 3928 | - | - | - | - | X | - | - | - | - |
| 3929 | X | - | - | - | - | - | - | - | - |
| 3930 | X | - | - | - | - | - | - | - | - |
| 3931 | - | - | - | - | - | X | - | - | - |
| 3932 | - | - | - | - | X | - | - | - | - |
| 3933 | - | - | - | - | X | - | - | - | - |
| 3934 | - | - | - | - | X | - | - | - | - |
| 3935 | - | - | - | - | - | X | - | - | - |
| 3936 | - | - | - | - | - | X | - | - | - |
| 3937 | - | - | - | - | - | X | - | - | - |
| 3938 | - | - | - | - | - | X | - | - | - |
| 3939 | - | - | - | - | - | X | - | - | - |
| 8071 | - | X | - | - | - | - | - | - | - |
| 8072 | - | X | - | - | - | - | - | - | - |
| 4529 | - | - | - | - | - | - | - | X | - |
| 8073 | - | - | X | - | - | - | - | - | - |
| 3943 | X | - | - | - | - | - | - | - | - |
| 3944 | X | - | - | - | - | - | - | - | - |
| 3945 | X | - | - | - | - | - | - | - | - |
| 3946 | X | - | - | - | - | - | - | - | - |
| 3947 | X | - | - | - | - | - | - | - | - |
| 3948 | X | - | - | - | - | - | - | - | - |
| 3949 | X | - | - | - | - | - | - | - | - |
| 8080 | - | - | - | - | - | - | - | - | X |
| 488 | X | - | - | - | - | - | - | - | - |
| 1966 | X | - | - | - | - | - | - | - | - |
| 3950 | - | - | - | - | - | X | - | - | - |
| 1120 | - | - | - | - | - | X | - | - | - |
| 3951 | - | - | - | - | - | X | - | - | - |
| 815 | - | - | - | - | - | X | - | - | - |
| 3952 | - | - | - | - | - | X | - | - | - |
| 1359 | X | - | - | - | - | - | - | - | - |
| 3953 | X | - | - | - | - | - | - | - | - |
| 3954 | - | - | - | - | - | X | - | - | - |
| 3955 | X | - | - | - | - | - | - | - | - |
| 3956 | X | - | - | - | - | - | - | - | - |

(continued)

| SEQ ID | A | B | C | D | E | F | G | H | I |
|--------|---|---|---|---|---|---|---|---|---|
| 3957 | - | - | - | - | X | - | - | - | - |
| 3958 | - | - | - | - | X | - | - | - | - |
| 1114 | - | - | - | - | - | X | - | - | - |
| 3959 | - | - | - | - | - | X | - | - | - |
| 3960 | - | - | - | - | - | X | - | - | - |
| 3961 | - | - | - | - | - | X | - | - | - |
| 3962 | - | - | - | - | - | X | - | - | - |
| 3963 | - | - | - | - | - | X | - | - | - |
| 3964 | - | - | - | X | - | - | - | - | - |
| 3965 | - | - | - | - | - | X | - | - | - |
| 6045 | - | - | - | - | - | - | - | X | - |
| 3099 | - | - | - | - | - | X | - | - | - |
| 1806 | - | - | - | - | - | X | - | - | - |
| 3966 | - | - | - | - | - | X | - | - | - |
| 3967 | - | - | - | - | - | X | - | - | - |
| 3968 | - | - | - | - | - | X | - | - | - |
| 3969 | - | - | - | - | - | X | - | - | - |
| 3970 | - | - | - | - | - | X | - | - | - |
| 3971 | - | - | - | - | - | X | - | - | - |
| 3972 | - | - | - | - | - | X | - | - | - |
| 3973 | - | - | - | - | - | X | - | - | - |
| 3974 | X | - | - | - | - | - | - | - | - |
| 3975 | X | - | - | - | - | - | - | - | - |
| 3976 | X | - | - | - | - | - | - | - | - |
| 3977 | X | - | - | - | - | - | - | - | - |
| 3978 | X | - | - | - | - | - | - | - | - |
| 1829 | X | - | - | - | - | - | - | - | - |
| 3979 | - | - | - | - | - | X | - | - | - |
| 3980 | - | - | - | - | - | X | - | - | - |
| 3981 | - | - | - | - | X | - | - | - | - |
| 3982 | - | - | - | - | X | - | - | - | - |
| 3983 | - | - | - | - | X | - | - | - | - |
| 3984 | - | - | - | - | X | - | - | - | - |
| 3985 | - | - | - | - | X | - | - | - | - |
| 3986 | - | - | - | - | - | X | - | - | - |
| 3987 | X | - | - | - | - | - | - | - | - |
| 3988 | X | - | - | - | - | - | - | - | - |
| 3989 | X | - | - | - | - | - | - | - | - |
| 3990 | X | - | - | - | - | - | - | - | - |
| 3991 | X | - | - | - | - | - | - | - | - |
| 3992 | X | - | - | - | - | - | - | - | - |
| 3993 | - | - | - | - | - | X | - | - | - |
| 3994 | - | - | - | - | - | X | - | - | - |
| 2089 | X | - | - | - | - | - | - | - | - |
| 3995 | X | - | - | - | - | - | - | - | - |
| 3996 | X | - | - | - | - | - | - | - | - |
| 3997 | X | - | - | - | - | - | - | - | - |
| 3998 | X | - | - | - | - | - | - | - | - |
| 3999 | X | - | - | - | - | - | - | - | - |
| 2544 | - | - | - | X | - | - | - | - | - |

(continued)

| SEQ ID | A | B | C | D | E | F | G | H | I |
|--------|---|---|---|---|---|---|---|---|---|
| 4000 | X | - | - | - | - | - | - | - | - |
| 546 | X | - | - | - | - | - | - | - | - |
| 4001 | X | - | - | - | - | - | - | - | - |
| 4002 | X | - | - | - | - | - | - | - | - |
| 4003 | X | - | - | - | - | - | - | - | - |
| 4004 | X | - | - | - | - | - | - | - | - |
| 4005 | - | - | - | - | - | X | - | - | - |
| 8137 | - | - | X | - | - | - | - | - | - |
| 4007 | - | - | - | - | - | X | - | - | - |
| 4008 | - | - | - | - | X | - | - | - | - |
| 4009 | - | - | - | - | - | X | - | - | - |
| 4010 | - | - | - | - | - | X | - | - | - |
| 5683 | - | - | - | - | - | - | - | X | - |
| 4011 | - | - | - | - | - | X | - | - | - |
| 4012 | - | - | - | - | X | - | - | - | - |
| 4013 | - | - | - | - | - | X | - | - | - |
| 4014 | - | - | - | - | - | X | - | - | - |
| 4015 | - | - | - | - | - | X | - | - | - |
| 4016 | - | - | - | - | - | X | - | - | - |
| 4017 | - | - | - | - | - | X | - | - | - |
| 4018 | - | - | - | - | - | X | - | - | - |
| 4508 | - | - | - | - | - | - | - | X | - |
| 4019 | - | - | - | - | - | X | - | - | - |
| 856 | X | - | - | - | - | - | - | - | - |
| 4020 | X | - | - | - | - | - | - | - | - |
| 4021 | - | - | - | - | - | X | - | - | - |
| 4022 | - | - | - | - | X | - | - | - | - |
| 4023 | - | - | - | - | X | - | - | - | - |
| 4024 | X | - | - | - | - | - | - | - | - |
| 4025 | X | - | - | - | - | - | - | - | - |
| 4026 | - | - | - | - | X | - | - | - | - |
| 4027 | - | - | - | - | X | - | - | - | - |
| 4028 | - | - | - | - | X | - | - | - | - |
| 4029 | - | - | - | - | X | - | - | - | - |
| 4030 | - | - | - | - | - | X | - | - | - |
| 4031 | - | - | - | - | - | X | - | - | - |
| 4032 | - | - | - | - | X | - | - | - | - |
| 4033 | - | - | - | - | X | - | - | - | - |
| 6968 | - | - | - | - | - | - | - | X | - |
| 86 | - | - | - | - | - | X | - | - | - |
| 4034 | - | - | - | - | - | X | - | - | - |
| 4035 | - | - | - | - | - | X | - | - | - |
| 2419 | - | - | - | - | X | - | - | - | - |
| 4036 | - | - | - | - | X | - | - | - | - |
| 4037 | - | - | - | - | - | X | - | - | - |
| 4038 | - | - | - | - | - | X | - | - | - |
| 961 | X | - | - | - | - | - | - | - | - |
| 4039 | X | - | - | - | - | - | - | - | - |
| 4040 | X | - | - | - | - | - | - | - | - |
| 4041 | - | - | - | - | - | X | - | - | - |

(continued)

| SEQ ID | A | B | C | D | E | F | G | H | I |
|--------|---|---|---|---|---|---|---|---|---|
| 1617 | - | - | - | X | - | - | - | - | - |
| 4042 | X | - | - | - | - | - | - | - | - |
| 4043 | X | - | - | - | - | - | - | - | - |
| 1517 | X | - | - | - | - | - | - | - | - |
| 4044 | X | - | - | - | - | - | - | - | - |
| 4045 | - | - | - | - | - | X | - | - | - |
| 4046 | X | - | - | - | - | - | - | - | - |
| 4047 | X | - | - | - | - | - | - | - | - |
| 4048 | X | - | - | - | - | - | - | - | - |
| 4049 | X | - | - | - | - | - | - | - | - |
| 1815 | X | - | - | - | - | - | - | - | - |
| 2008 | X | - | - | - | - | - | - | - | - |
| 409 | X | - | - | - | - | - | - | - | - |
| 4050 | X | - | - | - | - | - | - | - | - |
| 4051 | - | - | - | - | - | X | - | - | - |
| 117 | X | - | - | - | - | - | - | - | - |
| 4052 | - | - | - | - | - | X | - | - | - |
| 224 | X | - | - | - | - | - | - | - | - |
| 4053 | X | - | - | - | - | - | - | - | - |
| 4054 | X | - | - | - | - | - | - | - | - |
| 4055 | X | - | - | - | - | - | - | - | - |
| 4056 | - | - | - | - | - | X | - | - | - |
| 4057 | - | - | - | - | - | X | - | - | - |
| 4783 | - | - | - | - | - | - | - | X | - |
| 4058 | X | - | - | - | - | - | - | - | - |
| 7242 | - | - | - | - | - | - | - | X | - |
| 4059 | X | - | - | - | - | - | - | - | - |
| 4060 | X | - | - | - | - | - | - | - | - |
| 406 | X | - | - | - | - | - | - | - | - |
| 6320 | - | - | - | - | - | - | - | X | - |
| 4062 | - | - | - | - | - | X | - | - | - |
| 8183 | - | - | X | - | - | - | - | - | - |
| 4063 | - | - | - | - | - | X | - | - | - |
| 4064 | - | - | - | - | - | X | - | - | - |
| 4065 | - | - | - | - | - | X | - | - | - |
| 4066 | - | - | - | - | - | X | - | - | - |
| 4067 | - | - | - | - | - | X | - | - | - |
| 4068 | - | - | - | - | - | X | - | - | - |
| 4069 | - | - | - | - | - | X | - | - | - |
| 1028 | - | - | - | - | X | - | - | - | - |
| 4070 | - | - | - | - | - | X | - | - | - |
| 4071 | - | - | - | - | - | X | - | - | - |
| 4072 | - | - | - | - | - | X | - | - | - |
| 4073 | - | - | - | - | - | X | - | - | - |
| 348 | - | - | - | - | X | - | - | - | - |
| 4074 | X | - | - | - | - | - | - | - | - |
| 4075 | X | - | - | - | - | - | - | - | - |
| 6142 | - | - | - | - | - | - | - | X | - |
| 4076 | - | - | - | - | - | X | - | - | - |
| 2911 | - | - | - | - | - | X | - | - | - |

(continued)

| SEQ ID | A | B | C | D | E | F | G | H | I |
|---|---|---|---|---|---|---|---|---|---|
| 4077 | - | - | - | - | - | X | - | - | - |
| 4078 | X | - | - | - | - | - | - | - | - |
| 4079 | X | - | - | - | - | - | - | - | - |
| 4080 | X | - | - | - | - | - | - | - | - |
| 4081 | X | - | - | - | - | - | - | - | - |
| 4082 | X | - | - | - | - | - | - | - | - |
| 2903 | X | - | - | - | - | - | - | - | - |
| 4083 | X | - | - | - | - | - | - | - | - |
| 4084 | X | - | - | - | - | - | - | - | - |
| 4085 | - | - | - | - | - | X | - | - | - |
| 4086 | X | - | - | - | - | - | - | - | - |
| 4087 | X | - | - | - | - | - | - | - | - |
| 4088 | - | - | - | - | - | X | - | - | - |
| 4089 | X | - | - | - | - | - | - | - | - |
| 4090 | X | - | - | - | - | - | - | - | - |
| 4091 | - | - | - | - | X | - | - | - | - |
| 6270 | - | - | - | - | - | - | - | X | - |
| 5093 | - | - | - | - | - | - | - | X | - |
| 492 | X | - | - | - | - | - | - | - | - |
| 4092 | X | - | - | - | - | - | - | - | - |
| 3023 | X | - | - | - | - | - | - | - | - |
| 4093 | X | - | - | - | - | - | - | - | - |
| 178 | X | - | - | - | - | - | - | - | - |
| 2701 | X | - | - | - | - | - | - | - | - |
| 5799 | - | - | - | - | - | - | - | X | - |
| 4094 | - | - | - | - | - | X | - | - | - |
| 4095 | - | - | - | - | - | X | - | - | - |
| 4096 | - | - | - | - | - | X | - | - | - |
| 4097 | X | - | - | - | - | - | - | - | - |
| 4098 | - | - | - | - | X | - | - | - | - |
| 4099 | - | - | - | - | - | X | - | - | - |
| 4100 | - | - | - | - | - | X | - | - | - |
| 4101 | - | - | - | - | X | - | - | - | - |
| 4102 | - | - | - | - | X | - | - | - | - |
| 4103 | - | - | - | - | X | - | - | - | - |
| 4104 | - | - | - | - | X | - | - | - | - |
| 4105 | - | - | - | - | X | - | - | - | - |
| 4106 | - | - | - | - | X | - | - | - | - |
| 4107 | - | - | - | - | X | - | - | - | - |
| 4108 | - | - | - | - | X | - | - | - | - |
| 5804 | - | - | - | - | - | - | - | X | - |
| 4109 | - | - | - | - | - | X | - | - | - |
| 4110 | - | - | - | - | - | X | - | - | - |
| 4111 | X | - | - | - | - | - | - | - | - |
| 4112 | X | - | - | - | - | - | - | - | - |
| 4113 | X | - | - | - | - | - | - | - | - |
| 6635 | - | - | - | - | - | - | - | X | - |
| 4114 | - | - | - | - | X | - | - | - | - |
| 4115 | - | - | - | - | X | - | - | - | - |
| 4116 | - | - | - | - | - | X | - | - | - |

(continued)

| SEQ ID | A | B | C | D | E | F | G | H | I |
|---|---|---|---|---|---|---|---|---|---|
| 5548 | - | - | - | - | - | - | - | X | - |
| 4117 | X | - | - | - | - | - | - | - | - |
| 4118 | X | - | - | - | - | - | - | - | - |
| 4119 | X | - | - | - | - | - | - | - | - |
| 4120 | X | - | - | - | - | - | - | - | - |
| 4121 | X | - | - | - | - | - | - | - | - |
| 4122 | X | - | - | - | - | - | - | - | - |
| 4123 | X | - | - | - | - | - | - | - | - |
| 2308 | X | - | - | - | - | - | - | - | - |
| 4124 | - | - | - | - | X | - | - | - | - |
| 4125 | - | - | - | - | X | - | - | - | - |
| 4126 | - | - | - | - | X | - | - | - | - |
| 4127 | - | - | - | - | X | - | - | - | - |
| 4128 | - | - | - | - | X | - | - | - | - |
| 4129 | - | - | - | - | X | - | - | - | - |
| 4130 | - | - | - | - | X | - | - | - | - |
| 4131 | - | - | - | - | X | - | - | - | - |

Table 2. Expressed sequence tags from banana, wheat and maize having homology to the abiotic stress responsive polynucleotides of the present invention.

| SeqID | BANANA | WHEAT | MAIZE |
|---|---|---|---|
| 1 | - | 10978 | - |
| 2 | 9424 | 10548 | 12773 |
| 3 | - | 10164 | 12546 |
| 4 | - | 11828 | 13040 |
| 6 | 9321 | 11158 | 12911 |
| 7 | - | 10172 | - |
| 8 | 9704 | 11791 | 12284 |
| 9 | - | 11268 | 12668 |
| 10 | 9455 | 9956 | 13298 |
| 11 | - | 9920 | - |
| 13 | - | 11383 | 13282 |
| 14 | - | 11224 | 12268 |
| 15 | - | 11281 | - |
| 16 | 9511 | 11430 | 13049 |
| 17 | 9654 | 10905 | 12734 |
| 18 | 9779 | 10199 | 12291 |
| 19 | - | 11020 | 13191 |
| 21 | 9396 | 10584 | 12226 |
| 22 | - | 11885 | - |
| 23 | - | 11932 | - |
| 24 | 9365 | 10132 | 13043 |
| 26 | 9830 | 11796 | 12992 |
| 27 | 9237 | 11511 | 12372 |
| 28 | - | 10674 | - |
| 29 | - | 11813 | 13301 |
| 30 | - | - | 12187 |
| 31 | - | 11583 | 13157 |
| 32 | 9643 | 11085 | 12066 |

(continued)

| SeqID | BANANA | WHEAT | MAIZE |
|---|---|---|---|
| 33 | - | 11605 | 12696 |
| 34 | 9814 | 11093 | 12829 |
| 35 | 9684 | 11774 | 12345 |
| 36 | - | 9972 | 12926 |
| 37 | 9566 | 11383 | 12927 |
| 38 | - | 10334 | - |
| 39 | 9436 | 10137 | 12013 |
| 41 | - | 11552 | 12894 |
| 42 | 9770 | 9938 | 12698 |
| 43 | 9476 | 10860 | 12729 |
| 45 | - | - | 12514 |
| 46 | - | 10983 | - |
| 47 | 9599 | 10373 | 13032 |
| 48 | - | 10216 | - |
| 49 | 9303 | 11394 | - |
| 50 | 9289 | 9892 | - |
| 51 | 9475 | 10811 | 12378 |
| 52 | - | 11737 | - |
| 53 | 9342 | 10276 | 12421 |
| 54 | - | 11386 | 12293 |
| 55 | - | 11457 | 12503 |
| 56 | 9313 | 10417 | 12338 |
| 57 | - | 11064 | 12324 |
| 58 | - | - | 12472 |
| 59 | 9225 | 10611 | 12365 |
| 60 | 9391 | 11870 | 13366 |
| 61 | - | 11695 | 12510 |
| 62 | 9586 | 11317 | 13336 |
| 63 | 9726 | 11595 | 12072 |
| 64 | - | 11172 | 13368 |
| 65 | - | 11857 | 12910 |
| 66 | - | 10265 | 12447 |
| 67 | 9799 | 11140 | 13349 |
| 68 | - | 10784 | 12372 |
| 69 | - | 11146 | 12618 |
| 70 | - | 10315 | - |
| 71 | - | 10462 | - |
| 72 | 9573 | 11817 | 12950 |
| 73 | - | 9890 | 13060 |
| 74 | 9600 | 11127 | 12702 |
| 75 | - | - | 12033 |
| 76 | 9360 | 11324 | 12916 |
| 77 | 9687 | 11795 | 13222 |
| 78 | - | - | 12861 |
| 79 | 9720 | 11255 | 12103 |
| 80 | 9456 | 10373 | 13032 |
| 81 | - | 10530 | 12290 |
| 82 | - | 10026 | 13279 |
| 83 | 9235 | 10431 | 12832 |
| 84 | 9504 | 10917 | - |

(continued)

| SeqID | BANANA | WHEAT | MAIZE |
|---|---|---|---|
| 85 | - | 11840 | 12385 |
| 86 | 9546 | 11314 | 12697 |
| 88 | 9250 | 11675 | 13371 |
| 89 | - | 11049 | 12557 |
| 90 | - | 10193 | 13225 |
| 93 | 9312 | 11339 | 12858 |
| 94 | 9402 | 11707 | 13297 |
| 96 | - | 11506 | - |
| 98 | 9841 | 11859 | 12364 |
| 99 | 9429 | 11190 | 12186 |
| 100 | 9507 | 9900 | - |
| 101 | - | 10415 | 12308 |
| 102 | 9390 | 11852 | - |
| 103 | 9682 | 11047 | 12151 |
| 104 | - | 11522 | 12998 |
| 105 | - | 10649 | - |
| 106 | 9851 | 10789 | 12347 |
| 107 | 9446 | 10955 | 12129 |
| 108 | - | 10319 | - |
| 109 | - | 9946 | 12614 |
| 110 | - | 9883 | 13065 |
| 111 | 9557 | 10563 | - |
| 112 | 9456 | 11149 | 12663 |
| 114 | - | 9992 | - |
| 115 | 9498 | 10943 | 12329 |
| 116 | 9772 | 11378 | - |
| 117 | 9227 | 11411 | - |
| 119 | - | 10235 | - |
| 120 | - | 10172 | - |
| 121 | 9382 | 10202 | 13285 |
| 122 | 9421 | 10262 | 12183 |
| 123 | 9701 | 11671 | 12997 |
| 124 | 9589 | 11490 | 12069 |
| 125 | 9870 | 10942 | 13257 |
| 126 | - | 10265 | 12447 |
| 127 | - | 11046 | 12056 |
| 128 | 9698 | 11832 | 12527 |
| 129 | 9799 | 11356 | 12660 |
| 131 | 9688 | 11501 | 13250 |
| 132 | - | 11145 | 12663 |
| 133 | - | 10197 | 12170 |
| 134 | - | 11634 | 12573 |
| 135 | 9465 | - | 12155 |
| 136 | - | 11803 | - |
| 137 | - | 11161 | - |
| 138 | 9755 | 11647 | 12433 |
| 139 | - | 10322 | - |
| 140 | 9423 | 10417 | 12338 |
| 141 | - | 11657 | 13137 |
| 142 | - | 11196 | 12307 |

(continued)

| SeqID | BANANA | WHEAT | MAIZE |
|---|---|---|---|
| 143 | - | 10021 | - |
| 144 | - | 11319 | 13263 |
| 145 | 9630 | 9886 | 12302 |
| 146 | - | 11060 | - |
| 147 | - | 11161 | - |
| 148 | 9244 | 10978 | - |
| 149 | 9555 | 10368 | 12781 |
| 150 | 9658 | 11914 | 12695 |
| 151 | - | 10253 | 12826 |
| 152 | - | - | 12499 |
| 153 | 9756 | - | 12744 |
| 154 | - | 10498 | - |
| 155 | - | 11844 | 13019 |
| 157 | - | 11383 | 11991 |
| 158 | 9626 | 11508 | 12834 |
| 159 | - | 9990 | - |
| 160 | - | 9997 | 13071 |
| 161 | - | 10150 | 12304 |
| 162 | - | 10697 | - |
| 163 | - | 10321 | 13367 |
| 165 | - | 11097 | 12094 |
| 166 | - | 11706 | 13048 |
| 167 | - | 10157 | 12981 |
| 168 | - | 10515 | - |
| 169 | - | 10628 | 12573 |
| 170 | 9456 | 11145 | 12663 |
| 171 | - | 11016 | 12355 |
| 172 | - | 9986 | 13302 |
| 173 | - | 11544 | 13083 |
| 174 | - | 10990 | 12134 |
| 175 | 9742 | 11076 | 12914 |
| 176 | 9441 | 11199 | 12260 |
| 177 | - | 10232 | 13090 |
| 178 | 9324 | 10319 | 12967 |
| 179 | - | 10040 | - |
| 180 | 9548 | 11141 | 12596 |
| 181 | 9799 | 11356 | 12660 |
| 182 | - | 10640 | 12886 |
| 183 | - | 10868 | - |
| 184 | 9494 | 10249 | 13161 |
| 186 | - | 11776 | 12372 |
| 187 | - | 10949 | 12106 |
| 188 | 9377 | 10429 | 12467 |
| 189 | 9477 | 10527 | 13110 |
| 190 | - | - | 12107 |
| 192 | - | 10980 | - |
| 193 | - | 10537 | - |
| 194 | - | 10091 | 13210 |
| 195 | 9543 | 10769 | 12440 |
| 196 | - | - | 12056 |

(continued)

| SeqID | BANANA | WHEAT | MAIZE |
|---|---|---|---|
| 197 | - | 11667 | 13171 |
| 198 | - | - | 12170 |
| 199 | - | 10173 | 13265 |
| 201 | 9405 | 11964 | 12023 |
| 202 | 9806 | 10744 | 12302 |
| 204 | - | 10465 | - |
| 206 | - | 11728 | - |
| 208 | - | 11525 | 12076 |
| 209 | 9489 | 11145 | 12663 |
| 210 | 9362 | 10343 | 12440 |
| 211 | - | 10458 | - |
| 212 | - | 11172 | - |
| 213 | 9260 | 11692 | 12575 |
| 214 | - | 11778 | 12361 |
| 215 | - | 11136 | 12766 |
| 216 | - | 10682 | - |
| 217 | - | 11374 | 13347 |
| 218 | - | 10634 | - |
| 220 | - | 10207 | - |
| 221 | - | 10640 | 13118 |
| 222 | 9251 | - | - |
| 223 | - | 10040 | - |
| 224 | 9612 | 11070 | 13172 |
| 226 | 9365 | 11738 | 13237 |
| 228 | 9427 | 10640 | 12361 |
| 229 | 9430 | 11663 | 12945 |
| 230 | - | 10674 | 12378 |
| 231 | 9615 | 11590 | 12867 |
| 232 | - | 11941 | - |
| 233 | 9365 | 11739 | 13297 |
| 234 | 9529 | - | - |
| 235 | 9720 | 11633 | 12103 |
| 236 | - | 10063 | - |
| 237 | 9736 | 11128 | 12030 |
| 238 | - | 11610 | 12668 |
| 239 | 9388 | 9919 | 12269 |
| 240 | 9798 | 11395 | 13349 |
| 241 | 9434 | 11724 | 13050 |
| 242 | - | 11226 | 12738 |
| 243 | - | 10640 | 13118 |
| 244 | - | 10916 | - |
| 245 | - | 11779 | - |
| 246 | - | 9949 | - |
| 247 | - | 10532 | - |
| 248 | - | 10232 | 13090 |
| 249 | - | 9990 | 12706 |
| 250 | - | 11133 | 13255 |
| 253 | 9528 | 10227 | 12750 |
| 254 | 9402 | 10982 | 12212 |
| 255 | 9870 | 10950 | 13088 |

(continued)

| SeqID | BANANA | WHEAT | MAIZE |
|---|---|---|---|
| 257 | 9321 | 11777 | 12361 |
| 258 | 9446 | 10759 | 12380 |
| 259 | 9246 | 11730 | 13087 |
| 260 | - | 10728 | - |
| 261 | - | 10389 | 12932 |
| 262 | 9448 | 11634 | 13220 |
| 264 | 9601 | 10800 | 11996 |
| 265 | 9521 | 11209 | - |
| 266 | - | 10983 | - |
| 267 | - | 11594 | - |
| 268 | 9292 | 10417 | 12337 |
| 269 | 9793 | 10540 | 13049 |
| 270 | - | 10023 | 12778 |
| 271 | 9247 | 10297 | 11973 |
| 272 | 9384 | 11733 | 12942 |
| 273 | - | 11215 | - |
| 274 | 9720 | 11123 | 12133 |
| 275 | 9371 | 10230 | - |
| 276 | - | 11540 | 12682 |
| 277 | - | 11290 | 13251 |
| 278 | 9704 | 11057 | 12822 |
| 279 | 9404 | 11422 | 13053 |
| 280 | - | 10006 | - |
| 281 | - | 11135 | 12881 |
| 282 | - | 10255 | 13213 |
| 283 | - | - | 13056 |
| 284 | - | 9949 | 12676 |
| 285 | - | 10667 | - |
| 287 | 9838 | 11479 | - |
| 289 | - | 10440 | - |
| 290 | - | - | 13293 |
| 291 | 9751 | 11365 | 12340 |
| 295 | - | 10735 | - |
| 296 | - | 11256 | 13176 |
| 297 | 9853 | 10489 | 12847 |
| 298 | - | 11288 | 12558 |
| 301 | 9366 | 11125 | 12133 |
| 304 | - | 10440 | - |
| 305 | 9324 | 11063 | 13178 |
| 306 | 9308 | 11526 | 13184 |
| 307 | 9832 | 11529 | 12687 |
| 308 | 9812 | 10653 | 12376 |
| 309 | 9478 | 11185 | 12016 |
| 310 | 9589 | 11260 | - |
| 311 | - | 10795 | 12511 |
| 312 | 9692 | 10899 | 12286 |
| 313 | - | 11050 | 12056 |
| 314 | - | 11172 | 13368 |
| 315 | 9499 | 10209 | 12148 |
| 316 | 9880 | 10084 | 11975 |

(continued)

| SeqID | BANANA | WHEAT | MAIZE |
|---|---|---|---|
| 317 | 9671 | 11094 | 12826 |
| 318 | 9532 | 11867 | 12032 |
| 319 | - | 10000 | 13177 |
| 320 | 9446 | 10955 | 12129 |
| 321 | - | 9949 | - |
| 322 | 9708 | 11134 | 12840 |
| 324 | 9416 | 10612 | 12856 |
| 325 | - | 10799 | - |
| 326 | 9829 | 10036 | 12143 |
| 327 | - | 11540 | 12368 |
| 328 | 9589 | 11112 | 12256 |
| 329 | - | 11629 | - |
| 330 | - | 11382 | 12751 |
| 331 | - | 11493 | - |
| 332 | 9666 | 11103 | 12055 |
| 333 | - | 10362 | - |
| 334 | - | 10696 | - |
| 335 | - | 11040 | 13092 |
| 336 | - | 11819 | 11988 |
| 337 | - | 11346 | 13144 |
| 338 | - | 11386 | - |
| 339 | 9777 | 11726 | 13101 |
| 340 | 9313 | 10417 | 12338 |
| 341 | 9768 | - | - |
| 342 | 9880 | 11733 | 12942 |
| 343 | 9869 | 10652 | 12393 |
| 344 | - | 10005 | - |
| 346 | - | 10209 | - |
| 348 | - | 10332 | 12043 |
| 349 | - | 10945 | 13051 |
| 350 | - | 9925 | - |
| 351 | 9735 | 10119 | 13260 |
| 352 | 9880 | 10103 | 12942 |
| 353 | - | 10640 | 12361 |
| 355 | 9810 | 11519 | 12654 |
| 356 | 9447 | 10993 | 12952 |
| 357 | - | 10017 | - |
| 358 | - | - | 12014 |
| 359 | 9392 | 11707 | 13043 |
| 361 | 9741 | 11077 | 13186 |
| 362 | 9436 | 10137 | - |
| 364 | - | 10783 | - |
| 365 | 9262 | 9953 | 13129 |
| 366 | - | 11850 | - |
| 367 | - | 11058 | 12567 |
| 368 | 9789 | 10544 | 13234 |
| 369 | - | 11386 | - |
| 370 | 9698 | 11832 | 12527 |
| 371 | 9331 | 11389 | - |
| 372 | 9869 | 11776 | 12393 |

(continued)

| SeqID | BANANA | WHEAT | MAIZE |
|---|---|---|---|
| 374 | - | - | 12868 |
| 375 | 9437 | 10146 | - |
| 376 | - | 9926 | 12460 |
| 377 | 9880 | 11733 | 12942 |
| 378 | 9506 | 11632 | 12784 |
| 379 | - | 10689 | - |
| 380 | - | 11889 | 12533 |
| 381 | - | 11666 | - |
| 382 | - | - | 12123 |
| 383 | 9269 | 11846 | - |
| 384 | 9808 | 10214 | 12954 |
| 385 | 9412 | 11038 | 12147 |
| 386 | 9237 | 10594 | 13336 |
| 387 | - | 10001 | - |
| 388 | 9446 | 10759 | 12380 |
| 389 | 9305 | 11397 | 12859 |
| 390 | - | 9945 | - |
| 391 | - | 10728 | - |
| 392 | 9376 | 11250 | 13318 |
| 394 | 9302 | 10777 | 12909 |
| 395 | - | 10230 | 13213 |
| 396 | - | 11488 | - |
| 397 | 9742 | 11072 | 12491 |
| 400 | 9635 | 11091 | 12713 |
| 401 | 9742 | 11078 | 13352 |
| 402 | 9833 | 11478 | 13167 |
| 403 | - | 10850 | - |
| 404 | 9678 | 10062 | 12592 |
| 405 | - | 10054 | 13090 |
| 406 | - | 10946 | 12599 |
| 407 | 9587 | 10733 | 12573 |
| 408 | 9606 | 10388 | 13122 |
| 409 | - | 10259 | 12811 |
| 410 | - | 11595 | 12669 |
| 412 | 9851 | 10985 | 12183 |
| 413 | 9704 | 11057 | 12984 |
| 414 | 9365 | 10143 | 12458 |
| 415 | - | 10285 | 13201 |
| 417 | 9575 | 11234 | 12402 |
| 418 | - | 10451 | - |
| 419 | 9256 | 11199 | 12260 |
| 420 | 9765 | 11440 | 12603 |
| 421 | - | 11214 | - |
| 422 | - | 10224 | 12293 |
| 424 | 9553 | 10458 | - |
| 425 | - | 10331 | 12360 |
| 426 | - | 10199 | 13118 |
| 428 | - | 11876 | - |
| 429 | - | 11642 | - |
| 430 | - | 10503 | 13070 |

(continued)

| SeqID | BANANA | WHEAT | MAIZE |
|---|---|---|---|
| 431 | - | 9948 | - |
| 432 | - | 11465 | 12554 |
| 433 | - | 10040 | - |
| 435 | 9629 | 10838 | 12463 |
| 436 | - | - | 13086 |
| 437 | 9376 | 11856 | 12784 |
| 438 | 9324 | 11205 | 12967 |
| 439 | 9779 | 11248 | 13259 |
| 440 | 9782 | 9974 | - |
| 441 | 9366 | 11125 | 12133 |
| 442 | - | 11576 | 12792 |
| 443 | 9653 | 11318 | 12499 |
| 444 | - | 10329 | 12866 |
| 445 | - | 11254 | 12847 |
| 446 | - | 11811 | 13051 |
| 447 | 9496 | 11163 | 13022 |
| 448 | 9518 | - | 12583 |
| 449 | 9720 | 11632 | 12133 |
| 450 | - | 10422 | - |
| 451 | - | 9958 | 12119 |
| 452 | 9401 | 9950 | 12022 |
| 453 | - | 10564 | - |
| 454 | 9446 | 11202 | 12428 |
| 455 | 9391 | 10127 | 12497 |
| 456 | - | 11593 | 12012 |
| 457 | 9668 | 10390 | 12872 |
| 459 | 9380 | 11797 | 12041 |
| 460 | 9446 | 11202 | 12428 |
| 461 | - | 10690 | 12351 |
| 462 | - | 10138 | 13000 |
| 463 | 9589 | 11490 | - |
| 464 | 9819 | 11409 | 12434 |
| 465 | 9871 | 10942 | 13187 |
| 466 | - | 11948 | 12366 |
| 467 | - | 9905 | - |
| 468 | 9682 | 11044 | 12044 |
| 469 | - | 10028 | - |
| 470 | - | 10453 | - |
| 471 | 9819 | 11425 | 12434 |
| 472 | 9401 | 9913 | 12424 |
| 473 | - | 10656 | - |
| 474 | 9802 | 11257 | 12279 |
| 475 | 9363 | 10743 | 12324 |
| 476 | - | - | 12285 |
| 478 | - | 11618 | 12652 |
| 480 | - | 10745 | - |
| 481 | 9330 | 10900 | 12482 |
| 482 | - | - | 12008 |
| 483 | - | 11696 | 13051 |
| 484 | 9564 | 10500 | 12583 |

(continued)

| SeqID | BANANA | WHEAT | MAIZE |
|---|---|---|---|
| 485 | 9228 | 10505 | 12485 |
| 486 | - | 10685 | - |
| 487 | 9586 | 11353 | 12903 |
| 488 | 9355 | 11092 | 12656 |
| 489 | 9852 | 10934 | - |
| 490 | 9248 | 10913 | - |
| 491 | 9301 | 11373 | 12922 |
| 493 | - | 11665 | - |
| 494 | 9548 | 11474 | 12596 |
| 495 | - | 10764 | 12324 |
| 498 | 9363 | 10512 | 12094 |
| 500 | - | 10804 | - |
| 501 | - | 11385 | - |
| 502 | 9684 | 11765 | 12318 |
| 503 | 9392 | - | 12577 |
| 504 | - | 10222 | - |
| 505 | 9687 | 11172 | 12761 |
| 506 | - | 10184 | - |
| 507 | 9811 | 10667 | 13031 |
| 508 | 9739 | 10654 | 12889 |
| 509 | - | 11786 | - |
| 511 | - | 10286 | 12084 |
| 512 | 9443 | 11278 | 12815 |
| 513 | 9720 | 11523 | 12316 |
| 514 | - | 10305 | - |
| 515 | 9450 | 11304 | 12429 |
| 516 | - | 10750 | 13166 |
| 517 | 9244 | 9998 | 12140 |
| 518 | 9781 | 11269 | 12153 |
| 519 | - | 11650 | 12681 |
| 520 | - | 11810 | 13051 |
| 523 | 9704 | 10079 | 12530 |
| 524 | - | 10982 | 13074 |
| 525 | - | 11145 | 12912 |
| 526 | - | 10165 | 12077 |
| 527 | - | 10562 | 12168 |
| 529 | 9705 | 11104 | 12341 |
| 530 | - | 11425 | 13215 |
| 531 | - | 11556 | 13255 |
| 533 | 9376 | 11250 | 12784 |
| 534 | - | 9984 | - |
| 535 | - | 11024 | - |
| 536 | - | 11046 | 12056 |
| 537 | - | 10937 | 13219 |
| 538 | - | 10745 | - |
| 539 | - | 11275 | 13033 |
| 540 | 9436 | 11910 | 12013 |
| 541 | 9880 | 10103 | 12942 |
| 542 | - | 9915 | 12419 |
| 543 | - | 9890 | 13060 |

(continued)

| SeqID | BANANA | WHEAT | MAIZE |
|-------|--------|-------|-------|
| 544 | 9540 | 9947 | - |
| 545 | - | 11826 | 12859 |
| 546 | - | 9976 | 13155 |
| 547 | 9308 | 11461 | - |
| 548 | - | 11359 | 12052 |
| 549 | 9731 | 10923 | - |
| 550 | - | 10472 | 12483 |
| 551 | - | 11835 | 12705 |
| 552 | - | 9994 | 12355 |
| 553 | 9313 | 10417 | 12338 |
| 556 | - | 11464 | - |
| 557 | - | 11160 | - |
| 558 | 9719 | 11902 | 12690 |
| 559 | - | 11607 | 12400 |
| 560 | 9537 | 11514 | 12233 |
| 561 | - | 11405 | 13001 |
| 562 | - | - | 12378 |
| 563 | - | 11277 | 12056 |
| 564 | 9880 | 11685 | 12837 |
| 565 | - | 10526 | 12156 |
| 566 | - | 11539 | 12449 |
| 567 | 9400 | 11298 | 13185 |
| 569 | 9722 | 10626 | 12374 |
| 570 | 9222 | 10693 | 12332 |
| 571 | - | - | 12885 |
| 572 | 9308 | 9962 | - |
| 573 | - | 10224 | 12293 |
| 574 | - | 10098 | - |
| 575 | - | 10672 | - |
| 576 | 9446 | 11201 | 12428 |
| 577 | - | 10850 | 12388 |
| 578 | - | 10023 | 12778 |
| 579 | 9285 | 11664 | 12273 |
| 580 | 9868 | 11137 | 12610 |
| 581 | 9851 | 11722 | 13031 |
| 582 | - | 11763 | 12373 |
| 583 | 9639 | 10480 | 12209 |
| 584 | 9841 | 11088 | 12298 |
| 585 | 9426 | 11437 | 12275 |
| 586 | - | 11374 | 13347 |
| 587 | - | 11825 | - |
| 588 | - | 9949 | - |
| 590 | - | 11383 | 12886 |
| 591 | 9436 | 10589 | 12013 |
| 592 | 9423 | 11511 | 12372 |
| 593 | 9532 | 10752 | 12031 |
| 594 | - | 11014 | - |
| 595 | - | 11524 | 13230 |
| 596 | - | 10032 | - |
| 597 | - | 9977 | - |

(continued)

| SeqID | BANANA | WHEAT | MAIZE |
|---|---|---|---|
| 598 | 9267 | 11450 | 13093 |
| 599 | 9600 | 11127 | 12702 |
| 600 | - | 10983 | 12427 |
| 601 | - | 10784 | 12372 |
| 603 | - | 11045 | 12737 |
| 604 | 9871 | 10942 | 13257 |
| 605 | 9277 | 10403 | 12675 |
| 606 | - | 10858 | - |
| 607 | 9870 | 10942 | 13257 |
| 609 | - | 11077 | 13186 |
| 612 | - | 10230 | 13189 |
| 613 | - | 11061 | 12306 |
| 614 | 9819 | 10457 | 12434 |
| 615 | - | 11155 | - |
| 616 | 9520 | 10635 | 12926 |
| 617 | 9687 | 11057 | 12984 |
| 619 | 9368 | 11716 | 13235 |
| 621 | - | 11573 | 12486 |
| 622 | - | - | 13158 |
| 623 | 9653 | 11318 | 13272 |
| 624 | - | 10180 | 13363 |
| 625 | - | 10983 | - |
| 626 | - | 11823 | 12304 |
| 627 | - | 10173 | 12505 |
| 628 | - | 11940 | 12528 |
| 629 | - | - | 13164 |
| 630 | 9423 | 11367 | 12338 |
| 631 | - | 10245 | 13100 |
| 632 | 9608 | 11057 | 12984 |
| 633 | - | 10983 | - |
| 634 | - | 10409 | 13106 |
| 635 | - | - | 12789 |
| 636 | 9831 | 11321 | 13175 |
| 638 | - | 10853 | 13277 |
| 639 | 9446 | 11200 | 12428 |
| 640 | - | 10576 | 12568 |
| 641 | - | 11386 | 12293 |
| 642 | 9339 | 11708 | 13081 |
| 643 | - | 11385 | 12293 |
| 644 | - | - | 12886 |
| 645 | - | 10427 | - |
| 647 | - | 11628 | - |
| 648 | 9384 | 9899 | 12756 |
| 649 | - | 9898 | 12773 |
| 650 | 9381 | 10156 | 12880 |
| 651 | 9261 | 10401 | 13160 |
| 652 | 9396 | 10583 | 12226 |
| 653 | 9656 | 10385 | 12879 |
| 654 | - | 10515 | - |
| 655 | - | - | 12249 |

(continued)

| SeqID | BANANA | WHEAT | MAIZE |
|---|---|---|---|
| 656 | 9404 | 10741 | 13053 |
| 657 | 9481 | 10073 | 12854 |
| 659 | 9340 | 11233 | 12819 |
| 661 | - | 11001 | - |
| 662 | - | 11960 | 13010 |
| 663 | - | 10682 | 13311 |
| 664 | - | 10166 | 13128 |
| 665 | 9456 | 11146 | 12663 |
| 668 | - | 10729 | 12817 |
| 669 | 9684 | 10610 | 12318 |
| 670 | - | 11851 | 12830 |
| 671 | - | 10858 | 12976 |
| 673 | - | 11154 | 12118 |
| 674 | 9382 | 11751 | - |
| 676 | - | 11544 | 12567 |
| 677 | - | 11940 | 12528 |
| 678 | - | 10825 | 12768 |
| 680 | - | 10751 | 12487 |
| 681 | 9477 | 11560 | 12161 |
| 682 | 9720 | 11123 | 12578 |
| 683 | 9523 | 11206 | 13081 |
| 684 | - | 10170 | 12850 |
| 685 | - | 11053 | 12661 |
| 686 | - | 11245 | - |
| 687 | 9831 | 11803 | - |
| 688 | - | 10820 | 13240 |
| 689 | 9358 | 10151 | 12775 |
| 690 | 9566 | 9937 | 12042 |
| 691 | - | 11481 | - |
| 692 | 9595 | 11079 | 12623 |
| 693 | 9878 | 11216 | 13078 |
| 694 | 9425 | 10040 | - |
| 695 | 9681 | 11579 | 12718 |
| 696 | 9570 | 11371 | 12442 |
| 697 | 9642 | 10477 | 12294 |
| 698 | - | 9949 | - |
| 700 | - | 10851 | 13322 |
| 701 | - | 11861 | 13339 |
| 702 | 9784 | 9906 | 12350 |
| 703 | 9824 | 11253 | 12821 |
| 704 | 9404 | 11596 | 12960 |
| 705 | 9563 | 10410 | 13208 |
| 706 | - | 10620 | 13092 |
| 707 | 9692 | 11306 | 13212 |
| 708 | - | 11380 | 12419 |
| 709 | 9394 | 11188 | 12672 |
| 710 | - | 10407 | - |
| 711 | - | 9933 | 12964 |
| 712 | - | 11741 | 13252 |
| 713 | - | 10314 | 12663 |

(continued)

| SeqID | BANANA | WHEAT | MAIZE |
|-------|--------|-------|-------|
| 714 | - | 11128 | 13084 |
| 715 | - | 10964 | - |
| 716 | 9363 | 11089 | 12094 |
| 717 | - | 11162 | - |
| 718 | - | 10974 | 12632 |
| 719 | 9574 | 10622 | 13069 |
| 720 | 9527 | 11487 | 12595 |
| 721 | 9475 | 11609 | - |
| 722 | 9408 | 10534 | 12586 |
| 723 | - | 9936 | 12109 |
| 724 | 9263 | 11511 | 12372 |
| 725 | - | 11523 | 13263 |
| 726 | - | 11499 | 13246 |
| 727 | - | 10801 | 12868 |
| 728 | - | 10107 | 13034 |
| 729 | - | 10009 | - |
| 730 | - | 10313 | - |
| 731 | - | 10858 | - |
| 732 | - | 10640 | 12363 |
| 733 | - | 11956 | 12574 |
| 734 | - | - | 13044 |
| 735 | - | 10678 | 12751 |
| 736 | - | 9949 | - |
| 737 | - | 11746 | 12604 |
| 739 | 9828 | 11096 | 12199 |
| 740 | - | 10765 | 12380 |
| 741 | 9820 | 10440 | - |
| 742 | 9821 | 11090 | 12026 |
| 743 | - | 10682 | - |
| 745 | - | 10739 | - |
| 746 | 9692 | 10390 | 12425 |
| 747 | - | 10078 | - |
| 749 | 9363 | 11526 | - |
| 750 | - | 10561 | - |
| 751 | - | 10139 | 12094 |
| 752 | 9575 | 11445 | 12044 |
| 753 | - | 10369 | - |
| 754 | 9800 | 10624 | 12326 |
| 755 | 9779 | 11249 | 12166 |
| 756 | - | 10405 | 13240 |
| 757 | 9778 | 11893 | 12040 |
| 758 | 9502 | 10751 | 12775 |
| 759 | 9518 | 10599 | 13289 |
| 760 | 9243 | 11371 | 12442 |
| 761 | 9719 | 11161 | - |
| 763 | - | 10423 | - |
| 764 | - | 10223 | - |
| 765 | - | 10486 | - |
| 766 | 9855 | 11460 | 12872 |
| 767 | 9497 | 11065 | 12261 |

(continued)

| SeqID | BANANA | WHEAT | MAIZE |
|---|---|---|---|
| 768 | - | 11697 | 12089 |
| 769 | - | - | 12591 |
| 770 | 9685 | 11280 | 12444 |
| 771 | - | 10204 | - |
| 772 | - | 11820 | 13282 |
| 773 | 9558 | 11012 | 12951 |
| 774 | - | - | 12707 |
| 775 | - | 10061 | 13147 |
| 776 | 9461 | 10286 | 12084 |
| 777 | 9734 | 11018 | 12281 |
| 778 | - | 10515 | - |
| 779 | - | 10631 | - |
| 781 | - | 10266 | 12380 |
| 782 | - | 10034 | 13325 |
| 783 | 9880 | 10103 | 12942 |
| 784 | - | 11608 | 12400 |
| 785 | - | 10015 | 12328 |
| 786 | - | 11390 | - |
| 787 | - | 10543 | 12888 |
| 788 | 9742 | 11075 | 12914 |
| 789 | - | 9935 | 12109 |
| 790 | - | 10690 | 12183 |
| 792 | 9474 | 10273 | 13359 |
| 793 | - | 10725 | - |
| 795 | 9783 | 11230 | 13255 |
| 796 | 9217 | 11860 | 12466 |
| 797 | - | - | 12014 |
| 799 | - | 9943 | - |
| 800 | 9799 | 11392 | 13303 |
| 802 | - | 11902 | 12681 |
| 803 | 9528 | 11204 | 12261 |
| 804 | 9761 | 10907 | 12913 |
| 805 | - | 11143 | - |
| 806 | 9363 | - | - |
| 807 | 9535 | 11475 | 12819 |
| 808 | - | 11472 | 12719 |
| 809 | 9621 | 10510 | 12771 |
| 810 | - | 10218 | 13244 |
| 811 | - | 11899 | 12265 |
| 812 | - | 10116 | - |
| 813 | - | 10706 | 12446 |
| 815 | - | 11900 | 13138 |
| 816 | 9291 | 11067 | 12035 |
| 817 | 9308 | 10671 | - |
| 818 | 9522 | 10330 | - |
| 820 | - | 11097 | 12094 |
| 821 | 9384 | 11922 | 12756 |
| 822 | - | 11459 | 12665 |
| 823 | 9319 | 10569 | 12562 |
| 824 | 9376 | 11126 | 12578 |

(continued)

| SeqID | BANANA | WHEAT | MAIZE |
|-------|--------|-------|-------|
| 825 | - | 11173 | 12822 |
| 826 | - | 9926 | - |
| 827 | 9768 | - | - |
| 828 | 9494 | 11054 | 12536 |
| 829 | 9365 | 10143 | 13043 |
| 830 | - | 10920 | - |
| 831 | - | 11543 | - |
| 832 | 9575 | 10595 | 12044 |
| 833 | - | - | 12625 |
| 834 | 9430 | 10164 | 12130 |
| 835 | - | 11276 | 13240 |
| 836 | 9461 | 11334 | 13046 |
| 837 | - | 10770 | - |
| 839 | - | 10745 | - |
| 840 | - | 10539 | - |
| 841 | 9544 | 11191 | 13109 |
| 842 | 9687 | 11237 | 12822 |
| 843 | 9255 | 10008 | - |
| 844 | 9313 | 10699 | 11977 |
| 845 | - | 9990 | - |
| 846 | - | 10058 | 12387 |
| 847 | 9446 | 11202 | 12428 |
| 849 | 9663 | 10120 | 12297 |
| 850 | - | 11445 | 12925 |
| 851 | 9707 | 9966 | 12648 |
| 852 | - | 10801 | - |
| 853 | - | 10558 | - |
| 854 | - | 11220 | 12091 |
| 855 | 9290 | 10096 | - |
| 856 | 9449 | 10587 | 12674 |
| 857 | 9848 | 10356 | - |
| 858 | 9620 | 11630 | 12282 |
| 859 | - | - | 12423 |
| 861 | 9656 | 10443 | 12375 |
| 862 | - | - | 12078 |
| 863 | - | 10432 | 12915 |
| 864 | - | 10433 | 12857 |
| 866 | - | 11888 | 12138 |
| 867 | 9445 | 11762 | 12966 |
| 868 | - | 10962 | 12943 |
| 869 | - | 11344 | 12053 |
| 870 | - | 11271 | 13345 |
| 872 | - | 11115 | 12547 |
| 873 | - | 11352 | 12959 |
| 874 | - | 11880 | 12727 |
| 875 | 9880 | 10103 | 12942 |
| 877 | - | 11732 | - |
| 878 | - | 10932 | 12396 |
| 880 | - | 11538 | 12253 |
| 882 | 9693 | 10129 | 12462 |

(continued)

| SeqID | BANANA | WHEAT | MAIZE |
|---|---|---|---|
| 883 | 9384 | 10699 | 12099 |
| 884 | - | 10645 | 12617 |
| 885 | - | 10083 | - |
| 886 | 9669 | 11567 | - |
| 888 | - | 11863 | 12417 |
| 889 | - | 10798 | 13305 |
| 890 | - | 11871 | 12187 |
| 892 | - | 9903 | - |
| 893 | - | 11745 | - |
| 894 | - | 10387 | 12627 |
| 895 | - | 10232 | - |
| 896 | 9534 | 11198 | - |
| 898 | 9435 | 10691 | 12079 |
| 899 | 9728 | 10325 | 12711 |
| 900 | - | 9896 | - |
| 901 | - | - | 13085 |
| 902 | - | 11559 | 12071 |
| 903 | 9585 | 11048 | 12615 |
| 905 | - | 11312 | 12821 |
| 906 | - | - | 12107 |
| 907 | - | 11366 | 13133 |
| 908 | - | 11210 | - |
| 909 | - | 10928 | 12919 |
| 910 | - | 10658 | 12963 |
| 911 | - | - | 12381 |
| 912 | - | 10085 | 12213 |
| 913 | 9460 | 11645 | - |
| 914 | - | - | 13212 |
| 915 | - | 11213 | 12217 |
| 916 | 9314 | - | - |
| 917 | - | 11326 | 13287 |
| 918 | - | 10155 | - |
| 921 | - | 10013 | - |
| 922 | - | - | 13228 |
| 923 | - | 10617 | 12990 |
| 924 | - | 10112 | 12211 |
| 926 | - | 11750 | - |
| 927 | 9466 | 11453 | 12723 |
| 928 | - | 10966 | 12716 |
| 929 | - | 11469 | 12748 |
| 930 | - | 11898 | - |
| 931 | 9411 | 10545 | 12871 |
| 934 | - | 10082 | 12145 |
| 935 | - | 9908 | - |
| 936 | - | 10287 | 12541 |
| 937 | - | 11294 | 12625 |
| 939 | - | 11660 | 12667 |
| 940 | - | 10885 | 13181 |
| 941 | - | 10863 | - |
| 942 | 9327 | 10618 | 12075 |

(continued)

| SeqID | BANANA | WHEAT | MAIZE |
|---|---|---|---|
| 943 | 9714 | 10044 | - |
| 944 | - | 11358 | 12799 |
| 945 | - | 11219 | 13274 |
| 948 | 9455 | 11100 | 12176 |
| 949 | - | 11891 | 12211 |
| 950 | - | 11052 | 12615 |
| 951 | - | 10010 | - |
| 952 | - | - | 12534 |
| 953 | - | 11285 | 12413 |
| 954 | - | 11682 | - |
| 955 | 9384 | 11922 | 12756 |
| 956 | - | - | 13369 |
| 957 | - | - | 12700 |
| 958 | 9774 | 11129 | 13311 |
| 960 | - | 10155 | - |
| 962 | 9858 | 11178 | 12392 |
| 963 | - | - | 12177 |
| 964 | - | 10796 | 12870 |
| 965 | 9337 | - | - |
| 966 | 9455 | 11764 | - |
| 967 | - | 11067 | - |
| 968 | 9614 | 10508 | 13021 |
| 970 | - | 10233 | - |
| 971 | - | 11926 | - |
| 972 | - | - | 12506 |
| 974 | - | 11534 | - |
| 975 | 9623 | 10509 | 12295 |
| 976 | 9867 | 10340 | 12452 |
| 977 | - | - | 13216 |
| 978 | - | 10191 | |
| 979 | - | 10763 | 13357 |
| 980 | 9538 | 10999 | 11976 |
| 981 | - | 9928 | 12646 |
| 982 | 9506 | 11126 | 13264 |
| 983 | - | - | 12581 |
| 984 | - | 10472 | - |
| 985 | 9562 | 10684 | - |
| 986 | 9837 | 11372 | - |
| 987 | - | 11033 | 12581 |
| 988 | 9789 | 11283 | 12149 |
| 989 | - | 10495 | 13317 |
| 990 | - | 10250 | 12847 |
| 991 | - | 10773 | 12162 |
| 992 | - | 10130 | 13143 |
| 993 | - | 11087 | 12344 |
| 994 | 9241 | 11068 | 12516 |
| 995 | - | - | 12918 |
| 996 | - | - | 12848 |
| 997 | 9306 | 10095 | 12993 |
| 998 | - | 10506 | - |

(continued)

| SeqID | BANANA | WHEAT | MAIZE |
|---|---|---|---|
| 999 | 9874 | 11410 | 12227 |
| 1000 | - | 11361 | 12620 |
| 1001 | 9774 | 11129 | 13134 |
| 1002 | 9571 | 11428 | 12102 |
| 1003 | - | 10775 | 12418 |
| 1004 | - | 11749 | 13009 |
| 1005 | - | 10848 | - |
| 1008 | - | - | 12047 |
| 1009 | 9438 | 10419 | 13028 |
| 1010 | 9774 | 11129 | 13134 |
| 1011 | 9818 | 11436 | 13273 |
| 1012 | - | 10839 | - |
| 1013 | 9596 | 9923 | 12445 |
| 1014 | - | 11330 | 13017 |
| 1015 | - | 11967 | 13360 |
| 1016 | - | 11636 | - |
| 1017 | 9712 | 11761 | 12679 |
| 1018 | - | - | 12806 |
| 1019 | - | - | 12925 |
| 1021 | 9694 | 10590 | 13042 |
| 1022 | 9317 | 11965 | 12969 |
| 1023 | - | - | 11971 |
| 1024 | - | 11187 | 12920 |
| 1025 | - | 11513 | - |
| 1026 | - | - | 12354 |
| 1027 | 9469 | - | 12678 |
| 1028 | - | - | 12031 |
| 1030 | - | 11244 | 13238 |
| 1031 | - | - | 12450 |
| 1032 | - | 11462 | 12606 |
| 1033 | - | 10281 | - |
| 1034 | - | 10012 | 12064 |
| 1035 | - | 11177 | 12917 |
| 1036 | - | 11541 | - |
| 1037 | - | 10490 | - |
| 1038 | 9656 | 10258 | 12375 |
| 1039 | - | - | 12580 |
| 1040 | - | 10348 | 13173 |
| 1041 | - | 10661 | - |
| 1042 | - | 11418 | 12865 |
| 1043 | - | 11336 | 12572 |
| 1044 | - | 10547 | - |
| 1045 | - | 11295 | 13248 |
| 1046 | - | 11809 | 12770 |
| 1047 | - | 11627 | 13054 |
| 1048 | 9393 | 11701 | - |
| 1050 | - | 11042 | 12710 |
| 1051 | - | 11274 | 13320 |
| 1052 | - | 11124 | 13176 |
| 1053 | - | 10450 | 12641 |

(continued)

| SeqID | BANANA | WHEAT | MAIZE |
|---|---|---|---|
| 1054 | - | 10379 | 12735 |
| 1055 | - | 11801 | - |
| 1056 | - | 11414 | 12280 |
| 1057 | - | 11264 | 12195 |
| 1058 | - | 10963 | - |
| 1059 | - | 10488 | - |
| 1060 | - | 10056 | - |
| 1061 | 9411 | 10546 | - |
| 1062 | - | 9884 | 13248 |
| 1064 | 9636 | 11736 | 13249 |
| 1065 | - | 10902 | - |
| 1066 | - | 10865 | 12634 |
| 1067 | 9827 | 10575 | 12823 |
| 1068 | - | - | 13067 |
| 1069 | 9776 | 11212 | 12740 |
| 1070 | 9594 | 10446 | 12112 |
| 1071 | - | 10476 | 12015 |
| 1072 | - | 10746 | - |
| 1073 | - | 11443 | 12408 |
| 1074 | - | 10292 | - |
| 1075 | 9769 | 11705 | - |
| 1076 | 9748 | 10860 | 12745 |
| 1077 | - | 11320 | 12720 |
| 1078 | - | 10192 | 12216 |
| 1080 | - | 11470 | - |
| 1081 | 9774 | 10792 | 13311 |
| 1083 | 9503 | 11935 | 12096 |
| 1084 | - | 11898 | - |
| 1085 | - | 10136 | - |
| 1086 | - | 10081 | 12841 |
| 1087 | - | 11929 | - |
| 1088 | 9617 | 10111 | 12642 |
| 1091 | - | 10198 | 12522 |
| 1092 | - | 11164 | - |
| 1093 | - | 10196 | - |
| 1094 | - | 11807 | 12092 |
| 1095 | - | 11214 | 12255 |
| 1096 | - | 10716 | - |
| 1097 | 9850 | 11145 | 12609 |
| 1098 | - | 10253 | 12851 |
| 1099 | 9333 | 10133 | 12465 |
| 1100 | 9490 | - | 12650 |
| 1102 | 9375 | 10873 | 12197 |
| 1103 | - | 10688 | - |
| 1104 | 9229 | 11678 | 13055 |
| 1105 | - | 10567 | 13168 |
| 1106 | 9509 | 10126 | 13003 |
| 1107 | - | 10251 | 12024 |
| 1108 | - | 10727 | 13150 |
| 1109 | - | 10719 | - |

(continued)

| SeqID | BANANA | WHEAT | MAIZE |
|---|---|---|---|
| 1110 | 9649 | 11485 | 11970 |
| 1111 | 9774 | 11130 | 13311 |
| 1112 | - | 10295 | 12833 |
| 1113 | - | 10843 | - |
| 1114 | - | 11122 | 13139 |
| 1116 | - | 10560 | - |
| 1117 | - | 10988 | 12310 |
| 1118 | - | 11477 | - |
| 1119 | - | - | 12258 |
| 1120 | - | 11900 | 13138 |
| 1121 | - | 11221 | 13146 |
| 1122 | 9648 | 11581 | 12814 |
| 1124 | - | 9999 | 12589 |
| 1125 | - | - | 12381 |
| 1126 | - | 11848 | 12416 |
| 1127 | 9322 | - | - |
| 1128 | 9578 | 11551 | 12336 |
| 1129 | - | 11035 | - |
| 1130 | - | 11841 | 12371 |
| 1132 | - | 10521 | 12184 |
| 1133 | - | 10790 | 12293 |
| 1134 | 9857 | - | - |
| 1136 | - | 11229 | 12501 |
| 1137 | - | 11399 | 12963 |
| 1138 | - | 11918 | 12957 |
| 1139 | 9875 | 11809 | 12500 |
| 1140 | 9428 | 11007 | - |
| 1141 | - | 11384 | - |
| 1142 | - | 11533 | 12635 |
| 1143 | - | - | 12686 |
| 1144 | 9603 | 10018 | - |
| 1145 | - | 11886 | 13364 |
| 1146 | - | 10604 | - |
| 1147 | - | 11808 | 12633 |
| 1148 | - | 10174 | 12550 |
| 1149 | 9506 | 11124 | 13176 |
| 1150 | 9344 | 10623 | 13192 |
| 1151 | - | 10275 | - |
| 1152 | - | - | 12394 |
| 1153 | - | 11109 | 12246 |
| 1155 | - | 11553 | 12593 |
| 1156 | - | - | 12590 |
| 1157 | - | 10175 | 12230 |
| 1158 | - | 11384 | - |
| 1160 | 9774 | 11132 | 13311 |
| 1161 | - | 10501 | - |
| 1162 | - | 10168 | 12250 |
| 1163 | - | 10776 | 12851 |
| 1164 | - | - | 12065 |
| 1166 | 9510 | 11631 | 12502 |

(continued)

| SeqID | BANANA | WHEAT | MAIZE |
|---|---|---|---|
| 1167 | - | 10074 | 12007 |
| 1168 | 9679 | 10105 | 12644 |
| 1170 | 9318 | 9902 | - |
| 1171 | 9347 | 10012 | 13212 |
| 1172 | - | - | 12590 |
| 1173 | - | 10279 | 12938 |
| 1174 | - | 10148 | - |
| 1175 | 9349 | 11223 | 12390 |
| 1176 | - | 10794 | - |
| 1177 | 9439 | - | - |
| 1178 | - | 10615 | - |
| 1179 | - | - | 12636 |
| 1180 | - | - | 13180 |
| 1181 | - | 10475 | - |
| 1183 | 9652 | 11291 | - |
| 1184 | 9763 | 11184 | 12758 |
| 1185 | - | 10601 | - |
| 1187 | 9847 | 11180 | 12644 |
| 1188 | 9370 | 10898 | 12231 |
| 1189 | 9839 | 9887 | 12097 |
| 1190 | - | - | 12918 |
| 1191 | 9713 | 10701 | - |
| 1192 | 9480 | 11646 | 13009 |
| 1194 | - | 10582 | - |
| 1195 | 9386 | 11717 | - |
| 1197 | - | 10033 | - |
| 1198 | - | 10020 | - |
| 1199 | - | 11081 | 12408 |
| 1200 | - | 9975 | 12502 |
| 1201 | - | 10071 | 12801 |
| 1202 | - | 10792 | - |
| 1203 | - | 10962 | - |
| 1204 | 9556 | 10585 | 12753 |
| 1205 | - | 11961 | - |
| 1207 | - | 10527 | - |
| 1208 | - | 10395 | - |
| 1209 | - | 10792 | 12535 |
| 1210 | - | 11495 | - |
| 1211 | - | 11211 | 12795 |
| 1212 | - | 11823 | 12224 |
| 1214 | - | 10310 | - |
| 1215 | - | 10535 | - |
| 1216 | - | 9929 | - |
| 1218 | 9470 | 11917 | - |
| 1219 | 9711 | 10885 | 13181 |
| 1220 | - | - | 13150 |
| 1221 | - | 10397 | 12785 |
| 1222 | - | 11101 | - |
| 1223 | - | 10615 | 13338 |
| 1224 | - | 11822 | - |

(continued)

| SeqID | BANANA | WHEAT | MAIZE |
|-------|--------|-------|-------|
| 1225 | 9592 | 10097 | 12464 |
| 1226 | - | 11171 | 12693 |
| 1227 | 9324 | 10985 | 13286 |
| 1229 | 9641 | 10730 | 11980 |
| 1232 | 9674 | 10722 | 12765 |
| 1233 | - | 10862 | 12419 |
| 1234 | - | 10294 | - |
| 1236 | 9774 | 11132 | 13311 |
| 1237 | 9645 | 10068 | 12294 |
| 1239 | 9836 | 10650 | 13112 |
| 1240 | 9389 | 10969 | - |
| 1241 | 9422 | 11571 | 12621 |
| 1242 | - | 11214 | 12255 |
| 1243 | - | - | 12410 |
| 1247 | 9455 | 11102 | 13298 |
| 1248 | - | 11554 | 12936 |
| 1249 | - | 10871 | 12028 |
| 1250 | 9361 | 10320 | 12046 |
| 1251 | 9723 | 10878 | 12576 |
| 1252 | - | 10570 | 13082 |
| 1253 | - | 11784 | - |
| 1254 | - | 11565 | 12532 |
| 1255 | - | 10835 | - |
| 1256 | - | - | 13057 |
| 1257 | 9487 | 10828 | 12357 |
| 1258 | 9367 | 11270 | 12525 |
| 1260 | - | 11533 | 13126 |
| 1261 | - | 9917 | 13306 |
| 1262 | 9696 | 11265 | - |
| 1264 | 9389 | 10625 | - |
| 1266 | 9508 | 11062 | 12113 |
| 1267 | 9380 | 11797 | 12041 |
| 1268 | - | 10732 | - |
| 1269 | - | 11725 | - |
| 1270 | 9316 | 10481 | 13132 |
| 1271 | - | 11023 | 12883 |
| 1272 | - | 10509 | 12295 |
| 1273 | 9415 | 10896 | 12664 |
| 1274 | 9438 | 10419 | 13028 |
| 1275 | 9840 | 11952 | 12000 |
| 1276 | - | 11303 | 12002 |
| 1277 | - | 11231 | - |
| 1278 | 9806 | 11845 | 13020 |
| 1279 | - | 11754 | - |
| 1281 | - | 10345 | 12876 |
| 1283 | - | 10832 | 12149 |
| 1284 | - | 10125 | - |
| 1285 | 9627 | 11364 | 12479 |
| 1286 | 9792 | 11570 | 13356 |
| 1288 | - | 10267 | - |

(continued)

| SeqID | BANANA | WHEAT | MAIZE |
|-------|--------|-------|-------|
| 1289 | - | 9955 | - |
| 1290 | 9636 | 10885 | 13181 |
| 1291 | - | 10381 | - |
| 1292 | 9702 | 9961 | 12643 |
| 1294 | - | 10931 | 12237 |
| 1296 | - | 11864 | 12456 |
| 1297 | 9326 | 11505 | - |
| 1299 | - | 11156 | - |
| 1300 | - | 11116 | 12011 |
| 1301 | - | 11463 | - |
| 1303 | 9551 | 10713 | 12923 |
| 1304 | - | - | 12085 |
| 1305 | - | 11019 | 12024 |
| 1306 | - | - | 12908 |
| 1307 | 9451 | 11262 | - |
| 1308 | - | 10891 | 12619 |
| 1310 | 9354 | 10398 | 13174 |
| 1311 | - | 11384 | - |
| 1314 | - | 10659 | 12070 |
| 1315 | - | 10383 | 13229 |
| 1316 | 9241 | 11068 | 12192 |
| 1317 | - | 11227 | 12607 |
| 1318 | - | 9963 | 12156 |
| 1319 | - | 11712 | 12725 |
| 1321 | 9268 | 11317 | 13336 |
| 1322 | - | 11662 | 12515 |
| 1324 | 9704 | 9973 | 12582 |
| 1325 | - | 11101 | 12176 |
| 1326 | - | 10742 | - |
| 1327 | 9348 | 10104 | 12422 |
| 1328 | 9454 | 11350 | 13052 |
| 1329 | 9640 | 11170 | 12828 |
| 1330 | - | 11758 | - |
| 1331 | 9586 | 10514 | 12480 |
| 1332 | - | 11703 | 12642 |
| 1333 | - | 10760 | - |
| 1334 | 9775 | 10792 | 12535 |
| 1335 | 9418 | - | - |
| 1336 | - | 10818 | - |
| 1337 | - | 10609 | 12163 |
| 1339 | 9541 | 11569 | 13271 |
| 1340 | - | - | 13194 |
| 1341 | 9237 | 10140 | 12756 |
| 1342 | - | 11731 | 12845 |
| 1343 | - | 11901 | 12808 |
| 1344 | 9862 | 10664 | - |
| 1345 | 9514 | 11939 | 13076 |
| 1346 | - | 11110 | 12898 |
| 1347 | - | 10768 | - |
| 1348 | - | 11906 | - |

(continued)

| SeqID | BANANA | WHEAT | MAIZE |
|-------|--------|-------|-------|
| 1351 | - | 10293 | 12054 |
| 1353 | 9545 | 10669 | - |
| 1355 | - | 11513 | - |
| 1356 | 9486 | 10301 | 12041 |
| 1358 | - | 11043 | 12938 |
| 1359 | 9293 | 10483 | - |
| 1360 | - | 11457 | 12498 |
| 1361 | - | 10102 | - |
| 1362 | - | 11404 | 13063 |
| 1363 | - | 11721 | - |
| 1364 | - | 11568 | 12949 |
| 1365 | 9372 | 10904 | 13295 |
| 1367 | - | 10177 | - |
| 1368 | - | - | 13209 |
| 1369 | 9495 | - | - |
| 1370 | 9872 | 10586 | 12939 |
| 1371 | - | 10513 | 12436 |
| 1372 | - | 11214 | 12255 |
| 1373 | - | 10288 | 12941 |
| 1374 | - | 10648 | 13214 |
| 1375 | - | 9904 | 12230 |
| 1377 | - | 11743 | - |
| 1379 | 9774 | 11396 | 13256 |
| 1380 | - | 11711 | 12271 |
| 1381 | - | 11598 | - |
| 1382 | 9745 | 10418 | 12714 |
| 1384 | 9774 | 11131 | 13134 |
| 1386 | 9613 | 11101 | - |
| 1387 | - | 11937 | - |
| 1388 | 9801 | 10559 | 12156 |
| 1389 | 9598 | 11954 | 12767 |
| 1390 | - | 11369 | 12870 |
| 1391 | - | 10895 | 13323 |
| 1392 | - | 10282 | 12474 |
| 1393 | 9691 | 11184 | 12758 |
| 1395 | - | 11734 | - |
| 1396 | 9598 | 10866 | 13342 |
| 1397 | - | 11476 | 12933 |
| 1398 | 9753 | 11300 | 12891 |
| 1400 | - | 9885 | 12061 |
| 1401 | - | - | 13299 |
| 1402 | - | 11490 | 12014 |
| 1403 | - | 11599 | - |
| 1404 | 9274 | 10791 | - |
| 1405 | - | - | 13117 |
| 1407 | - | 11241 | 12564 |
| 1408 | 9549 | 9970 | 13300 |
| 1409 | - | 10975 | 12477 |
| 1410 | - | 11245 | 12606 |
| 1411 | - | 10816 | 12746 |

(continued)

| SeqID | BANANA | WHEAT | MAIZE |
|---|---|---|---|
| 1412 | - | 10856 | - |
| 1413 | 9647 | 10660 | 13313 |
| 1414 | - | 10877 | 12892 |
| 1415 | - | 10962 | 12382 |
| 1417 | - | 9964 | 12494 |
| 1418 | 9664 | 9988 | 12075 |
| 1419 | 9534 | 11198 | - |
| 1421 | 9813 | 11391 | 13320 |
| 1422 | 9216 | 10906 | - |
| 1423 | - | 10995 | 12508 |
| 1424 | - | - | 12444 |
| 1425 | 9856 | 11098 | 13365 |
| 1426 | 9345 | 11924 | - |
| 1427 | - | 10387 | 12420 |
| 1430 | 9743 | 11655 | 12314 |
| 1431 | - | 11292 | - |
| 1432 | - | 10802 | 12236 |
| 1433 | - | 10195 | - |
| 1434 | - | 11470 | - |
| 1435 | - | 11638 | 12758 |
| 1436 | - | 10212 | 12433 |
| 1437 | - | 11603 | - |
| 1439 | 9633 | 11176 | - |
| 1440 | - | 9896 | - |
| 1441 | 9238 | 9993 | 12325 |
| 1442 | - | 11156 | 12865 |
| 1443 | - | 11878 | 12518 |
| 1444 | - | 11429 | 12677 |
| 1445 | 9636 | 10885 | 13103 |
| 1446 | - | - | 12443 |
| 1450 | - | 11374 | - |
| 1452 | - | 10355 | 12670 |
| 1453 | - | - | 12608 |
| 1455 | - | - | 12918 |
| 1456 | - | 10263 | - |
| 1457 | 9341 | 11381 | 12605 |
| 1458 | - | 10720 | - |
| 1459 | - | 11547 | 12666 |
| 1460 | - | 11644 | 12165 |
| 1462 | 9374 | 10039 | 13012 |
| 1463 | - | 11567 | - |
| 1464 | - | - | 12004 |
| 1465 | 9283 | 11507 | 12559 |
| 1466 | 9598 | 11954 | 12767 |
| 1468 | - | 10926 | 12270 |
| 1469 | - | 11538 | 12253 |
| 1470 | - | 11918 | 12113 |
| 1471 | 9512 | 11883 | 12730 |
| 1472 | - | 11681 | 13095 |
| 1473 | - | 10680 | - |

(continued)

| SeqID | BANANA | WHEAT | MAIZE |
|---|---|---|---|
| 1474 | - | 11614 | - |
| 1475 | - | 11942 | 12533 |
| 1476 | - | 10891 | 12619 |
| 1477 | 9369 | 11881 | - |
| 1479 | - | 11879 | - |
| 1480 | - | 10591 | - |
| 1481 | - | 10869 | 13149 |
| 1482 | - | - | 12507 |
| 1483 | - | - | 13266 |
| 1484 | - | 10554 | - |
| 1485 | - | 11003 | - |
| 1486 | - | 11845 | 12137 |
| 1487 | - | 10799 | - |
| 1488 | 9786 | 10958 | 12222 |
| 1489 | - | 10793 | 12141 |
| 1490 | - | 10479 | - |
| 1491 | - | 10070 | - |
| 1493 | 9384 | 11317 | 12756 |
| 1494 | - | 10781 | - |
| 1496 | - | 10288 | 12941 |
| 1497 | 9456 | 11146 | 12663 |
| 1498 | 9517 | 11266 | 12195 |
| 1499 | 9271 | 10411 | 12457 |
| 1500 | - | 10794 | - |
| 1501 | - | 11871 | 12925 |
| 1502 | - | 10183 | - |
| 1504 | 9300 | 11587 | 13179 |
| 1505 | - | 10010 | - |
| 1506 | - | 10304 | 12968 |
| 1507 | - | 10835 | - |
| 1508 | - | 11619 | - |
| 1509 | - | 10821 | 12362 |
| 1510 | - | 10002 | - |
| 1511 | - | 11400 | 12208 |
| 1512 | - | - | 12506 |
| 1513 | 9743 | 11497 | 12266 |
| 1514 | - | - | 12570 |
| 1515 | - | - | 13165 |
| 1516 | - | 11898 | - |
| 1518 | 9298 | 10925 | 13239 |
| 1519 | 9357 | 11601 | 12301 |
| 1520 | - | 10753 | - |
| 1521 | 9632 | 10211 | 12499 |
| 1522 | 9385 | 10110 | - |
| 1524 | - | 10501 | 13196 |
| 1525 | 9405 | 10268 | - |
| 1526 | 9223 | 10886 | - |
| 1528 | - | 10516 | - |
| 1529 | 9774 | 11130 | 13311 |
| 1530 | 9309 | 11753 | 12835 |

(continued)

| SeqID | BANANA | WHEAT | MAIZE |
|---|---|---|---|
| 1531 | 9516 | 10244 | 13041 |
| 1533 | - | 10763 | 13357 |
| 1534 | - | 10989 | - |
| 1535 | 9702 | 11837 | 13291 |
| 1536 | - | 9940 | - |
| 1537 | - | 11679 | - |
| 1538 | - | 10377 | - |
| 1539 | 9414 | 11640 | 12484 |
| 1540 | - | 10663 | 12870 |
| 1541 | - | 1115I | - |
| 1542 | - | - | 12509 |
| 1543 | 9373 | 11654 | - |
| 1544 | - | 11357 | - |
| 1546 | 9661 | 11928 | - |
| 1547 | - | 10885 | 13181 |
| 1549 | - | 11139 | 12766 |
| 1551 | - | 10670 | - |
| 1552 | - | 11574 | 12404 |
| 1553 | - | 11242 | 13064 |
| 1554 | - | 11768 | 12612 |
| 1555 | 9840 | 11952 | 12000 |
| 1556 | - | 11785 | - |
| 1557 | - | 11500 | - |
| 1558 | - | 11836 | - |
| 1559 | - | 10012 | 12064 |
| 1560 | - | 10312 | - |
| 1561 | - | 11578 | 12838 |
| 1562 | 9697 | 10350 | 12899 |
| 1563 | - | 11919 | - |
| 1564 | - | 11211 | 12977 |
| 1566 | 9315 | 10372 | - |
| 1567 | - | 11457 | 12895 |
| 1568 | 9552 | 11447 | 12860 |
| 1569 | - | 10740 | - |
| 1570 | - | 10167 | - |
| 1571 | - | 11232 | - |
| 1572 | - | 10511 | 12896 |
| 1573 | - | 11854 | 12721 |
| 1574 | - | 11853 | 12739 |
| 1575 | - | 11537 | 12451 |
| 1577 | 9363 | 11940 | 12324 |
| 1578 | - | - | 12730 |
| 1579 | - | 10076 | 12398 |
| 1580 | - | 10439 | 13093 |
| 1581 | - | 10545 | - |
| 1582 | - | 11101 | - |
| 1583 | - | 10081 | 12841 |
| 1584 | 9805 | 11174 | 12158 |
| 1585 | - | - | 12310 |
| 1586 | 9588 | 11013 | 12060 |

(continued)

| SeqID | BANANA | WHEAT | MAIZE |
|-------|--------|-------|-------|
| 1587 | - | 9930 | 12073 |
| 1588 | - | 11006 | - |
| 1589 | - | 10804 | - |
| 1590 | - | 9949 | - |
| 1591 | - | 10328 | 13221 |
| 1592 | - | 10986 | - |
| 1593 | 9545 | - | - |
| 1594 | - | 10004 | 13327 |
| 1596 | 9534 | 11198 | - |
| 1597 | - | 10118 | - |
| 1598 | - | 11458 | - |
| 1599 | - | 10805 | - |
| 1600 | - | 11530 | - |
| 1601 | - | 9907 | 13265 |
| 1602 | - | 10987 | 12136 |
| 1603 | - | 10639 | 11981 |
| 1605 | 9628 | 11152 | 12441 |
| 1606 | 9464 | 11572 | 12717 |
| 1608 | - | 11335 | 12844 |
| 1609 | 9245 | 9980 | 12602 |
| 1610 | - | 11111 | 12095 |
| 1611 | 9446 | 11201 | 12475 |
| 1612 | 9825 | 10181 | 12897 |
| 1613 | - | 10592 | - |
| 1614 | - | 10962 | 12393 |
| 1615 | - | 10533 | - |
| 1616 | 9316 | 11263 | 12389 |
| 1617 | - | 10482 | 13146 |
| 1619 | - | 10484 | 13126 |
| 1621 | - | 11120 | 13309 |
| 1623 | - | 10592 | 12557 |
| 1624 | 9676 | 10080 | 12779 |
| 1625 | - | 10424 | - |
| 1626 | - | - | 12824 |
| 1627 | - | 10771 | 12585 |
| 1628 | 9801 | 10394 | 12868 |
| 1629 | - | 9924 | 12150 |
| 1630 | - | 10580 | - |
| 1631 | - | 11368 | 12870 |
| 1634 | - | 11750 | - |
| 1635 | - | 10124 | 12048 |
| 1636 | - | 11520 | 12956 |
| 1637 | - | 10414 | - |
| 1639 | 9767 | 10254 | 12673 |
| 1640 | - | 10680 | - |
| 1641 | - | 11622 | 12139 |
| 1642 | 9877 | 11567 | 13373 |
| 1643 | 9539 | 10694 | 12144 |
| 1644 | 9259 | 10306 | - |
| 1646 | - | 11623 | - |

(continued)

| SeqID | BANANA | WHEAT | MAIZE |
|---|---|---|---|
| 1647 | - | 11635 | 12470 |
| 1648 | - | 10294 | 12747 |
| 1649 | - | 10159 | 13138 |
| 1650 | 9730 | 11095 | 12101 |
| 1651 | 9686 | 11613 | - |
| 1653 | 9773 | 9909 | 13107 |
| 1654 | 9699 | 11307 | 12741 |
| 1655 | - | 11945 | 12403 |
| 1656 | 9787 | 10822 | 12617 |
| 1658 | 9353 | 11839 | - |
| 1659 | 9753 | 11166 | 12613 |
| 1660 | 9389 | 11424 | 12059 |
| 1661 | - | 10205 | 12573 |
| 1662 | - | 10367 | 12063 |
| 1663 | - | 11246 | 12920 |
| 1664 | - | 9952 | 12160 |
| 1665 | - | 11492 | 12879 |
| 1666 | 9323 | 11296 | 12594 |
| 1667 | 9648 | 11831 | 12814 |
| 1668 | - | 10551 | - |
| 1669 | 9295 | 11865 | - |
| 1671 | - | - | 13005 |
| 1672 | - | 11239 | 12111 |
| 1673 | - | 10129 | 12746 |
| 1674 | - | 11639 | - |
| 1675 | 9446 | 10759 | 12380 |
| 1676 | 9486 | 11798 | 12041 |
| 1677 | - | 11546 | 12418 |
| 1678 | - | 10461 | - |
| 1679 | 9580 | 10100 | - |
| 1680 | 9311 | 10326 | 13257 |
| 1682 | - | 10513 | - |
| 1683 | - | 10638 | 12010 |
| 1685 | - | 9967 | 12100 |
| 1686 | - | 11946 | 12776 |
| 1687 | - | 10274 | 12912 |
| 1689 | - | 10346 | 12724 |
| 1690 | - | 10756 | 13136 |
| 1691 | - | 10178 | - |
| 1692 | 9329 | 11721 | 12774 |
| 1693 | 9241 | 10190 | 12120 |
| 1694 | - | 11282 | 12020 |
| 1695 | 9266 | 11575 | 12401 |
| 1696 | - | 11150 | 12323 |
| 1697 | 9534 | 11197 | 13275 |
| 1698 | - | 10715 | 12504 |
| 1699 | 9350 | 11011 | 12356 |
| 1700 | - | 10445 | 12742 |
| 1701 | - | 9891 | 13248 |
| 1702 | - | 11905 | 12292 |

(continued)

| SeqID | BANANA | WHEAT | MAIZE |
|-------|--------|-------|-------|
| 1703 | 9657 | 11175 | 12787 |
| 1704 | - | 10757 | 12492 |
| 1705 | - | 10012 | 12064 |
| 1706 | 9383 | 11354 | - |
| 1707 | - | 10630 | 11971 |
| 1708 | 9475 | 10811 | - |
| 1709 | - | 10296 | 13346 |
| 1710 | - | 11157 | - |
| 1712 | 9673 | 10927 | 12017 |
| 1713 | - | 10616 | 12390 |
| 1714 | - | 11718 | 12529 |
| 1715 | - | 10027 | 12218 |
| 1716 | - | 10568 | 12742 |
| 1717 | 9407 | 11074 | 13315 |
| 1718 | 9679 | 11181 | 13242 |
| 1719 | - | 10114 | - |
| 1720 | - | 11272 | - |
| 1721 | - | 10766 | - |
| 1722 | - | 11372 | - |
| 1723 | - | 10625 | - |
| 1725 | - | 11309 | 12531 |
| 1726 | - | 11955 | - |
| 1728 | - | 11495 | - |
| 1729 | - | 10212 | 12830 |
| 1730 | - | 9991 | 12988 |
| 1732 | - | 10183 | - |
| 1733 | - | 10153 | 12965 |
| 1734 | - | 11384 | - |
| 1736 | - | 10342 | 12918 |
| 1738 | - | 10335 | - |
| 1740 | 9490 | 11327 | - |
| 1742 | - | 10220 | 13341 |
| 1743 | - | 10387 | 12627 |
| 1745 | 9379 | 11005 | 11984 |
| 1746 | - | 10400 | 12755 |
| 1747 | - | 11211 | 12795 |
| 1748 | - | 10883 | 12369 |
| 1749 | - | 11113 | 12593 |
| 1750 | - | 10614 | 12986 |
| 1751 | - | 10176 | 12198 |
| 1752 | - | 11329 | 12848 |
| 1753 | - | 9907 | 13265 |
| 1754 | 9609 | 10410 | 13208 |
| 1755 | - | 11918 | - |
| 1756 | - | 10998 | 12659 |
| 1757 | - | - | 12079 |
| 1758 | 9613 | 11101 | - |
| 1759 | - | 11683 | 13361 |
| 1760 | - | 11342 | 12861 |
| 1761 | - | 10565 | - |

(continued)

| SeqID | BANANA | WHEAT | MAIZE |
|---|---|---|---|
| 1762 | - | 11496 | 12112 |
| 1763 | - | 11124 | 13176 |
| 1765 | - | 11368 | 13015 |
| 1766 | 9471 | 10517 | 12174 |
| 1768 | 9460 | 10552 | - |
| 1769 | - | 11536 | - |
| 1770 | - | 10337 | - |
| 1771 | 9585 | 10810 | 12615 |
| 1772 | 9625 | 11584 | 12407 |
| 1773 | - | 11431 | 11984 |
| 1774 | - | 10882 | - |
| 1775 | - | 10072 | 12784 |
| 1776 | 9753 | 11299 | 12124 |
| 1777 | - | 11454 | 12420 |
| 1778 | 9279 | 11427 | - |
| 1779 | - | - | 12310 |
| 1781 | 9440 | 11099 | 12438 |
| 1783 | - | 11710 | 13029 |
| 1784 | - | 11295 | - |
| 1785 | - | 10435 | 12642 |
| 1786 | - | 10970 | - |
| 1787 | - | 10298 | - |
| 1788 | - | - | 12885 |
| 1789 | - | 10186 | - |
| 1790 | - | 10383 | 12038 |
| 1791 | 9656 | 10884 | - |
| 1792 | - | 10666 | - |
| 1793 | - | - | 12538 |
| 1794 | - | 11484 | 12647 |
| 1795 | 9250 | 10602 | 12219 |
| 1796 | - | 10187 | 12874 |
| 1798 | - | 10404 | - |
| 1799 | - | 10213 | 12820 |
| 1800 | - | 11498 | 12524 |
| 1802 | - | 10169 | 12462 |
| 1803 | 9325 | 10553 | 11982 |
| 1804 | - | 10821 | 12038 |
| 1805 | - | 9901 | - |
| 1806 | 9524 | 11542 | - |
| 1807 | - | 10263 | - |
| 1808 | 9859 | 11179 | 12392 |
| 1809 | 9406 | 11119 | 12813 |
| 1810 | - | 10271 | 12957 |
| 1811 | - | 10662 | 13094 |
| 1812 | 9822 | 9944 | 12637 |
| 1814 | - | 11911 | 12863 |
| 1815 | - | 11597 | - |
| 1816 | - | 11235 | 13274 |
| 1817 | - | 11316 | 12848 |
| 1818 | - | 10252 | - |

(continued)

| SeqID | BANANA | WHEAT | MAIZE |
|-------|--------|-------|-------|
| 1819 | 9241 | 11068 | 12516 |
| 1820 | 9801 | 11528 | 13243 |
| 1821 | - | 11810 | 13051 |
| 1822 | 9336 | 10700 | - |
| 1824 | 9752 | 10528 | 12638 |
| 1825 | - | 11467 | 12544 |
| 1826 | - | 11833 | - |
| 1827 | - | - | 12315 |
| 1828 | 9275 | 10939 | 13355 |
| 1829 | - | 10077 | 12704 |
| 1830 | - | 10633 | 12566 |
| 1831 | - | 10059 | - |
| 1832 | - | - | 12611 |
| 1833 | 9656 | 10972 | 12853 |
| 1835 | - | 11225 | 12320 |
| 1836 | - | - | 12294 |
| 1837 | 9614 | 10806 | 13021 |
| 1838 | - | 10470 | 12723 |
| 1841 | - | 10643 | - |
| 1842 | - | 10025 | 12050 |
| 1844 | - | 10092 | - |
| 1845 | - | 11247 | 12831 |
| 1846 | - | 10872 | 13113 |
| 1847 | - | 10291 | - |
| 1848 | - | - | 12550 |
| 1849 | - | - | 11985 |
| 1850 | - | 10115 | 12267 |
| 1851 | 9234 | 11658 | 12635 |
| 1852 | - | 11189 | 12790 |
| 1853 | - | 11951 | 13255 |
| 1854 | 9750 | 10289 | 12972 |
| 1855 | 9232 | 11184 | 12758 |
| 1856 | 9542 | 10494 | - |
| 1858 | 9876 | 11349 | - |
| 1859 | - | 10016 | - |
| 1860 | - | 11108 | 12246 |
| 1861 | - | 11900 | 13138 |
| 1863 | 9356 | 9980 | 12602 |
| 1864 | 9394 | 11721 | - |
| 1865 | - | 10627 | - |
| 1866 | - | - | 12811 |
| 1867 | - | 10542 | 12752 |
| 1868 | 9530 | 11742 | 13152 |
| 1869 | - | 10387 | 12627 |
| 1871 | - | 10057 | 13041 |
| 1872 | - | 11855 | - |
| 1873 | 9532 | 11036 | 11997 |
| 1874 | - | 11370 | 12999 |
| 1876 | - | 10358 | 12809 |
| 1877 | - | 11964 | 12023 |

**EP 1 925 672 A1**

(continued)

| SeqID | BANANA | WHEAT | MAIZE |
|---|---|---|---|
| 1878 | 9359 | 11259 | - |
| 1879 | 9716 | 11167 | 12613 |
| 1880 | - | 9896 | - |
| 1881 | - | - | 12175 |
| 1882 | 9548 | 11474 | 12596 |
| 1884 | - | 11750 | - |
| 1885 | - | 10259 | 12271 |
| 1886 | - | 11080 | 12764 |
| 1887 | - | 11888 | 12549 |
| 1888 | - | 11950 | - |
| 1890 | - | 10994 | - |
| 1891 | 9879 | 11616 | - |
| 1892 | 9299 | 11243 | 13238 |
| 1894 | 9608 | 9916 | 12005 |
| 1895 | - | 10592 | 12108 |
| 1897 | - | 10204 | 13200 |
| 1898 | - | - | 12419 |
| 1900 | - | 11496 | - |
| 1901 | - | 11298 | 11972 |
| 1902 | - | 10971 | 12578 |
| 1904 | 9846 | - | 12901 |
| 1906 | 9241 | 11069 | 12516 |
| 1907 | 9272 | 11228 | 12902 |
| 1908 | - | 10232 | - |
| 1909 | 9413 | 10229 | 12537 |
| 1911 | - | 11862 | - |
| 1912 | - | 11183 | 13140 |
| 1913 | 9579 | 11686 | 12190 |
| 1914 | 9332 | 11261 | 13316 |
| 1915 | 9514 | 10736 | 12879 |
| 1916 | - | 11626 | 12689 |
| 1917 | - | 11311 | 12743 |
| 1918 | - | 10812 | 12171 |
| 1919 | 9583 | 11258 | 12540 |
| 1921 | 9814 | 11217 | 12829 |
| 1922 | 9612 | 11377 | 13102 |
| 1924 | - | 9987 | - |
| 1925 | - | 10876 | 12762 |
| 1926 | - | 10171 | - |
| 1927 | - | 10478 | 12062 |
| 1928 | - | 11556 | - |
| 1929 | - | 9882 | 12563 |
| 1930 | - | 11073 | 13142 |
| 1931 | - | - | 12379 |
| 1932 | - | 10599 | - |
| 1935 | 9748 | 11858 | 12182 |
| 1936 | 9282 | 11947 | 13115 |
| 1937 | - | 10787 | 12555 |
| 1938 | 9223 | 10837 | - |
| 1939 | - | 11767 | 12409 |

(continued)

| SeqID | BANANA | WHEAT | MAIZE |
|-------|--------|-------|-------|
| 1940 | - | 11161 | - |
| 1941 | - | 11332 | - |
| 1944 | 9394 | 11721 | - |
| 1946 | - | 10893 | 12478 |
| 1947 | - | 10499 | - |
| 1948 | 9446 | 11202 | 12428 |
| 1950 | 9762 | 11393 | - |
| 1951 | - | - | 13075 |
| 1952 | 9271 | 11194 | 12457 |
| 1953 | - | 11588 | 12246 |
| 1954 | 9404 | 10741 | 13053 |
| 1955 | - | 10588 | 12810 |
| 1956 | - | 11944 | 13351 |
| 1957 | 9659 | 11456 | 13348 |
| 1958 | - | 11159 | - |
| 1959 | - | 11416 | - |
| 1960 | - | 11821 | - |
| 1961 | - | 11788 | 12989 |
| 1963 | - | - | 12258 |
| 1965 | - | 10323 | - |
| 1967 | - | 11953 | 13077 |
| 1969 | - | 11580 | 12185 |
| 1970 | - | 11927 | - |
| 1972 | - | 11830 | - |
| 1973 | 9695 | 11376 | 12018 |
| 1974 | - | 11147 | 12906 |
| 1977 | - | 11723 | - |
| 1980 | - | - | 12786 |
| 1981 | - | 11557 | - |
| 1985 | - | 10679 | - |
| 1987 | 9796 | 11236 | 13335 |
| 1988 | 9220 | 11769 | 12313 |
| 1989 | - | - | 12788 |
| 1991 | 9618 | 11420 | 12403 |
| 1992 | - | - | 12283 |
| 1993 | - | 10324 | 12430 |
| 1994 | - | 9995 | - |
| 1996 | - | 11562 | - |
| 1997 | - | 9995 | - |
| 2000 | - | 9942 | 12035 |
| 2002 | - | 9995 | - |
| 2004 | 9866 | 11452 | - |
| 2006 | - | 10161 | - |
| 2007 | - | 10437 | 13023 |
| 2008 | - | - | 13193 |
| 2009 | 9849 | 10290 | 13202 |
| 2011 | 9791 | 11563 | 12213 |
| 2014 | - | 10376 | 13151 |
| 2017 | - | 10807 | - |
| 2018 | - | 9985 | - |

(continued)

| SeqID | BANANA | WHEAT | MAIZE |
|---|---|---|---|
| 2020 | - | 10703 | - |
| 2024 | - | 11688 | 12228 |
| 2025 | - | 11723 | 13358 |
| 2028 | - | - | 13126 |
| 2030 | 9835 | 11273 | 13308 |
| 2035 | - | 11486 | 13119 |
| 2037 | - | 9995 | 11987 |
| 2041 | - | 11615 | 13105 |
| 2042 | - | 10531 | 13127 |
| 2043 | - | 10236 | 12519 |
| 2044 | - | 11362 | 12701 |
| 2045 | - | - | 13079 |
| 2046 | 9790 | - | - |
| 2049 | - | 10030 | 12205 |
| 2050 | - | 10754 | 13014 |
| 2051 | - | 9995 | - |
| 2054 | - | - | 12743 |
| 2055 | 9780 | 11735 | 12467 |
| 2057 | - | 10880 | 12018 |
| 2060 | 9834 | 11273 | 13111 |
| 2063 | 9581 | 11849 | 13277 |
| 2064 | - | 9939 | 12083 |
| 2065 | 9320 | - | 12624 |
| 2066 | - | 9959 | - |
| 2067 | - | 11868 | 13236 |
| 2068 | - | - | 12541 |
| 2069 | - | 11142 | 12772 |
| 2070 | - | 10380 | 12439 |
| 2073 | 9618 | 11421 | 13077 |
| 2074 | - | 11494 | 12391 |
| 2076 | - | 10088 | 11998 |
| 2077 | - | 11747 | 12884 |
| 2079 | - | 10938 | - |
| 2081 | - | - | 12890 |
| 2083 | - | 10324 | 12887 |
| 2085 | 9287 | 11953 | 12353 |
| 2086 | - | 10361 | 13135 |
| 2087 | - | 11923 | 12354 |
| 2088 | - | 10464 | - |
| 2089 | 9618 | 11420 | 12584 |
| 2091 | - | 10042 | 12981 |
| 2092 | - | 10468 | 13207 |
| 2093 | - | - | 11993 |
| 2094 | - | 9995 | - |
| 2095 | - | 10043 | 12458 |
| 2096 | 9618 | 11421 | 12180 |
| 2097 | - | - | 13269 |
| 2098 | - | 9910 | 12384 |
| 2101 | 9606 | 10388 | - |
| 2103 | 9610 | 10914 | - |

(continued)

| SeqID | BANANA | WHEAT | MAIZE |
|---|---|---|---|
| 2104 | - | 10530 | 12290 |
| 2105 | - | 9939 | 13262 |
| 2107 | - | 9921 | - |
| 2109 | - | 11207 | - |
| 2113 | 9410 | 11805 | - |
| 2116 | - | 9939 | 13262 |
| 2117 | 9287 | 11953 | 12353 |
| 2118 | - | 11494 | 12391 |
| 2119 | - | 9921 | 12981 |
| 2122 | - | 10113 | - |
| 2123 | - | - | 11969 |
| 2125 | - | 11827 | - |
| 2126 | 9650 | 9911 | 12552 |
| 2128 | 9835 | 11287 | 13308 |
| 2129 | - | 9995 | - |
| 2130 | - | 11752 | - |
| 2131 | 9864 | - | 12142 |
| 2132 | - | - | 12712 |
| 2133 | - | 10924 | 13056 |
| 2135 | - | 10324 | 12430 |
| 2136 | 9618 | 11032 | 12905 |
| 2137 | - | - | 12743 |
| 2139 | 9797 | 11502 | 12080 |
| 2143 | - | - | 12743 |
| 2144 | - | 9893 | - |
| 2145 | - | 9995 | - |
| 2147 | - | 9995 | - |
| 2148 | 9519 | 11770 | 12802 |
| 2149 | - | 11574 | 12691 |
| 2153 | - | 11915 | - |
| 2154 | - | 10324 | 12430 |
| 2156 | - | 11163 | 12106 |
| 2158 | 9791 | 10855 | 12523 |
| 2164 | - | 10731 | - |
| 2166 | - | - | 13268 |
| 2168 | - | 11548 | - |
| 2169 | - | 11341 | 13173 |
| 2170 | - | 10420 | - |
| 2172 | - | 11535 | 12934 |
| 2174 | - | 10541 | 12110 |
| 2175 | - | 10208 | - |
| 2176 | - | 9971 | - |
| 2177 | - | 11800 | 13353 |
| 2178 | - | 10496 | - |
| 2181 | - | 10045 | 12014 |
| 2183 | - | - | 12172 |
| 2184 | 9842 | 11438 | 12181 |
| 2185 | - | 11659 | - |
| 2186 | 9550 | 10834 | - |
| 2187 | - | 10655 | - |

(continued)

| SeqID | BANANA | WHEAT | MAIZE |
|---|---|---|---|
| 2188 | - | 10217 | - |
| 2189 | - | 11789 | 13190 |
| 2190 | - | 9941 | - |
| 2192 | - | 11512 | 12051 |
| 2193 | - | 11838 | - |
| 2195 | - | - | 12657 |
| 2196 | 9452 | 10598 | - |
| 2197 | - | 10619 | 12113 |
| 2198 | 9655 | 10977 | 13156 |
| 2199 | - | 10778 | 12852 |
| 2200 | - | 10065 | - |
| 2201 | - | - | 12398 |
| 2202 | - | 10003 | - |
| 2209 | - | 11521 | - |
| 2210 | - | 10210 | - |
| 2213 | - | 11398 | 12538 |
| 2214 | 9759 | 11010 | 12561 |
| 2215 | - | 11894 | - |
| 2216 | - | 10890 | 12272 |
| 2218 | - | 11379 | - |
| 2219 | 9233 | 10378 | 12671 |
| 2220 | - | 10809 | 13204 |
| 2221 | - | 10875 | 12159 |
| 2223 | - | 11896 | - |
| 2224 | - | 11105 | 12210 |
| 2229 | - | 10392 | - |
| 2230 | - | 10121 | - |
| 2231 | - | 11766 | 12507 |
| 2232 | - | 10092 | 13309 |
| 2233 | - | - | 13253 |
| 2234 | - | - | 12723 |
| 2235 | - | 10311 | 12021 |
| 2237 | - | 10629 | - |
| 2239 | 9638 | - | 12651 |
| 2240 | - | 10359 | - |
| 2241 | - | 10067 | 12300 |
| 2242 | - | 10235 | - |
| 2245 | - | 10463 | 13159 |
| 2246 | - | 9910 | 12384 |
| 2247 | - | 11034 | 13233 |
| 2248 | - | 11289 | 12658 |
| 2249 | - | 11799 | 12827 |
| 2250 | - | 10519 | - |
| 2251 | - | - | 12775 |
| 2252 | - | 11772 | - |
| 2253 | - | - | 12861 |
| 2255 | - | 10277 | - |
| 2257 | - | 10580 | 13209 |
| 2258 | - | 11186 | - |
| 2259 | - | 10447 | - |

(continued)

| SeqID | BANANA | WHEAT | MAIZE |
|---|---|---|---|
| 2260 | - | 10877 | 12892 |
| 2265 | - | 10441 | - |
| 2267 | - | 10121 | - |
| 2268 | - | 11331 | 12252 |
| 2269 | - | 10232 | - |
| 2270 | - | 10145 | 12034 |
| 2274 | - | 11286 | - |
| 2279 | - | 11388 | - |
| 2280 | - | 10922 | - |
| 2281 | - | 11363 | 12743 |
| 2284 | - | 11748 | - |
| 2285 | - | 10142 | - |
| 2286 | 9740 | 10782 | 11995 |
| 2288 | 9397 | 10364 | 13057 |
| 2289 | - | 10952 | 12953 |
| 2290 | - | 11843 | - |
| 2291 | - | - | 12825 |
| 2292 | - | - | 13130 |
| 2295 | - | 10341 | 12105 |
| 2297 | - | - | 12925 |
| 2298 | - | 10842 | 13004 |
| 2299 | - | 9932 | 12869 |
| 2301 | 9653 | 11661 | 12405 |
| 2302 | - | 11402 | - |
| 2304 | - | - | 12254 |
| 2305 | 9766 | - | 12645 |
| 2308 | - | 11021 | 12262 |
| 2309 | 9576 | - | - |
| 2310 | - | 10179 | - |
| 2311 | - | 11868 | 13236 |
| 2312 | - | 9894 | - |
| 2313 | - | 11847 | 12754 |
| 2317 | - | 10472 | 12001 |
| 2318 | - | 10911 | 12928 |
| 2319 | 9591 | 11442 | 12726 |
| 2320 | - | 11466 | 12882 |
| 2321 | - | 11121 | 13263 |
| 2322 | - | 11719 | 13304 |
| 2325 | - | 10613 | 13344 |
| 2326 | - | 11806 | - |
| 2329 | 9584 | 10566 | - |
| 2332 | - | 10605 | 13247 |
| 2334 | - | 11387 | - |
| 2335 | - | 11812 | 12556 |
| 2336 | - | 11814 | 13068 |
| 2337 | - | 11002 | - |
| 2338 | - | - | 12499 |
| 2340 | - | 10304 | 12968 |
| 2344 | 9727 | 10050 | - |
| 2346 | - | 10085 | 12213 |

(continued)

| SeqID | BANANA | WHEAT | MAIZE |
|-------|--------|-------|-------|
| 2348 | - | - | 13211 |
| 2349 | 9718 | - | - |
| 2351 | - | 10309 | 12861 |
| 2352 | - | 11799 | 13353 |
| 2354 | - | 10188 | - |
| 2356 | - | - | 13301 |
| 2357 | - | 10382 | 13038 |
| 2359 | 9432 | 10881 | 12461 |
| 2360 | - | 11432 | 13337 |
| 2361 | 9219 | 10550 | 12780 |
| 2365 | - | 10686 | 13306 |
| 2366 | - | 10525 | - |
| 2368 | - | 9918 | 12029 |
| 2369 | - | 10619 | 13309 |
| 2370 | - | 11473 | 12732 |
| 2371 | - | 10747 | 12722 |
| 2372 | - | - | 13114 |
| 2374 | 9675 | - | 13188 |
| 2375 | 9479 | 10352 | 12122 |
| 2378 | - | 10840 | 12415 |
| 2379 | - | 10449 | - |
| 2381 | 9644 | - | 12115 |
| 2382 | - | 10022 | 13123 |
| 2384 | - | 10761 | 13108 |
| 2386 | - | 10283 | 12980 |
| 2390 | - | 11818 | - |
| 2391 | - | 10772 | - |
| 2392 | 9236 | 10813 | 13223 |
| 2395 | - | - | 12861 |
| 2396 | - | 10392 | - |
| 2397 | - | 10336 | 12093 |
| 2400 | - | 11114 | 12058 |
| 2401 | - | 11516 | - |
| 2402 | 9276 | 10338 | 13305 |
| 2405 | - | 11757 | - |
| 2406 | - | - | 12157 |
| 2407 | 9665 | - | - |
| 2408 | 9457 | - | 11995 |
| 2409 | - | 11652 | 12037 |
| 2410 | - | - | 12476 |
| 2411 | - | 10712 | - |
| 2412 | - | 10846 | - |
| 2413 | - | 11031 | 12743 |
| 2417 | - | 10847 | - |
| 2419 | - | 11897 | 12370 |
| 2422 | - | 11624 | 12215 |
| 2425 | - | 10948 | 12311 |
| 2429 | - | 11407 | - |
| 2432 | - | 11455 | - |
| 2435 | 9294 | 10897 | 13100 |

(continued)

| SeqID | BANANA | WHEAT | MAIZE |
|---|---|---|---|
| 2437 | - | - | 12448 |
| 2439 | - | 10093 | - |
| 2441 | - | 11186 | - |
| 2442 | - | 11715 | 12805 |
| 2443 | - | 10396 | - |
| 2444 | - | - | 12531 |
| 2445 | - | 11670 | - |
| 2446 | - | 10912 | 12928 |
| 2447 | 9525 | 10870 | - |
| 2448 | - | 11439 | 13013 |
| 2449 | - | 10887 | - |
| 2450 | - | 11047 | 12044 |
| 2451 | - | 10516 | - |
| 2452 | - | 10442 | 11974 |
| 2453 | - | 11782 | - |
| 2454 | - | 10705 | 13310 |
| 2456 | - | 11933 | - |
| 2458 | - | 11744 | - |
| 2459 | - | 11773 | 12090 |
| 2461 | 9873 | 10786 | 12946 |
| 2462 | 9788 | 11375 | 12807 |
| 2465 | - | 10673 | 12985 |
| 2467 | - | 10240 | 12974 |
| 2471 | - | - | 11979 |
| 2472 | - | 11021 | 12334 |
| 2474 | - | 11021 | 12189 |
| 2476 | 9420 | - | - |
| 2480 | - | 11186 | - |
| 2482 | - | 10808 | - |
| 2483 | 9597 | 11471 | 12542 |
| 2485 | - | 10215 | - |
| 2486 | - | 11740 | 12680 |
| 2487 | - | 10055 | - |
| 2492 | - | 10459 | - |
| 2495 | - | 11011 | 12985 |
| 2496 | - | - | 12598 |
| 2499 | - | 10269 | - |
| 2501 | - | 10717 | - |
| 2503 | - | 10392 | - |
| 2504 | 9655 | 10520 | 12493 |
| 2507 | - | 11799 | 13353 |
| 2510 | - | 10709 | 12962 |
| 2511 | 9637 | 11838 | - |
| 2512 | - | 10284 | 12098 |
| 2515 | - | - | 13016 |
| 2516 | - | 10452 | - |
| 2519 | - | - | 12138 |
| 2521 | - | 10412 | 12248 |
| 2524 | - | - | 12481 |
| 2528 | - | - | 12864 |

(continued)

| SeqID | BANANA | WHEAT | MAIZE |
|---|---|---|---|
| 2529 | - | - | 12537 |
| 2530 | - | 10726 | 13333 |
| 2532 | - | - | 12772 |
| 2533 | - | - | 12367 |
| 2534 | - | 10031 | - |
| 2536 | - | - | 12589 |
| 2537 | - | 10695 | - |
| 2538 | - | 10854 | 13312 |
| 2539 | - | 10035 | 11990 |
| 2541 | - | - | 12473 |
| 2542 | - | 11421 | 12180 |
| 2544 | - | 10237 | 12768 |
| 2545 | - | 10089 | 12890 |
| 2547 | - | 10421 | 12875 |
| 2549 | 9550 | 11589 | 13245 |
| 2550 | 9278 | 10908 | 13008 |
| 2551 | - | 9897 | 12768 |
| 2552 | - | 11408 | 12593 |
| 2553 | - | 10593 | - |
| 2555 | - | 11736 | 13080 |
| 2557 | - | - | 12240 |
| 2559 | - | 10487 | 13162 |
| 2562 | - | 10436 | 12685 |
| 2564 | 9710 | 10226 | 12397 |
| 2567 | - | 10734 | 13331 |
| 2571 | - | - | 12617 |
| 2573 | - | 10874 | 12683 |
| 2575 | - | 11586 | 13091 |
| 2578 | - | 11959 | 12861 |
| 2579 | - | - | 13326 |
| 2585 | 9766 | - | 12251 |
| 2587 | - | 10399 | 13329 |
| 2588 | - | 11943 | 13104 |
| 2589 | - | 10574 | 13324 |
| 2590 | - | 10221 | - |
| 2591 | - | 11766 | 12507 |
| 2592 | - | - | 13126 |
| 2593 | - | 10779 | - |
| 2594 | - | 10491 | 12775 |
| 2595 | - | 10304 | 12968 |
| 2597 | - | 11873 | - |
| 2598 | - | 10698 | 12861 |
| 2600 | - | . 10557 | - |
| 2601 | - | - | 13269 |
| 2602 | 9662 | 10347 | - |
| 2604 | - | 10824 | - |
| 2606 | - | - | 12453 |
| 2607 | - | - | 12864 |
| 2608 | - | 10467 | |
| 2610 | - | - | 12699 |

(continued)

| SeqID | BANANA | WHEAT | MAIZE |
|---|---|---|---|
| 2613 | - | 10785 | - |
| 2614 | - | - | 13038 |
| 2615 | 9804 | 11884 | 12346 |
| 2619 | 9865 | 11434 | 12836 |
| 2622 | 9514 | 11755 | 12979 |
| 2623 | | 11107 | 12319 |
| 2627 | - | 10163 | 12239 |
| 2628 | - | 10122 | - |
| 2635 | 9655 | 10977 | 13156 |
| 2638 | - | 10677 | 12917 |
| 2639 | - | 10256 | - |
| 2640 | - | 11009 | 13224 |
| 2641 | - | 11694 | 12991 |
| 2642 | - | - | 13241 |
| 2646 | - | 10973 | 12901 |
| 2647 | - | - | 12071 |
| 2648 | 9809 | 10241 | 13141 |
| 2649 | 9501 | 10370 | - |
| 2652 | - | 10474 | 12276 |
| 2653 | - | 10386 | - |
| 2656 | - | 10087 | 13045 |
| 2658 | - | 11279 | 12204 |
| 2659 | - | - | 12241 |
| 2660 | - | 11621 | - |
| 2661 | 9604 | 11451 | 13270 |
| 2662 | - | 11908 | 13206 |
| 2664 | - | 11804 | - |
| 2665 | - | 10455 | 12904 |
| 2666 | - | 11783 | - |
| 2667 | - | 11620 | 12955 |
| 2670 | - | - | 13354 |
| 2673 | - | 10556 | 13247 |
| 2675 | 9709 | 10260 | 12245 |
| 2676 | - | 11423 | 12225 |
| 2678 | - | 11869 | 12296 |
| 2679 | - | - | 12628 |
| 2680 | - | 10154 | - |
| 2681 | - | 10019 | 12240 |
| 2683 | - | 10933 | 13362 |
| 2685 | - | 11516 | - |
| 2686 | - | 10232 | - |
| 2688 | - | 11347 | 12684 |
| 2691 | - | 10668 | - |
| 2692 | - | 10788 | 12800 |
| 2694 | - | - | 13039 |
| 2695 | - | 11882 | 12074 |
| 2696 | - | - | 13290 |
| 2697 | - | 10890 | - |
| 2699 | - | 10861 | - |
| 2700 | - | 10642 | 12685 |

(continued)

| SeqID | BANANA | WHEAT | MAIZE |
|-------|--------|-------|-------|
| 2701 | - | - | 12193 |
| 2705 | - | 11690 | - |
| 2706 | - | 10047 | - |
| 2707 | 9270 | 11700 | - |
| 2708 | - | - | 12890 |
| 2710 | - | - | 13312 |
| 2711 | - | - | 12878 |
| 2713 | - | - | 12188 |
| 2715 | - | 10459 | - |
| 2716 | - | 11677 | - |
| 2717 | - | 11083 | - |
| 2718 | - | 11252 | 12220 |
| 2719 | 9860 | - | 13249 |
| 2720 | - | 9942 | 13121 |
| 2725 | - | 10438 | 13183 |
| 2727 | - | 10692 | - |
| 2728 | - | - | 13018 |
| 2730 | - | 10847 | 12105 |
| 2731 | - | - | 12733 |
| 2732 | - | - | 12890 |
| 2733 | - | - | 13089 |
| 2735 | - | 9881 | - |
| 2737 | - | 10046 | - |
| 2739 | - | 10384 | - |
| 2740 | 9281 | 10936 | 12630 |
| 2741 | - | 11829 | - |
| 2742 | - | 10257 | - |
| 2743 | - | 11575 | 13283 |
| 2744 | - | 10555 | - |
| 2745 | - | 11293 | - |
| 2746 | - | - | 12511 |
| 2748 | 9215 | 10194 | 13024 |
| 2749 | - | - | 12342 |
| 2750 | - | 10228 | - |
| 2752 | - | - | 12367 |
| 2753 | - | 10755 | 13357 |
| 2754 | - | 10141 | - |
| 2755 | - | 10504 | - |
| 2756 | - | 11903 | - |
| 2757 | 9275 | 10940 | 12242 |
| 2759 | - | 10538 | 12441 |
| 2760 | 9849 | 11310 | 12842 |
| 2761 | - | 11625 | 12692 |
| 2762 | - | - | 12688 |
| 2763 | - | - | 13293 |
| 2768 | - | 11451 | 13270 |
| 2772 | - | 10767 | - |
| 2773 | - | 11030 | - |
| 2775 | 9483 | 11714 | 12703 |
| 2776 | - | 10447 | - |

(continued)

| SeqID | BANANA | WHEAT | MAIZE |
|---|---|---|---|
| 2778 | - | 10354 | 12639 |
| 2780 | - | 10134 | - |
| 2783 | - | 11444 | 12490 |
| 2784 | - | 10578 | - |
| 2786 | - | 11561 | - |
| 2787 | - | - | 13125 |
| 2788 | - | 11890 | - |
| 2789 | - | 11909 | 12937 |
| 2792 | 9598 | - | 13342 |
| 2793 | - | 10988 | 12506 |
| 2795 | - | 10109 | 12515 |
| 2796 | - | 10182 | - |
| 2797 | - | - | 12996 |
| 2799 | - | - | 12235 |
| 2801 | - | 10037 | - |
| 2802 | - | - | 12081 |
| 2803 | - | 10737 | - |
| 2805 | - | 11026 | 12128 |
| 2809 | 9351 | 10448 | 12235 |
| 2810 | 9547 | 10959 | 11999 |
| 2811 | - | - | 13294 |
| 2813 | - | - | 12317 |
| 2814 | - | 10234 | 13305 |
| 2816 | - | 11907 | - |
| 2817 | - | 11000 | - |
| 2818 | - | - | 12203 |
| 2820 | - | 10144 | 12649 |
| 2821 | - | 10698 | - |
| 2822 | 9683 | - | 12117 |
| 2825 | - | 10189 | - |
| 2826 | - | 10492 | 12121 |
| 2827 | - | 10677 | 13198 |
| 2829 | - | - | 12126 |
| 2831 | - | 10371 | - |
| 2832 | 9559 | - | 12978 |
| 2834 | - | 10710 | - |
| 2836 | - | 10303 | - |
| 2838 | 9860 | 11449 | 13124 |
| 2839 | - | 11222 | 12839 |
| 2841 | - | - | 12399 |
| 2842 | - | - | 12961 |
| 2844 | - | 11426 | - |
| 2846 | - | - | 12352 |
| 2847 | - | 11719 | 13304 |
| 2848 | 9550 | 10456 | 13217 |
| 2849 | - | 10632 | - |
| 2851 | - | 11337 | 13169 |
| 2853 | 9611 | 9983 | 12694 |
| 2854 | - | 10830 | 13276 |
| 2855 | - | 10683 | - |

(continued)

| SeqID | BANANA | WHEAT | MAIZE |
|---|---|---|---|
| 2856 | - | 10375 | - |
| 2857 | - | 10831 | 13036 |
| 2858 | - | 11875 | 12335 |
| 2860 | - | - | 12511 |
| 2861 | - | 10099 | - |
| 2863 | - | - | 13119 |
| 2864 | 9860 | 11084 | 12358 |
| 2867 | - | 10497 | - |
| 2869 | - | 9982 | - |
| 2870 | 9533 | - | 11983 |
| 2871 | - | 10083 | - |
| 2872 | 9435 | 10430 | - |
| 2874 | - | - | 12928 |
| 2876 | - | 10327 | 13287 |
| 2878 | 9462 | 11687 | 13007 |
| 2879 | - | 11604 | 12787 |
| 2880 | - | 10011 | - |
| 2881 | 9655 | - | 12493 |
| 2882 | - | - | 12196 |
| 2884 | - | 11480 | - |
| 2886 | - | 10676 | 13343 |
| 2887 | 9493 | 10708 | 12616 |
| 2889 | - | - | 12278 |
| 2890 | - | - | 12494 |
| 2891 | - | 11345 | 12489 |
| 2892 | 9717 | 10956 | 12194 |
| 2893 | 9406 | 11118 | 12813 |
| 2895 | - | 11773 | 12090 |
| 2899 | 9265 | 10106 | - |
| 2900 | - | 11693 | 12794 |
| 2901 | - | - | 12495 |
| 2902 | - | 11545 | 12045 |
| 2904 | - | 10086 | - |
| 2906 | 9458 | 10271 | 12843 |
| 2907 | - | 10041 | - |
| 2908 | - | 10261 | - |
| 2909 | - | 10864 | 13011 |
| 2910 | - | 10425 | - |
| 2912 | 9670 | 10814 | - |
| 2913 | - | 11934 | - |
| 2914 | - | 11509 | - |
| 2916 | 9395 | - | - |
| 2918 | 9515 | 10270 | 12545 |
| 2919 | - | - | 12105 |
| 2920 | - | 10064 | - |
| 2921 | - | 10702 | 13148 |
| 2922 | - | 10826 | - |
| 2924 | - | 11468 | 12116 |
| 2926 | - | - | 12622 |
| 2927 | - | 10524 | - |

(continued)

| SeqID | BANANA | WHEAT | MAIZE |
|---|---|---|---|
| 2929 | - | 11448 | 13331 |
| 2932 | - | 10857 | - |
| 2934 | - | - | 13226 |
| 2935 | - | - | 13229 |
| 2936 | - | 9889 | 13332 |
| 2937 | - | 10339 | 12274 |
| 2938 | - | 11760 | - |
| 2939 | - | 10048 | - |
| 2940 | - | 10353 | - |
| 2942 | - | - | 12039 |
| 2943 | - | 10953 | 12953 |
| 2945 | - | - | 12206 |
| 2948 | 9816 | 11148 | 13002 |
| 2950 | - | 11958 | 12343 |
| 2952 | - | 10523 | - |
| 2953 | - | 10031 | - |
| 2954 | - | 11689 | - |
| 2955 | - | 10147 | 12240 |
| 2956 | - | - | 11992 |
| 2959 | 9823 | - | - |
| 2960 | - | 10714 | - |
| 2961 | - | 10408 | - |
| 2966 | 9843 | 10549 | - |
| 2971 | - | - | 13314 |
| 2972 | - | 10299 | - |
| 2973 | - | 11611 | 12234 |
| 2976 | - | 10819 | - |
| 2977 | - | 11648 | 13209 |
| 2980 | 9297 | 10052 | 12728 |
| 2983 | - | 11240 | 13072 |
| 2984 | - | 9931 | - |
| 2986 | - | 9951 | 12132 |
| 2988 | - | 9922 | 12006 |
| 2989 | - | 11218 | - |
| 2991 | - | 11086 | - |
| 2993 | 9861 | 11895 | 12358 |
| 2995 | - | 11704 | 12238 |
| 2997 | - | 10131 | - |
| 2998 | - | 10123 | - |
| 2999 | - | - | 12760 |
| 3001 | - | 11355 | 12468 |
| 3002 | - | - | 13280 |
| 3004 | - | 10846 | 12179 |
| 3005 | - | 10439 | - |
| 3006 | 9732 | 10219 | 12791 |
| 3007 | 9453 | 11251 | - |
| 3010 | - | 11725 | - |
| 3012 | 9582 | 11651 | 12214 |
| 3013 | - | 10349 | 12929 |
| 3014 | - | 11021 | 12189 |

(continued)

| SeqID | BANANA | WHEAT | MAIZE |
|---|---|---|---|
| 3016 | - | 10723 | 12104 |
| 3017 | - | 11549 | - |
| 3018 | 9619 | 10947 | - |
| 3019 | 9795 | 10596 | 12818 |
| 3021 | 9240 | - | - |
| 3022 | 9399 | 11360 | - |
| 3023 | - | 11842 | 12798 |
| 3024 | - | - | 13154 |
| 3028 | - | 10823 | 12917 |
| 3029 | - | 10049 | 12471 |
| 3031 | - | 11027 | 12982 |
| 3032 | - | 11518 | - |
| 3034 | - | 10152 | 13370 |
| 3036 | 9485 | 10841 | - |
| 3037 | - | 11649 | 12987 |
| 3039 | - | 10307 | 12759 |
| 3041 | - | 11412 | 12057 |
| 3043 | - | 11585 | 12777 |
| 3044 | - | - | 13164 |
| 3045 | - | 11503 | - |
| 3046 | 9803 | - | - |
| 3047 | 9754 | - | 13066 |
| 3048 | - | 11600 | - |
| 3049 | - | 10774 | - |
| 3050 | - | 11866 | 12087 |
| 3052 | - | 11106 | 12569 |
| 3053 | - | 10101 | 12662 |
| 3057 | - | 11531 | - |
| 3059 | - | 11887 | - |
| 3062 | - | - | 13255 |
| 3063 | - | 10845 | - |
| 3064 | - | 11913 | 12201 |
| 3065 | - | 10248 | - |
| 3068 | - | 10090 | 13328 |
| 3069 | - | 11824 | 12551 |
| 3071 | - | 11916 | 12708 |
| 3072 | 9854 | - | 12431 |
| 3074 | - | 11802 | - |
| 3076 | 9493 | 10708 | 11968 |
| 3078 | - | 10704 | 13130 |
| 3079 | - | 11790 | 12093 |
| 3080 | - | 10269 | 13058 |
| 3081 | - | 10502 | 12768 |
| 3082 | - | - | 12377 |
| 3083 | - | 10647 | - |
| 3085 | - | 10961 | - |
| 3086 | 9616 | 10238 | 12200 |
| 3088 | - | - | 12088 |
| 3089 | - | 11921 | - |
| 3090 | - | 10051 | - |

(continued)

| SeqID | BANANA | WHEAT | MAIZE |
|-------|--------|-------|-------|
| 3091 | - | 10300 | 13218 |
| 3092 | 9572 | 11305 | 13321 |
| 3094 | - | 11441 | 12003 |
| 3099 | 9660 | 10644 | 12288 |
| 3100 | - | 10523 | - |
| 3101 | - | 10200 | - |
| 3102 | - | 10117 | 12975 |
| 3103 | - | - | 13059 |
| 3104 | - | 10162 | 13207 |
| 3105 | - | 10053 | - |
| 3107 | - | 11904 | 13199 |
| 3109 | 9729 | - | 12305 |
| 3111 | - | 11340 | 13227 |
| 3112 | - | 11121 | 12520 |
| 3113 | - | 11892 | 13119 |
| 3114 | 9565 | 10366 | - |
| 3115 | - | 10396 | - |
| 3117 | - | - | 12994 |
| 3118 | - | 10243 | 12496 |
| 3119 | - | 10758 | 12947 |
| 3120 | - | 11325 | 12131 |
| 3124 | - | 10681 | - |
| 3125 | - | 11816 | 12849 |
| 3126 | - | - | 12247 |
| 3127 | - | 10749 | 12068 |
| 3128 | 9590 | 11144 | - |
| 3129 | - | 10581 | 13205 |
| 3131 | - | 9881 | - |
| 3132 | - | - | 11986 |
| 3134 | 9867 | 10246 | 12452 |
| 3135 | - | 11792 | 13148 |
| 3136 | - | 11303 | - |
| 3137 | 9567 | 11674 | - |
| 3138 | - | - | 12927 |
| 3140 | - | 10399 | 13329 |
| 3141 | - | 11417 | - |
| 3142 | - | 10128 | - |
| 3143 | - | 10210 | - |
| 3148 | - | 10454 | 12898 |
| 3150 | - | - | 12223 |
| 3153 | - | 10827 | - |
| 3155 | 9452 | 10598 | 12521 |
| 3157 | - | 9968 | 13232 |
| 3159 | - | 11643 | - |
| 3160 | - | 10066 | 13148 |
| 3162 | - | 10038 | 12709 |
| 3163 | - | 10829 | - |
| 3166 | 9651 | - | 12749 |
| 3168 | - | - | 12893 |
| 3169 | - | 11301 | - |

(continued)

| SeqID | BANANA | WHEAT | MAIZE |
|---|---|---|---|
| 3173 | - | 11004 | - |
| 3174 | - | - | 13283 |
| 3175 | - | - | 12861 |
| 3176 | - | 10149 | - |
| 3179 | 9304 | 11446 | - |
| 3180 | - | 11558 | 12459 |
| 3181 | 9417 | - | - |
| 3182 | - | 10579 | - |
| 3183 | 9335 | 10968 | - |
| 3196 | 9467 | 10646 | - |
| 3200 | - | 10571 | 13229 |
| 3204 | - | 11781 | 12983 |
| 3208 | 9721 | 11066 | 13258 |
| 3214 | - | - | 12921 |
| 3216 | - | 11219 | 13274 |
| 3218 | - | - | 12027 |
| 3220 | 9226 | 9979 | 12763 |
| 3221 | - | 10225 | - |
| 3223 | - | - | 12743 |
| 3224 | 9252 | 11592 | 12930 |
| 3225 | 9484 | 10888 | - |
| 3226 | 9760 | 11912 | 12543 |
| 3228 | - | 9989 | - |
| 3230 | - | 11923 | - |
| 3240 | - | 11515 | - |
| 3247 | - | - | 13292 |
| 3248 | - | 10815 | - |
| 3249 | 9257 | 11483 | 13197 |
| 3250 | - | 10836 | - |
| 3252 | 9425 | 11722 | 12757 |
| 3253 | 9242 | 11612 | 12169 |
| 3256 | - | 11925 | 12414 |
| 3258 | - | 10641 | - |
| 3261 | - | 10792 | 13037 |
| 3268 | 9526 | 10518 | - |
| 3269 | 9264 | 11482 | 12173 |
| 3271 | - | 9960 | 13323 |
| 3273 | - | - | 12806 |
| 3285 | - | - | 12900 |
| 3286 | - | 10930 | - |
| 3287 | - | 10413 | - |
| 3288 | 9513 | 10892 | 12232 |
| 3289 | - | 11328 | - |
| 3292 | - | - | 12507 |
| 3295 | 9622 | 10721 | 12548 |
| 3299 | - | 11504 | 12803 |
| 3301 | 9536 | 9954 | 12259 |
| 3302 | - | 10803 | - |
| 3303 | 9280 | 10317 | - |
| 3304 | - | - | 13261 |

(continued)

| SeqID | BANANA | WHEAT | MAIZE |
|-------|--------|-------|-------|
| 3307 | - | 10393 | - |
| 3308 | 9785 | 11401 | 12309 |
| 3313 | - | 10160 | 12411 |
| 3315 | 9352 | 9895 | 12287 |
| 3325 | 9488 | 10416 | - |
| 3326 | - | 10278 | - |
| 3336 | 9484 | 11713 | - |
| 3337 | 9631 | 11591 | 12855 |
| 3341 | - | 11794 | - |
| 3344 | - | - | 12303 |
| 3346 | - | 10929 | 12036 |
| 3347 | - | 10402 | - |
| 3348 | - | 10069 | - |
| 3351 | 9737 | 11931 | 12935 |
| 3357 | - | 11071 | 12082 |
| 3358 | - | 10185 | 12797 |
| 3361 | - | 10471 | 12634 |
| 3362 | - | 11169 | - |
| 3363 | 9364 | 9981 | 13281 |
| 3365 | - | 11489 | 13009 |
| 3367 | - | 10718 | - |
| 3369 | - | 11637 | 12243 |
| 3370 | 9328 | 10608 | - |
| 3372 | - | - | 13284 |
| 3373 | - | 11672 | - |
| 3379 | 9733 | 11564 | 12736 |
| 3380 | 9826 | 11957 | - |
| 3381 | - | 11348 | - |
| 3382 | - | 10859 | - |
| 3387 | - | 10393 | 13255 |
| 3388 | - | 11028 | - |
| 3389 | - | - | 12731 |
| 3390 | - | 10797 | - |
| 3391 | - | 11963 | - |
| 3393 | - | - | 13327 |
| 3394 | - | 11527 | 12135 |
| 3396 | - | 11025 | 13267 |
| 3399 | - | 10485 | - |
| 3400 | 9757 | 10536 | 13098 |
| 3401 | 9431 | 11302 | - |
| 3404 | - | 11333 | 12191 |
| 3405 | - | 10991 | - |
| 3409 | 9646 | 11419 | 13296 |
| 3410 | - | 11962 | 12796 |
| 3411 | 9846 | 11114 | 12359 |
| 3413 | - | 11834 | - |
| 3414 | 9715 | 11313 | 12454 |
| 3416 | 9605 | 11510 | 12426 |
| 3419 | 9593 | 9957 | 12395 |
| 3424 | - | - | 13340 |

(continued)

| SeqID | BANANA | WHEAT | MAIZE |
| --- | --- | --- | --- |
| 3425 | - | 11641 | - |
| 3426 | - | 11771 | - |
| 3427 | - | 10572 | - |
| 3428 | 9403 | - | 12579 |
| 3429 | - | - | 13284 |
| 3431 | 9239 | 10206 | 13203 |
| 3432 | 9758 | 10941 | 13350 |
| 3434 | 9569 | 9927 | - |
| 3438 | 9500 | 11669 | 13027 |
| 3441 | - | 10308 | - |
| 3442 | 9419 | 11413 | 12629 |
| 3445 | 9249 | 10909 | 12244 |
| 3446 | - | - | 12513 |
| 3447 | - | 10374 | 13016 |
| 3454 | 9771 | 10094 | - |
| 3459 | - | 11702 | - |
| 3460 | - | 11756 | 13026 |
| 3467 | - | 11059 | 12655 |
| 3469 | - | 10901 | - |
| 3470 | - | 11673 | 12152 |
| 3472 | - | 11114 | - |
| 3473 | - | 10507 | - |
| 3475 | - | 11780 | - |
| 3476 | - | 11267 | - |
| 3480 | 9667 | 11308 | - |
| 3482 | - | 10979 | 12846 |
| 3484 | 9429 | 11193 | 12186 |
| 3487 | 9224 | 11351 | 13025 |
| 3490 | 9706 | 11315 | - |
| 3491 | 9747 | 10201 | 12715 |
| 3492 | 9747 | 10201 | 12973 |
| 3493 | - | 10360 | - |
| 3496 | - | 10817 | 12333 |
| 3497 | 9738 | 10272 | 12597 |
| 3501 | - | 10333 | 12321 |
| 3506 | 9407 | 11077 | 13186 |
| 3508 | - | 11709 | - |
| 3509 | - | - | 13288 |
| 3510 | - | 10318 | - |
| 3512 | - | 11488 | - |
| 3513 | - | 10203 | - |
| 3516 | 9310 | 10921 | - |
| 3521 | - | - | 12907 |
| 3522 | - | - | 12086 |
| 3523 | - | 11037 | - |
| 3524 | - | - | 12394 |
| 3526 | - | 10944 | - |
| 3528 | 9560 | 10651 | 13330 |
| 3530 | - | 10434 | 12264 |
| 3531 | - | 11491 | - |

(continued)

| SeqID | BANANA | WHEAT | MAIZE |
|-------|--------|-------|-------|
| 3532 | 9433 | 11323 | 12114 |
| 3533 | - | 11015 | 12221 |
| 3534 | - | - | 11978 |
| 3535 | - | 11729 | - |
| 3536 | - | 11720 | 12469 |
| 3546 | 9338 | 10280 | 12154 |
| 3547 | - | - | 12553 |
| 3548 | 9444 | 10024 | - |
| 3549 | - | 11051 | 12995 |
| 3551 | 9624 | 11415 | 13153 |
| 3552 | - | 11566 | - |
| 3553 | - | 11920 | 12146 |
| 3556 | - | 10460 | - |
| 3562 | - | 11195 | 12327 |
| 3565 | - | 10529 | - |
| 3568 | - | 10918 | - |
| 3570 | - | 10428 | 12804 |
| 3571 | - | - | 13061 |
| 3572 | - | 11251 | - |
| 3575 | - | 10351 | 12971 |
| 3576 | 9680 | 11041 | 13120 |
| 3577 | - | 11055 | 13097 |
| 3578 | - | 11203 | - |
| 3579 | 9343 | - | - |
| 3581 | - | - | 12322 |
| 3585 | - | 11322 | 12383 |
| 3587 | - | 10426 | - |
| 3588 | 9561 | 10577 | - |
| 3592 | - | 10981 | - |
| 3595 | - | - | 12342 |
| 3603 | 9764 | 11684 | 12924 |
| 3604 | 9342 | 11017 | 13163 |
| 3605 | - | 10302 | - |
| 3607 | - | 10365 | - |
| 3608 | 9746 | 10201 | 12715 |
| 3609 | - | 10844 | - |
| 3612 | 9346 | 11617 | - |
| 3615 | - | 11815 | - |
| 3619 | - | - | 11978 |
| 3621 | 9744 | - | 12873 |
| 3623 | 9815 | 10606 | 12995 |
| 3633 | - | - | 12207 |
| 3634 | - | 10573 | 12277 |
| 3635 | 9398 | 11787 | 12164 |
| 3636 | - | 11872 | - |
| 3637 | 9703 | 10976 | 12565 |
| 3639 | - | - | 13307 |
| 3642 | 9639 | - | 12418 |
| 3644 | - | 11577 | 12793 |
| 3646 | - | 10007 | 12455 |

(continued)

| SeqID | BANANA | WHEAT | MAIZE |
|-------|--------|-------|-------|
| 3651 | - | 10984 | - |
| 3652 | 9492 | - | - |
| 3654 | - | - | 12348 |
| 3659 | 9288 | 10967 | 13030 |
| 3660 | - | 10603 | - |
| 3661 | - | 10493 | 12067 |
| 3662 | - | - | 12321 |
| 3666 | - | 10657 | - |
| 3667 | - | - | 12748 |
| 3669 | - | - | 13035 |
| 3670 | - | 10469 | 12760 |
| 3671 | - | - | 12851 |
| 3674 | - | 10231 | - |
| 3675 | - | 11759 | - |
| 3677 | - | 11008 | - |
| 3682 | 9807 | 10637 | 12299 |
| 3687 | 9484 | 11713 | - |
| 3688 | - | - | 12283 |
| 3693 | - | 11606 | 13145 |
| 3694 | 9517 | 11266 | 12195 |
| 3696 | - | 10954 | - |
| 3698 | - | 11168 | - |
| 3699 | - | 10935 | 13278 |
| 3700 | - | - | 12049 |
| 3701 | 9468 | 11208 | - |
| 3703 | - | 11039 | - |
| 3704 | 9847 | - | 12958 |
| 3707 | 9634 | 9888 | - |
| 3708 | 9505 | 10108 | 12940 |
| 3713 | - | 10135 | 13116 |
| 3715 | - | - | 12775 |
| 3718 | - | 10247 | 12412 |
| 3721 | - | - | 12691 |
| 3722 | - | 9914 | - |
| 3725 | - | 10636 | - |
| 3728 | 9845 | 10344 | 12816 |
| 3732 | 9554 | 11668 | - |
| 3733 | - | 11653 | - |
| 3735 | 9794 | - | 13284 |
| 3738 | - | 11117 | - |
| 3740 | - | 10607 | - |
| 3741 | - | 10516 | - |
| 3749 | - | 11793 | 12769 |
| 3750 | - | 10029 | - |
| 3756 | 9844 | - | - |
| 3758 | - | 11517 | 13334 |
| 3759 | 9755 | 10621 | 12433 |
| 3761 | - | 10992 | 13131 |
| 3765 | 9482 | - | 12402 |
| 3766 | - | 10675 | - |

(continued)

| SeqID | BANANA | WHEAT | MAIZE |
|---|---|---|---|
| 3772 | - | 10707 | - |
| 3773 | 9218 | 9978 | - |
| 3775 | - | 9912 | - |
| 3777 | - | - | 13207 |
| 3779 | 9254 | 11403 | - |
| 3782 | - | 11082 | 13002 |
| 3783 | - | - | 13062 |
| 3784 | 9378 | 10919 | 12167 |
| 3785 | 9724 | 10951 | 12851 |
| 3786 | - | 10473 | 12432 |
| 3787 | 9473 | 11406 | - |
| 3797 | 9568 | 11930 | 12571 |
| 3798 | - | 10406 | - |
| 3799 | 9221 | 10889 | 12178 |
| 3801 | - | - | 12573 |
| 3803 | 9817 | 9996 | 12970 |
| 3804 | 9457 | - | - |
| 3806 | 9749 | 10894 | 11989 |
| 3812 | - | - | 12349 |
| 3814 | - | 10444 | 12202 |
| 3816 | - | - | 13195 |
| 3817 | - | 11698 | - |
| 3818 | - | 11699 | - |
| 3822 | - | 10008 | - |
| 3826 | - | - | 13006 |
| 3827 | - | - | 13013 |
| 3833 | - | 10394 | - |
| 3834 | 9625 | 10833 | 12406 |
| 3839 | - | 11877 | 12587 |
| 3842 | - | 10997 | - |
| 3847 | 9409 | 10879 | 13254 |
| 3849 | - | 10075 | - |
| 3850 | 9725 | 11532 | 12263 |
| 3851 | - | 11602 | 12526 |
| 3852 | - | - | 11994 |
| 3857 | - | 11182 | - |
| 3858 | - | 10014 | 12983 |
| 3861 | 9307 | 11401 | 12309 |
| 3867 | - | 10182 | 12626 |
| 3877 | - | 10762 | - |
| 3895 | 9863 | 11029 | - |
| 3898 | 9463 | 11775 | 13372 |
| 3902 | - | - | 12560 |
| 3908 | - | 10316 | 12691 |
| 3914 | 9231 | 9969 | 12386 |
| 3918 | - | 10363 | 12025 |
| 3919 | - | 10600 | 12331 |
| 3920 | 9284 | 10158 | - |
| 3921 | - | 10780 | 12009 |
| 3923 | 9700 | 11966 | 12437 |

(continued)

| SeqID | BANANA | WHEAT | MAIZE |
|---|---|---|---|
| 3924 | - | 10965 | - |
| 3925 | - | 10880 | 12018 |
| 3926 | 9531 | 10910 | - |
| 3932 | - | 10960 | 12600 |
| 3933 | - | - | 13319 |
| 3935 | - | - | 13207 |
| 3940 | - | 11138 | 12601 |
| 3941 | 9258 | 11338 | 13073 |
| 3943 | 9273 | 11691 | - |
| 3944 | 9602 | 10597 | - |
| 3945 | - | 9965 | 12944 |
| 3946 | - | 10466 | 13229 |
| 3948 | - | - | 13121 |
| 3949 | 9334 | 11022 | 12782 |
| 3952 | - | 11949 | - |
| 3957 | 9387 | 11153 | - |
| 3958 | 9459 | 10264 | - |
| 3960 | - | - | 12812 |
| 3965 | - | 11433 | 13182 |
| 3966 | - | 11936 | - |
| 3970 | - | 10665 | - |
| 3971 | - | - | 13255 |
| 3972 | - | - | 13255 |
| 3976 | - | 11555 | 12936 |
| 3977 | - | 10903 | - |
| 3978 | - | - | 12877 |
| 3979 | - | 10915 | - |
| 3980 | - | 10357 | - |
| 3981 | 9253 | 10687 | 13170 |
| 3985 | 9689 | 10738 | 12330 |
| 3986 | - | 10239 | 12127 |
| 3990 | - | 11938 | - |
| 3991 | - | - | 12956 |
| 3997 | - | - | 12631 |
| 3998 | 9286 | 11284 | 12019 |
| 4004 | - | 11727 | 13207 |
| 4005 | - | - | 12289 |
| 4006 | - | 10505 | 12485 |
| 4010 | - | - | 12468 |
| 4020 | - | 10060 | - |
| 4021 | - | 11582 | - |
| 4022 | 9442 | 10957 | 12862 |
| 4024 | - | 10996 | 12512 |
| 4025 | 9690 | - | 12488 |
| 4026 | 9577 | 10522 | 12257 |
| 4029 | - | - | 12435 |
| 4031 | - | - | 11995 |
| 4032 | - | 10849 | 12339 |
| 4034 | - | 10852 | 13096 |
| 4040 | 9672 | 10724 | 13047 |

(continued)

| SeqID | BANANA | WHEAT | MAIZE |
| --- | --- | --- | --- |
| 4041 | - | 11343 | - |
| 4044 | - | - | 12890 |
| 4048 | - | 10681 | - |
| 4049 | - | 10867 | - |
| 4050 | - | - | 12312 |
| 4052 | 9472 | 11550 | 12640 |
| 4053 | - | 11680 | 12588 |
| 4059 | - | 11192 | - |
| 4063 | 9491 | 10711 | 12229 |
| 4069 | - | - | 12948 |
| 4074 | 9323 | 11297 | 12594 |
| 4076 | - | - | 12156 |
| 4077 | - | - | 12517 |
| 4080 | - | - | 13231 |
| 4086 | - | 10636 | - |
| 4088 | 9677 | 11056 | 12783 |
| 4093 | - | 11676 | 12653 |
| 4100 | - | - | 12539 |
| 4101 | 9607 | 10391 | 12125 |
| 4105 | - | 11165 | - |
| 4111 | - | 11874 | - |
| 4112 | - | 11656 | - |
| 4113 | 9230 | 11435 | - |
| 4117 | 9296 | 9934 | - |
| 4121 | - | - | 12931 |
| 4122 | - | 10748 | 12816 |
| 4126 | - | 10242 | - |
| 4128 | - | - | 13099 |
| 4129 | - | 11238 | - |

**Conclusion**

In light of the detailed description of the invention and the examples presented above, it can be appreciated that the several aspects of the invention are achieved.

It is to be understood that the present invention has been described in detail by way of illustration and example in order to acquaint others skilled in the art with the invention, its principles, and its practical application. Particular formulations and processes of the present invention are not limited to the descriptions of the specific embodiments presented, but rather the descriptions and examples should be viewed in terms of the claims that follow and their equivalents. While some of the examples and descriptions above include some conclusions about the way the invention may function, the inventor does not intend to be bound by those conclusions and functions, but puts them forth only as possible explanations.

It is to be further understood that the specific embodiments of the present invention as set forth are not intended as being exhaustive or limiting of the invention, and that many alternatives, modifications, and variations will be apparent to those of ordinary skill in the art in light of the foregoing examples and detailed description. Accordingly, this invention is intended to embrace all such alternatives, modifications, and variations that fall within the spirit and scope of the invention.

Table 3

>CBFT3 - SEQ ID NO:1 (EP Application SEQ ID NO:14025)
ATGAATGTCGACAAGCTTAAGAAGATGGCGGGTGCCGTGCGCACCGGTGGCAAGGGCAGCATGCGCAGGAAGAAG
AAGGCAGTTCACAAGACTACCACCACTGATGACAAGAGGCTTCAAAGCACCTTGAAAAGAGTAGGAGTGAACAAC
ATTCCTGGTATCGAAGAGGTCAATATCTTCAAGGATGATGTGGTTATCCAATTTCAGAATCCAAAAGTGCAAGCA
TCCATTGGTGCAAATACATGGGTAGTGAGTGGAACACCACAGACGAAGAAGCTGCAAGATCTGCTTCCAACAATC
ATCAACCAGTTGGGACCTGATAACCTGGACAACCTCAGGAGGCTTGCTGAGCAGTTCCAGAAGCAGGTACCCGGT
GCTGAGGCTGGTGCCAGCGCAGGTAACGCTCAGGACGACGACGATGATGTCCCTGAGCTTGTCCCTGGAGAGACG
TTCGAGGAGGCTGCAGAGGAGAAGGAGCCTGAGGAGAAGAAGGAAGCGGAGGTGGAAGAGAAGAAAGAGTCGTCC

>OS002908 - SEQ ID NO:2  (EP Application SEQ ID NO:720)
ATGGGTGTATTGGACAGCCTCTCTGATATGTGCAGCCTGACAGAGACCAAGGAAGCCCTCAAGCTAAGGAAGAAG
CGGCCACTGCAGACGGTGAACATCAAGGTGAAGATGGACTGCGAGGGGTGCGAGAGGAGGGTGAAGAACGCGGTG
AAGTCGATGCGAGGGGTGACGAGCGTGGCGGTGAACCCGAAGCAGAGCCGGTGCACGGTGACCGGGTACGTGGAG
GCGAGCAAGGTGCTGGAGCGCGTGAAGAGCACCGGGAAGGCGGCGGAGATGTGGCCCTACGTCCCGTACACCATG
ACCACCTACCCGTACGTCGGCGGCGCCTACGACAAGAAGGCCCCCGCCGGCTTCGTCCGCGGCAACCCCGCCGCC
ATGGCCGACCCCTCCGCCCCCGAGGTCCGCTACATGACCATGTTCAGCGACGAGAACGTCGACTCCTGCTCCATC
ATGTAA

>OS015403 - SEQ ID NO:3 (EP Application SEQ ID NO:8204)
ATGTATGATGAGTTGGCCAGCAAGGGCAATGTTGAATATATTGCCGGAGGAGCCACCCAGAACTCTATCAGGGTT
GCTCAATGGATGCTTCAAACTCCTGGTGCAACAAGTTACATGGGTTGCATTGGAAAGGATAAGTTTGGTGAGGAG
ATGAAGAAGAATGCCCAAGCTGCTGGTGTTACTGCTCATTACTACGAGGATGAGGCTGCTCCCACGGGCACATGT
GCTGTCTGTGTTGTTGGTGGTGAAAGATCACTGGTTGCAAACTTATCAGCAGCAAACTGCTACAAATCTGAGCAT
CTGAAGAAACCGGAGAACTGGGCACTAGTGGAGAAAGCAAAATACATCTACATTGCTGGCTTTTTCCTTACGGTC
TCCCCAGATTCTATTCAGCTTGTTGCTGAGCATGCTGCCGCTAACAACAAGGTGTTCCTGATGAACCTCTCTGCA
CCCTTTATCTGTGAGTTTTTTCCGTGATGCCCAGGAGAAGGTTCTTCCGTTTGTGGACTACATCTTCGGTAACGAA
ACAGAAGCAAGAATCTTTGCTAAAGTCCGTGGATGGGAGACTGAGAATGTTGAGGAGATCGCGTTGAAGATTTCC
CAGCTTCCATTGGCCTCTGGAAAACAAAAGAGGATTGCCGTGATTACTCAAGGTGCTGATCCAGTAGTTGTCGCT
GAGGATGGACAGGTGAAAACATTCCCTGTGATCCTACTGCCAAAGGAGAAGCTTGTTGACACCAATGGCGCTGGT
GATGCCTTTGTTGGAGGCTTCCTCTCACAATTGGTTCAACAAAAGAGCATTGAGGACTCTGTGAAGGCTGGTTGC
TATGCCGCAAATGTTATCATCCAGCGTTCTGGCTGCACTTACCCTGAGAAGCCTGATTTCAACTAG

>ERA1 (FT) - SEQ ID NO:4 (EP Application SEQ ID NO:14907)
ATGGACCCCCCCTCGCCGCCGCCGCCGCCGCCATATCCTCCTGCTGCTGCTGAGGGCGGTCCGGCAGCGGATAGC
CAGGCCGCTGAGCTGCCCCGGCTGACTGTGACGCAGGTGGAGCAGATGAAGGTGGAGGCGAAGGTGGGCGAAATC
TACCGCGTCCTCTTCGGCAACGCGCCCAACGCCAATTCCCTCATGTTAGAGCTGTGGCGTGAGCAGCATGTTGAG
TATTTGACGAGAGGGCTGAAACATCTTGGACCAAGCTTCCATGTGCTCGATGCCAATCGACCTTGGCTGTGCTAC
TGGATTATTCATGCACTTGCTCTGTTGGATGAAATACCTGACGATGTTGAGGATGATATTGTGGACTTCTTATCT
CGATGTCAGGACAAAGATGGTGGTTATGGCGGAGGACCTGGACAGGGACAACCTGTACAAGTTCATGCTTCGGAT
GAAAGATACATCGGGAGCTTTCAGAAATGCATGAATGGTGGTGAAATAGATGTTCGTGCTAGCTATACTGCAATA
TCGGTTGCCAGCCTTGTGAACATTCTTGATGGTGAACTAGCAAAAGGTGTTGGAAATTACATAACAAGGTGTCAA
ACCTATGAAGGTGGCATTGCTGGGGAACCGTATGCTGAAGCTCATGGTGGGTACACTTTTTGTGGGCTGGCTACG
ATGATCCTGCTTAACGAAGTGGACAAACTTGATTTGGCTAGCTTGATTGTTAATGCCATACCTGTTTTTTTTTTC
CTGGCATCCTCCACTCTATCTGACAAACTTCTGGTGTATGACCAGGGAGCTGCTCTTGCTTTAACACAAAAACTA
ATGACAGTTGTTGATGAGCAATTAAAATCATCATATTCCAGCAAAAGGCCTCCAGGAGATGATGCTTGTGGTACG
AGCTCTTCTACTGAAGCAGCATATTATGCTAAGTTTGGATTTGATTTTATAGAGAAGAGCAACCAAATAGGCCCA
CTGTTCCACAACATCGCGCTGCAGCAATACATCCTGCTTTGCGCACAGGTGCTGGATGGAGGGTTGAGGGATAAG
CCTGGGAAGAACAGAGATCACTACCACTCGTGCTACTGCCTGAGTGGTCTGTCAGTTAGCCAGTACAGCGCCATG
GTTGATTCTGATGCGTGCCCCTTGCCGCAGCACGTGCTTGGTCCTTACTCAAACTTGCTAGAGCCGATCCATCCG
CTCTACAATGTTGTACTAGACAAATACCATACGGCCTATGAGTTCTTTTCAAGCTAG

**Conclusion**

# EP 1 925 672 A1

**Claims**

1. An isolated nucleic acid molecule comprising a polynucleotide selected from the group consisting of:

   a) any one of the nucleotide sequences selected from the group of SEQ ID NOs. 10425, 720, 8204, and 14907;
   b) a functional portion of any of the sequences of a);
   c) a polynucleotide that is substantially similar to a sequence of a) or b);
   d) a sequence of at least 15 nucletides that hybridizes under stringent conditions to a polynucleotide of a), b) or c);
   e) the complement of any sequence of a), b), c) or d);
   f) the reverse complement of any sequence of a), b), c) or d);
   and
   g) an allelic variant of any of the above.

2. The nucleic acid molecule of claim 1, wherein said polynucleotide is present in a drought tolerance QTL heated on rice chromosome 3.

3. The nucleic acid molecule of claim 1, wherein said polynucleotide is from a genomic region syntenic with a maize cold tolerance QTL.

4. The nucleic acid molecule of claim 1, wherein said polynucleotide is present in a drought tolerance QTL on rice chromosome 8,

5. The nucleic acid molecule of claim 1, wherein said polynucleotide encodes a protein containing a Universal Stress Protein A domain.

6. The nucleic acid molecule of any of the preceding claims, wherein said polynucleotide is from a plant.

7. The nucleic acid molecule of any of the preceding claims, wherein said polynucleotide is from a monocot or a dicot.

8. The nucleic acid molecule of any of the preceding claims, wherein said polynucleotide is from a cereal.

9. The nucleic acid molecule of 8 wherein said cereal is selected from the group consisting of maize, rice, wheat, barley, oat, rye, millet, milo, triticale, orchardgrass, guinea grass, sorghum and turfgrass.

10. The nucleic acid of any of the preceding claims, wherein expression of said nucleic acid is altered 2X up or down in response to an abiotic stress.

11. The nucleic acid of claim 10 wherein said abiotic stress is selected from the group consisting of cold stress, salt stress, osmotic stress or any combination thereof.

12. A vector comprising a polynucleotide of claim 1.

13. The vector of claim 12, wherein said vector is a cloning vector or an expression vector.

14. An expression cassette comprising a polynucleotide of claim 1.

15. A host cell comprising a vector of claim 12 or 13 or a expression cassette of claim 14.

16. The host cell of claim 15, wherein said host cell is a prokaryotic cell or a eukaryotic cell.

17. The host cell of claim 15 or 16, wherein said host cell is a bacterial cell, an insect cell, a yeast cell, a plant cell or an animal cell.

18. The host cell of any of claim 15 to 17, wherein said host cell is a plant cell.

19. A plant comprising a vector of claim 12 or 13 or expression cassette of claim 14.

20. A plant comprising a host cell of claim 18.

**21.** A seed from the plant of claim 20.

**22.** A seed comprising a vector of claim 12 or 13 or expression cassette of claim 14.

**23.** A plant produced from the seed of claim 22.

**24.** The progeny of a plant of claim 23.

**25.** A hybrid derived from a plant of claim 23.

**26.** A method for altering the tolerance of a plant to an abiotic stress composing introducing into said plant a recombinant nucleic acid construct comprising at least one of the polynucleotides selected from the group consisting of SEQ ID NOs. 10425, 720, 8204, and 14907.

**27.** A method for altering the tolerance of a plant to an abiotic stress comprising introducing into said plant a recombinant nucleic acid construct comprising a polynucleotide of at least 15 nucleotides in length that hybridizes under high stringency conditions to the complement of a sequence selected from the group consisting of SEQ ID NOs. 10425, 720, 8204, and 14907.

**28.** The method of claim 27, wherein said polynucleotide is operably linked to a heterologous promoter.

**29.** A method for altering the tolerance of a plant to an abiotic stress comprising introducing into said plant a recombinant nucleic acid construct, wherein said construct encodes a molecule which alters expression of an abiotic stress regulated coding region selected from the group consisting of SEQ ID NOs. 10425, 720, 8204, and 14907, or the complement thereof.

**30.** The method of claim 29 wherein said molecule comprises an antisense RNA, double stranded RNAi sequence, a triplexing agent, and a molecule containing a dominant negative, mutation,

**31.** A plant produced by the method of any of claim 26 to 30.

**32.** A seed from a plant of claim 31.

**33.** A method for selecting an agent that alters abiotic stress regulated polynucleotide expression in a plant cell comprising:

contacting at least one plant cell with is test agent;
subjecting said plant cell to an abiotic stress before, during or after contacting said plant cell with said test agent;
obtaining an expression profile of said plant cell wherein said expression profile comprises expression data for at least one abiotic stress regulated sequence selected from the group consisting of SEQ ID NOs. 10425, 720, 8204, and 14907;
comparing the expression profile of said cell to the expression profile obtained from at least one plant cell not exposed to said agent, but exposed to said abiotic stress;
selecting said agent if said agent alters expression of said abiotic stress regulated sequence.

European Patent Office

**EUROPEAN SEARCH REPORT**

Application Number

EP 08 10 2091

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE EMBL [Online] 7 August 2000 (2000-08-07), "WHE0963_H06_011ZS Wheat pre-anthesis spike cDNA library Triticum aestivum cDNA clone WHE0963_H06_011, mRNA sequence." XP002477385 retrieved from EBI accession no. EMBL:BE498238 Database accession no. BE498238 * abstract * | 1,6-9, 12,13, 15-17 | INV. C12N15/82 C12N15/29 C07K14/415 C12N5/10 C07K16/16 A01H5/10 G01N33/50 C12Q1/68 G11B27/00 |
| X | DATABASE EMBL [Online] 22 February 2001 (2001-02-22), "Oryza sativa Japonica Group genomic DNA, chromosome 1, BAC clone:OSJNBb0021A09." XP002477386 retrieved from EBI accession no. EMBL:AP003218 Database accession no. AP003218 * abstract * overlap is between base pairs 60080-62080 | 1,6-9, 12,13, 15-17 | |
| A | DATABASE EMBL [Online] 22 February 2001 (2001-02-22), "Oryza sativa Japonica Group genomic DNA, chromosome 1, BAC clone:OSJNBa0094H06." XP002477387 retrieved from EBI accession no. EMBL:AP003217 Database accession no. AP003217 * abstract * | 1,6-9, 12,13, 15-17 | TECHNICAL FIELDS SEARCHED (IPC) C12N C07K |

-/--

The present search report has been drawn up for all claims

| Place of search | Date of completion of the search | Examiner |
|---|---|---|
| The Hague | 22 April 2008 | Holtorf, Sönke |

EPO FORM 1503 03.82 (P04C01)

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DATABASE EMBL SEQUENCE LIBRARY [Online] ebi, Hinxton, UK; linear genomic DNA 10 July 2001 (2001-07-10), SASAKI,T.; MATSUMOTO,T.; YAMAMOTO,K.;: "Oryza sativa Japonica Group genomic DNA, chromosome 8, BAC clone:OJ1123_G08" XP002477388 retrieved from WWW.EBI.CO.UK accession no. http://srs.ebi.ac.uk Database accession no. AP003880 * abstract * | 1 | |
| P,X | & DATABASE UniProt [Online] 5 July 2004 (2004-07-05), "Putative uncharacterized protein OJ1123_G08.9." retrieved from EBI accession no. UNIPROT:Q6ZKC6 Database accession no. Q6ZKC6 * abstract * | 1 | |
| A | TAKAHASHI ET AL: "Induction of chilling resistance by water stress, and cDNA sequence analysis and expression of water stress regulated genes in rice" PLANT MOLECULAR BIOLOGY, SPRINGER, DORDRECHT, NL, vol. 26, 1994, pages 339-352, XP002078431 ISSN: 0167-4412 * the whole document * | | TECHNICAL FIELDS SEARCHED (IPC) |

-/--

The present search report has been drawn up for all claims

| Place of search | Date of completion of the search | Examiner |
|---|---|---|
| The Hague | 22 April 2008 | Holtorf, Sönke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EUROPEAN SEARCH REPORT

**European Patent Office**

**Application Number**

EP 08 10 2091

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CLAES B ET AL: "Characterization of a rice gene showing organ-specific expression in response to salt stress and drought" PLANT CELL, AMERICAN SOCIETY OF PLANT PHYSIOLOGISTS, ROCKVILLE, MD, US, vol. 2, no. 1, January 1990 (1990-01), pages 19-27, XP002956018 ISSN: 1040-4651 * the whole document * | | |
| A | JOSHEE N ET AL: "ISOLATION AND CHARACTERIZATION OF A WATER STRESS-SPECIFIC GENOMIC GENE PWSI 18, FROM RICE" PLANT AND CELL PHYSIOLOGY, JAPANESE SOCIETY OF PLANT PHYSIOLOGISTS, vol. 39, no. 1, January 1998 (1998-01), pages 64-72, XP008018585 ISSN: 0032-0781 * the whole document * | | |
| A | WO 99/13067 A (PURDUE RESEARCH FOUNDATION [US]; HODGES THOMAS K [US]; HUQ ENAMUL [US]) 18 March 1999 (1999-03-18) * the whole document * | | |
| P,X | DATABASE Geneseq [Online] 22 September 2005 (2005-09-22), "Rice genome derived DNA sequence, SEQ ID 2292." XP002477389 retrieved from EBI accession no. GSN:AEB67147 Database accession no. AEB67147 * abstract * | 1 | |

**TECHNICAL FIELDS SEARCHED (IPC)**

-/--

The present search report has been drawn up for all claims

| Place of search | Date of completion of the search | Examiner |
|---|---|---|
| The Hague | 22 April 2008 | Holtorf, Sönke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent
Office

**EUROPEAN SEARCH REPORT**

Application Number

EP 08 10 2091

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| P,X | DATABASE EMBL [Online] 15 January 2002 (2002-01-15), "EBem06_SQ004_E16_R embryo, 21 DPA, no treatment, cv Optic, EBem06 Hordeum vulgare cDNA clone EBem06_SQ004_E16 5', mRNA sequence." XP002477390 retrieved from EBI accession no. EMBL:BM375744 Database accession no. BM375744 * abstract * | 1,6-9, 12,13, 15-17 | |
| | ----- | | |
| | | | TECHNICAL FIELDS SEARCHED (IPC) |

The present search report has been drawn up for all claims

| Place of search | Date of completion of the search | Examiner |
|---|---|---|
| The Hague | 22 April 2008 | Holtorf, Sönke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent
Office

Application Number

EP 08 10 2091

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

| | | | |
|---|---|---|---|
| European Patent Office | **LACK OF UNITY OF INVENTION**<br>**SHEET B** | Application Number<br>EP 08 10 2091 | |

The Search Division considers that the present European patent application does not comply with the requirements of unity of invention and relates to several inventions or groups of inventions, namely:

Invention 1 : claims 1-33 partially

> Isolated nucleic acid sequence as characterized by SEQID720, 10425; the recombinant expression of the same in host cells; a method of altering the tolerance of a plant to an abiotic stress by introduction of a recombinant nucleic acid construct comprising the polynucleotide sequence as characterized by SEQIDs720, 10425 or by introducing a construct wherein said construct alters the expression of an abiotic stress regulated coding region as characterized by SEQId720, 10425; method for selecting an agent that alters abiotic stress regulated polynucleotide expression by evalutaing a test agent for an effect on the expression profile of said polynucleotide sequence as characterized by SEQID720, 10425.
>
> ---

Invention 2: claims 1 partially

> Isolated polypeptide as characterized by SEQID 8204.
>
> ---

Invention 3: claims 1,6-9,12-27,31-33 partially

> A method for altering the expression of a stress-responsive polynucleotide by linking said polynucleotide with a regulatory region as characterized by SEQIDs 14907.
>
> ---

## ANNEX TO THE EUROPEAN SEARCH REPORT
## ON EUROPEAN PATENT APPLICATION NO.

EP 08 10 2091

This annex lists the patent family members relating to the patent documents cited in the above-mentioned European search report.
The members are as contained in the European Patent Office EDP file on
The European Patent Office is in no way liable for these particulars which are merely given for the purpose of information.

22-04-2008

| Patent document cited in search report | | Publication date | Patent family member(s) | | Publication date |
|---|---|---|---|---|---|
| WO 9913067 | A | 18-03-1999 | CA | 2303402 A1 | 18-03-1999 |
| | | | EP | 1012317 A2 | 28-06-2000 |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 30011201 P
- US 31466201 P
- US 32527701 A
- US 33213201 P
- WO 0070067 A
- EP 0332104 A
- US 5614395 A
- US 5641876 A
- EP 255378 A
- US 5625136 A
- US 4943674 A
- US 4535060 A
- US 4769061 A
- US 4801590 A
- US 5107065 A
- US 5589379 A
- US 5364780 A
- WO 9706269 A
- WO 9706268 A
- WO 00760067 A
- US 5605793 A
- WO 9420627 A
- US 5767373 A
- WO 9947552 A
- EP 164575 A
- EP 120516 A
- WO 97108331 A
- US 1985 A
- EP 0709462 A2

- US 5451513 A
- US 5545817 A
- US 5545818 A
- WO 9516783 A
- WO 9515972 A
- US 5501967 A
- US 5270163 A
- US 5143854 A
- WO 9015070 A
- WO 9210092 A
- US 5412087 A
- WO 9511995 A
- WO 9412305 A
- WO 9411530 A
- WO 9729212 A
- WO 9731256 A
- US 5817479 A
- EP 0707592 A
- WO 9220702 A
- US 5185444 A
- US 5034506 A
- US 5142047 A
- FR 7810975
- EP 0225807 A
- WO 9704112 A
- WO 006837 A
- WO 9806860 A
- US 5766847 A

**Non-patent literature cited in the description**

- **AHN et al.** *Mol Gen Genet,* 1993, vol. 241, 483
- **BENNETZEN et al.** *Proc Natl Acad Sci,* 1998, vol. 95, 1975
- **DUDITS.** Plant Cell Division. Portland Press, 1997, 21
- **BERGOUNIOUX.** *Protoplasma,* 1988, vol. 142, 127-136
- **SAMBROOK et al.** Molecular Cloning. Cold Spring Harbor Press, 1989
- **DAVIS et al.** Basic Methods in Molecular Biology. Appleton and Lange, 1994, 6-8
- **SMITH ; WATERMAN.** *Adv. Appl. Math.,* 1981, vol. 2, 482
- **NEEDLEMAN ; WUNSCH.** *J. Mol. Biol.,* 1970, vol. 48, 443

- **PERSON ; LIPMAN.** *Proc. Natl. Acad. Sci., USA,* 1988, vol. 85, 2444
- **ALTSCHUL et al.** *Nucleic Acids Res.,* 1977, vol. 25, 3389-3402
- *J. Mol. Biol.,* 1990, vol. 215, 403-410
- **HENIKOFF ; HENIKOFF.** *Proc. Natl. Acad. Sci., USA,* 1989, vol. 89, 10915
- **KARLIN ; ALTSCHUL.** *Proc. Natl. Acad. Sci., USA,* 1993, vol. 90, 5873
- **GONNET et al.** *Science,* 1992, vol. 256, 1443-1445
- **HENIKOFF ; HENIKOFF.** *Proteins,* 1993, vol. 17, 49-61
- Matrices for Detecting Distance Relationships: Atlas of Protein Sequence and Structure. National Biomedical Research Foundation. 1978

- **SCHUCH et al.** *Plant Mol. Biol.,* 1989, vol. 13, 303
- **DIEKMAN ; FISCHER.** *EMBO J.,* 1988, vol. 7, 3315
- **PEAR et al.** *Plant Mol. Biol.,* 1989, vol. 13, 639
- **WANG et al.** *Molec. Cell Biol.,* 1992, vol. 12, 3399
- **ODELL et al.** *Nature,* 1985, vol. 313, 810
- **LAWTON et al.** *Mol. Cell Biol.,* 1987, vol. 7, 335
- **VODKIN.** *Prog. Clin Biol. Res.,* 1983, vol. 138, 87
- **LINDSTROM et al.** *Der. Genet.,* 1990, vol. 11, 160
- **VOGEL et al.** *EMBO J.,* 1992, vol. 11, 157
- **DENNIS et al.** *Nuc. Acid Res.,* 1984, vol. 12, 3983
- **BANSAL et al.** *Proc. Natl. Acad. Sci. USA,* 1992, vol. 89, 3654
- **POULSEN et al.** *Mol. Gen. Genet.,* 1986, vol. 205, 193
- **LANGRIDGE et al.** *Proc. Natl. Acad. Sci., USA,* 1989, vol. 86, 3219
- **VANTUNEN et al.** *EMBO J.,* 1988, vol. 7, 1257
- **KELLER et al.** *Genes Dev.,* 1989, vol. 3, 1639
- **WENZLER et al.** *Plant Molec. Biol.,* 1989, vol. 13, 347
- **YAMAMOTO et al.** *Nuc. Acid Res.,* 1990, vol. 18, 7449
- **REINA et al.** *Nuc. Acids Res.,* 1990, vol. 18, 6425-26
- **KRIZ et al.** *Mol. Gen. Genet.,* 1987, vol. 207, 90
- **WANDELT et al.** *Nuc. Acids Res.,* 1989, vol. 17, 2354
- **LANGRIDGE et al.** *Cell,* 1983, vol. 34, 1015
- **BELANGER.** *Genetics,* 1991, vol. 129, 863
- **SULLIVAN et al.** *Mol. Gen. Genet.,* 1989, vol. 215, 431
- **HUDSPETH ; GRULA.** *Plant Molec. Biol.,* 1989, vol. 12, 579
- **CHANDLER et al.** *Plant Cell,* 1989, vol. 1, 1175
- **FRANKEN et al.** *EMBO J.,* 1991, vol. 10, 2605
- **FROMM et al.** *Bio/Technology,* 1989, vol. 8, 833
- **KRIDL et al.** *Seed Sci. Res.,* 1991, vol. 1, 209
- **CZAKO et al.** *Mol. Gen. Genet.,* 1992, vol. 235, 33
- **GAN et al.** *Science,* 1995, vol. 270, 1986
- **JOHN et al.** *Proc. Natl. Acad. Sci. USA,* 1992, vol. 89, 5769
- **MANSSON et al.** *Gen. Genet.,* 1985, vol. 200, 356
- **SLATER et al.** *Plant Molec. Biol.,* 1985, vol. 5, 137
- **HEMPEL et al.** *Development,* 1997, vol. 124, 3845-3853
- **TSUGEKI ; FEDOROFF.** *Proc. Natl. Acad., USA,* 1999, vol. 96, 12941-12946
- **SMITH ; FEDOROFF.** *Plant Cell,* 1995, vol. 7, 735
- **BLAZQUEZ et al.** *Development,* 1997, vol. 124, 3835-3844
- **HEMPEL et al.** *supra,* 1997
- **PLANT et al.** *Plant Mol. Biol.,* 1994, vol. 25, 193-205
- **HAMILTON et al.** *Plant Mol. Biol.,* 1992, vol. 18, 211-218
- **ATANASSOVA et al.** *Plant J.,* 1992, vol. 2, 291
- **ITO et al.** *Plant Mol. Biol.,* 1994, vol. 24, 863
- **MARTINEZ et al.** *Proc. Natl. Acad. Sci., USA,* 1992, vol. 89, 7360
- **MEDFORD et al.** *Plant Cell,* 1991, vol. 3, 359
- **TERADA et al.** *Plant J.,* 1993, vol. 3, 241
- **WISSENBACH et al.** *Plant J.,* 1993, vol. 4, 411
- **JACK et al.** *Cell,* 1994, vol. 76, 703
- **BEALS et al.** *Plant Cell,* 1997, vol. 9, 1527
- **GATZ.** *Cur. Opin. Biotech,* 1996, vol. 7, 168
- **GATZ.** *Ann. Rev. Plant. Physiol. Plant Mol. Biol.,* 1997, vol. 48, 89
- **AOYAMA et al.** *N-H Plant J.,* 1997, vol. 11, 605
- **SCHWOB et al.** *Plant J.,* 1993, vol. 4, 423
- **RALSTON et al.** *Genetics,* 1988, vol. 119, 185
- **CORDERO et al.** *Plant J.,* 1994, vol. 6, 141
- **KOHLER et al.** *Plant Molec. Biol.,* 1995, vol. 29, 1293
- **QUIGLEY et al.** *J. Mol. Evol.,* 1989, vol. 29, 412
- **MARTINEZ et al.** *J. Mol. Biol.,* 1989, vol. 208, 551
- **FURST et al.** *Cell,* 1988, vol. 55, 705-717
- **METT et al.** *Proc. Natl. Acad. Sci., USA,* 1993, vol. 90, 4567-4571
- **GATZ et al.** *Plant J.,* 1992, vol. 2, 397-404
- **RODER et al.** *Mol. Gen. Genet.,* 1994, vol. 243, 32-38
- **CHRISTOPHERSON et al.** *Proc. Natl. Acad. Sci., USA,* 1992, vol. 89, 6314-6318
- **SCHENA et al.** *Proc. Natl. Acad. Sci., USA,* 1991, vol. 88, 10421-10425
- **TAKAHASHI et al.** *Plant Physiol.,* 1992, vol. 99, 383-390
- **BACK et al.** *Plant Mol. Biol.,* 1991, vol. 17, 9
- **FEINBAUM et al.** *Mol. Gen. Genet.,* 1991, vol. 226, 449
- **LAM ; CHUA.** *Science,* 1990, vol. 248, 471
- **YAMAGUCHI-SHINOZAKI et al.** *Plant Mol. Biol.,* 1990, vol. 15, 905

- **KARES et al.** *Plant Mol. Biol.,* 1990, vol. 15, 225
- **BEVAN et al.** *Nature,* 1983, vol. 304, 184
- **GALLIE et al.** *Nuc. Acids Res.,* 1987, vol. 15, 8693
- **SKUZESKI et al.** *Plant Mol. Biol.,* 1990, vol. 15, 65
- **ELROY-STEIN et al.** *Proc. Natl. Acad. Sci. USA,* 1989, vol. 86, 6126
- **MACEJAK et al.** *Nature,* 1991, vol. 353, 90
- **JOBLING et al.** *Nature,* 1987, vol. 325, 622
- **GALLIE et al.** *Molecular Biology of RNA,* 1989, vol. 237
- **LOMMEL et al.** *Virology,* 1991, vol. 81, 382
- **CIOPPA et al.** *Plant Physiol.,* 1987, vol. 84, 965
- **CALLIS et al.** *Genes Devel.,* 1987, vol. 1, 1183
- **VASIL.** *Plant Physiol.,* 1989, vol. 91, 1575
- **GALLIE et al.** *Molecular Biology of RNA,* 1989, vol. 237, 1989
- **ELLIS.** *EMBO J.,* 1987, vol. 6, 3203
- **VASIL et al.** *Plant Physiol.,* 1989, vol. 91, 1575
- **GALLIE et al.** *Molecular Biology of RNA,* 1989
- **MA et al.** *Nature,* 1988, vol. 334, 631
- **ELLIS et al.** *EMBO J.,* 1987, vol. 6, 3203
- **BOUCHEZ et al.** *EMBO J.,* 1989, vol. 8, 4197
- **STEMMER et al.** *Nature,* 1994, vol. 370, 389-391
- **FRANK-KAMENETSKII ; MIRKIN.** *Ann. Rev. Biochem.,* 1995, vol. 64, 65-95
- **REISS.** Life Sci. Adv. *Plant Physiol.,* 1994, vol. 13, 143-149
- **HERRERA-ESTRELLA.** *EMBO J.,* 1983, vol. 2, 987-995
- **MARSH.** *Gene,* 1984, vol. 32, 481-485
- **HARTMAN.** *Proc. Natl. Acad. Sci., USA,* 1988, vol. 85, 8047
- **MCCONLOGUE.** Current Communications in Molecular Biology. Cold Spring Harbor Laboratory, 1987
- **TAMURA.** *Biosci. Biotechnol. Biochem.,* 1995, vol. 59, 2336-2338
- **WHITE et al.** *Nucl. Acids Res.,* 1990, vol. 18, 1062
- **SPENCER et al.** *Theor. Appl. Genet.,* 1990, vol. 79, 625-631
- **HINCHEE et al.** *Bio/Technology,* 1998, vol. 91, 915-922
- **LEE et al.** *EMBO J.,* 1988, vol. 7, 1241-1248
- **SMEDA et al.** *Plant Physiol.,* 1993, vol. 103, 911-917
- **GIACOMIN.** *Plant Sci.,* 1996, vol. 116, 59-72
- **SCIKANTHA.** *J. Bacteriol.,* 1996, vol. 178, 121
- **GERDES.** *FEBS Lett.,* 1996, vol. 389, 44-47
- **JEFFERSON.** *EMBO J.,* 1997, vol. 6, 3901-3907
- **SAMBROOK et al.** Molecular Cloning; A Laboratory Manual. Cold Spring Harbor Laboratory Press, 1989
- **SEIMIYA et al.** *J. Biol. Chem.,* 1997, vol. 272, 4631-4636
- **MAHER et al.** *Antisense Res. Devel.,* 1991, vol. 1, 227
- **HELENE.** *Anticancer Drug Design,* 1991, vol. 6, 569
- **MALIGA et al.** Methods in Plant Molecular Biology. Cold Spring Harbor Laboratory Press, 1995, 39
- **VON HEIJNE et al.** *Plant Mol. Biol. Rep.,* 1991, vol. 9, 104
- **CLARK et al.** *J. Biol. Chem.,* 1989, vol. 264, 17544
- **DELLA-CIOPPA et al.** *Plant Physiol.,* 1987, vol. 84, 965
- **ROMER et al.** *Biochem. Biophys. Res. Commun.,* 1993, vol. 196, 1414
- **SHAH et al.** *Science,* 1986, vol. 233, 478
- **ARCHER et al.** *J. Bioenerg Biomembr.,* 1990, vol. 22, 789
- **SCANDALIOS.** *Prog. Clin. Biol. Res,* 1990, vol. 344, 515
- **WEISBEEK et al.** *J. Cell Sci. Suppl.,* 1989, vol. 11, 199
- **BRUCE.** *Trends Cell Biol.,* 2000, vol. 10, 440
- **HORSCH et al.** *Science,* 1985, vol. 227, 1229-1231
- **BANCROFT ; DEAN.** *Genetics,* 1993, vol. 134, 1221-1229
- **AARTS et al.** *Mol. Gen. Genet.,* 1995, vol. 247, 555-564
- **BITTER et al.** *Meth. Enzymol.,* 1987, vol. 153, 516-544
- *Meth. Enzymol.,* vol. 118
- **KLEE et al.** *Ann. Rev. Plant Physiol.,* 1987, vol. 38, 467
- **HORSCH et al.** *Science,* 1985, vol. 227, 1229
- **WEISSBACH ; WEISSBACH.** Methods for Plant Molecular Biology. Academic Press, 1988, 421-463
- **GRIERSON ; COREY.** Plant Molecular Biology. Blackie, 1988
- **BECHTOLD.** *C.R. Acad. Sci. Paris,* 1993, vol. 316, 1194
- **DEFRAMOND.** *BioTechnology,* 1983, vol. 1, 262
- **HOEKEMA et al.** *Nature,* 1983, vol. 303, 179
- **GLICK ; THOMPSON.** Methods in Plant Molecular Biology and Biotechnology. CRC Press, 1993
- **WANG et al.** Transformation of Plants and Soil Microorganisms. Cambridge University Press, 1995

- **HIEI et al.** *Plant J.,* 1994, vol. 6, 271-282
- **SHIMAMOTO.** *Science,* 1995, vol. 270, 1772-1773
- **KONCZ.** *Mol. Gen. Genet.,* 1986, vol. 204, 383-396
- **DEBLAERE.** *Nucl. Acid Res.,* 1985, vol. 13, 4777
- **BEVAN.** *Nucleic Acid Res.,* 1984, vol. 12, 8711
- **KONCZ.** *Proc. Natl. Acad. Sci.,* 1986, vol. 86, 8467-8471
- **KONCZ.** *Plant Mol. Biol.,* 1992, vol. 20, 963-976
- Specialized vectors for gene tagging and expression studies. **KONCZ.** Plant Molecular Biology Manual. Kluwer Academic Publ, 1994, vol. 2, 1-22
- **HOEKEMA.** The Binary Plant Vector System. Offset-drukkerij Kanters B. V, 1985
- **FRALEY.** *Crit. Rev. Plant. Sci.,* vol. 4, 1-46
- **AN.** *EMBO J.,* 1985, vol. 4, 277-287
- **LYZNIK.** *Plant Mol. Biol.,* 1989, vol. 13, 151-161
- **PENG.** *Plant Mol. Biol.,* 1995, vol. 27, 91-104
- **BAYLEY.** *Plant Mol. Biol.,* 1992, vol. 18, 353-361
- **LLOYD.** *Mol. Gen. Genet.,* 1994, vol. 242, 653-657
- **MAESER.** *Mol. Gen. Genet.,* 1991, vol. 230, 170-176
- **ONOUCHI.** *Nucl. Acids Res.,* 1991, vol. 19, 6373-6378
- **FROMM et al.** *Proc. Natl. Acad. Sci., USA,* vol. 82, 5824
- Gene Transfer To Plants. Springer Verlag, 1995
- **KLEIN et al.** *Nature,* 1987, vol. 327, 70-73
- **WAN.** *Plant Physiol.,* 1984, vol. 104, 37-48
- **VASIL.** *Bio/Technology,* 1993, vol. 11, 1553-1558
- **CHRISTOU.** *Trends in Plant Science,* 1996, vol. 1, 423-431
- **DUAN et al.** *Nature Biotech.,* 1996, vol. 14, 494-498
- **SHIMAMOTO.** *Curr. Opin. Biotech.,* 1994, vol. 5, 158-162
- **MCBRIDE et al.** *Proc. Natl. Acad. Sci., USA,* 1994, vol. 91, 7301-7305
- **SVAB et al.** *Proc. Natl. Acad. Sci., USA,* 1990, vol. 87, 8526-8530
- **STAUB ; MALIGA.** *Plant Cell,* 1992, vol. 4, 39-45
- **STAUB ; MALIGA.** *EMBO J.,* 1993, vol. 12, 601-606
- **SVAB ; MALIGA.** *Proc. Natl. Acad. Sci., USA,* 1993, vol. 90, 913-917
- **HERSKOWITZ.** *Nature,* 1987, vol. 329, 219-222
- **HEMMATI-BRIVANLOU.** *Curr. Topics Devel. Biol.,* 1998, vol. 36, 75-98
- **KASUGA et al.** *Nat. Biotechnol.,* 1999, vol. 17, 287-291
- *TIBTECH,* 1997, vol. 15, 441-447
- **KREN.** *Hepatology,* 1997, vol. 25, 1462-1468
- **COLE-STRAUSS.** *Science,* 1996, vol. 273, 1386-1389
- **CHEE et al.** *Science,* 1996, vol. 274, 610
- **LEFFEL et al.** *Biotechniques,* 1997, vol. 23, 912
- **LI et al.** *Mol Gen Genet,* 1999, vol. 261, 58
- **ZHANG et al.** *Thero. Appl. Genet.,* 2000, vol. 103, 19-29
- **FLOWERS et al.** *J Exp Bot,* 2000, vol. 51, 99
- **TANKSLEY ; MCCOUCH.** *Science,* 1997, vol. 277, 1063
- **NARANG et al.** *Meth Enzymol,* 1979, vol. 68, 90
- **MATTEUCCI et al.** *J Am Chem Soc,* 1981, vol. 103, 3185
- **URDEA et al.** *Proc Natl Acad Sci. USA,* 1981, vol. 80, 7461
- **URDEA et al.** *Nuc Acids Res,* 1988, vol. 16, 4937
- **TAKATA et al.** for RAD51B paralogs. *Mol Cell Biol,* 2000, vol. 20, 6476
- **BOUVIER et al.** *Eur J Biochem,* 2000, vol. 267, 6346
- **OSPINA-GIRALDO et al.** *Fungal Genet Biol,* 2000, vol. 29, 81
- **WEISE et al.** *Plant Cell,* 2000, vol. 12, 1345
- **SAMBROOK et al.** Molecular Cloning: A Laboratory Manual. Cold Spring Harbor Laboratory Press, 1989
- **AUSUBEL et al.** Current Protocols in Molecular Biology. Greene Publishing, 1992
- **TANKSLEY et al.** *Science,* 1997, vol. 277, 1063
- **BEETHAM et al.** *Proc. Natl. Acad. Sci. USA,* 1999, vol. 96, 8774
- **ZHU et al.** *Proc. Natl. Acad. Sci., USA,* 1999, vol. 96, 8768
- **CARELS et al.** *J Mol Evol,* 1998, vol. 46, 45
- **FENNOY et al.** *Nucl Acids Res,* 1993, vol. 21, 5294
- **NYSTROM ; NEIDHARDT.** *Mol. Microbiol.,* 1992, vol. 6, 3187-3198
- **MAO et al.** *Genome Res,* 2000, vol. 10, 982
- **ZHU ; WANG.** *Plant Physiol.,* 2000, vol. 124, 1472-1476
- **KRYSAN.** *Plant Cell,* 1999, vol. 11, 2283-2290
- **TISSER et al.** *Plant Cell,* 1999, vol. 11, 1841-1852
- **SPEULMAN et al.** *Plant Cell,* 1999, vol. 11, 1853-1866

- **PARINOV et al.** *Plant Cell,* 1999, vol. 11, 2263-2270
- **PARINOV ; SUNDARESAN.** *Biotechnology,* 2000, vol. 11, 157-161
- **SUSSMAN et al.** *Plant Physiology,* 2000, vol. 124, 1465-1467
- **LIU et al.** *Plant J.,* 1995, vol. 8, 457-463
- **KLIMYUK et al.** *Plant J.,* 1993, vol. 3, 493-494
- **HORTON et al.** *Gene,* 1989, vol. 77, 61-68
- **PAZOUR et al.** *J. Bacteriol.,* 1992, vol. 174, 4169-4174
- **HANSEN et al.** *Proc. Natl. Acad. Sci. USA,* 1994, vol. 91, 7603-7607
- **ITOH et al.** *Plasmid,* 1984, vol. 11, 206-220
- **ITOH ; HAAS.** *Gene,* 1985, vol. 36, 27-36
- **DITTA et al.** *Proc. Natl. Acad. Sci USA,* 1980, vol. 77, 7347-7351
- **YANNISCH-PERRON et al.** *Gene,* 1985, vol. 33, 103-119
- **FLING et al.** *Nucl. Acids Res.,* 1985, vol. 13, 7095
- **AMMAN et al.** *Gene,* 1983, vol. 25, 167-178
- **YADAV et al.** *Proc. Natl. Acad. Sci. USA,* 1982, vol. 79, 6322-6326
- **NEGROTTO et al.** *Plant Cell Reports,* 2000, vol. 19, 798-803
- **OOMS et al.** *Plasmid,* 1982, vol. 7, 15-29
- **ISHIDA et al.** *Nat. Biotechnol.,* 1996, vol. 14, 745-750
- **GRITZ ; DAVIES.** *Gene,* 1983, vol. 25, 179-188
- **KEEGAN et al.** *Science,* 1986, vol. 231, 699
- **GREENE et al.** *Science,* 1986, vol. 231, 1150
- **TRIEZENBERG et al.** *Genes Dev.,* 1988, vol. 2, 718-729
- **CHU et al.** *Sci. Sin.,* 1975, vol. 18, 659-668
- **WU et al.** *Plant Cell,* 1996, vol. 8, 617-627
- **KASUGA et al.** *Nat. Biotech.,* 1999, vol. 17, 287-291
- **BABU et al.** *Crop Sci.,* 1999, vol. 39, 150-158
- **SAIJO et al.** *Plant J.,* 2000, vol. 23, 319-327
- **MOONS et al.** *Plant Physiol.,* 1995, vol. 107, 177-186
- **KAWASKI et al.** *Plant Cell,* 2001, vol. 13, 889-905
- **MIYAO et al.** *DNA Res.,* 1996, vol. 3, 233
- **YANG et al.** *Mol. Gen. Genet.,* 1994, vol. 245, 187
- **AHN et al.** *Mol. Gen. Genet.,* 1993, vol. 241, 483
- **REFSETH et al.** *Electrophoresis,* 1997, vol. 18, 1519
- **AKAGI et al.** *Genome,* 1996, vol. 39, 205
- **ZIETKIEWICZ et al.** *Genomics,* 1994, vol. 20, 176
- **LEROY et al.** *Electron. J. Biotechnol.,* 2000, vol. 3 (2, http://www.ejb.org
- **LEROY et al.** *Electron. J Biotechnol,* 2000, vol. 3 (2
- **LI et al.** *Mol. Gen. Genet.,* 1999, vol. 261, 58
- **FLOWERS et al.** *J. Exp. Bot.,* 2000, vol. 51, 99